# EUROPEAN PATENT APPLICATION

(11) **EP 4 173 675 A1**
(43) Date of publication of application: **03.05.2023**
(21) Application number: 21306489.2
(22) Date of filing: 26.10.2021
(51) Int. Cl.: A61P 25/16, A61P 25/28, C07D 401/14, C07D 403/06, C07D 405/14, C07D 413/06, C07D 413/14, C07D 417/14, A61P 3/10, A61P 19/02, A61P 31/16, A61P 31/18, A61P 31/22, A61P 33/02, A61P 33/06, A61P 35/00

(54) **IMIDAZOLONE DERIVATIVES AS INHIBITORS OF PROTEIN KINASES IN PARTICULAR DYRK1A, CLK1 AND/OR CLK4**

(71) Applicant: Perha Pharmaceuticals, 29680 Roscoff (FR)
(72) Inventor: DEAU, Emmanuel, 29680 Roscoff (FR); GEORGE, Pascal, 78730 LONGVILLIERS (FR); MEIJER, Laurent, 29680 Roscoff (FR); MIEGE, Frédéric, 29680 Roscoff (FR)
(74) Representative: Cabinet Nony

(57) **Abstract**

« New imidazolone derivatives as inhibitors of protein kinases, in particular DYRK1A, CLK1 and/or CLK4»

The present invention relates to a compound of formula (I) 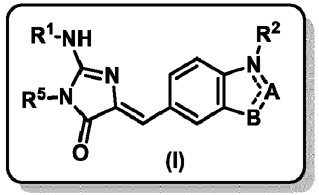 wherein R¹ represents a (C₄-C₆)alkyl group, a (C₃-C₈)cycloalkyl group, a bridged (C₆-C₁₀)cycloalkyl group, a fused phenyl group, a substituted phenyl group, a R'-L- group, wherein L is either a single bond or a (C₁-C₃)alkanediyl group, and R' represents, a (C₃-C₈)heterocycloalkyl group, or a (C₃-C₈)heteroaryl group, or a R"-L- group wherein L is a (C₁-C₃)alkanediyl group, and R" is an optionally substituted phenyl group; R² is selected from the group consisting of a hydrogen atom and a (C₁-C₃)alkyl group; R⁵ represents a hydrogen atom, a (C₁-C₄)alkyl group or a (C₃-C₆)cycloalkyl group or any of its pharmaceutically acceptable salt.

The present invention further relates to a composition comprising a compound of formula (I) and a process for manufacturing said compound as well as its synthesis intermediates.

It also relates to said compound for use as a medicament, in particular in the treatment and/or prevention of cognitive deficits and neuroinflammation associated with Down syndrome; Alzheimer's disease; dementia; tauopathies; Parkinson's disease; CDKL5 Deficiency Disorder; Phelan-McDermid syndrome; autism; type 1 and type 2 diabetes; abnormal folate and methionine metabolism; osteoarthritis and tendinopathy; Duchenne muscular dystrophy; cancers and leukemias; neuroinflammation, anemia, infections caused by unicellular parasites, viral infections and for regulating body temperature.

## Description

### FIELD OF THE INVENTION

The present invention relates to Leucettinibs, a class of new compounds useful as a medicament. Said new compounds are in particular useful as kinase inhibitors, and even more particularly as inhibitors of DYRK1A and/or CLK1 and/or CLK4. They are efficient for treating and/or preventing cognitive deficits associated with Down syndrome; Alzheimer's disease and related diseases; dementia; tauopathies; Parkinson's disease; other neurodegenerative diseases; CDKL5 Deficiency Disorder; type 1 and type 2 diabetes; abnormal folate and methionine metabolism; osteoarthritis and tendinopathy; Duchenne muscular dystrophy; several cancers and leukemias; viral infections and for regulating body temperature.

Some of said compounds are further inhibitors of other kinases and namely other DYRKs (DYRK1B, 2, 3, 4) and the closely related cdc2-like kinases (CLKs) (CLK 2, 3, 4). Said compounds may then further be efficient for treating and/or preventing Phelan-McDermid syndrome; autism; viral infections, cancers, neuroinflammation, anemia and infections caused by unicellular parasites.

It further relates to the pharmaceutical compositions containing said new compounds and to the chemical synthesis processes for obtaining them.

### BACKGROUND

The **DYRK** and **CLK** kinase families belong to the CMGC group of kinases which also includes the mitogen-activated protein kinases (MAPK), cyclin-dependent kinases (CDKs) and glycogen synthase kinase-3 (GSK-3). They phosphorylate many substrates involved in signaling pathways. **DYRKs** and **CLKs** play key roles in mRNA splicing, chromatin transcription, DNA damage repair, cell survival, cell cycle, differentiation, homocysteine/methionine/folate regulation, endocytosis, neuronal development and functions, synaptic plasticity (review in Lindberg, M. and Meijer, L., 2021. Dual-specificity, tyrosine phosphorylation-regulated kinases (DYRKs) and cdc2-like kinases (CLKs) in human disease, an overview. Internat. J. Mol. Sci. 22, 6047).

### DYRK1A and Down syndrome (DS)

The gene encoding **DYRK1A** is located on chromosome 21, in particular in the **"Down syndrome critical region"** (DSCR), the triploidy of which is responsible for most DS-associated deficiencies. There is considerable genetic and pharmacological evidence showing that the mere 1.5-fold overexpression of **DYRK1A** is responsible for most cognitive deficits, especially memory and learning deficits, observed in DS patients (Feki, A., Hibaoui, Y., 2018. DYRK1A protein, a promising therapeutic target to improve cognitive deficits in Down syndrome. Brain Sci. 8, 187; Rueda N et al., 2020. Translational validity and implications of pharmacotherapies in preclinical models of Down syndrome. Prog Brain Res 251, 245). Pharmacological or genetical normalization of **DYRK1A** levels restores cognitive functions (Nguyen TL et al., 2017. Dual-specificity tyrosine phosphorylation-regulated kinase 1A (DYRK1A) inhibitors: a survey of recent patent literature. Expert Opin. Ther. Pat. 27, 1183-1199; Nguyen TL et al., 2018. Correction of cognitive deficits in mouse models of Down syndrome by pharmacological inhibitor of DYRK1A. Dis. Model Mech. 11, dmm035634).

### DYRK1A and Alzheimer's disease (AD), Tauopathies

There is mounting evidence for a role of **DYRK1A** in the onset of AD. **DYRK1A** phosphorylates key substrates involved in AD and dementia: Tau, septin 4, amyloid precursor protein (APP), presenilin 1, neprilysin, Munc18-1, α-synuclein, RCAN1, β-Tubulin. There is evidence for abnormal expression and post-translational modifications of **DYRK1A** in AD. By modulating alternative splicing of exon 10, **DYRK1A** favors the production of the 3R-Tau splice isoform (characteristic for DS/AD/tauopathy) over the normal 4R-Tau isoform. **DYRK1A** inhibition promotes autophagy which could counterbalance the autophagy deficit seen in AD. There is a clear association of AD with DS (Fortea J. et al., 2021. Alzheimer's disease associated with Down syndrome: a genetic form of dementia. The Lancet 20, 930-942): most DS patients show AD neuropathology at an early age (40's) and high prevalence of dementia in later age (>60).

### DYRK1A and Parkinson's disease (PD) and Pick disease

GWAS studies have revealed that **DYRK1A** is a risk factor for PD (Nalls MA et al., 2019. Identification of novel risk loci, causal insights, and heritable risk for Parkinson's disease: a meta-analysis of genome-wide association studies. Lancet Neurol 18, 1091). **DYRK1A** phosphorylates key factors for PD such as Parkin, septin 4, α-synuclein. Upregulation of micro-RNA specific for PD target **DYRK1A** expression (Chiu CC et al., 2019. Upregulated expression of microRNA-204-5p leads to the death of dopaminergic cells by targeting DYRK1A-mediated apoptotic signaling cascade. Front Cell Neurosci 13, 399). There is further evidence that **DYRK1A** expression is increased in PD. **DYRK1A** is overexpressed in Pick disease.

### DYRK1A and viral infections

**DYRK1A** and **DYRK1B** are utilized during HCMV placental replication. Inhibition of **DYRKs** prevent replication of various viruses including Herpes virus, cytomegalovirus and HIV-1.

### DYRK1A and type 1 and type 2 diabetes

**DYRK1A** inhibitors stimulate the proliferation of pancreatic, insulin-producing β-cells. This constitutes a promising approach to diabetes, both type 1 (which displays low number of β-cells) and type 2 (where the β-cell mass is reduced by half) (Ackeifi C et al., 2020. Pharmacologic and genetic approaches define human pancreatic β cell mitogenic targets of DYRK1A inhibitors. JCI Insight 5, e132594; Kumar K et al., 2021. DYRK1A inhibitors as potential therapeutics for β-cell regeneration for diabetes. J Med Chem 64, 2901-2922. Barzowska A, et al., 2021. DYRK1A kinase inhibitors promote β-cell survival and insulin homeostasis. Cells. 10, 2263; Wang P et al., 2021. Human beta cell regenerative drug therapy for diabetes: past achievements and future challenges. Front Endocrinol 12, 671946). Besides direct application to diabetic patients, **DYRK1A** inhibitors could be applied to stimulate β-cell proliferation in vitro or ex vivo to increase β-cell mass prior to grafting.

### DYRK1A, cancers and leukemias

There is abundant literature linking **DYRK1A** with cancer. The most prominent examples are megakaryoblastic leukemia (Malinge S et al., 2012. Increased dosage of the chromosome 21 ortholog Dyrkla promotes megakaryoblastic leukemia in a murine model of Down syndrome. J Clin Invest 122, 948-962), acute lymphoblastic leukemia (Bhansali RS et al., 2021. DYRK1A regulates B cell Acute Lymphoblastic Leukemia through phosphorylation of FOXO1 and STAT3. J Clin Investig, 131, e135937), pancreatic cancer and brain tumor (glioblastoma) (see review in Lindberg and Meijer, 2021. cited above).

Accordingly, abnormalities in **DYRK1A** dosage are associated with cognitive disorders observed in Down syndrome, and Alzheimer's disease. **DYRK1A** is a risk factor for Parkinson's disease. Inhibition of **DYRK1A** additionally triggers the proliferation of pancreatic, insulin-producing β-cells. **DYRK1A** inhibitors may thus find applications in preventing and/or treating DS, AD, and other Tauopathies (particularly cognitive deficits associated with these pathologies), dementia, PD, Niemann-Pick Type C Disease, CDKL5 deficiency disorder, type 1 and type 2 diabetes, viral infections, several cancers (leukemia, pancreatic cancer, glioblastoma), tendinopathy and osteoarthritis, infections caused by unicellular parasites and for regulating body temperature.

### Other DYRKs and human disease

**DYRK1B** is involved in the replication of various viruses including hepatitis C virus, Chikungunya virus, Dengue virus and SARS coronavirus, cytomegalovirus and human papillomavirus. Like **DYRK1A, DYRK1B** inhibition leads to the proliferation of pancreatic, insulin-producing β-cells. **DYRK1B** is involved in neuroinflammation. Targeting **DYRK1B** provides a new rationale for treatment of various cancers such as liposarcoma or breast cancers.

**DYRK2,** in association with GSK-3β, regulates neuronal morphogenesis. **DYRK2** is involved in various ways in cancer development.

**DYRK3** promotes hepatocellular carcinoma. **DYRK3** couples stress granule condensation/dissolution to mTORC1 signaling. **DYRK3** regulates phase transition of membraneless organelles in mitosis. **DYRK3** and **DYRK4** are involved in the regulation of cytoskeletal organization and process outgrowth in neurons.

**DYRK1A** decreases axon growth, **DYRK3** and **DYRK4** increase dendritic branching and **DYRK2** decreases both axon and dendrite growth and branching.

### CLKs and human disease

Note that **CLK** is a confusing abbreviation as it has the following meanings: (**a**) monooxygenase CLK-1 (human homologue COQ7); (**b**) Collectin-K1 (CL-K1, or CL-11), a multifunctional Ca(2+)-dependent lectin; (**c**) MAPK gene of the maize pathogen *Curvularia lunata,* Clk1; (**d**) mitochondrial membrane-bound enzyme Clock-1 (CLK-1); (**e**) *Colletotrichum lindemuthianum* kinase 1 (clk1).

**CLKs** play essential functions in alternative splicing. **CLKs** act as a body-temperature sensor which globally controls alternative splicing and gene expression. The activity of CLKs is indeed highly responsive to physiological temperature changes, which is conferred by structural rearrangements within the kinase activation segment (Haltenhof T et al., 2020. A conserved kinase-based body-temperature sensor globally controls alternative splicing and gene expression. Mol Cell 78, 57).

### CLK1 and human disease

**CLK1** triggers periodic alternative splicing during the cell division cycle. **CLK1** regulates influenza A virus mRNA splicing and its inhibition prevents viral replication. **CLK1** and **CLK2** also regulates HIV-1 gene expression. **CLK1** is an autophagy inducer. **CLK1** inhibition may prevent chemoresistance in glioma and **CLK1** inhibition by TG693 allows the skipping of mutated exon 31 of the dystrophin gene in Duchenne Muscular Dystrophy.

### Other CLKs and human disease

Inhibition of **CLK2** has been proposed as a way to improve neuronal functions and combat intellectual disability and autism in Phelan-McDermid syndrome (PMDS). Dual inhibition of **CLK2** and DYRK1A by Lorecivivint is a potential disease-modifying approach for knee osteoarthritis. **CLK2** inhibition compromises MYC-driven breast tumors, triple negative breast cancer and glioblastoma. Inhibition of **CLK2** improves autistic features in Phelan-McDermid syndrome (PMDS). Alternative splicing of Tau exon 10 is regulated by **CLK2** and **other CLKs,** leading to changes in the 3R/4R isoforms ratio and neurodegeneration in sporadic AD. Inhibition of **CLK2, CLK3, CLK4** blocks HIV-1 production. By regulating alternative splicing **CLKs** modulate the balance between pro-apoptotic and anti-apoptotic regulators, and inhibition of **CLKs** may thus find applications in the treatment of numerous cancers, in particular prostate cancer and hepatocellular carcinoma.

The following Table 1 summarizes the implication of the DYRKs and CLKs kinases in various diseases.

**Table 1**

| **Kinase target** | **Disease** |
|---|---|
| DYRK1A | Down syndrome (DS) |
| DYRK1A | Alzheimer's disease (AD) and other Tauopathies |
| DYRK1A | Parkinson's disease |
| DYRK1A | Pick disease |
| DYRK1A | CDKL5 Deficiency Disorder |
| DYRK1A | Type 1 and Type 2 diabetes |
| DYRK1A | Abnormalities in folate and methionine metabolism |
| DYRK1A | Glioblastoma |
| DYRK1A | Head and neck squamous cell carcinoma |
| DYRK1A | Pancreatic ductal adenocarcinoma |
| DYRK1A | Mepakaryoblastic leukemia |
| DYRK1A | Acute Lymphoblastic Leukemia (ALL) |
| DYRK1A | Knee osteoarthritis, tendinopathy |
| DYRK1A | Human immunodeficiency virus type 1 (HIV-1) |
| DYRK1A, DYRK1B | Human cytomegalovirus (HCMV) |
| DYRK1B | Hepatitis C virus, Chikungunya virus, Dengue virus and Severe acute respiratory syndrome coronavirus, Cytomegalovirus, Human papillomavirus |
| DYRK1B | Type 1 and Type 2 diabetes |
| DYRK1B | Neuroinflammation |
| DYRK1B | Liposarcoma, Breast cancer, Hedgehog/GLI-dependent cancer |
| DYRK2 | Triple-negative breast cancer (TNBC) and multiple myeloma (MM) |
| DYRK2 | Glioblastoma |
| DYRK3 | Hepatocellular carcinoma |
| DYRK3 | Influenza virus replication |
| DYRK3 | Anemia |
| DYRKs | Glioblastoma |
| DYRKs | Herpes simplex virus, cytomegalovirus, varicella-zoster virus |
| LmDYRK1 | Leishmaniasis |
| TbDYRK | *Trypanosoma brucei* |
| | |
| CLK1 | Glioblastoma |
| CLK1 | Duchenne muscular dystrophy |
| CLK1 | Influenza A |
| CLK2 | HIV-1 |
| CLK1/CLK2 | Triple-negative breast cancer |
| CLK2 | Autism, Phelan-McDermid syndrome (PMDS) |
| CLK2 | Knee osteoarthritis, tendinopathy |
| CLK2 | Breast cancer, Triple negative breast cancer, Glioblastoma |
| CLK2 | Alzheimer's disease (alternative splicing of Tau exon 10) |
| CLK3 | Hepatocellular carcinoma, Prostate cancer |
| CLKs | Body temperature |
| CLKs | Prostate cancer, Gastrointestinal cancer |
| PfCLKs | Malaria |
| | |
| DYRKs/CLKs | Glioblastoma and numerous other cancers |

### DYRKs and CLK inhibitors

Several DYRK1A inhibitors have been reported in recent years. Most DYRK1A inhibitors also inhibit DYRK1B, 2, 3, 4, as well as the closely related CLK1, 2, 3, 4, with several possible inhibition profiles.

Some imidazolone derivatives, named **Leucettines** in the text below, were disclosed in WO 2009/050352 as kinase inhibitors and more particularly as inhibitors of the DYRK1A kinase.

WO 2021/114314 and WO 2021/115489 disclose compounds useful in cardiomyocyte proliferation activity for the treatment of heart diseases. However, these documents comprise a very broad range of compounds illustrated in a very partial way and focused on benzothiazoles derivatives (i.e., in the terms of the present invention, wherein A = =C- and B = -S-). In other words, none of the compounds disclosed are comprised in the formula (I) of the present invention. Furthermore, the compounds disclosed in these applications do not have an optimized activity with regards to DYRK1A and CLK1 and are not directed to the PATHOLOGIES as defined herein after.

There is still a need to identify new compounds for treating and/or preventing the diseases as recited above, and particularly through the inhibition, and in particular selective inhibition of DYRK1A, other DYRKs and the related CLKs kinases.

### SUMMARY OF THE INVENTION

It has now been found that the compounds as defined in formula (I) herein after are useful in the treatment and/or prevention of a disease selected from cognitive deficits and neuroinflammation associated with Down syndrome, Alzheimer's disease and related diseases, dementia and tauopathies; Parkinson's disease and other neurodegenerative diseases; CDKL5 Deficiency Disorder; Phelan-McDermid syndrome; autism; type 1 and type 2 diabetes; abnormal folate and methionine metabolism; osteoarthritis and tendinopathy; several cancers and leukemias, neuroinflammation, anemia, infections caused by unicellular parasites, viral infections and for regulating body temperature.

The present invention therefore relates to a compound of formula (I), as defined below.

The present invention further relates to a compound of formula (I) as defined below for use as a medicament.

The present invention further relates to a compound of formula (I) as defined below for use in the treatment and/or prevention of a disease selected from cognitive deficits and neuroinflammation associated with Down syndrome, Alzheimer's disease and related diseases, dementia or tauopathies; Parkinson's disease; other neurodegenerative diseases; CDKL5 Deficiency Disorder; Phelan-McDermid syndrome; autism; type 1 and type 2 diabetes; abnormal folate and methionine metabolism; osteoarthritis and tendinopathy; several cancers and leukemias, neuroinflammation, anemia, infections caused by unicellular parasites, viral infections and for regulating body temperature.

The present invention further relates to a pharmaceutical composition comprising it and to a process for manufacturing it.

The present invention at last relates to synthetic intermediates of formula (III), (IX) and (XI) as defined below.

### DEFINITIONS

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means preventing, reversing, alleviating, inhibiting the progress of, or preventing the disease and its cognitive, motor or metabolic changes resulting from high DYRK1A kinase and/or CLK1 expression and activity, and optionally associated with the abnormalities in other DYRKs (DYRK1B, 2, 3, 4) and the closely related further cdc2-like kinases (CLKs) (CLK 2, 3, 4) and more particularly in connection to the diseases as described herein after in the paragraph "PATHOLOGIES".

Therefore, the term "treating" or "treatment" encompasses within the framework of the present invention the improvement of medical conditions of patients suffering from the diseases as described herein after in the paragraph "PATHOLOGIES", related to high expression and activity of any of the DYRK1A and CLK1 kinases, and optionally associated with the abnormalities in other DYRKs (DYRK1B, 2, 3, 4) and the closely related further cdc2-like kinases (CLKs) (CLK 2, 3, 4).

As used herein, an "effective amount" refers to an amount of a compound of the present invention which is effective in preventing, reducing, eliminating, treating or controlling the symptoms of the herein-described diseases and conditions.

The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "effective amount" includes "prophylaxis-effective amount" as well as "treatment-effective amount".

The term "preventing", as used herein, means reducing the risk of onset or slowing the occurrence of a given phenomenon, namely in the present invention, a disease resulting from abnormal DYRKs/CLKs kinase activity, in particular DYRK1A kinase activity.

As used herein, *"preventing"* also encompasses *"reducing the likelihood of occurrence*" or *"reducing the likelihood of reoccurrence*".

The term *"prophylaxis-effective amount"* refers to a concentration of compound of this invention that is effective in inhibiting, preventing, decreasing the likelihood of anyone of the hereabove described diseases.

Likewise, the term "treatment-effective amount" refers to a concentration of compound that is effective in treating the hereabove described diseases, *e.g*. leads to a reduction or normalization DYRK1A and/or CLK1 kinase activity, and optionally additionally of DYRKs/CLKs kinase activity in general, following examination when administered after disease has occurred.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### DETAILED DESCRIPTION OF THE INVENTION

The inventors have surprisingly found that the compounds of formula (I) as disclosed herein after inhibit the DYRK1A, other DYRKs (DYRK1B, DYRK2, DYRK3, DYRK4) and CLKs (CLK1, CLK2, CLK3, CLK4). This assertion is based on data as illustrated in the following examples and more detailed herein after.

According to a first aspect, a subject-matter of the present invention relates to a compound of formula (I) 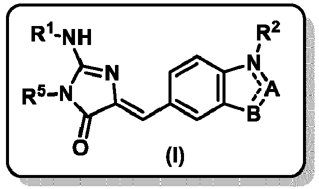 wherein:
A is selected from the group consisting of =CH-, -N= and -NR³-,
B is selected from the group consisting of -O-, =CH-, =N-, -NH- and -NR⁴-,
at least one of A and B is =CH-,
A and B can not both represent a group comprising a C atom,
R² is selected from the group consisting of a hydrogen atom and a (C₁-C₃)alkyl group,
R³ and R⁴ are independently selected from (C₁-C₃)alkyl groups,
R⁵ represents a hydrogen atom, a (C₁-C₄)alkyl group or a (C₃-C₆)cycloalkyl group, and when A represents =CH- and B represents =N- or -NH-, then R² respectively represents a hydrogen atom or does not exist,
when N is trivalent, then R² does not exist, and
wherein R¹ represents:
   (i). a (C₄-C₆)alkyl group substituted by a group selected from a hydroxy group, a halogen and a (C₁-C₃)alkoxy group,
   (ii). a (C₃-C₈)cycloalkyl group, optionally substituted by a group selected from a hydroxy group and a (C₁-C₃) alkoxy group,
   (iii). a bridged (C₆-C₁₀)cycloalkyl group, optionally substituted by a group selected from a (C₁-C₄)alkyl group, a (C₁-C₄)alkoxy group, a halogen atom and a hydroxy group,
   (iv). a fused phenyl group, selected from phenyl groups fused with a (C₅-C₆)cycloalkyl, which (C₅-C₆)cycloalkyl is optionally substituted by a hydroxy group,
   (v). a phenyl group, substituted by a (C₄-C₇)heterocycloalkyl group, said (C₄-C₇)heterocycloalkyl group being itself optionally substituted by a (C₁-C₄) alkyl group, or
   (vi). a R'-L- group, wherein L is either a single bond or a (C₁-C₃)alkanediyl group, optionnally susbstituted by a group selected from a hydroxy group and a (C₁-C₃)alkoxy group, and
      R' represents:
      (vi.1). a (C₃-C₈)heterocycloalkyl group, optionally substituted by one or two groups selected from a hydroxy group, and a (C₁-C₄)alkyl group, or
      (vi.2). a (C₃-C₈)heteroaryl group, optionally substituted by a (C₁-C₄)alkyl group, or
   (vii). a R"-L- group wherein L is a (C₁-C₃)alkanediyl group, optionally substituted by a group selected from a -NR^{b}R^{c} group, a (C₁-C₃)alkoxy group and a hydroxy group, and
      R" is a phenyl group, optionally substituted by a fluoro(C₁-C₄)alkyl group,
R^{b} and R^{c} independently represent a (C₁-C₆)alkyl group or a hydrogen atom, or any of its pharmaceutically acceptable salt.

The inventors have surprisingly discovered that compounds having the following scaffolds (A) to (H) displayed much reduced kinase inhibitory activities on DYRK1A and other related kinases compared to their homologs (compounds according to the invention): IC₅₀ values were reduced by 10 to 1000 -fold factors, and some compounds were completely inactive at the highest dose tested (10 µM).

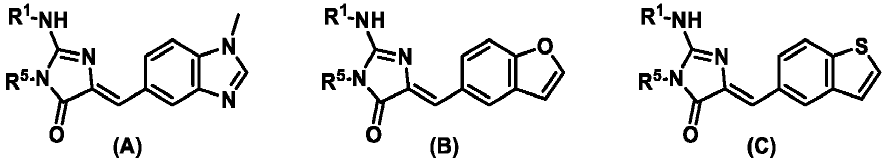

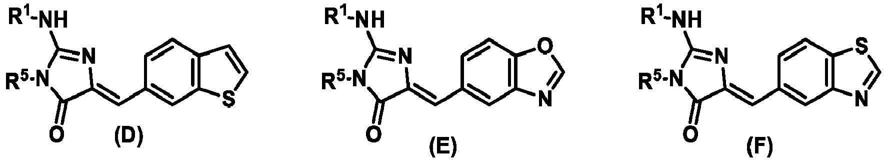

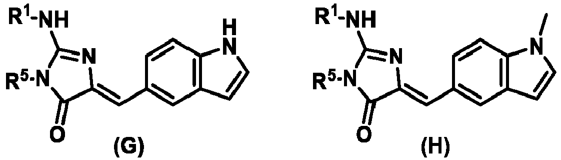

These much-reduced kinase inhibitory activities have been verified, for example, by individual comparison of a compound of formula (I) and of a compound having a scaffold of formula (A) to (H), wherein in both, R¹ is selected from the group consisting of cyclohexyl, cycloheptyl, cyclooctyl, 1-(hydroxymethyl)-3-methyl-butyl, 1-(methoxymethyl)-3-methyl-butyl, 2-methoxy-1-phenyl-ethyl, 3-noradamantyl, 1-adamantyl, 3-hydroxy-1-adamantyl, 3-fluoro-1-adamantyl and wherein in both, R⁵ is selected from the group consisting of a methyl group or a hydrogen atom.

According to a particular embodiment, the present invention relates to a compound of formula (I) as defined herein above, wherein R¹ represents:
(i). a (C₅-C₆)alkyl group substituted by a group selected from a hydroxy group, a halogen and a (C₁-C₃)alkoxy group,
(ii). a (C₅-C₈)cycloalkyl group, optionally substituted by a group selected from a hydroxy group and a (C₁-C₃) alkoxy group,
(iii). a bridged (C₉-C₁₀)cycloalkyl group, optionally substituted by a group selected from a (C₁-C₄)alkyl group, a (C₁-C₄)alkoxy group, a halogen atom and a hydroxy group,
(iv). a phenyl group fused with a cyclopentyl, which cyclopentyl is optionally substituted by a hydroxy group,
(v). a phenyl group, substituted by a (C₄-C₇)heterocycloalkyl group, said (C₄-C₇)heterocycloalkyl group being itself optionally substituted by a (C₁-C₄) alkyl group, or
(vi). a R'-L- group wherein
   - L is either a single bond or a (C₁-C₃)alkanediyl group, optionnally susbstituted by a group selected from a hydroxy group and a (C₁-C₃)alkoxy group, and
   - R' represents:
      (vi.1). a (C₆-C₇)heterocycloalkyl group, optionally substituted by one or two groups selected from a hydroxy group and a (C₁-C₄)alkyl group, or
      (vi.2). a heteroaryl group, optionally substituted by a (C₁-C₄)alkyl group,
(vi). a R"-L- group wherein
   - L is a (C₁-C₃)alkanediyl group, optionally substituted by a group selected from a -NR^{b}R^{c} group, a (C₁-C₃)alkoxy group and a hydroxy group, and
   - R" is a phenyl group, optionally substituted by a fluoro(C₁-C₄)alkyl group, and
R^{b} and R^{c} independently represent a (C₁-C₆)alkyl group or a hydrogen atom, or any of its pharmaceutically acceptable salt.

According to another particular embodiment, the present invention relates to a compound of formula (I) as defined herein above, wherein R¹ represents:
(i). a (C₅-C₆)alkyl group substituted by a group selected from a hydroxy group, a fluorine atom and a methoxy group,
(ii). a (C₅-C₈)cycloalkyl group in particular a cyclopentyl, a cyclohexyl, a cycloheptyl or a cyclooctyl, optionally substituted by a group selected from a hydroxy group and a methoxy group,
(iii). a bridged (C₉-C₁₀)cycloalkyl group in particular an adamantyl or a noradamantyl, optionally substituted by a group selected from a methoxy group, a fluorine atom and a hydroxy group,
(iv). a phenyl group fused with a cyclopentyl, which cyclopentyl is substituted by a hydroxy group,
(v). a phenyl group, substituted by a *N*-methylpiperazinyl group, or
(vi). a R'-L- group wherein L is either a single bond or a (C₁-C₃)alkanediyl group, optionnally susbstituted by a group chosen from a hydroxy group and a methoxy group,
   and R' is selected from the group consisting of:
   (vi.1). a (C₆-C₇)heterocycloalkyl group, in particular a tetrahydropyranyl or an oxepanyl, optionally substituted by one or two groups selected from a hydroxy group and a methyl group,
   (vi.2). a heteroaryl group, in particular a pyridinyl or a thiazolyl, optionally substituted by a methyl group, or
(vii). a R"-L- group wherein L is a (C₁-C₃)alkanediyl group, optionally substituted by a group selected from the group consisting of a -NH₂ group, a methoxy group, a hydroxy group, a -COOR^{a} group and a halogen atom, in particular a fluorine atom, and
   R" is a phenyl group, optionally substituted by a trifluoromethyl group,
or any of its pharmaceutically acceptable salts.

According to another particular embodiment, the present invention relates to a compound of formula (I) as defined herein above wherein L is selected from a group consisting of a -CH₂- group, a -CH(CH₂OH)- group, a -CH(CH₂OCH₃)- group, a -CH(OH)-CH₂- group and a -CH(CH₂NH₂)- group, or any of its pharmaceutically acceptable salts.

According to another particular embodiment, the present invention relates to a compound of formula (I) as defined herein above wherein:
(vi.1). when R' is a (C₅-C₈)heterocycloalkyl group, L is a -CH₂-group,
(vi.2). when R' is a phenyl, L is selected from the group consisting of a -CH₂- group, a -CH(CH₂OH)- group, a -CH(CH₂OCH₃)- group, a -CH(OH)-CH₂- group and a -CH(CH₂NH₂)- group,
(vi.3). when R' is a heteroaryl group, L is a -CH₂- group,
or any of its pharmaceutically acceptable salts.

According to another particular embodiment, the present invention relates to a compound selected from the following compounds 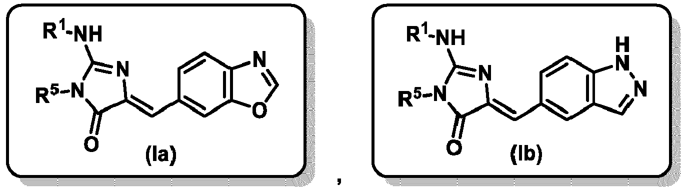 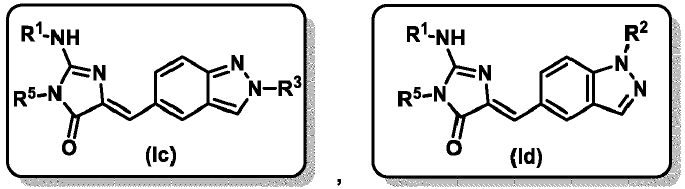 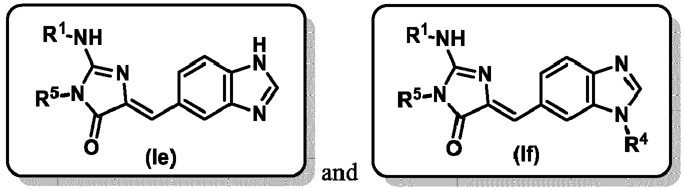
wherein R¹, R², R³, R⁴ and R⁵ are as defined above,
or any pharmaceutically acceptable salt thereof.

According to another particular embodiment, the present invention relates to a compound of formula (I) as defined herein above wherein when the compound of formula (I) is chosen from the subformulae (Ib), (Id), (Ie) and (If), R¹ represents:
(i). a (C₄-C₆)alkyl group substituted by a group selected from a hydroxy group, a halogen and a (C₁-C₃)alkoxy group,
(ii). a (C₃-C₈)cycloalkyl group, optionally substituted by a group selected from a hydroxy group and a (C₁-C₃) alkoxy group,
(iii). a bridged (C₆-C₁₀)cycloalkyl group, optionally substituted by a group selected from a (C₁-C₄)alkyl group, a (C₁-C₄)alkoxy group, a halogen atom and a hydroxy group, or
(iv). a R'-L- group, wherein L is either a single bond or a (C₁-C₃)alkanediyl group, optionnally susbstituted by a group selected from a hydroxy group and a (C₁-C₃)alkoxy group, and
   R' represents:
   (iv.1). a (C₃-C₈)heterocycloalkyl group, optionally substituted by one or two groups selected from a hydroxy group, and a (C₁-C₄)alkyl group, or
   (iv.2). a (C₃-C₈)heteroaryl group, optionally substituted by a (C₁-C₄)alkyl group, or
(v). a R"-L- group wherein L is a (C₁-C₃)alkanediyl group, optionally substituted by a group selected from a -NR^{b}R^{c} group, a (C₁-C₃)alkoxy group and a hydroxy group, and
   R" is a phenyl group, optionally substituted by a fluoro(C₁-C₄)alkyl group,
R^{b} and R^{c} independently represent a (C₁-C₆)alkyl group or a hydrogen atom, or any of its pharmaceutically acceptable salt.

According to another particular embodiment, the present invention relates to a compound of formula (I) as defined herein above wherein R¹ represents:
(i). a (C₄-C₆)alkyl group substituted by a group selected from a hydroxy group, a halogen and a (C₁-C₃)alkoxy group,
(ii). a (C₃-C₈)cycloalkyl group, optionally substituted by a group selected from a hydroxy group and a (C₁-C₃) alkoxy group,
(iii). a bridged (C₆-C₁₀)cycloalkyl group, optionally substituted by a group selected from a (C₁-C₄)alkyl group, a (C₁-C₄)alkoxy group, a halogen atom and a hydroxy group, or
(iv). a R'-L- group, wherein L is either a single bond or a (C₁-C₃)alkanediyl group, optionnally susbstituted by a group selected from a hydroxy group and a (C₁-C₃)alkoxy group, and
   R' represents:
   (iv.1). a (C₃-C₈)heterocycloalkyl group, optionally substituted by one or two groups selected from a hydroxy group, and a (C₁-C₄)alkyl group, or
   (iv.2). a (C₃-C₈)heteroaryl group, optionally substituted by a (C₁-C₄)alkyl group, or
(v). a R"-L- group wherein L is a (C₁-C₃)alkanediyl group, optionally substituted by a group selected from a -NR^{b}R^{c} group, a (C₁-C₃)alkoxy group and a hydroxy group, and
   R" is a phenyl group, optionally substituted by a fluoro(C₁-C₄)alkyl group,
R^{b} and R^{c} independently represent a (C₁-C₆)alkyl group or a hydrogen atom, or any pharmaceutically acceptable salt thereof.

According to another particular embodiment, the present invention relates to a compound of formula (I) as defined herein above wherein R⁵ represents a hydrogen atom or a (C₁-C₄)alkyl group.

According to another particular embodiment, the present invention relates to a compound of formula (Ia) 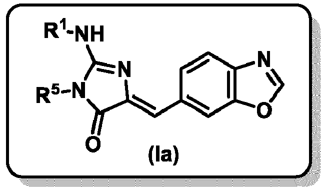 wherein R¹ represents:
- a (C₅-C₆)alkyl group substituted by a group selected from a hydroxy group and a methoxy group,
- a (C₅-C₈)cycloalkyl group in particular a cyclopentyl, a cyclohexyl, a cycloheptyl or a cyclooctyl, optionally substituted by a group selected from a hydroxy group and a methoxy group,
- an adamantyl group, optionally substituted by a group selected from a methoxy group and a hydroxy group
- a phenyl group fused with a cyclopentyl, which cyclopentyl is optionally substituted by a hydroxy group,
- a phenyl group, substituted by a *N*-methylpiperazinyl group,
- a R'-L- group, L is either a single bond or a methylene group, and
   R' is selected from the group consisting of:
   ∘ a (C₆-C₇)heterocycloalkyl group, in particular a tetrahydropyranyl or an oxepanyl, optionally substituted by one or two groups selected from a hydroxy group and a methyl group, and
   ∘ a heteroaryl group, in particular a pyridyl or a thiazolyl, optionally substituted by a methyl group, or
- a R"-L- group wherein
   ∘ L is a (C₁-C₂)alkanediyl group, optionnally susbstituted by a group chosen from a hydroxy group and a methoxy group, and
   ∘ R" is a phenyl group, optionally substituted by a trifluoromethyl, and R⁵ is as defined above,
or any pharmaceutically acceptable salt thereof.

According to another particular embodiment, the present invention relates to a compound of formula (Ib) 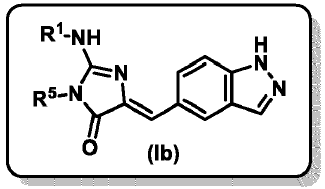 wherein R¹ represents:
- a (C₅-C₆)alkyl group substituted by a group selected from a hydroxy group and a methoxy group,
- a cyclohexyl group,
- an adamantyl group, or
- a R"-L- group wherein
   ∘ L is a methoxymethylmethylene group, and
   ∘ R" is a phenyl group, and
   R⁵ is as defined above,
or any pharmaceutically acceptable salt thereof.

According to another particular embodiment, the present invention relates to a compound of formula (Ic) 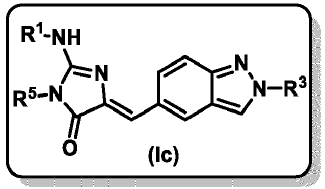 wherein R¹ represents:
- a (C₅-C₆)alkyl group substituted by a group selected from a hydroxy group and a methoxy group,
- a cyclohexyl group,
- an adamantyl group, optionally substituted by a group selected from a halogen atom and a hydroxy group, or
- a R"-L- group wherein
   ∘ L is a methoxymethylmethylene group, and
   ∘ R" is a phenyl group, and
   wherein R³ represents a methyl group, and
   R⁵ is as defined above,
or any pharmaceutically acceptable salt thereof.

According to another particular embodiment, the present invention relates to a compound of formula (Id) 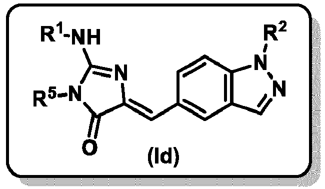 wherein R¹ represents:
- a (C₅-C₆)alkyl group substituted by a group selected from a hydroxy group, a methoxy group and a fluorine atom,
- a (C₅-C₈)cycloalkyl group in particular a cyclopentyl, a cyclohexyl, a cycloheptyl or a cyclooctyl group, optionally substituted by a group selected from a hydroxy group and a methoxy group,
- an adamantyl group, optionally substituted by a group selected from a methoxy group, a hydroxy group and a fluorine atom,
- a phenyl group fused with a cyclopentyl, which cyclopentyl is optionally substituted by a hydroxy group,
- a phenyl group, substituted by a *N*-methylpiperazinyl group,
- a R'-L- group, L is either a single bond or a methylene group, and
   R' is selected from the group consisting of:
   ∘ a (C₆-C₇)heterocycloalkyl group, in particular a tetrahydropyranyl or an oxepanyl, optionally substituted by one or two groups selected from a hydroxy group and a methyl group, and
   ∘ a heteroaryl group, in particular a pyridyl or a thiazolyl group, optionally substituted by a methyl group, or
- a R"-L- group wherein
   ∘ L is a (C₁-C₂)alkanediyl group, optionnally susbstituted by a group chosen from a hydroxy group, a methoxy group and a -NH₂ group, and
   ∘ R" is a phenyl group, optionally substituted by a trifluoromethyl, and
   wherein R² represents a methyl group, and
   R⁵ is as defined above,
or any pharmaceutically acceptable salt thereof.

According to another particular embodiment, the present invention relates to a compound of formula (Ie) 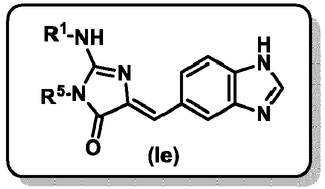 wherein R¹ represents:
- a (C₅-C₆)alkyl group substituted by a group selected from a hydroxy group, a methoxy group and a fluorine atom,
- a (C₅-C₈)cycloalkyl group in particular a cyclopentyl, a cyclohexyl, a cycloheptyl or a cyclooctyl, optionally substituted by a group selected from a hydroxy group and a methoxy group,
- a bridged (C₉-C₁₀)cycloalkyl group, in particular an adamantyl or a noradamantyl, optionally substituted by a group selected from a methoxy group, a fluorine atom and a hydroxy group,
- a phenyl group fused with a cyclopentyl, which cyclopentyl is optionally substituted by a hydroxy group,
- a phenyl group, substituted by a *N*-methylpiperazinyl group,
- a R'-L- group wherein L is either a single bond or a methylene group, and R' is selected from the group consisting of:
   ∘ a (C₆-C₇)heterocycloalkyl group, in particular a tetrahydropyranyl or an oxepanyl, optionally substituted by a hydroxy group, and
   ∘ a heteroaryl group, in particular a pyridinyl or a thiazolyl group, optionally substituted by a methyl group, or
- a R"-L- group wherein
   ∘ L is a (C₁-C₂)alkanediyl group, optionnally susbstituted by a group chosen from a hydroxy group, a methoxy group and a -NH₂ group, and
   ∘ R" is a phenyl group, optionally substituted by a trifluoromethyl, and
   R⁵ is as defined above,
or any pharmaceutically acceptable salt thereof.

According to another particular embodiment, the present invention relates to a compound of formula (If) 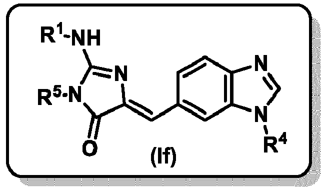 wherein R¹ represents:
- a (C₅-C₆)alkyl group substituted by a group selected from a hydroxy group, a methoxy group and a fluorine atom,
- a (C₆-C₈)cycloalkyl group in particular a cyclohexyl, a cycloheptyl or a cyclooctyl,
- a bridged (C₉-C₁₀)cycloalkyl group in particular an adamantyl or a noradamantyl, optionally substituted by a group selected from a methoxy group, a fluorine atom and a hydroxy group,
- a phenyl group fused with a cyclopentyl, which cyclopentyl is optionally substituted by a hydroxy group,
- a phenyl group, substituted by a *N*-methylpiperazinyl group,
- a R'-L- group wherein L is either a single bond or a methylene group, and R' is a heteroaryl group, in particular a pyridyl or a thiazolyl, optionally substituted by a methyl group,
- a Rₐ'-L- group wherein L is a methylene group and Ra' is a tetrahydropyranyl group, or
- a R"-L- group wherein
   ∘ L is a (C₁-C₂)alkanediyl group, optionnally susbstituted by a group chosen from a hydroxy group, a methoxy group and a -NH₂ group, and
   ∘ R" is a phenyl group, optionally substituted by a trifluoromethyl, and
   wherein R⁴ represents a methyl group, and
   R⁵ is as defined above,
or any pharmaceutically acceptable salt thereof.

According to another particular embodiment, the present invention relates to a compound of formula (I) as defined herein above, wherein R¹ represents:
- a (C₄-C₆)alkyl group substituted by a group selected from a hydroxy group, a halogen and a (C₁-C₃)alkoxy, or
- a (C₃-C₈)cycloalkyl group, optionally substituted by a group selected from a hydroxy group and a (C₁-C₃) alkoxy group,
- a bridged (C₆-C₁₀)cycloalkyl group, optionally substituted by a group selected from a (C₁-C₄)alkyl group, a (C₁-C₄)alkoxy group, a halogen atom and a hydroxy group,
or any pharmaceutically acceptable salt thereof.

Said sub-group of compounds are gathered under the "A1" type of compounds within the herein after table 1.

Still according to said embodiment, R¹ may more particularly represent a cyclohexyl, a cycloheptyl, a cyclooctyl, a 1-adamantyl, a 3-hydroxy-1-adamantyl, a 3-methoxy-1-adamantyl, a 1-(hydroxymethyl)-3-methyl-butyl, a 1-(methoxymethyl)-3-methyl-butyl, a 1-(fluoromethyl)-3-methyl-butyl, a 2-methoxycyclopentyl, a 4-hydroxycycloheptyl, a 3-methoxycycloheptyl, a 4-methoxycycloheptyl, a 3-noradamantyl, 3-fluoro-1-adamantyl.

According to another particular embodiment, the present invention relates to a compound of formula (I) as defined herein above wherein R¹ represents:
- a fused phenyl group, selected from phenyl groups fused with a (C₅-C₆)cycloalkyl, which (C₅-C₆)cycloalkyl is optionally substituted by a hydroxy group,
- a phenyl group, substituted by a (C₄-C₇)heterocycloalkyl group, said (C₄-C₇)heterocycloalkyl group being itself optionally substituted by a (C₁-C₄) alkyl group, or
- a R"-L- group, wherein
   ∘ L is a (C₁-C₃)alkanediyl group, optionnally susbstituted by a group chosen from a hydroxy group, a (C₁-C₃)alkoxy group and a -NR^{b}R^{c} group, and
   ∘ R" is a phenyl group, optionally substituted by a fluoro(C₁-C₄)alkyl group, wherein R^{b} and R^{c} are independently chosen from (C₁-C₆)alkyl and a hydrogen atom
or any pharmaceutically acceptable salt thereof.

Said sub-group of compounds are gathered under the "A2" and "A5" type of compounds within the herein after table 1.

Still according to said embodiment, R¹ may more particularly represent a a [2-(trifluoromethyl)phenyl]methyl, a 2-hydroxyindan-1-yl, a 2-amino-1-phenyl-ethyl, a 2-hydroxy-1-phenyl-ethyl, a 2-methoxy-1-phenyl-ethyl, a 2-hydroxy-2-phenyl-ethyl, a 4-(4-methylpiperazin- 1 -yl)phenyl.

According to another particular embodiment, the present invention relates to a compound of formula (I) as defined herein above wherein R¹ represents a R'-L- group wherein:
- R' is a (C₃-C₈)heteroaryl group, optionally substituted by a (C₁-C₄)alkyl group and
- L is a (C₁-C₃)alkanediyl or a single bond, or any pharmaceutically acceptable salt thereof.

Said sub-group of compounds are gathered under the "A3" and "A6" type of compounds within the herein after table 1.

Still according to said embodiment, R¹ may more particularly represent a (4-methylthiazol-2-yl)methyl or a 2-pyridyl.

According to another particular embodiment, the present invention relates to a compound of formula (I) as defined herein above wherein R¹ represents a R'-L- group wherein:
- R' is a (C₃-C₈)heterocycloalkyl group, optionally substituted by one to two groups selected from a hydroxy group and a (C₁-C₄)alkyl, and
- L is a (C₁-C₃)alkanediyl or a single bond, or any pharmaceutically acceptable salt thereof.

Said sub-group of compounds are gathered under the "A4" and "A7" type of compounds within the herein after table 1.

Still according to said embodiment, R¹ may more particularly a tetrahydropyran-4-yl-methyl, a tetrahydropyran-3-yl, a 6,6-dimethyltetrahydropyran-3-yl, a 4-hydroxytetrahydropyran-3-yl, or an oxepan-3-yl.

In the context of the present invention, the term:
- "halogen" is understood to mean chlorine, fluorine, bromine, or iodine, and in particular denotes chlorine, fluorine or bromine,
- "(Cₓ-C_{y})alkyl", as used herein, respectively refers to a Cₓ-C_{y} normal, secondary or tertiary monovalent saturated hydrocarbon radical, for example (C₁-C₆)alkyl. Examples are, but are not limited to, methyl, ethyl, propyl, *n*-propyl, isopropyl, butyl, isobutyl, *sec-*butyl, *tert*-butyl, pentyl, isopentyl, hexyl and isohexyl groups, and the like.
- "(C₁-C₃)alkanediyl", as used herein, refers to a divalent saturated hydrocarbon radical which is branched or linear, comprises from 1 to 3 carbon atoms, and more particularly a methylene, ethylene or propylene, such as linear propylene or isopropylene, said alkanediyl may be substituted as it is apparent from the following description.
- "(C₃-C₈)cycloalkyl", as used herein, refers to a cyclic saturated hydrocarbon, from 3 to 8 carbon atoms, saturated or partially unsaturated and unsubstituted or substituted. Examples are, but are not limited to, cyclopropyl, cyclobutyl, cyclopentyl, cyclohexyl, cycloheptyl and cyclooctyl.
- "(C₃-C₈)heterocycloalkyl group", as used herein, refers to a (C₃-C₈)cycloalkyl group wherein one or two of the carbon atoms are replaced with a heteroatom such as oxygen, nitrogen or sulphur, and more particularly such as an oxygen or a nitrogen atom. Such heterocycloalkyl group may be saturated or partially saturated and unsubstituted or substituted. Examples are, but are not limited to, morpholinyl, piperazinyl, piperidinyl, pyrrolidinyl, aziridinyl, oxanyl, oxetanyl, tetrahydropyranyl, morpholinyl, tetrahydrofuranyl, oxepanyl, diazepanyl, dioxanyl and tetrahydrothiopyranyl, and more particularly piperidinyl and piperazinyl, and even more particularly piperazinyl.
- "(C₁-Cₓ)alkoxy", as used herein, refers to a -O-(C₁-Cₓ)alkyl or -O-(C₃-Cₓ)cycloalkyl moiety, wherein alkyl and cycloalkyl are as defined above, for example (C₁-C₆)alkoxy. Examples are, but are not limited to, methoxy, ethoxy, 1-propoxy, 2-propoxy, cyclopropoxy, butoxy, *tert*-butoxy and pentoxy.
- a "bridged (C₆-C₁₀)cycloalkyl" group, as used herein, refers to a bi- or tricyclic compound where the cycles are cycloalkyls, the rings share three or more atoms and the bridge contains at least one atom, for example 1, 2 or 3 atoms. Such bridged cycloalkyl groups may be substituted by one or more C₁-C₃ alkyl. Examples are, but not limited to adamantyl and noradamantyl.
- a "fused phenyl group" refers to a bicyclic radical that contains a phenyl moiety and may be substituted. Said fused phenyl group may be fused to a cycloalkyl or to a heterocycloalkyl and bound to the rest of the molecule either by its phenyl moeity or by said cycloalkyl or heterocycloalkyl. Examples are, but are not limited to indanyl, acetylindolinyl, methylindazolyl, hydroxyindanyl, benzothiazolyl, indolyl, indazolyl, methoxyindanyl and the like.
- a (C₅-C₁₁)heteroaryl group, as used herein, refers to a monocyclic aromatic group or to a bicyclic aromatic group where at least one of the ring is aromatic and wherein one to three ring carbon atom is replaced by a heteroatom, such as nitrogen, oxygen or sulphur. By way of examples of heteroaryl groups, mention may be made of, but not limited to: oxazole, isoxazole, pyridine, pyrimidine, pyridazine, triazine, pyrazine, oxadiazole, furane, pyrazole, thiazole, isothiazole, thiadiazole, imidazole, triazole and the like. In the framework of the present invention, the heteroaryl is advantageously pyridine, imidazole, pyrazine, furane, thiazole, pyrazole, thiadiazole, pyridazine and pyrimidine.
- an aromatic ring means, according to Hückel's rule, that a molecule has 4*n*+2 π-electrons.
- a (C₁-Cₓ)fluoroalkyl group, as used herein, refers to a (C₁-Cₓ)alkyl as defined herein above in which one or more hydrogen atom(s) have been substituted by fluorines. In one embodiment all the hydrogen atoms are replaced by fluorine atoms, forming perfluoroalkyl groups, such as trifluoromethyl.

In the context of the present invention, the terms "aromatic ring", and "heteroaryl" include all the positional isomers.

The nomenclature of the following compounds (1) to (266) was generated according to the principles of the International Union of Pure and Applied Chemistry, using Accelrys Draw 4.1 SP1. To avoid any confusion, the "(±)" symbol added to designates a racemic mixture; *"cis"* and *"trans"* prefixes were also used to assign the relative stereochemistry of two adjacent chiral centers.

According to a preferred embodiment of the present invention, the compound of formula (I) is chosen from:
(1) (4*Z*)-4-(1,3-Benzoxazol-6-ylmethylene)-2-(cyclohexylamino)-1*H-*imidazol-5-one,
(2) (4*Z*)-4-(1,3-Benzoxazol-6-ylmethylene)-2-(cycloheptylamino)-1*H-*imidazol-5-one,
(3) (4*Z*)-4-(1,3-Benzoxazol-6-ylmethylene)-2-(cyclooctylamino)-1*H-*imidazol-5-one,
(4) (4*Z*)-4-(1,3-Benzoxazol-6-ylmethylene)-2-[[(1*R*)-1-(hydroxymethyl)-3-methyl-butyl]amino]-1*H*-imidazol-5-one,
(5) (4*Z*)-4-(1,3-Benzoxazol-6-ylmethylene)-2-[[(1*R*)-1-(methoxymethyl)-3-methyl-butyl]amino]-1*H*-imidazol-5-one,
(6) (4*Z*)-4-(1,3-Benzoxazol-6-ylmethylene)-2-[[(1*R*,2*R*)-2-methoxycyclopentyl]amino]-1*H*-imidazol-5-one,
(7) (4*Z*)-4-(1,3-Benzoxazol-6-ylmethylene)-2-[[(1*S*,2*S*)-2-methoxycyclopentyl]amino]-1*H*-imidazol-5-one,
(8) (±)-(4*Z*)-4-(1,3-Benzoxazol-6-ylmethylene)-2-[[*trans*-4-hydroxycycloheptyl]amino]-1*H*-imidazol-5-one,
(9) (±)-(4*Z*)-4-(1,3-Benzoxazol-6-ylmethylene)-2-[[*trans*-4-methoxycycloheptyl]amino]-1*H*-imidazol-5-one,
(10) (±)-(4*Z*)-4-(1,3-Benzoxazol-6-ylmethylene)-2-[[*cis*-3-methoxycycloheptyl]amino]-1*H*-imidazol-5-one,
(11) (4*Z*)-2-(1-Adamantylamino)-4-(1,3-benzoxazol-6-ylmethylene)-1*H-*imidazol-5-one,
(12) (4*Z*)-4-(1,3-Benzoxazol-6-ylmethylene)-2-[(3-hydroxy-1-adamantyl)amino]-1*H*-imidazol-5-one,
(13) (4*Z*)-4-(1,3-Benzoxazol-6-ylmethylene)-2-[(3-methoxy-1-adamantyl)amino]-1*H*-imidazol-5-one.
(14) (4*Z*)-4-(1,3-Benzoxazol-6-ylmethylene)-2-[[2-(trifluoromethyl)phenyl)methylamino]-1*H*-imidazol-5-one,
(15) (4Z)-4-(1,3-Benzoxazol-6-ylmethylene)-2-[[(1*S*,2*S*)-2-hydroxyindan-1-yl]amino]-1*H*-imidazol-5-one,
(16) (4*Z*)-4-(1,3-Benzoxazol-6-ylmethylene)-2-[[(1*R*)-2-hydroxy-1-phenylethyl]amino]-1*H*-imidazol-5-one,
(17) (4*Z*)-4-(1,3-Benzoxazol-6-ylmethylene)-2-[[(1*S*)-2-hydroxy-1-phenylethyl]amino]-1*H*-imidazol-5-one,
(18) (4*Z*)-4-(1,3-Benzoxazol-6-ylmethylene)-2-[[(1*R*)-2-methoxy-1-phenylethyl]amino]-1*H*-imidazol-5-one,
(19) (4*Z*)-4-(1,3-Benzoxazol-6-ylmethylene)-2-[(4-methylthiazol-2-yl)methylamino]-1*H*-imidazol-5-one,
(20) (4Z)-4-(1,3-Benzoxazol-6-ylmethylene)-2-(tetrahydropyran-4-ylmethylamino)-1*H*-imidazol-5-one,
(21) (4*Z*)-4-(1,3-Benzoxazol-6-ylmethylene)-2-[4-(4-methylpiperazin-1-yl)anilino]-1*H*-imidazol-5-one,
(22) (4*Z*)-4-(1,3-Benzoxazol-6-ylmethylene)-2-(2-pyridylamino)-1*H-*imidazol-5-one,
(23) (±)-(4*Z*)-4-(1,3-Benzoxazol-6-ylmethylene)-2-[(6,6-dimethyltetrahydropyran-3-yl)amino]-1*H*-imidazol-5-one.
(24) (4Z)-4-(1,3-Benzoxazol-6-ylmethylene)-2-[[(3*S*)-tetrahydropyran-3-yl]amino]-1*H*-imidazol-5-one,
(25) (±)-(4*Z*)-4-(1,3-Benzoxazol-6-ylmethylene)-2-(oxepan-3-ylamino)-1*H-*imidazol-5-one,
(26) (4*Z*)-2-(Cyclohexylamino)-4-(1*H*-indazol-5-ylmethylene)-1*H*-imidazol-5-one,
(27) 4*Z*)-2-(Cycloheptylamino)-4-(1*H*-indazol-5-ylmethylene)-1*H*-imidazol-5-one,
(28) 4*Z*)-2-(Cyclooctylamino)-4-(1*H*-indazol-5-ylmethylene)-1*H*-imidazol-5-one,
(29) (4*Z*)-2-[[(1*R*)-1-(Hydroxymethyl)-3-methyl-butyl]amino]-4-(1*H-*indazol-5-ylmethylene)-1*H*-imidazol-5-one,
(30) (4Z)-4-(1*H*-Indazol-5-ylmethylene)-2-[[(1*R*)-1-(methoxymethyl)-3-methyl-butyl]amino]-1H-imidazol-5-one,
(31) (4*Z*)-2-(1-Adamantylamino)-4-(1*H*-indazol-5-ylmethylene)-1*H-*imidazol-5-one,
(32) (4*Z*)-2-[(3-Hydroxy-1-adamantyl)amino]-4-(1*H*-indazol-5-ylmethylene)-1*H*-imidazol-5-one,
(33) (4*Z*)-4-(1*H*-Indazol-5-ylmethylene)-2-[[(1*R*)-2-methoxy-1-phenylethyl]amino]-1*H*-imidazol-5-one,
(34) (4*Z*)-2-(Cyclohexylamino)-4-[(2-methylindazol-5-yl)methylene]-1*H-*imidazol-5-one,
(35) (4*Z*)-2-(Cycloheptylamino)-4-[(2-methylindazol-5-yl)methylene]-1*H-*imidazol-5-one,
(36) (4*Z*)-2-(Cyclooctylamino)-4-[(2-methylindazol-5-yl)methylene]-1*H-*imidazol-5-one,
(37) (4*Z*)-2-[[(1*R*)-1-(Hydroxymethyl)-3-methyl-butyl]amino]-4-[(2-methylindazol-5-yl)methylene]-1*H*-imidazol-5-one,
(38) (4*Z*)-2-[[(1*R*)-1-(Methoxymethyl)-3-methyl-butyl]amino]-4-[(2-methylindazol-5-yl)methylene]-1*H*-imidazol-5-one,
(39) (4*Z*)-2-(1-Adamantylamino)-4-[(2-methylindazol-5-yl)methylenel-1*H-*imidazol-5-one,
(40) (4*Z*)-2-[(3-Hydroxy-1-adamantyl)amino]-4-[(2-methylindazol-5-yl)methylene]-1*H*-imidazol-5-one,
(41) (4*Z*)-2-[(3-Fluoro-1-adamantyl)amino]-4-[(2-methylindazol-5-yl)methylene]-1*H*-imidazol-5-one,
(42) (4*Z*)-2-[[(1*R*)-2-Methoxy-1-phenyl-ethyl]amino]-4-[(2-methylindazol-5-yl)methylene]-1*H*-imidazol-5-one,
(43) (4*Z*)-2-(Cyclohexylamino)-4-[(1-methylindazol-5-yl)methylene]-1*H-*imidazol-5-one,
(44) (4*Z*)-2-(Cycloheptylamino)-4-[(1-methylindazol-5-yl)methylene]-1*H-*imidazol-5-one,
(45) (4*Z*)-2-(Cyclooctylamino)-4-[(1-methylindazol-5-yl)methylene]-1*H-*imidazol-5-one,
(46) (4*Z*)-2-[[(1*R*)-1-(Hydroxymethyl)-3-methyl-butyl]amino]-4-[(1-methylindazol-5-yl)methylene]-1*H*-imidazol-5-one,
(47) (*4*Z)-2-[[(1*R*)-1-(Methoxymethyl)-3-methyl-butyl]amino]-4-[(1-methylindazol-5-yl)methylene]-1*H*-imidazol-5-one,
(48) (4*Z*)-2-[[(1*R*)-1-(Fluoromethyl)-3-methyl-butyl]amino]-4-[(1-methylindazol-5-yl)methylene]-1*H*-imidazol-5-one,
(49) (4*Z*)-2-[[(1*S*)-1-(Fluoromethyl)-3-methyl-butyl]amino]-4-[(1-methylindazol-5-yl)methylene]-1*H*-imidazol-5-one,
(50) (4*Z*)-2-[[(1*R*,2*R*)-2-Methoxycyclopentyl]amino]-4-[(1-methylindazol-5-yl)methylene]-1*H*-imidazol-5-one,
(51) (4Z)-2- [[(1*S*,2*S*)-2-Methoxycyclopentyl]amino]-4-[(1 -methylindazol-5-yl)methylene]-1*H*-imidazol-5-one,
(52) (±)-(4Z)-2-[[*trans*-4-Hydroxycycloheptyl]amino]-4-[(1-methylindazol-5-yl)methylene]-1H-imidazol-5-one,
(53) (±)-(4*Z*)-2-[[*trans*-4-Methoxycycloheptyl]amino]-4-[(1-methylindazol-5-yl)methylene]-1*H*-imidazol-5-one,
(54) (±)-(4*Z*)-2-[[*cis*-3-Methoxycycloheptyl]amino]-4-[(1-methylindazol-5-yl)methylene]-1*H*-imidazol-5-one,
(55) (4*Z*)-2-(1-Adamantylamino)-4-[(1-methylindazol-5-yl)methylene]-1*H-*imidazol-5-one,
(56) (4*Z*)-2-[(3-Hydroxy-1-adamantyl)amino]-4-[(1-methylindazol-5-yl)methylene]-1*H*-imidazol-5-one,
(57) (4Z)-2-[(3-Methoxy-1-adamantyl)amino]-4-[(1-methylindazol-5-yl)methylene]-1*H*-imidazol-5-one,
(58) (4Z)-2-[(3-Fluoro-1-adamantyl)amino]-4-[(1-methylindazol-5-yl)methylene]-1*H*-imidazol-5-one,
(59) (4*Z*)-4-[(1-Methylindazol-5-yl)methylene]-2-[[2-(trifluoromethyl)phenyl]methylamino]-1*H*-imidazol-5-one,
(60) (4*Z*)-2-[[(1*S*,2*S*)-2-Hydroxyindan-1-yl]amino]-4-[(1-methylindazol-5-yl)methylene]-1*H*-imidazol-5-one,
(61) (4Z)-2-[[(1*R*)-2-Hydroxy-1-phenyl-ethyl]amino]-4-[(1-methylindazol-5-yl)methylene]-1*H*-imidazol-5-one.
(62) (4*Z*)-2-[[(1*S*)-2-Hydroxy-1-phenyl-ethyl]amino]-4-[(1-methylindazol-5-yl)methylene]-1*H*-imidazol-5-one,
(63) (4*Z*)-2-[[(1*R*)-2-Methoxy-1-phenyl-ethyl]amino]-4-[(1-methylindazol-5-yl)methylene]-1*H*-imidazol-5-one,
(64) (4*Z*)-2-[[(2*R*)-2-Hydroxy-2-phenyl-ethyl]amino]-4-[(1-methylindazol-5-yl)methylene]-1*H*-imidazol-5-one,
(65) (4Z)-2-[[(1*R*)-2-Amino-1-phenyl-ethyl]amino]-4-[(1-methylindazol-5-yl)methylene]-1*H*-imidazol-5-one dihydrochloride,
(66) (4*Z*)-2-[[(1*S*)-2-Amino-1-phenyl-ethyl]amino]-4-[(1-methylindazol-5-yl)methylene]-1*H*-imidazol-5-one dihydrochloride,
(67) (4*Z*)-4-[(1-Methylindazol-5-yl)methylene]-2-[(4-methylthiazol-2-yl)methylamino]-1*H*-imidazol-5-one,
(68) (4*Z*)-4-[(1-Methylindazol-5-yl)methylene]-2-(tetrahydropyran-4-ylmethylamino)-1*H*-imidazol-5-one,
(69) (4*Z*)-4-[(1-Methylindazol-5-yl)methylene]-2-[4-(4-methylpiperazin-1-yl)anilino]-1*H*-imidazol-5-one,
(70) (4*Z*)-4-[(1-Methylindazol-5-yl)methylene]-2-(2-pyridylamino)-1*H-*imidazol-5-one,
(71) (4*Z*)-2-[(6,6-Dimethyltetrahydropyran-3-yl)amino]-4-[(1-methylindazol-5-yl)methylene]-1*H*-imidazol-5-one,
(72) (4*Z*)-4-[(1-Methylindazol-5-yl)methylene]-2-[[(3*S*)-tetrahydropyran-3-yl]amino]-1*H*-imidazol-5-one,
(73) (4*Z*)-2-[[(3*R*,4*R*)-4-Hydroxytetrahydropyran-3-yl]amino]-4-[(1-methylindazol-5-yl)methylene]-1*H*-imidazol-5-one,
(74) (±)-(4*Z*)-4-[(1-Methylindazol-5-yl)methylene]-2-(oxepan-3-ylamino)-1*H*-imidazol-5-one,
(75) (4*Z*)-4-(1*H*-Benzimidazol-5-ylmethylene)-2-(cyclohexylamino)-1*H-*imidazol-5-one,
(76) (4*Z*)-4-(1*H*-Benzimidazol-5-ylmethylene)-2-(cycloheptylamino)-1*H-*imidazol-5-one.
(77) (4*Z*)-4-(1*H*-Benzimidazol-5-ylmethylene)-2-(cyclooctylamino)-1*H-*imidazol-5-one,
(78) (4*Z*)-4-(1*H*-Benzimidazol-5-ylmethylene)-2-[[(1*R*)-1-(hydroxymethyl)-3-methyl-butyl]amino]-1*H*-imidazol-5-one,
(79) (4*Z*)-4-(1*H*-Benzimidazol-5-ylmethylene)-2-[[(1*R*)-1-(methoxymethyl)-3-methyl-butyl]amino]-1*H*-imidazol-5-one,
(80) (4*Z*)-4-(1*H*-Benzimidazol-5-ylmethylene)-2-[[(1*R*)-1-(fluoromethyl)-3-methyl-butyl]amino]-1*H*-imidazol-5-one,
(81) (4*Z*)-4-(1*H*-Benzimidazol-5-ylmethylene)-2-[[(1*S*)-1-(fluoromethyl)-3-methyl-butyl]amino]-1*H*-imidazol-5-one,
(82) (4*Z*)-4-(1*H*-Benzimidazol-5-ylmethylene)-2-[[(1*R*,2*R*)-2-methoxycyclopentyl]amino]-1*H*-imidazol-5-one,
(83) (4*Z*)-2-(3-Noradamantylamino)-4-(1*H*-benzimidazol-5-ylmethylene)-1*H*-imidazol-5-one,
(84) (4*Z*)-2-(1-Adamantylamino)-4-(1*H*-benzimidazol-5-ylmethylene)-1*H-*imidazol-5-one,
(85) (4*Z*)-4-(1*H*-Benzimidazol-5-ylmethylene)-2-[(3-hydroxy-1-adamantyl)amino]-1*H*-imidazol-5-one,
(86) (4*Z*)-4-(1*H*-Benzimidazol-5-ylmethylene)-2-[(3-methoxy-1-adamantyl)amino]-1*H*-imidazol-5-one,
(87) (4*Z*)-4-(1*H*-benzimidazol-5-ylmethylene)-2-[(3-fluoro-1-adamantyl)amino]-1*H*-imidazol-5-one,
(88) (4*Z*)-4-(1*H*-Benzimidazol-5-ylmethylene)-2-[[2-(trifluoromethyl)phenyl)methylamino]-1*H*-imidazol-5-one,
(89) (4*Z*)-4-(1*H*-Benzimidazol-5-ylmethylene)-2-[[(1*S*,2*S*)-2-hydroxyindan-1-yl]amino]-1*H*-imidazol-5-one,
(90) (4*Z*)-4-(1*H*-Benzimidazol-5-ylmethylene)-2-[[(1*R*)-2-hydroxy-1-phenyl-ethyl]aminol-1*H*-imidazol-5-one,
(91) (4*Z*)-4-(1*H*-Benzimidazol-5-ylmethylene)-2-[[(1*S*)-2-hydroxy-1-phenyl-ethyl]aminol-1*H*-imidazol-5-one,
(92) (4*Z*)-4-(1*H*-Benzimidazol-5-ylmethylene)-2-[[(1*R*)-2-methoxy-1-phenyl-ethyl]aminol-1*H*-imidazol-5-one,
(93) (4*Z*)-4-(1*H*-Benzimidazol-5-ylmethylene)-2-[[(2*R*)-2-hydroxy-2-phenyl-ethyl]aminol-1*H*-imidazol-5-one,
(94) (4*Z*)-4-(1*H*-Benzimidazol-5-ylmethylene)-2-[[(2*S*)-2-hydroxy-2-phenyl-ethyl]aminol-1*H*-imidazol-5-one,
(95) (4*Z*)-2-[[(1*R*)-2-Amino-1-phenyl-ethyl]amino]-4-(1*H*-benzimidazol-5-ylmethylene)-1*H*-imidazol-5-one dihydrochloride,
(96) (4*Z*)-2-[[(1*S*)-2-Amino-1-phenyl-ethyl]amino]-4-(1*H*-benzimidazol-5-ylmethylene)-1*H*-imidazol-5-one dihydrochloride,
(97) (4*Z*)-4-(1*H*-Benzimidazol-5-ylmethylene)-2-[(4-methylthiazol-2-yl)methylamino]-1*H*-imidazol-5-one,
(98) (4*Z*)-4-(1*H*-Benzimidazol-5-ylmethylene)-2-(tetrahydropyran-4-ylmethylamino)-1*H*-imidazol-5-one,
(99) (4*Z*)-4-(1*H*-Benzimidazol-5-ylmethylene)-2-[4-(4-methylpiperazin-1-yl)anilino]-1*H*-imidazol-5-one,
(100) (4*Z*)-4-(1*H*-Benzimidazol-5-ylmethylene)-2-(2-pyridylamino)-1*H-*imidazol-5-one,
(101) (4*Z*)-4-(1*H*-Benzimidazol-5-ylmethylene)-2-[[(3*S*)-tetrahydropyran-3-yl]amino]-1*H*-imidazol-5-one,
(102) (4*Z*)-4-(1*H*-Benzimidazol-5-ylmethylene)-2-[[(3*R*,4*R*)-4-hydroxytetrahydropyran-3-yl]amino]-1*H*-imidazol-5-one,
(103) (4*Z*)-4-(1*H*-Benzimidazol-5-ylmethylene)-2-[[(3*S*,4*S*)-4-hydroxytetrahydropyran-3-yl]amino]-1*H*-imidazol-5-one,
(104) (4*Z*)-4-(1*H*-Benzimidazol-5-ylmethylene)-2-(oxepan-3-ylamino)-1*H-*imidazol-5-one,
(105) (4Z)-2-(Cyclohexylamino)-4-[(3-methylbenzimidazol-5-yl)methylene]-1*H*-imidazol-5-one,
(106) (4Z)-2-(Cycloheptylamino)-4-[(3-methylbenzimidazol-5-yl)methylene]-1*H*-imidazol-5-one,
(107) (4Z)-2-(Cyclooctylamino)-4-[(3-methylbenzimidazol-5-yl)methylene]-1*H*-imidazol-5-one,
(108) (4*Z*)-2-[[(1*R*)-1-(Hydroxymethyl)-3-methyl-butyl]amino]-4-[(3-methylbenzimidazol-5-yl)methylene]-1*H*-imidazol-5-one,
(109) (4*Z*)-2-[[(1*R*)-1-(Methoxymethyl)-3-methyl-butyl]amino]-4-[(3-methylbenzimidazol-5-yl)methylene]-1*H*-imidazol-5-one,
(110) (4*Z*)-2-[[(1*R*)-1-(Fluoromethyl)-3-methyl-butyl]amino]-4-[(3-methylbenzimidazol-5-yl)methylene]-1*H*-imidazol-5-one,
(111) (4*Z*)-2-[[(1*S*)-1-(Fluoromethyl)-3-methyl-butyl]amino]-4-[(3-methylbenzimidazol-5-yl)methylene]-1*H*-imidazol-5-one,
(112) (4*Z*)-2-[[(1*R*,2*R*)-2-Methoxycyclopentyl]amino]-4-[(3-methylbenzimidazol-5-yl)methylene]-1*H*-imidazol-5-one,
(113) (4*Z*)-2-[[(1*S*,2*S*)-2-Methoxycyclopentyl]amino]-4-[(3-methylbenzimidazol-5-yl)methylene]-1*H*-imidazol-5-one,
(114) (4Z)-2-(3-Noradamantylamino)-4-[(3-methylbenzimidazol-5-yl)methylene]-1*H*-imidazol-5-one,
(115) (4*Z*)-2-(1-Adamantylamino)-4-[(3-methylbenzimidazol-5-yl)methylene]-1*H*-imidazol-5-one,
(116) (4*Z*)-2-[(3-Hydroxy-1-adamantyl)amino]-4-[(3-methylbenzimidazol-5-yl)methylene]-1*H*-imidazol-5-one,
(117) (4*Z*)-2-[(3-Methoxy-1-adamantyl)amino]-4-[(3-methylbenzimidazol-5-yl)methylene]-1*H*-imidazol-5-one,
(118) (4*Z*)-2-[(3-Fluoro-1-adamantyl)amino]-4-[(3-methylbenzimidazol-5-yl)methylene]-1*H*-imidazol-5-one,
(119) (4Z)-4-[(3-Methylbenzimidazol-5-yl)methylene]-2-[[2-(trifluoromethyl)phenyl]methylamino]-1*H*-imidazol-5-one,
(120) (4*Z*)-2-[[(1*S*,2*S*)-2-Hydroxyindan-1-yl]amino]-4-[(3-methylbenzimidazol-5-yl)methylene]-1*H*-imidazol-5-one,
(121) (4*Z*)-2-[[(1*R*)-2-Hydroxy-1-phenyl-ethyl]amino]-4-[(3-methylbenzimidazol-5-yl)methylene]-1*H*-imidazol-5-one,
(122) (4*Z*)-2-[[(1*S*)-2-Hydroxy-1-phenyl-ethyl]amino]-4-[(3-methylbenzimidazol-5-yl)methylene]-1*H*-imidazol-5-one,
(123) (4*Z*)-2-[[(1*R*)-2-Methoxy-1-phenyl-ethyl]amino]-4-[(3-methylbenzimidazol-5-yl)methylene]-1*H*-imidazol-5-one,
(124) (4*Z*)-2-[[(2*R*)-2-Hydroxy-2-phenyl-ethyl]amino]-4-[(3-methylbenzimidazol-5-yl)methylene]-1*H*-imidazol-5-one,
(125) (4*Z*)-2-[[(2*S*)-2-Hydroxy-2-phenyl-ethyl]amino]-4-[(3-methylbenzimidazol-5-yl)methylene]-1*H*-imidazol-5-one,
(126) (4*Z*)-2-[[(1*R*)-2-amino-1-phenyl-ethyl]amino]-4-[(3-methylbenzimidazol-5-yl)methylene]-1*H*-imidazol-5-one dihydrochloride,
(127) (4*Z*)-2-[[(1*S*)-2-amino-1-phenyl-ethyl]amino]-4-[(3-methylbenzimidazol-5-yl)methylene]-1*H*-imidazol-5-one dihydrochloride,
(128) (4*Z*)-4-[(3-Methylbenzimidazol-5-yl)methylene]-2-[(4-methylthiazol-2-yl)methylamino]-1*H*-imidazol-5-one,
(129) (4*Z*)-4-[(3-Methylbenzimidazol-5-yl)methylene]-2-(tetrahydropyran-4-ylmethylamino)-1*H*-imidazol-5-one,
(130) (4*Z*)-4-[(3-Methylbenzimidazol-5-yl)methylene]-2-[4-(4-methylpiperazin-1-yl)anilino]-1*H*-imidazol-5-one,
(131) (4*Z*)-4-[(3-Methylbenzimidazol-5-yl)methylene]-2-(2-pyridylamino)-1*H*-imidazol-5-one,
(132) (5Z)-5-(1,3-Benzoxazol-6-ylmethylene)-2-(cyclohexylamino)-3-methyl-imidazol-4-one,
(133) (5*Z*)-5-(1,3-Benzoxazol-6-ylmethylene)-2-(cyclooctylamino)-3-methyl-imidazol-4-one,
(134) (5*Z*)-5-(1,3-Benzoxazol-6-ylmethylene)-2-[[(1*R*)-1-(methoxymethyl)-3-methyl-butyl]amino]-3-methyl-imidazol-4-one,
(135) (5*Z*)-5-(1,3-Benzoxazol-6-ylmethylene)-2-[[(1*R*,2*R*)-2-methoxycyclopentyl]amino]-3-methyl-imidazol-4-one,
(136) (5*Z*)-5-(1,3-Benzoxazol-6-ylmethylene)-2-[[(1S*R*,2*S*)-2-methoxycyclopentyl]amino]-3-methyl-imidazol-4-one,
(137) (5*Z*)-2-(3-Noradamantylamino)-5-(1,3-benzoxazol-6-ylmethylene)-3-methyl-imidazol-4-one,
(138) (5*Z*)-2-(1-Adamantylamino)-5-(1,3-benzoxazol-6-ylmethylene)-3-methyl-imidazol-4-one,
(139) (5*Z*)-5-(1,3-Benzoxazol-6-ylmethylene)-2-[(3-hydroxy-1-adamantyl)amino]-3-methyl-imidazol-4-one,
(140) (5*Z*)-5-(1,3-Benzoxazol-6-ylmethylene)-2-[(3-methoxy-1-adamantyl)amino]-3-methyl-imidazol-4-one,
(141) (5*Z*)-5-(1,3-Benzoxazol-6-ylmethylene)-2-[(3-fluoro-1-adamantyl)amino]-3-methyl-imidazol-4-one,
(142) (5*Z*)-5-(1,3-Benzoxazol-6-ylmethylene)-3-methyl-2-[[2-(trifluoromethyl)phenyl]methylamino]imidazol-4-one,
(143) (5*Z*)-5-(1,3-Benzoxazol-6-ylmethylene)-2-[[(1*R*)-2-methoxy-1-phenylethyl]amino]-3-methyl-imidazol-4-one,
(144) (5*Z*)-5-(1,3-Benzoxazol-6-ylmethylene)-3-methyl-2-[(4-methylthiazol-2-yl)methylamino]imidazol-4-one,
(145) (5*Z*)-2-(Cyclohexylamino)-5-(1*H*-indazol-5-ylmethylene)-3-methyl-imidazol-4-one,
(146) (5*Z*)-2-(Cycloheptylamino)-5-(1*H*-indazol-5-ylmethylene)-3-methyl-imidazol-4-one,
(147) (5*Z*)-2-(Cyclooctylamino)-5-(1*H*-indazol-5-ylmethylene)-3-methyl-imidazol-4-one,
(148) (5*Z*)-2-[[(1*R*)-1-(Hydroxymethyl)-3-methyl-butyl]amino]-5-(1*H-*indazol-5-ylmethylene)-3-methyl-imidazol-4-one,
(149) (5*Z*)-5-(1*H*-Indazol-5-ylmethylene)-2-[[(1*R*)-1-(methoxymethyl)-3-methyl-butyl]amino]-3-methyl-imidazol-4-one,
(150) (5*Z*)-5-(1*H*-Indazol-5-ylmethylene)-2-[[(1*R*,2*R*)-2-methoxycyclopentyl]amino]-3-methyl-imidazol-4-one,
(151) (5*Z*)-5-(1*H*-Indazol-5-ylmethylene)-2-[[(1*S*,2*S*)-2-methoxycyclopentyl]amino]-3-methyl-imidazol-4-one,
(152) (5*Z*)-2-(3-Nordamantylamino)-5-(1*H*-indazol-5-ylmethylene)-3-methyl-imidazol-4-one,
(153) (5*Z*)-2-(1-Adamantylamino)-5-(1*H*-indazol-5-ylmethylene)-3-methyl-imidazol-4-one,
(154) (5*Z*)-2-[(3-Hydroxy-1-adamantyl)amino]-5-(1*H*-indazol-5-ylmethylene)-3-methyl-imidazol-4-one,
(155) (5*Z*)-5-(1*H*-Indazol-5-ylmethylene)-2-[(3-methoxy-1-adamantyl)amino]-3-methyl-imidazol-4-one,
(156) (5*Z*)-2-[(3-Fluoro-1-adamantyl)amino]-5-(1*H*-indazol-5-ylmethylene)-3-methyl-imidazol-4-one,
(157) (5*Z*)-5-(1*H*-Indazol-5-ylmethylene)-3-methyl-2-[[2-(trifluoromethyl)phenyl]methylamino]imidazol-4-one,
(158) (5Z)-2-[[(1*S*,2*S*)-2-Hydroxyindan-1-yl]amino]-5-(1*H*-indazol-5-ylmethylene)-3-methyl-imidazol-4-one,
(159) (5*Z*)-2-[[(1*R*)-2-Hydroxy-1-phenyl-ethyl]amino]-5-(1*H*-indazol-5-ylmethylene)-3-methyl-imidazol-4-one,
(160) (5*Z*)-2-[[(1*S*)-2-Hydroxy-1-phenyl-ethyl]amino]-5-(1*H*-indazol-5-ylmethylene)-3-methyl-imidazol-4-one,
(161) (5*Z*)-5-(1*H*-Indazol-5-ylmethylene)-2-[[(1*R*)-2-methoxy-1-phenylethyl]amino]-3-methyl-imidazol-4-one,
(162) (5*Z*)-2-[[(2*R*)-2-Hydroxy-2-phenyl-ethyl]amino]-5-(1*H*-indazol-5-ylmethylene)-3-methyl-imidazol-4-one,
(163) (5*Z*)-2-[[(1*R*)-2-Amino-1-phenyl-ethyl]amino]-5-(1*H*-indazol-5-ylmethylene)-3-methyl-imidazol-4-one dihydrochloride,
(164) (5*Z*)-5-(1*H*-Indazol-5-ylmethylene)-3-methyl-2-[(4-methylthiazol-2-yl)methylamino]imidazol-4-one,
(165) (5*Z*)-5-(1*H*-Indazol-5-ylmethylene)-3-methyl-2-(tetrahydropyran-4-ylmethylamino)imidazol-4-one,
(166) (5*Z*)-5-(1*H*-Indazol-5-ylmethylene)-3-methyl-2-[4-(4-methylpiperazin-1-yl)anilino]imidazol-4-one,
(167) (5*Z*)-5-(1*H*-Indazol-5-ylmethylene)-3-methyl-2-(2-pyridylamino)imidazol-4-one,
(168) (5*Z*)-5-(1*H*-Indazol-5-ylmethylene)-3-methyl-2-[[(3*S*)-tetrahydropyran-3-yl]amino]imidazol-4-one,
(169) (5*Z*)-2-[[(3*R*,4*R*)-4-Hydroxytetrahydropyran-3-yl]amino]-5-(1*H-*indazol-5-ylmethylene)-3-methyl-imidazol-4-one,
(170) (5*Z*)-2-(Cyclohexylamino)-3-methyl-5-[(2-methylindazol-5-yl)methylene]imidazol-4-one,
(171) (5*Z*)-2-(Cycloheptylamino)-3-methyl-5-[(2-methylindazol-5-yl)methylene]imidazol-4-one,
(172) (5*Z*)-2-(Cyclooctylamino)-3-methyl-5-[(2-methylindazol-5-yl)methylene]imidazol-4-one,
(173) (5*Z*)-2-[[(1*R*)-1-(Hydroxymethyl)-3-methyl-butyl]amino]-3-methyl-5-[(2-methylindazol-5-yl)methylene]imidazol-4-one,
(174) (5*Z*)-2-[[(1*R*)-1-(Methoxymethyl)-3-methyl-butyl]amino]-3-methyl-5-[(2-methylindazol-5-yl)methylene]imidazol-4-one,
(175) (5*Z*)-2-[[(1*R*,2*R*)-2-Methoxycyclopentyl]amino]-3-methyl-5-[(2-methylindazol-5-yl)methylene]imidazol-4-one,
(176) (5*Z*)-2-[[(1*S*,2*S*)-2-Methoxycyclopentyl]amino]-3-methyl-5-[(2-methylindazol-5-yl)methylene]imidazol-4-one,
(177) (5*Z*)-2-(3-Noradamantylamino)-3-methyl-5-[(2-methylindazol-5-yl)methylene]imidazol-4-one,
(178) (5*Z*)-2-(1-Adamantylamino)-3-methyl-5-[(2-methylindazol-5-yl)methylene]imidazol-4-one,
(179) (5*Z*)-2-[(3-Hydroxy-1-adamantyl)amino]-3-methyl-5-[(2-methylindazol-5-yl)methylene]imidazol-4-one,
(180) (5*Z*)-2-[(3-Methoxy-1-adamantyl)amino]-3-methyl-5-[(2-methylindazol-5-yl)methylene]imidazol-4-one,
(181) (5*Z*)-2-[(3-Fluoro-1-adamantyl)amino]-3-methyl-5-[(2-methylindazol-5-yl)methylene]imidazol-4-one,
(182) (5*Z*)-3-Methyl-5-[(2-methylindazol-5-yl)methylene]-2-[[2-(trifluoromethyl)phenyl]methylamino]imidazol-4-one,
(183) (5*Z*)-2-[[(1*S*,2*S*)-2-Hydroxyindan-1-yl]amino]-3-methyl-5-[(2-methylindazol-5-yl)methylene]imidazol-4-one,
(184) (5*Z*)-2-[[(1*R*)-2-Hydroxy-1-phenyl-ethyl]amino]-3-methyl-5-[(2-methylindazol-5-yl)methylene]imidazol-4-one,
(185) (5*Z*)-2-[[(1*S*)-2-Hydroxy-1-phenyl-ethyl]amino]-3-methyl-5-[(2-methylindazol-5-yl)methylene]imidazol-4-one,
(186) (5*Z*)-2-[[(1*R*)-2-Methoxy-1-phenyl-ethyl]amino]-3-methyl-5-[(2-methylindazol-5-yl)methylene]imidazol-4-one,
(187) (5*Z*)-2-[[(2*R*)-2-Hydroxy-2-phenyl-ethyl]amino]-3-methyl-5-[(2-methylindazol-5-yl)methylene]imidazol-4-one,
(188) (5*Z*)-2-[[(1*R*)-2-Amino-1-phenyl-ethyl]amino]-3-methyl-5-[(2-methylindazol-5-yl)methylene]imidazol-4-one dihydrochloride,
(189) (5*Z*)-3-Methyl-5-[(2-methylindazol-5-yl)methylene]-2-[(4-methylthiazol-2-yl)methylamino]imidazol-4-one,
(190) (5*Z*)-3-Methyl-5-[(2-methylindazol-5-yl)methylene]-2-(tetrahydropyran-4-ylmethylamino)imidazol-4-one,
(191) (5*Z*)-3-Methyl-5-[(2-methylindazol-5-yl)methylene]-2-[4-(4-methylpiperazin-1-yl)anilino]imidazol-4-one,
(192) (5*Z*)-3-Methyl-5-[(2-methylindazol-5-yl)methylene]-2-(2-pyridylamino)imidazol-4-one,
(193) (5*Z*)-3-Methyl-5-[(2-methylindazol-5-yl)methylene]-2-[[(3*S*)-tetrahydropyran-3-yl]amino]imidazol-4-one,
(194) (5*Z*)-2-[[(3*R*,4*R*)-4-Hydroxytetrahydropyran-3-yl]amino]-3-methyl-5-[(2-methylindazol-5-yl)methylene]imidazol-4-one,
(195) (5*Z*)-2-(Cyclohexylamino)-3-methyl-5-[(1-methylindazol-5-yl)methylene]imidazol-4-one,
(196) (5*Z*)-2-(Cycloheptylamino)-3-methyl-5-[(1-methylindazol-5-yl)methylene]imidazol-4-one,
(197) (5*Z*)-2-(Cyclooctylamino)-3-methyl-5-[(1-methylindazol-5-yl)methylene]imidazol-4-one,
(198) (5*Z*)-2-[[(1*R*)-1-(Hydroxymethyl)-3-methyl-butyl]amino]-3-methyl-5-[(1-methylindazol-5-yl)methylene]imidazol-4-one,
(199) (5*Z*)-2-[[(1*R*)-1-(Methoxymethyl)-3-methyl-butyl]amino]-3-methyl-5-[(1-methylindazol-5-yl)methylene]imidazol-4-one,
(200) (5*Z*)-2-[[(1*R*,2*R*)-2-Methoxycyclopentyl]amino]-3-methyl-5-[(1-methylindazol-5-yl)methylene]imidazol-4-one,
(201) (5*Z*)-2-[[(1*S*,2*S*)-2-Methoxycyclopentyl]amino]-3-methyl-5-[(1-methylindazol-5-yl)methylene]imidazol-4-one,
(202) (5*Z*)-2-(3-Noradamantylamino)-3-methyl-5-[(1-methylindazol-5-yl)methylene]imidazol-4-one,
(203) (5*Z*)-2-(1-Adamantylamino)-3-methyl-5-[(1-methylindazol-5-yl)methylene]imidazol-4-one,
(204) (5*Z*)-2-[(3-Hydroxy-1-adamantyl)amino]-3-methyl-5-[(1-methylindazol-5-yl)methylene]imidazol-4-one,
(205) (5*Z*)-2-[(3-Methoxy-1-adamantyl)amino]-3-methyl-5-[(1-methylindazol-5-yl)methylene]imidazol-4-one,
(206) (5*Z*)-2-[(3-Fluoro-1-adamantyl)amino]-3-methyl-5-[(1-methylindazol-5-yl)methylene]imidazol-4-one,
(207) (5*Z*)-3-Methyl-5-[(1-methylindazol-5-yl)methylene]-2-[[2-(trifluoromethyl)phenyl]methylamino]imidazol-4-one,
(208) (5*Z*)-2-[[(1*S*,2*S*)-2-Hydroxyindan-1-yl]amino]-3-methyl-5-[(1-methylindazol-5-yl)methylene]imidazol-4-one,
(209) (5*Z*)-2-[[(1*R*)-2-Hydroxy-1-phenyl-ethyl]amino]-3-methyl-5-[(1-methylindazol-5-yl)methylene]imidazol-4-one,
(210) (5*Z*)-2-[[(1*R*)-2-Hydroxy-1-phenyl-ethyl]amino]-3-methyl-5-[(1-methylindazol-5-yl)methylene]imidazol-4-one,
(211) (5*Z*)-2-[[(1*R*)-2-Methoxy-1-phenyl-ethyl]amino]-3-methyl-5-[(1-methylindazol-5-yl)methylene]imidazol-4-one,
(212) (5*Z*)-2-[[(2*R*)-2-Hydroxy-2-phenyl-ethyl]amino]-3-methyl-5-[(1-methylindazol-5-yl)methylene]imidazol-4-one,
(213) (5*Z*)-2-[[(1*R*)-2-Amino-1-phenyl-ethyl]amino]-3-methyl-5-[(1-methylindazol-5-yl)methylene]imidazol-4-one dihydrochloride,
(214) (5*Z*)-3-Methyl-5-[(1-methylindazol-5-yl)methylene]-2-[(4-methylthiazol-2-yl)methylamino]imidazol-4-one,
(215) (5*Z*)-3-Methyl-5-[(1-methylindazol-5-yl)methylene]-2-(tetrahydropyran-4-ylmethylamino)imidazol-4-one,
(216) (5*Z*)-3-Methyl-5-[(1-methylindazol-5-yl)methylene]-2-[4-(4-methylpiperazin-1-yl)anilino]imidazol-4-one,
(217) (5*Z*)-3-Methyl-5-[(1-methylindazol-5-yl)methylene]-2-(2-pyridylamino)imidazol-4-one,
(218) (5*Z*)-3-Methyl-5-[(1-methylindazol-5-yl)methylene]-2-[[(3*S*)-tetrahydropyran-3-yl]amino]imidazol-4-one,
(219) (5*Z*)-2-[[(3*R*,4*R*)-4-Hydroxytetrahydropyran-3-yl]amino]-3-methyl-5-[(1-methylindazol-5-yl)methylene]imidazol-4-one,
(220) (5*Z*)-5-(1*H*-Benzimidazol-5-ylmethylene)-2-(cyclohexylamino)-3-methyl-imidazol-4-one,
(221) (5*Z*)-5-(1*H*-Benzimidazol-5-ylmethylene)-2-(cycloheptylamino)-3-methyl-imidazol-4-one,
(222) (5*Z*)-5-(1*H*-Benzimidazol-5-ylmethylene)-2-(cyclooctylamino)-3-methyl-imidazol-4-one,
(223) (5*Z*)-5-(1*H*-Benzimidazol-5-ylmethylene)-2-[[(1*R*)-1-(hydroxymethyl)-3-methyl-butyl]amino]-3-methyl-imidazol-4-one,
(224) (5*Z*)-5-(1*H*-Benzimidazol-5-ylmethylene)-2-[[(1*R*)-1-(methoxymethyl)-3-methyl-butyl] amino]-3-methyl-imidazol-4-one,
(225) (5*Z*)-5-(1*H*-Benzimidazol-5-ylmethylene)-2-[[(1*R*,2*R*)-2-methoxycyclopentyl]amino]-3-methyl-imidazol-4-one,
(226) (5*Z*)-5-(1*H*-Benzimidazol-5-ylmethylene)-2-[[(1*S*,2*S*)-2-methoxycyclopentyl]amino]-3-methyl-imidazol-4-one,
(227) (5*Z*)-2-(3-Nordamantylamino)-5-(1*H*-benzimidazol-5-ylmethylene)-3-methyl-imidazol-4-one,
(228) (5*Z*)-2-(1-Adamantylamino)-5-(1*H*-benzimidazol-5-ylmethylene)-3-methyl-imidazol-4-one,
(229) (5*Z*)-5-(1*H*-Benzimidazol-5-ylmethylene)-2-[(3-hydroxy-1-adamantyl)amino]-3-methyl-imidazol-4-one,
(230) (5*Z*)-5-(1*H*-Benzimidazol-5-ylmethylene)-2-[(3-methoxy-1-adamantyl)amino]-3-methyl-imidazol-4-one,
(231) (5*Z*)-5-(1*H*-Benzimidazol-5-ylmethylene)-2-[(3-fluoro-1-adamantyl)amino]-3-methyl-imidazol-4-one,
(232) (5*Z*)-5-(1*H*-Benzimidazol-5-ylmethylene)-3-methyl-2-[[2-(trifluoromethyl)phenyl]methylamino]imidazol-4-one,
(233) (5*Z*)-5-(1*H*-Benzimidazol-5-ylmethylene)-2-[[(1*S*,2*S*)-2-hydroxyindan-1-yl]amino]-3-methyl-imidazol-4-one,
(234) (5*Z*)-5-(1*H*-Benzimidazol-5-ylmethylene)-2-[[(1*R*)-2-hydroxy-1-phenyl-ethyl]amino]-3-methyl-imidazol-4-one,
(235) (5*Z*)-5-(1*H*-Benzimidazol-5-ylmethylene)-2-[[(1*S*)-2-hydroxy-1-phenyl-ethyl]amino]-3-methyl-imidazol-4-one,
(236) (5*Z*)-5-(1*H*-Benzimidazol-5-ylmethylene)-2-[[(1*R*)-2-methoxy-1-phenyl-ethyl]amino]-3-methyl-imidazol-4-one,
(237) (5*Z*)-5-(1*H*-Benzimidazol-5-ylmethylene)-2-[[(2*R*)-2-hydroxy-2-phenyl-ethyl]amino]-3-methyl-imidazol-4-one,
(238) (5*Z*)-5-(1*H*-Benzimidazol-5-ylmethylene)-3-methyl-2-[(4-methylthiazol-2-yl)methylamino]imidazol-4-one,
(239) (5*Z*)-5-(1*H*-Benzimidazol-5-ylmethylene)-3-methyl-2-(tetrahydropyran-4-ylmethylamino)imidazol-4-one,
(240) (5*Z*)-5-(1*H*-Benzimidazol-5-ylmethylene)-3-methyl-2-[4-(4-methylpiperazin-1-yl)anilino]imidazol-4-one,
(241) (5*Z*)-5-(1*H*-Benzimidazol-5-ylmethylene)-3-methyl-2-(2-pyridylamino)imidazol-4-one,
(242) (5*Z*)-5-(1*H*-Benzimidazol-5-ylmethylene)-3-methyl-2-[[(3*S*)-tetrahydropyran-3-yl]amino]imidazol-4-one,
(243) (5*Z*)-5-(1*H*-Benzimidazol-5-ylmethylene)-2-[[(3*R*,4*R*)-4-hydroxytetrahydropyran-3-yl]amino]-3-methyl-imidazol-4-one,
(244) (5*Z*)-2-(Cyclohexylamino)-3-methyl-5-[(3-methylbenzimidazol-5-yl)methylene]imidazol-4-one,
(245) (5*Z*)-2-(Cycloheptylamino)-3-methyl-5-[(3-methylbenzimidazol-5-yl)methylene]imidazol-4-one,
(246) (5*Z*)-2-(Cyclooctylamino)-3-methyl-5-[(3-methylbenzimidazol-5-yl)methylene]imidazol-4-one,
(247) (5*Z*)-2-[[(1*R*)-1-(Hydroxymethyl)-3-methyl-butyl]amino]-3-methyl-5-[(3-methylbenzimidazol-5-yl)methylene]imidazol-4-one,
(248) (5*Z*)-2-[[(1*R*)-1-(Methoxymethyl)-3-methyl-butyl]amino]-3-methyl-5-[(3-methylbenzimidazol-5-yl)methylene]imidazol-4-one,
(249) (5*Z*)-2-[[(1*R*,2*R*)-2-Methoxycyclopentyl]amino]-3-methyl-5-[(3-methylbenzimidazol-5-yl)methylene]imidazol-4-one,
(250) (5*Z*)-2-[[(1*S*,2*S*)-2-Methoxycyclopentyl]amino]-3-methyl-5-[(3-methylbenzimidazol-5-yl)methylene]imidazol-4-one,
(251) (5Z)-2-(3-Noradamantylamino)-3-methyl-5-[(3-methylbenzimidazol-5-yl)methylene]imidazol-4-one
(252) (5*Z*)-2-(1-Adamantylamino)-3-methyl-5-[(3-methylbenzimidazol-5-yl)methylene]imidazol-4-one,
(253) (5*Z*)-2-[(3-Hydroxy-1-adamantyl)amino]-3-methyl-5-[(3-methylbenzimidazol-5-yl)methylene]imidazol-4-one,
(254) (5*Z*)-2-[(3-Methoxy-1-adamantyl)amino]-3-methyl-5-[(3-methylbenzimidazol-5-yl)methylene]imidazol-4-one,
(255) (5*Z*)-2-[(3-Fluoro-1-adamantyl)amino]-3-methyl-5-[(3-methylbenzimidazol-5-yl)methylene]imidazol-4-one,
(256) (5*Z*)-3-Methyl-5-[(3-methylbenzimidazol-5-yl)methylene]-2-[[2-(trifluoromethyl)phenyl]methylamino]imidazol-4-one,
(257) (5*Z*)-2-[[(1*R*)-2-Hydroxy-1-phenyl-ethyl]amino]-3-methyl-5-[(3-methylbenzimidazol-5-yl)methylene]imidazol-4-one,
(258) (5*Z*)-2-[[(1*S*)-2-Hydroxy-1-phenyl-ethyl]amino]-3-methyl-5-[(3-methylbenzimidazol-5-yl)methylene]imidazol-4-one,
(259) (5*Z*)-2-[[(1*R*)-2-Methoxy-1-phenyl-ethyl]amino]-3-methyl-5-[(3-methylbenzimidazol-5-yl)methylene]imidazol-4-one,
(260) (5*Z*)-2-[[(2*R*)-2-Hydroxy-2-phenyl-ethyl]amino]-3-methyl-5-[(3-methylbenzimidazol-5-yl)methylene]imidazol-4-one,
(261) (5*Z*)-3-Methyl-5-[(3-methylbenzimidazol-5-yl)methylene]-2-[(4-methylthiazol-2-yl)methylamino]imidazol-4-one,
(262) (5*Z*)-3-Methyl-5-[(3-methylbenzimidazol-5-yl)methylene]-2-(tetrahydropyran-4-ylmethylamino)imidazol-4-one,
(263) (5*Z*)-3-Methyl-5-[(3-methylbenzimidazol-5-yl)methylene]-2-[4-(4-methylpiperazin-1-yl)anilino]imidazol-4-one,
(264) (5*Z*)-3-Methyl-5-[(3-methylbenzimidazol-5-yl)methylene]-2-(2-pyridylamino)imidazol-4-one,
(265) (5*Z*)-3-Methyl-5-[(3-methylbenzimidazol-5-yl)methylene]-2-[[(3*S*)-tetrahydropyran-3-yl]amino]imidazol-4-one,
(266) (5*Z*)-2-[[(3*R*,4*R*)-4-Hydroxytetrahydropyran-3-yl]amino]-3-methyl-5-[(3-methylbenzimidazol-5-yl)methylene]imidazol-4-one,
and their pharmaceutically acceptable salts.

According to an even more preferred embodiment of the present invention, the compound of formula (I) is chosen from the group consisting of compounds (1), (2), (3), (4), (5), (6), (7), (8), (9), (10), (11), (12), (13), (14), (15), (16), (17), (18), (19), (21), (22), (23), (24), (25), (27), (28), (29), (30), (31), (32), (35), (36), (37), (38), (39), (40), (41), (44), (45), (46), (47), (48), (51), (52), (53), (55), (56), (57), (58), (60), (69), (72), (76), (77), (78), (79), (80), (83), (84), (85), (86), (87), (88), (89), (90), (96), (99), (100), (101), (105), (106), (107), (108), (109), (110), (112), (113), (114), (115), (116), (117), (118), (120), (121), (130), (132), (133), (134), (135), (136), (137), (138), (139), (140), (141), (142), (143), (152), (154), (156), (166), (172), (173), (174), (177), (178), (179), (180), (181), (183), (196), (198), (223), (225), (227), (228), (229), (230), (231), (247), (251), (252), (253), (255), (263) and their pharmaceutically acceptable salts.

According to an even more preferred embodiment of the present invention, the compound of formula (I) is chosen from the group consisting of compounds (1), (2), (3), (4), (5), (6), (7), (8), (9), (10), (11), (12), (13), (14), (15), (16), (17), (18), (19), (21), (22), (23), (24), (25), (27), (28), (29), (31), (32), (36), (37), (38), (39), (40), (41), (44), (45), (46), (48), (51), (56), (57), (58), (72), (78), (80), (83), (84), (85), (86), (87), (88), (89), (90), (108), (110), (114), (115), (116), (117), (118), (120), (130), (133), (134), (135), (137), (138), (139), (140), (141), (142), (156), (173), (177), (178), (179), (180), (181), (227), (228), (229), (231), (247), (251), (252), (253), (255) and their pharmaceutically acceptable salts.
According to an even more preferred embodiment of the present invention, the compound of formula (I) is chosen from the group consisting of compounds (3), (4), (5), (6), (7), (10), (11), (12), (13), (15), (16), (18), (21), (32), (39), (40), (41), (46), (58), (83), (84), (85), (86), (87), (114), (115), (116), (118), (137), (138), (139), (140), (141), (181), (231) and their pharmaceutically acceptable salts.

According to an alternative embodiment of the present invention, the compound of formula (I) is chosen from the group consisting of compounds (1), (2), (3), (4), (5), (6), (7), (8), (9), (10), (11), (12), (13), (14), (15), (16), (17), (18), (19), (20), (21), (22), (23), (24), (25), (28), (29), (31), (32), (35), (36), (37), (39), (40), (41), (42), (46), (58), (60), (75), (76), (77), (78), (80), (81), (83), (84), (85), (86), (87), (88), (89), (90), (91), (92), (93), (94), (95), (96), (97), (99), (100), (101), (102), (104), (105), (106), (107), (108), (109), (110), (111), (112), (113), (114), (115), (116), (117), (118), (119), (120), (121), (122), (123), (124), (125), (126), (127), (128), (129), (130), (131), (132), (133), (134), (135), (136), (137), (138), (139), (140), (141), (142), (143), (144), (147), (148), (150), (151), (152), (153), (154), (155), (156), (158), (172), (173), (177), (178), (179), (180), (181), (183), (184), (208), (220), (221), (222), (223), (225), (226), (227), (228), (229), (230), (231), (232), (233), (234), (235), (236), (237), (238), (239), (240), (241), (242), (243), (244), (245), (246), (247), (248), (249), (250), (251), (252), (253), (254), (255), (256), (257), (259), (260), (261), (262), (263), (264), (265), (266) and their pharmaceutically acceptable salts.

According to a preferred embodiment of the present invention, the compound of formula (I) is chosen from the group consisting of compounds (1), (2), (3), (4), (5), (6), (7), (8), (9), (10), (11), (12), (13), (14), (15), (16), (17), (18), (19), (20), (21), (22), (23), (24), (25), (28), (29), (31), (32), (36), (39), (40), (41), (46), (76), (77), (78), (80), (83), (84), (85), (86), (87), (88), (89), (90), (92), (93), (95), (96), (97), (99), (100), (101), (104), (105), (106), (107), (108), (109), (110), (111), (112), (113), (114), (115), (116), (117), (118), (119), (120), (121), (123), (124), (125), (126), (127), (128), (129), (130), (131), (132), (133), (134), (135), (136), (137), (138), (139), (140), (141), (142), (143), (144), (152), (153), (154), (155), (156), (158), (177), (179), (180), (181), (183), (184), (208), (221), (222), (223), (226), (227), (229), (230), (231), (232), (233), (234), (237), (242), (244), (245), (246), (247), (248), (249), (250), (251), (252), (253), (254), (255), (256), (257), (260), (262), (263), (265), (266) and their pharmaceutically acceptable salts.

According to an even more preferred embodiment of the present invention, the compound of formula (I) is chosen from the group consisting of compounds (7), (11), (12), (13), (15), (16), (31), (39), (40), (41), (83), (84), (85), (86), (87), (90), (107), (108), (114), (115), (116), (118), (136), (137), (139), (140), (156), (229), (230), (231), (245), (246), (247), (251), (253), (255) and their pharmaceutically acceptable salts.

According to a further embodiment of the present invention, the compound of formula (I) is chosen from the group consisting of compounds (3), (4), (5), (6), (7), (8), (9), (10), (11), (12), (13), (14), (15), (16), (18), (19), (21), (22), (23), (24), (25), (31), (39), (40), (41), (76), (77), (78), (80), (83), (84), (85), (86), (87), (88), (89), (90), (92), (95), (100), (101), (104), (105), (106), (107), (108), (110), (111), (112), (113), (114), (115), (116), (117), (118), (119), (120), (121), (124), (128), (129), (130), (131), (137), (139), (140), (141), (142), (156), (227), (229), (230), (231), (246), (247), (251), (252), (253), (254), (255), (257), (260) and their pharmaceutically acceptable salts.

The groups of compounds as defined by the list of compounds as identified in Example 4 below through tables 4A to table 4F and specifically identified as most potent kinase inhibitors, as well as multi-target kinase inhibitors, also form part of the present invention.

According to another aspect, a subject-matter of the present invention relates to a compound of formula (I) as defined above or any of its pharmaceutically acceptable salts, or at least any of compounds (1) to (266) or any of its pharmaceutically acceptable salts, for use as a medicament.

"Pharmaceutically acceptable salt thereof" refers to salts which are formed from acid addition salts formed with inorganic acids (e.g. hydrochloric acid, hydrobromic acid,), as well as salts formed with organic acids such as acetic acid, tartaric acid, succinic acid.

Suitable physiologically acceptable acid addition salts of compounds of formula (I) include hydrobromide, tartrate, hydrochloride, succinate and acetate.

The compounds of formula (I), and any of compounds (1) to (266) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, i.e. combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention. In particular, stereoisomers (Z) and (E) form part of the invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

### List of abbreviations:

| **Abbreviation/acronym** | **name** |
|---|---|
| **Ac** | Acetyl |
| **ACN** | Acetonitrile |
| **Alk** | Alkyl |
| **APTS** | *Para*-toluenesulfonic acid |
| **ATP** | Adenosine triphosphate |
| **br s** | Broad singlet |
| **c** | Molar concentration |
| **Cpd N°** | Compound number |
| **d** | Doublet |
| **DCM** | Methylene chloride |
| **dd** | Doublet of doublets |
| **DIPEA** | *N*,*N-*Diisopropylethylamine |
| **DMF** | Dimethylformamide |
| **DMSO** | Dimethylsulfoxide |
| **DTT** | Dithiothreitol |
| **Eq** | Equivalent |
| **ESI** | Electrospray ionization |
| **Et** | Ethyl |
| **FC** | Flash chromatography |
| **GP** | General protocol |
| **GST** | Glutathione S-transferase |
| **Hal** | Halogen |
| **HEPES** | 4-(2-Hydroxyethyl)-1-piperazineethanesulfonic acid |
| **His-tagged** | Polyhistidine-tagged |
| **HPLC** | High pressure liquid chromatography |
| **ICso** | Half maximal inhibitory concentration |
| **m** | Multiplet |
| **M** | Molarity |
| **Me** | Methyl |
| **MS** | Mass spectroscopy |
| **MW** | Molecular weight |
| **NMR** | Nuclear magnetic resonance |
| **N/A** | Not applicable |
| **n.t.** | Not tested |
| **Rac** | Racemic |
| **r.t.** | Room temperature |
| **s** | Singlet |
| **SDS-PAGE** | Sodium dodecyl sulfate-polyacrylamide gel electrophoresis |
| **t** | Triplet |
| **THF** | Tetrahydrofuran |
| **TLC** | Thin layer chromatography |
| **T °C** | Temperature in degrees Celsius |
| **PTLC** | Preparative thin layer chromatography |
| **UV** | Ultraviolet |
| **v/v** | Volume per volume |
| **w/v** | Weight per volume |
| **δ_{H}** | Hydrogen chemical shift |
| **µw** | Microwave irradiation |

The compounds of general formula (I) can be prepared according to schemes 1 to 2' below.

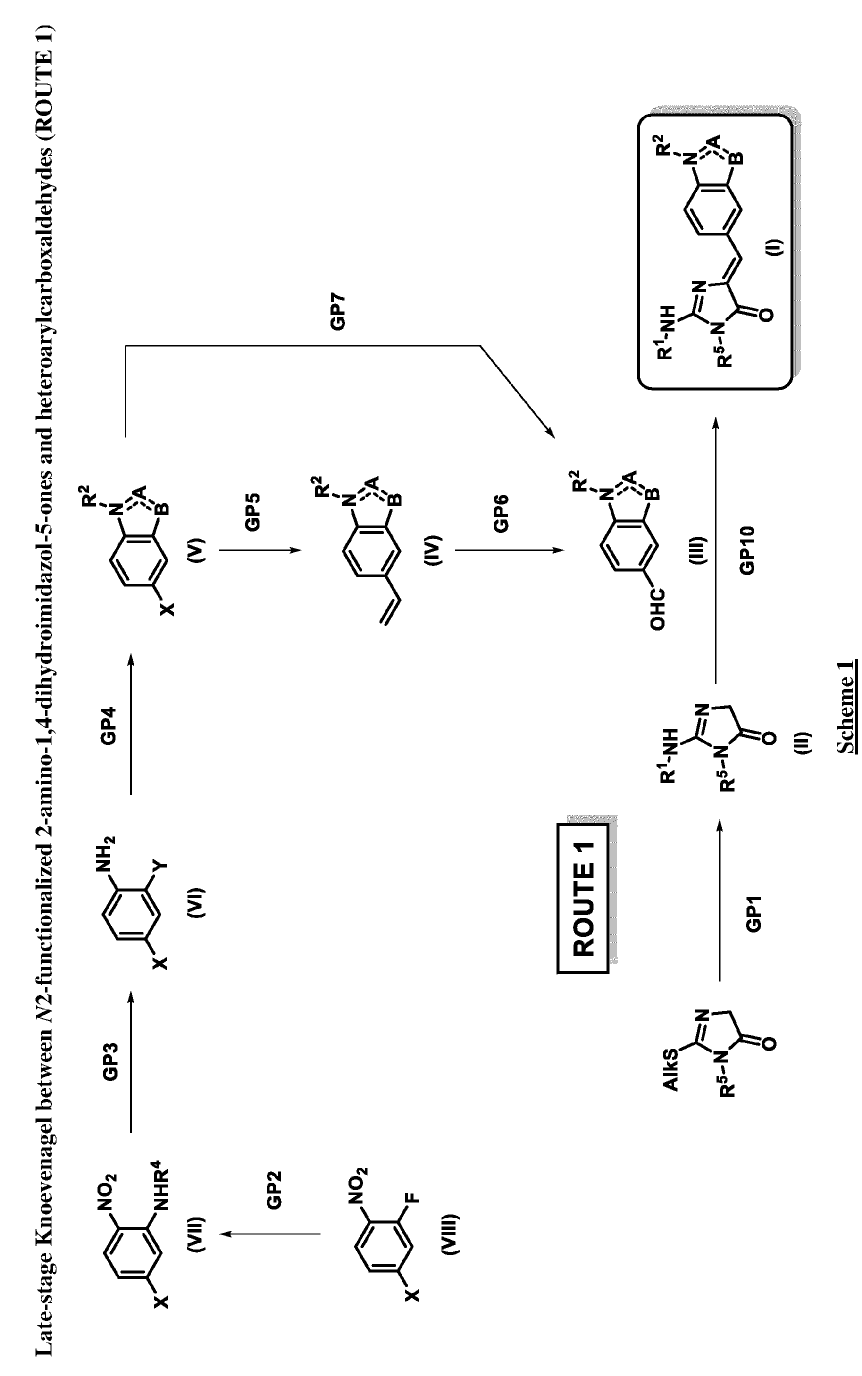

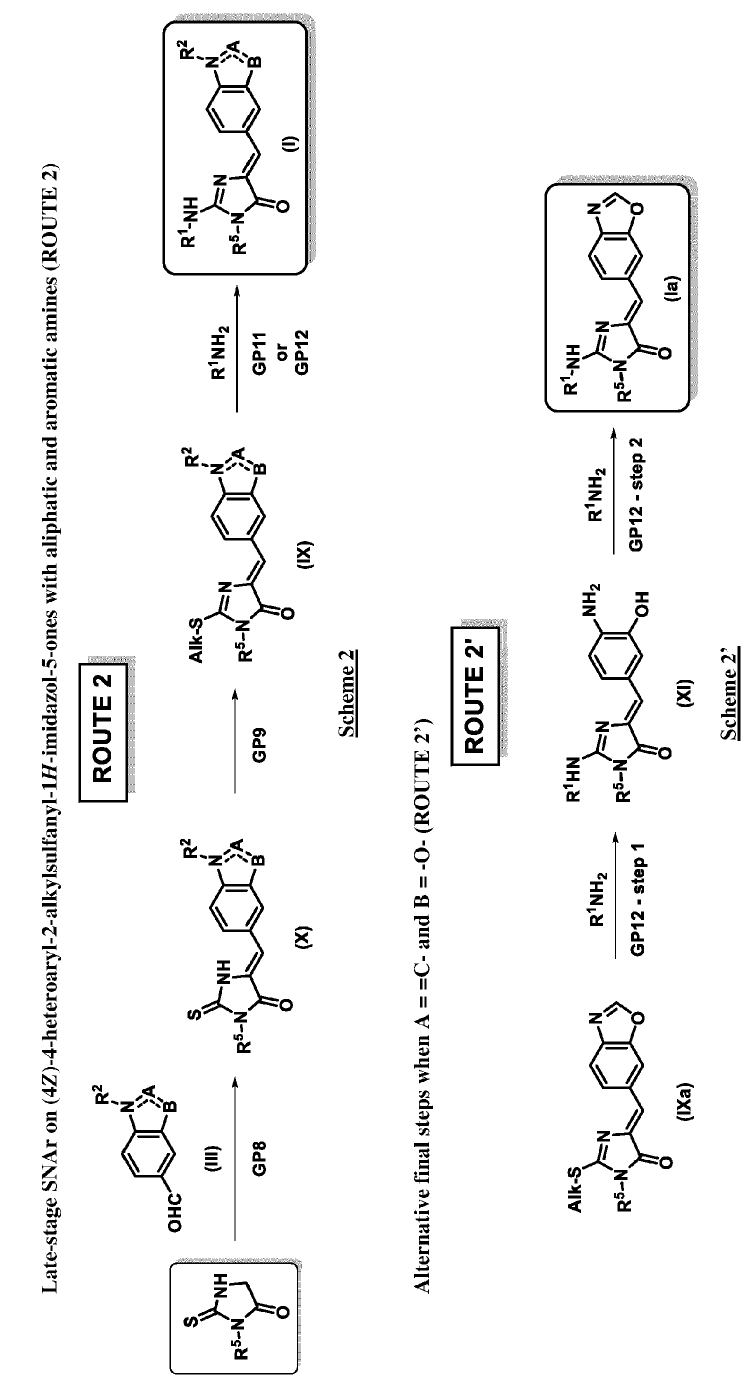

### ROUTE 1

The synthesis is based on a late-stage Knoevenagel reaction between a *N2-*substituted-1,4-dihydroimidazol-5-one of formula (II) wherein R¹ and R⁵ are as defined above and a heteroarylcarboxaldehyde of formula (III) wherein R², A and B are as defined above, following the general protocol 10 (GP10), described herein after.

According to GP10, the compounds (II) and (III) are placed in a protic solvent such as ethanol. Compounds (II) and (III) may be introduced for example in a molar ratio of compound (III) on compound (II) ranging from 1 to 5, for example of 1.2, in presence of an ammonium carboxylate such as ammonium formate, for example in a molar ratio with respect to compound (II) ranging from 1 to 5, for example of 1.2. The mixture may be irradiated, for example by microwaves, for example for a duration ranging from 1 to 5 hours, in particular 3 hours. The mixture may also be heated for example at a temperature ranging from 100 °C to 140 °C, for example from 110 °C to 130 °C and brought back to room temperature upon completion of the reaction.

The compound of formula (II) as defined above comprises an aliphatic or aromatic amine, it may be obtained by the addition of an amine of formula R¹NH₂ wherein R¹ is as defined above on a 2-alkylsulfanyl-1,4-dihydroimidazol-5-one, following to the general protocol 1 (GP1) described herein after.

According to GP1, the 2-alkylsulfanyl-1,4-dihydroimidazol-5-one may be placed in an aprotic solvent such as tetrahydrofuran (THF). An amine of formula R¹NH₂ may then be added, for example in a molar ratio ranging from 1.2 to 6, with respect to the 2-alkylsulfanyl-1,4-dihydroimidazol-5-one. Acetic acid may be added to the reaction mixture, in a molar ratio ranging from 2 to 5. The reaction mixture may be placed in a sealed tube, stirred and heated to a temperature ranging from 90 °C to 150 °C, for example of 120 °C. Upon completion of the reaction, the mixture may be brought back to room temperature and a solid may crystallize. The mixture may then be stirred at a temperature ranging from -10 °C to 10 °C, for example 0 °C, for a duration ranging from 30 to 90 minutes, for example of 60 minutes.

### ROUTE 2

The synthesis is based on a late-stage functionalization of a compound of formula (IX) wherein Alk, R², R⁵, A and B are as defined above by an amine of formula R¹NH₂ wherein R¹ is as defined above, following the general protocol 11 or 12 (GP11 and GP12), described herein after.

According to GP11, the compound of formula (IX) may be placed in an aprotic solvent such as THF or dioxane, or a mixture of both. The amine of formula R¹NH₂ may be added, for example in a molar ratio ranging from 2 to 6, in particular of 4, with respect to the compound of formula (IX). Acetic acid may be added to the reaction mixture, in a molar ratio with respect to the compound of formula (IX) ranging for example from 4 to 8. The reaction mixture may be placed in a sealed tube and may receive energy, for example from a heating block or from microwaves. Upon completion of the reaction, the mixture may be brought back to room temperature.

In an embodiment named GP11-A, the reaction mixture may be stirred at a temperature ranging from -10 °C to 10°C, for example at 0 °C, for a duration ranging from 30 minutes to 2 hours, for example for 1 hour.

Depending on the state of the product obtained (solid, precipitate), purification methods well-known by the person skilled in the art may be performed, for example filtering, washing, triturating, drying *in vacuo*, flash chromatography, precipitating and refluxing.

According to GP12, a compound of formula (IXa) wherein A is a =C- group, B is a -O- group and Alk and R⁵ are as defined above, may be placed in an aprotic solvent such as tetrahydrofuran (THF) together with the amine of formula R¹NH₂ wherein R¹ is as described above in a molar ratio of the amine to the compound of formula (IXa) ranging for example from 4 to 8, in particular of 6. The reaction mixture may be heated, for example in a sealed tube, at a temperature ranging for example from 90 °C to 130 °C, in particular of 110 °C. Upon completion, the reaction may be brought back to a temperature ranging for example from 15 °C to 30 °C, in particular at room temperature and isolated to yield a compound of formula (XI) wherein R¹ and R⁵ are as defined above.

In a second step of the GP12, the compound of formula (XI) may be placed in a solvent such as toluene together with triethyl orthoformate in a molar ratio of triethyl orthoformate to the compound of formula (XI) ranging for example ranging from 15 to 35, in particular of 25. The reaction mixture may be heated, for example in a sealed tube, at a temperature ranging for example from 100 °C to 200 °C, in particular at 150 °C, for a duration ranging for example from 30 to 90 minutes, in particular of 60 minutes.

A compound of formula (IX) as defined above may be obtained by the *S-*alkylation of a compound of formula (X) wherein R², R⁵, A and B are as defined above, following the general protocol 9 (GP9).

According to GP9, a compound of formula (X) may be placed in a polar aprotic solvent such as dimethylformamide (DMF). An alkylhalide of formula Alk-Hal wherein Hal is an halide such as iodide or bromide and Alk is a (C₁-C₅)alkyl such as methyl or ethyl may then be added dropwise, for example in a molar ratio ranging from 0.75 to 1.50, in particular of 1.05, with respect to the compound of formula (X), in presence of an inorganic base such as K2CO3, for example in a molar ratio ranging from 0.7 to 1.5, in particular of 1, still with respect to the compound of formula (X). The reaction mixture may be stirred during the addition of the alkylhalide. The resulting mixture may then be stirred, for example from 8 to 16 hours, in particular for 12 hours at a temperature ranging from -10 °C to 30 °C, in particular at 0 °C when Alk is a methyl or at room temperature when Alk is an ethyl.

A compound of formula (X) as defined above may be obtained from a compound of formula (III) wherein R², A and B are as defined above, following the general protocol 8 (GP8).

According to GP8, the compound of formula (III) may be placed in a protic solvent such as ethanol in presence of 2-thiohydantoin, for example in a molar ratio ranging from 0.85 to 1.15, in particular of 1, with respect to the compound of formula (III), in presence of an organic base such as piperidine or ethanolamine for example in a molar ratio ranging from 0.85 to 1.15, in particular of 1, still with respect to the compound of formula (III), in presence of an organic acid such as acetic acid for example in a molar ratio ranging from 0.85 to 1.15, in particular of 1, still with respect to the compound of formula (III). The reaction mixture may be placed in a sealed tube, stirred and heated at a temperature ranging for example from 60 °C to 130 °C, in particular from 80 °C to a duration ranging from 10 to 100 minutes, in particular from 15 to 90 minutes. The reaction mixture may be irradiated, for example by microwaves.

In both routes of synthesis described herein above, a compound of formula (III) as defined above, or heteroarylcarboxaldehyde, may be implemented.

The compound of formula (III) may be obtained by a vinylation of a heteroarylbromide of formula (V) wherein R², R³, R⁴, A and B are as defined above and X is a halogen atom, following the general protocol 5 (GP5) followed by at least a step of a Lemieux-Johnson oxidation, according to the general protocol 6 (GP6).

According to one embodiment, GP5 implements a palladium-catalyzed vinylation of a heteroarylbromide. The compound of formula (V) may be placed in a mixture together with potassium vinyltrifluoroborate, in presence of an inorganic base such as Cs₂CO₃ and of a source of Pd[0], in a solvent mixture containing at least a solvent able to stabilize cations, such as dioxane. The protocol GP5 encompasses two alternatives.

According to a first alternative of GP5 named GP5-A, the palladium source is a tetrakis(triphenylphosphine)palladium(0) (Pd(Ph3)4). In this embodiment, the compound of formula (V) may be placed in a mixture together with potassium vinyltrifluoroborate in a molar ratio with respect to the compound of formula (V) ranging for example from 1 to 1.4, in particular of 1.2, in presence of an inorganic base such as Cs₂CO₃, for example in a molar ratio ranging from 1 to 5, in particular 2, still with respect to the compound of formula (V), in the presence of Pd(PPh3)4 for example in an amount ranging from 2 to 10 mol%, in particular of 5 mol% relative to the total amount of compound of formula (V) and in a solvent mixture containing at least a solvent able to stabilize cations, such as dioxane. The mixture may be brought to reflux and heated for example for 6 to 18 hours, in particular 12 hours.

According to a second alternative of GP5 named GP5-B, the palladium source is a pyridine-enhanced precatalyst preparation stabilization and initation (PEPPSI) such as PEPPSI-iPr. The reaction mixture may contain the compound of formula (V), vinyltrifluoroborate in a molar ratio with respect to the compound of formula (V) ranging from 1.5 to 2.5, for example of 2, in presence of an inorganic base such as Cs₂CO₃, for example in a molar ratio ranging from 1 to 5, for example 3, still with respect to the compound of formula (V), in the presence of a PEPPSI catalyst such as PEPPSI-iPr in an amount ranging from 10 to 20 mol%, for example of 15 mol% relative to the total amount of compound of formula (V) and in a solvent mixture containing at least a solvent able to stabilize cations, such as dioxane. The reaction mixture may be irradiated, for example by microwaves, for example for a duration comprised between 2 and 6 hours, in particular 4 hours, and heated at a temperature ranging from 120 °C to 170 °C, for example 150 °C. Upon completion of the reaction, the mixture may be cooled down to room temperature and optionally purified.

According to the general protocol GP6, the vinylheteroaryl (IV) wherein R², A and B are as defined above, obtained from GP5 may be placed in a solvent mixture containing at least a solvent able to stabilize cations, such as dioxane. An oxidising agent such as periodate may then be added, for example in a molar ratio ranging from 2 to 6, in particular 4, with respect to the vinylheteroaryl (IV) obtained from GP5 in presence of an osmium compound such as osmium tetroxide (OsO₄), for example in an amount ranging from 1 to 10 mol%, in particular 5 mol%, relative to the total amount of vinylheteroaryl (IV) obtained from protocol GP5, in the presence of a non-nucleophilic base such as 2,6-lutidine, for example in a molar ratio ranging from 2 to 6, in particular 4, still with respect to the vinylheteroaryl (IV) obtained from protocol GP5. The reaction mixture may be stirred and maintained at a temperature for example ranging from -5 °C to 5 °C, in particular 0 °C, for example for a duration ranging from 30 minutes to 2 hours.

Alternatively, the heteroarylcarboxaldehyde of formula (III) as defined above may be obtained from the heteroarylcarbonitrile of formula (V) wherein R², A and B are as defined above and X is a cyanide group, following the general protocol 7 (GP7), described herein after.

According to GP7, the compound (V) may be placed in a solvent mixture containing at least a protic solvent such as formic acid. Adam's catalyst (platinum dioxide) may be added to the mixture, for example in a quantity ranging from 15 mol% to 30 mol%, in particular 23 mol%. The reaction mixture may be refluxed, for example for 24 hours to 72 hours, in particular for 48 hours. Upon completion of the reaction, the compound (III) may be isolated and purified.

A compound of formula (V) as defined above may be obtained by a cyclisation of a 2-amino-bromo-phenol or of a compound of formula (VI) wherein, X is chosen from the group consisting of a halogen atom and a cyanide group, Y is selected from the group consisting of a hydroxy group and a -NHR⁴ group wherein R⁴ is as defined above, following the general protocol 4 (GP4).

According to a first alternative of GP4 named GP4-A, a commercially available 2-amino-bromo-phenol wherein Y is a hydroxy group and X is a halogen atom, in particular a bromine atom, may be placed in solution with a solvent such as toluene and with triethyl orthoformate in a molar ratio of triethyl orthoformate to the 2-amino-bromo-phenol ranging for example ranging from 1.5 to 3, in particular of 2. The reaction mixture may be irradiated, for example at a temperature ranging from 100 °C to 200 °C, in particular at 130 °C, and for example for a duration ranging from 1 to 3 hours, in particular for 2 hours. Upon completion of the reaction, the mixture may be cooled down to room temperature.

According to a second alternative of GP4 named GP4-B, a compound of formula (VI) wherein X is a halogen atom, in particular a bromine atom, Y is a -NHR⁴ group and R⁴ is as defined above, may be placed in a solution with a solvent such as toluene and with triethyl orthoformate in a molar ratio of triethyl orthoformate to the compound of formula (VI) for example ranging from 1.5 to 3, in particular of 2. The solution may also contain para-toluenesulfonic acid in a content of, for example 1 mol% to 10 mol%, in particular of 5 mol%. The reaction mixture may be irradiated, for example at a temperature ranging from 100 °C to 200 °C, in particular at 130 °C, and for example for a duration ranging from 1 to 3 hours, in particular for 2 hours.

According to a third alternative of GP4 named GP4-C, a compound of formula (VI) wherein X is a cyanide group, Y is a -NHR⁴ group and R⁴ is as defined above, may be refluxed in solution in a protic solvent such as formic acid. The reaction may be carried out for a duration ranging from 2 to 6 hours, in particular for 4 hours. Upon completion of the reaction, the mixture may be cooled down to room temperature.

A compound of formula (VI) as defined above may be obtained from a compound of formula (VII) wherein R⁴ is as defined above and X is chosen from the group consisting of a halogen atom and a cyanide group, following the general protocol 3 (GP3).

According to a first alternative of GP3 named GP3-A, a compound of formula (VII) wherein R⁴ is as defined above and X is a halogen atom, in particular a bromine atom may be placed in a solution comprising at least an aprotic solvent such as tetrahydrofuran (THF) and a polar protic solvent such as methanol in a solvent ratio ranging, for example from 2/1 to 1/2, in particular of 1/1. The solution may also contain zinc dust in a molar ratio ranging for example from 5 to 15, in particular of 10. A hydrogen donor such as NH₄Cl may be added to the reaction mixture for a duration ranging for exemple from 15 to 60 minutes, in particular for 30 minutes. The reaction mixture may be maintained at a temperature ranging for example from -10 °C to 10 °C, in particular at 0 °C for a total duration ranging for example from 45 to 120 minutes, in particular of 90 minutes. Then, the reaction mixture may be stirred at a temperature ranging for example from 15 °C to 30 °C, in particular at room temperature, for a duration ranging from 3 to 12 hours, in particular of 6 hours.

According to a second alternative of GP3 named GP3-B, a compound of formula (VII) wherein R⁴ is as defined above and X is a cyanide groupe may be placed in a polar protic solvent such as methanol with ammonium formiate in a molar ratio of ammonium formiate to the compound of formula (VII) ranging for example from 5 to 15, in particular of 10. A hydrogenolysis catalyst such as palladium on carbon may be added to the reaction mixture in a content ranging for example from 5% to 15% by weight relative to the total weight of the compound of formula (VII), in particular of 10% by weight. The reaction mixture may be refluxed for a duration ranging from 3 to 12 hours, in particular of 6 hours.

A compound of formula (VII) as defined above may be obtained from a o-fluoro-nitro-benzene of formula (VIII) as described in the Scheme 1, wherein X is chosen from the group consisting of a halogen atom and a cyanide group, following the general protocol 2 (GP2).

The *o*-fluoro-nitro-benzene of formula (VIII) may be placed in a polar protic solvent such as ethanol and an amine of the formula R⁴NH₂ wherein R⁴ is as described herein above may be added to the reaction mixture in a molar ratio to the *o*-fluoro-nitro-benzene of formula (VIII) ranging for example from 1.5 to 5, in particular of 3. The reaction mixture may be maintained at a temperature ranging for example from -10 °C to 10°C, in particular of 0 °C for a duration ranging for example from 30 to 90 minutes. Then, the reaction mixture may be brought to a temperature ranging for example from 15 °C to 30 °C, in particular at room temperature for a duration ranging for example from 6 to 24 hours, in particular of 12 hours.

Accordingly, the present invention further relates to the synthesis process for manufacturing new compounds of formula (I) as defined above, comprising at least a step of coupling a compound of formula (III) with a compound of formula (II).

The present invention relates to a synthesis process for manufacturing a compound of formula (I) as defined above or any of its pharmaceutically acceptable salt or any of the compounds (1) to (266) as defined above or any of their pharmaceutically acceptable salts, comprising at least a step of coupling a compound of formula (II) below 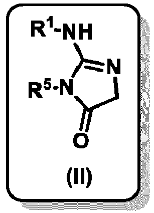
wherein R¹ and R⁵ are as defined above,
with a compound of formula (III) below 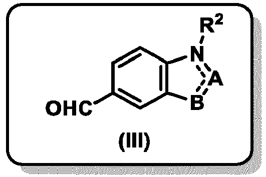
wherein R², A and B are as defined above.

The present invention further relates to a synthetic intermediate of formula (II) below 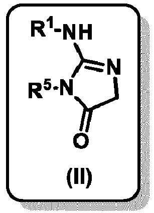 wherein R¹ and R⁵ are as defined above.

The present invention further relates to a synthetic intermediate of formula (III) below 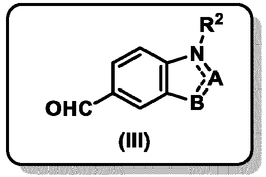 wherein R², A and B are as defined above.

Accordingly, the present invention further relates to the synthesis process for manufacturing new compounds of formula (I) as defined above, comprising at least a step of substituting a compound of formula (IX) with a primary amine.

The present invention relates to a synthesis process for manufacturing a compound of formula (I) as defined above or any of its pharmaceutically acceptable salt or any of the compounds (1) to (266) as defined above or any of its pharmaceutically acceptable salts, comprising at least a step of coupling a compound of formula (IX) below 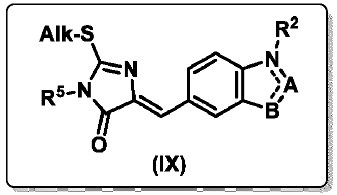
wherein R², R⁵, A and B are as defined above and Alk is a (C₁-C₅)alkyl,
with an amine of formula R¹NH₂ wherein R¹ is as defined above.

The present invention further relates to synthetic intermediates of formulae (IX) and (XI) below 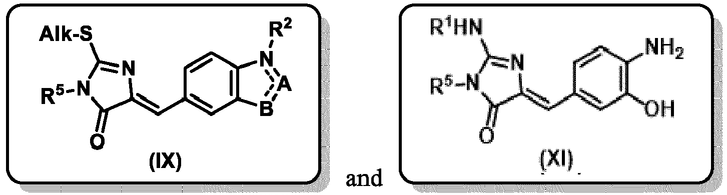 wherein R¹, R², R⁵, A and B are as defined above and Alk is a (C₁-C₅)alkyl, in particular Alk is selected from the group consisting of an ethyl and a methyl.

The present invention further relates to a synthetic intermediate selected from the compounds below 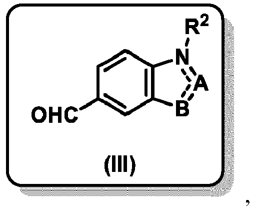 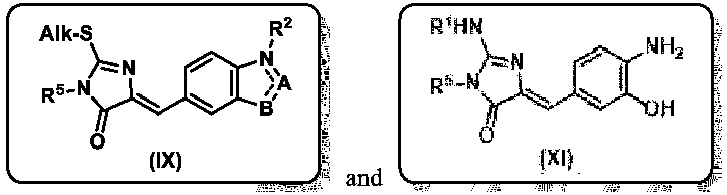 wherein Alk is a (C₁-C₅)alkyl, in particular Alk is selected from the group consisting of an ethyl and a methyl, and wherein R¹, R², R⁵, A and B are as defined above.

The chemical structures, the analytical and spectroscopic data of some compounds of formula (I) of the invention are illustrated respectively in the following Table 2 and Table 3.

Reactions were performed using oven-dried glassware under inert atmosphere of argon. Unless otherwise noted, all reagent-grade chemicals and solvents were obtained from commercial suppliers and were used as received. Reactions were monitored by thin-layer chromatography with silica gel 60 F254 pre-coated aluminium plates (0.25 mm). Visualization was performed under UV light and 254 or 312 nm, or with appropriate TLC stains including, but not limited to: phosphomolybdic acid, KMnO₄, ninhydrin, CAM, vanillin, *p*-anisaldehyde.

Microwave experiments were conducted in an Anton Paar Monowave 400^{®} microwave reactor. The experiments were conducted in a monomode cavity with a power delivery ranging from 0 to 850 W, allowing pressurized reactions (0 to 30 bars) to be performed in sealed glass vials (4 to 30 mL) equipped with snap caps and silicon septa. The temperature (0 to 300°C) was monitored by a contactless infrared sensor and calibrated with a ruby thermometer. Temperature, pressure, and power profiles were edited and monitored through a touch-screen control panel. Times indicated in the various protocols are the times measured when the mixtures reached the programmed temperature after a ramp period of 3 min.

Chromatographic purifications of compounds were achieved on an automated Interchim Puriflash XS420 equipped with 30 µm spherical silica-filled prepacked columns as stationary phase.

Some compounds of the invention are described with their structure in the below Table 2, which is merely illustrative and does not limit the scope of the present invention.

**Table 2: Structure of compounds (1) to (266). Formulas and molecular weights were generated using Perkin Elmer's Chemdraw^{®} version 17.1.0.105.**

| 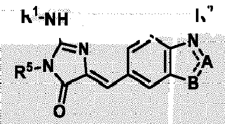 | | | | | | | |
|---|---|---|---|---|---|---|---|
| **Cpd N°** | **R¹-NH-** | **R⁵** | 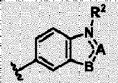 | **Class** | **Formula** | **MW** | **Stereo-chemistry** |
| **1** | 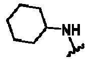 | H | 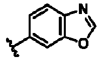 | A1 | C₁₇H₁₈N₄O₂ | 310.36 | N/A |
| **2** | 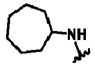 | H | 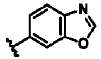 | A1 | C₁₈H₂₀N₄O₂ | 324.38 | N/A |
| **3** | 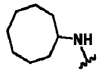 | H | 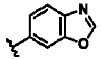 | A1 | C₁₉H₂₂N₄O₂ | 338.41 | N/A |
| **4** | 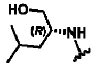 | H | 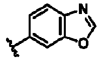 | A1 | C₁₇H₂₀N₄O₃ | 328.37 | (1*R*) |
| **5** | 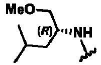 | H | 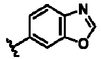 | A1 | C₁₈H₂₂N₄O₃ | 342.40 | (1*R*) |
| **6** | 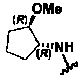 | H | 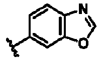 | A1 | C₁₇H₁₈N₄O₃ | 326.36 | (1*R*,2*R*) |
| **7** | 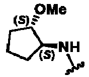 | H | 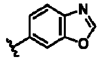 | A1 | C₁₇H₁₈N₄O₃ | 326.36 | (1*S*,2*S*) |
| **8** | 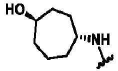 | H | 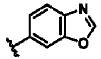 | A1 | C₁₈H₂₀N₄O₃ | 340.38 | *trans-*rac |
| **9** | 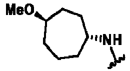 | H | 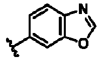 | A1 | C₁₉H₂₂N₄O₃ | 354.41 | *trans-*rac |
| **10** | 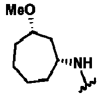 | H | 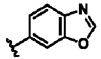 | A1 | C₁₉H₂₂N₄O₃ | 354.41 | *cis-*rac |
| **11** | 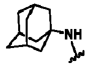 | H | 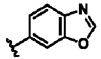 | A1 | C₂₁H₂₂N₄O₂ | 362.43 | N/A |
| **12** | 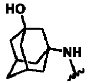 | H | 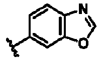 | A1 | C₂₁H₂₂N₄O₃ | 378.43 | N/A |
| **13** | 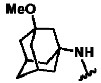 | H | 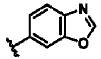 | A1 | C₂₂H₂₄N₄O₃ | 392.46 | N/A |
| **14** | 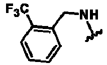 | H | 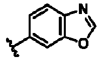 | A2 | C₁₉H₁₃F₃N₄O₂ | 386.33 | N/A |
| **15** | 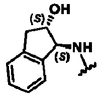 | H | 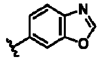 | A2 | C₂₀H₁₆N₄O₃ | 360.37 | (1*S*,2*S*) |
| **16** | 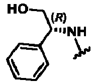 | H | 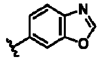 | A2 | C₁₉H₁₆N₄O₃ | 348.36 | (1*R*) |
| **17** | 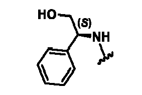 | H | 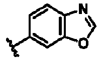 | A2 | C₁₉H₁₆N₄O₃ | 348.36 | (1*S*) |
| **18** | 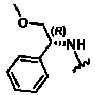 | H | 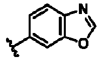 | A2 | C₂₀H₁₈N₄O₃ | 362.39 | (1*R*) |
| **19** | 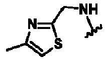 | H | 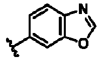 | A3 | C₁₆H₁₃N₅O₂S | 339.37 | N/A |
| **20** | 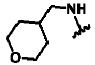 | H | 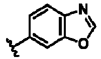 | A4 | C₁₇H₁₈N₄O₃ | 326.36 | N/A |
| **21** | 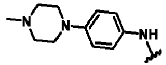 | H | 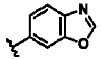 | A5 | C₂₂H₂₂N₆O₂ | 402.46 | N/A |
| **22** | 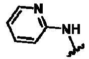 | H | 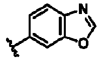 | A6 | C₁₆H₁₁N₅O₂ | 305.30 | N/A |
| **23** | 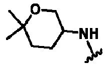 | H | 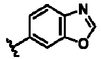 | A7 | C₁₈H₂₀N₄O₃ | 340.38 | rac |
| **24** | 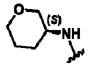 | H | 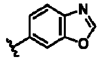 | A7 | C₁₆H₁₆N₄O₃ | 312.33 | (3*S*) |
| **25** | 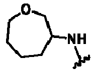 | H | 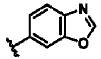 | A7 | C₁₇H₁₈N₄O₃ | 326.36 | rac |
| **26** | 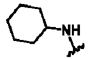 | H | 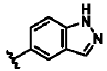 | A1 | C₁₇H₁₉N₅O | 309.37 | N/A |
| **27** | 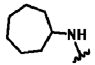 | H | 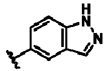 | A1 | C₁₈H₂₁N₅O | 323.40 | N/A |
| **28** | 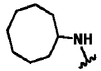 | H | 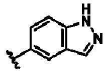 | A1 | C₁₉H₂₃N₅O | 337.43 | N/A |
| **29** | 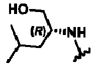 | H | 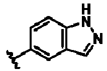 | A1 | C₁₇H₂₁N₅O₂ | 327.39 | (1*R*) |
| **30** | 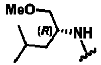 | H | 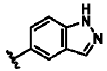 | A1 | C₁₈H₂₃N₅O₂ | 341.42 | (1*R*) |
| **31** | 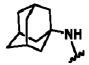 | H | 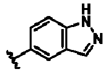 | A1 | C₂₁H₂₃N₅O | 361.45 | N/A |
| **32** | 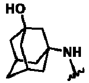 | H | 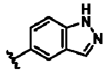 | A1 | C₂₁H₂₃N₅O₂ | 377.45 | N/A |
| **33** | 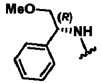 | H | 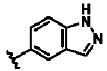 | A2 | C₂₀H₁₉NsO₂ | 361.405 | (1*R*) |
| **34** | 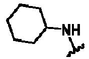 | H | 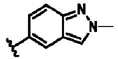 | A1 | C₁₈H₂₁N₅O | 323.40 | N/A |
| **35** | 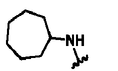 | H | 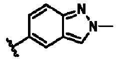 | A1 | C₁₉H₂₃N₅O | 337.43 | N/A |
| **36** | 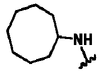 | H | 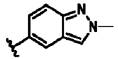 | A1 | C₂₀H₂₅N₅O | 351.45 | N/A |
| **37** | 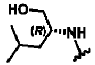 | H | 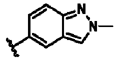 | A1 | C₁₈H₂₃N₅O₂ | 341.42 | (1*R*) |
| **38** | 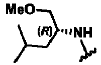 | H | 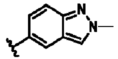 | A1 | C₁₉H₂₅N₅O₂ | 355.44 | (1*R*) |
| **39** | 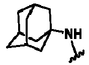 | H | 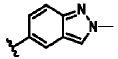 | A1 | C₂₂H₂₅N₅O | 375.48 | N/A |
| **40** | 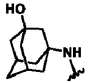 | H | 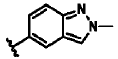 | A1 | C₂₂H₂₅N₅O₂ | 391.48 | N/A |
| **41** | 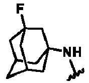 | H | 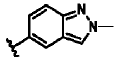 | A1 | C₂₂H₂₄FN₅O | 393.47 | N/A |
| **42** | 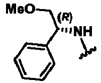 | H | 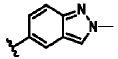 | A2 | C₂₁H₂₁N₅O₂ | 375.43 | (1*R*) |
| **43** | 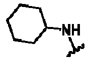 | H | 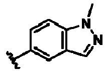 | A1 | C₁₈H₂₁N₅O | 323.40 | N/A |
| **44** | 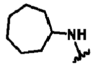 | H | 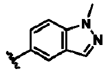 | A1 | C₁₉H₂₃N₅O | 337.43 | N/A |
| **45** | 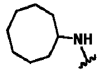 | H | 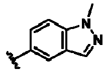 | A1 | C₂₀H₂₅N₅O | 351.45 | N/A |
| **46** | 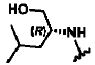 | H | 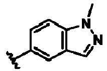 | A1 | C₁₈H₂₃N₅O₂ | 341.42 | (1*R*) |
| **47** | 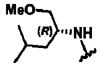 | H | 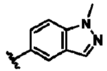 | A1 | C₁₉H₂₅N₅O₂ | 355.44 | (1*R*) |
| **48** | 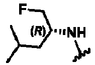 | H | 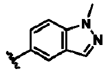 | A1 | C₁₈H₂₂FN₅O | 343.41 | (1*R*) |
| **49** | 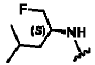 | H | 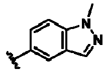 | A1 | C₁₈H₂₂FN₅O | 343.41 | (1*S*) |
| **50** | 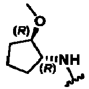 | H | 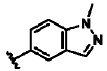 | A1 | C₁₈H₂₁N₅O₂ | 339.40 | (1*R*,2*R*) |
| **51** | 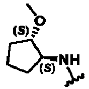 | H | 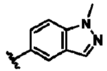 | A1 | C₁₈H₂₁N₅O₂ | 339.40 | (1*S*,2*S*) |
| **52** | 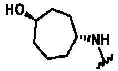 | H | 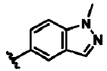 | A1 | C₁₉H₂₃N₅O₂ | 353.43 | *trans-rac* |
| **53** | 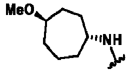 | H | 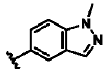 | A1 | C₂₀H₂₅N₅O₂ | 367.45 | *trans-rac* |
| **54** | 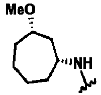 | H | 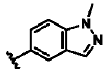 | A1 | C₂₀H₂₅N₅O₂ | 367.45 | *cis-*rac |
| **55** | 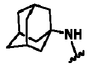 | H | 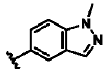 | A1 | C₂₂H₂₅N₅O | 375.48 | N/A |
| **56** | 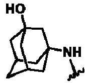 | H | 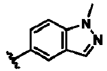 | A1 | C₂₂H₂₅N₅O₂ | 391.48 | N/A |
| **57** | 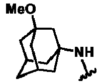 | H | 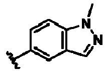 | A1 | C₂₃H₂₇N₅O₂ | 405.50 | N/A |
| **58** | 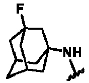 | H | 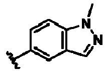 | A1 | C₂₂H₂₄FN₅O | 393.47 | N/A |
| **59** | 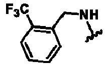 | H | 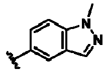 | A2 | C₂₀H₁₆F₃N₅O | 399.38 | N/A |
| **60** | 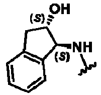 | H | 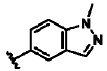 | A2 | C₂₁H₁₉N₅O₂ | 373.42 | (1*S*,2*S*) |
| **61** | 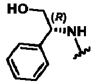 | H | 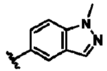 | A2 | C₂₀H₁₉NsO₂ | 361.41 | (1*R*) |
| **62** | 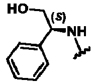 | H | 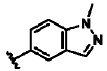 | A2 | C₂₀H₁₉NsO₂ | 361.41 | (1*S*) |
| **63** | 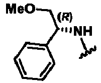 | H | 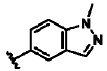 | A2 | C₂₁H₂₁N₅O₂ | 375.43 | (1*R*) |
| **64** | 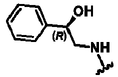 | H | 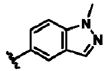 | A2 | C₂₀H₁₉NsO₂ | 361.41 | (2*R*) |
| **65** | 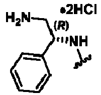 | H | 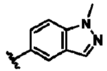 | A2 | C₂₀H₂₂Cl₂N₆O | 433.34 | (1*R*) |
| **66** | 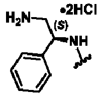 | H | 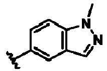 | A2 | C₂₀H₂₂Cl₂N₆O | 433.34 | (1*S*) |
| **67** | 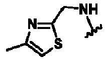 | H | 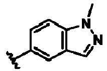 | A3 | C₁₇H₁₆N₆OS | 352.42 | N/A |
| **68** | 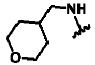 | H | 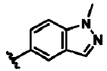 | A4 | C₁₈H₂₁N₅O₂ | 339.40 | N/A |
| **69** | 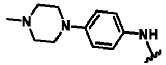 | H | 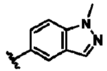 | A5 | C₂₃H₂₅N₇O | 415.50 | N/A |
| **70** | 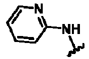 | H | 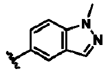 | A6 | C₁₇H₁₄N₆O | 318.34 | N/A |
| **71** | 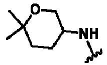 | H | 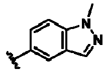 | A7 | C₁₉H₂₃N₅O₂ | 353.43 | rac |
| **72** | 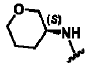 | H | 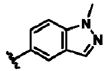 | A7 | C₁₇H₁₉N₅O₂ | 325.37 | (3*S*) |
| **73** | 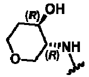 | H | 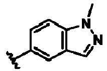 | A7 | C₁₇H₁₉N₅O₃ | 341.37 | (3*R*,4*R*) |
| **74** | 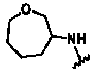 | H | 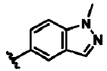 | A7 | C₁₈H₂₁N₅O₂ | 339.40 | rac |
| **75** | 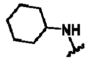 | H | 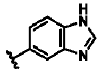 | A1 | C₁₇H₁₉N₅O | 309.37 | N/A |
| **76** | 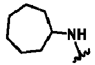 | H | 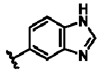 | A1 | C₁₈H₂₁N₅O | 323.4 | N/A |
| **77** | 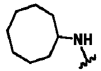 | H | 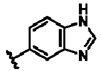 | A1 | C₁₉H₂₃N₅O | 337.43 | N/A |
| **78** | 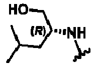 | H | 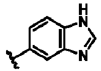 | A1 | C₁₇H₂₁N₅O₂ | 327.39 | (1*R*) |
| **79** | 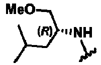 | H | 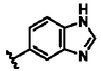 | A1 | C₁₈H₂₃N₅O₂ | 341.42 | (1*R*) |
| **80** | 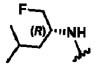 | H | 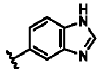 | A1 | C₁₇H₂₀FN₅O | 329.38 | (1*R*) |
| **81** | 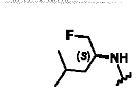 | H | 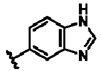 | A1 | C₁₇H₂₀FN₅O | 329.38 | (1*S*) |
| **82** | 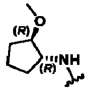 | H | 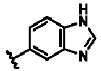 | A1 | C₁₇H₁₉N₅O₂ | 325.37 | (1*R*,2*R*) |
| **83** | 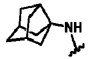 | H | 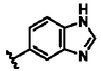 | A1 | C₂₀H₂₁N₅O | 347.42 | N/A |
| **84** | 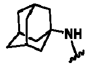 | H | 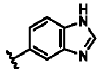 | A1 | C₂₁H₂₃N₅O | 361.45 | N/A |
| **85** | 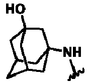 | H | 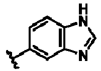 | A1 | C₂₁H₂₃N₅O₂ | 377.45 | N/A |
| **86** | 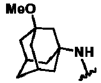 | H | 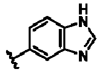 | A1 | C₂₂H₂₅N₅O₂ | 391.48 | N/A |
| **87** | 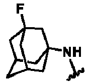 | H | 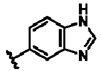 | A1 | C₂₁H₂₂FN₅O | 379,44 | N/A |
| **88** | 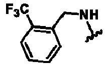 | H | 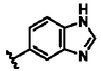 | A2 | C₁₉H₁₄F₃N₅O | 385.35 | N/A |
| **89** | 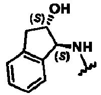 | H | 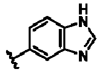 | A2 | C₂₀H₁₇N₅O₂ | 359.39 | (1*S*,2*S*) |
| **90** | 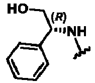 | H | 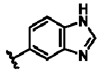 | A2 | C₁₉H₁₇N₅O₂ | 347.38 | (1*R*) |
| **91** | 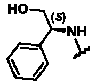 | H | 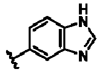 | A2 | C₁₉H₁₇N₅O₂ | 347.38 | (1*S*) |
| **92** | 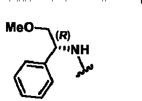 | H | 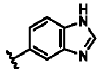 | A2 | C₂₀H₁₉NsO₂ | 361.41 | (1*R*) |
| **93** | 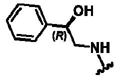 | H | 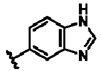 | A2 | C₁₉H₁₇N₅O₂ | 347.38 | (2*R*) |
| **94** | 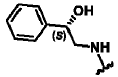 | H | 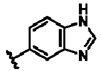 | A2 | C₁₉H₁₇N₅O₂ | 347.38 | (2*S*) |
| **95** | 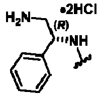 | H | 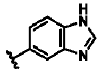 | A2 | C₁₉H₂₀Cl₂N₆O | 419.31 | (1*R*) |
| **96** | 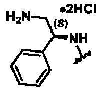 | H | 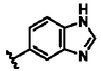 | A2 | C₁₉H₂₀Cl₂N₆O | 419.31 | (1*S*) |
| **97** | 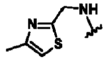 | H | 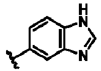 | A3 | C₁₆H₁₄N₆OS | 338.39 | N/A |
| **98** | 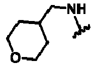 | H | 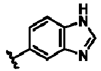 | A4 | C₁₇H₁₉N₅O₂ | 325.37 | N/A |
| **99** | 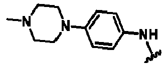 | H | 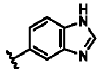 | A5 | C₂₂H₂₃N₇O | 401.47 | N/A |
| **100** | 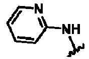 | H | 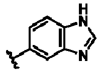 | A6 | C₁₆H₁₂N₆O | 304.31 | N/A |
| **101** | 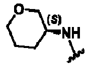 | H | 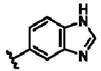 | A7 | C₁₆H₁₇N₅O₂ | 311.35 | (3*S*) |
| **102** | 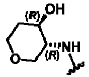 | H | 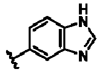 | A7 | C₁₆H₁₇N₅O₃ | 327.34 | (3*R*,4*R*) |
| **103** | 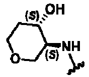 | H | 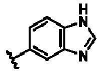 | A7 | C₁₆H₁₇N₅O₃ | 327.34 | (3*S*,4*S*) |
| **104** | 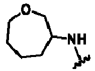 | H | 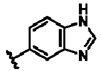 | A7 | C₁₇H₁₉N₅O₂ | 325.37 | rac |
| **105** | 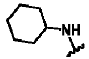 | H | 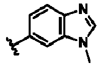 | A1 | C₁₈H₂₁N₅O | 323.40 | N/A |
| **106** | 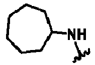 | H | 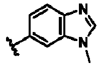 | A1 | C₁₉H₂₃N₅O | 337.43 | N/A |
| **107** | 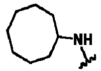 | H | 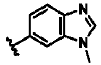 | A1 | C₂₀H₂₅N₅O | 351.45 | N/A |
| **108** | 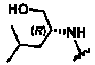 | H | 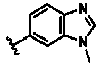 | A1 | C₁₈H₂₃N₅O₂ | 341.42 | (1*R*) |
| **109** | 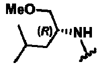 | H | 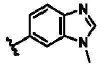 | A1 | C₁₉H₂₅N₅O₂ | 355.44 | (1*R*) |
| **110** | 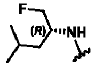 | H | 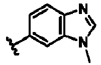 | A1 | C₁₈H₂₂FN₅O | 343.41 | (1*R*) |
| **111** | 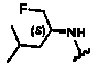 | H | 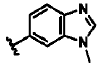 | A1 | C₁₈H₂₂FN₅O | 343.41 | (1*S*) |
| **112** | 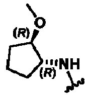 | H | 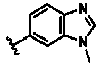 | A1 | C₁₈H₂₁N₅O₂ | 339.40 | (1*R*,2*R*) |
| **113** | 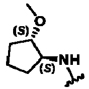 | H | 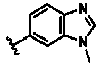 | A1 | C₁₈H₂₁N₅O₂ | 339.40 | (1*S*,2*S*) |
| **114** | 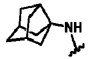 | H | 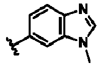 | A1 | C₂₁H₂₃N₅O | 361,45 | N/A |
| **115** | 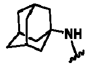 | H | 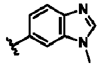 | A1 | C₂₂H₂₅N₅O | 375.48 | N/A |
| **116** | 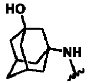 | H | 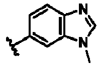 | A1 | C₂₂H₂₅N₅O₂ | 391.48 | N/A |
| **117** | 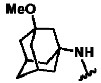 | H | 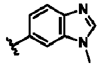 | A1 | C₂₃H₂₇N₅O₂ | 405.50 | N/A |
| **118** | 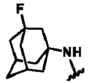 | H | 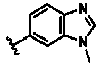 | A1 | C₂₂H₂₄FN₅O | 393,47 | N/A |
| **119** | 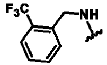 | H | 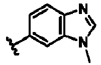 | A2 | C₂₀H₁₆F₃N₅O | 399.38 | N/A |
| **120** | 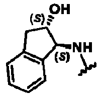 | H | 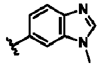 | A2 | C₂₁H₁₉N₅O₂ | 373.42 | (1*S*,2*S*) |
| **121** | 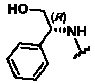 | H | 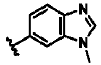 | A2 | C₂₀H₁₉N₅O₂ | 361.41 | (1*R)̅* |
| **122** | 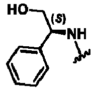 | H | 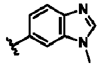 | A2 | C₂₀H₁₉N₅O₂ | 361.41 | (1*S*) |
| **123** | 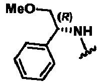 | H | 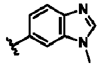 | A2 | C₂₁H₂₁N₅O₂ | 375.43 | (1*R*) |
| **124** | 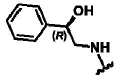 | H | 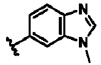 | A2 | C₂₀H₁₉N₅O₂ | 361.41 | (2*R*) |
| **125** | 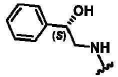 | H | 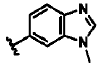 | A2 | C₂₀H₁₉N₅O₂ | 361.41 | (2*S*) |
| **126** | 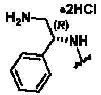 | H | 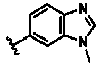 | A2 | C₂₀H₂₂Cl₂N₆O | 433.34 | (1*R*) |
| **127** | 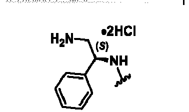 | H | 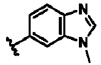 | A2 | C₂₀H₂₂Cl₂N₆O | 433.34 | (1*S*) |
| **128** | 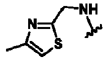 | H | 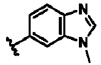 | A3 | C₁₇H₁₆N₆OS | 352.42 | N/A |
| **129** | 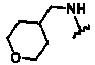 | H | 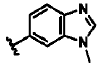 | A4 | C₁₈H₂₁N₅O₂ | 339.40 | N/A |
| **130** | 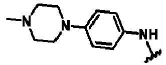 | H | 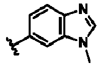 | A5 | C₂₃H₂₅N₇O | 415.50 | N/A |
| **131** | 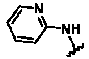 | H | 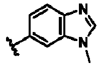 | A6 | C₁₇H₁₄N₆O | 318.34 | N/A |
| **132** | 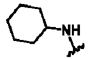 | Me | 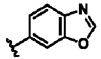 | A1 | C₁₈H₂₀N₄O₂ | 324.38 | N/A |
| **133** | 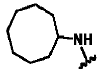 | Me | 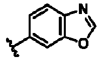 | A1 | C₂₀H₂₄N₄O₂ | 352.44 | N/A |
| **134** | 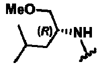 | Me | 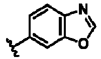 | A1 | C₁₉H₂₄N₄O₃ | 356.43 | (1*R*) |
| **135** | 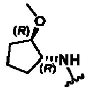 | Me | 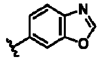 | A1 | C₁₈H₂₀N₄O₃ | 340.38 | (1*R,*2*R*) |
| **136** | 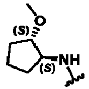 | Me | 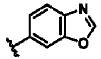 | A1 | C₁₈H₂₀N₄O₃ | 340.38 | (1*R*,2*R*) |
| **137** | 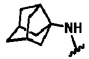 | Me | 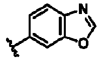 | A1 | C₂₁H₂₂N₄O₂ | 362.43 | N/A |
| **138** | 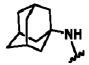 | Me | 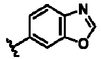 | A1 | C₂₂H₂₄N₄O₂ | 376.46 | N/A |
| **139** | 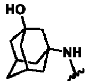 | Me | 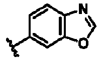 | A1 | C₂₂H₂₄N₄O₃ | 392.46 | N/A |
| **140** | 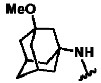 | Me | 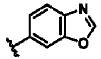 | A1 | C₂₃H₂₆N₄O₃ | 406.49 | N/A |
| **141** | 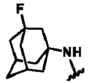 | Me | 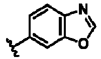 | A1 | C₂₂H₂₃FN₄O₂ | 394.45 | N/A |
| **142** | 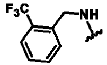 | Me | 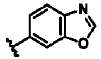 | A1 | C₂₀H₁₅F₃N₄O₂ | 400.36 | N/A |
| **143** | 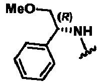 | Me | 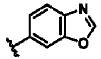 | A2 | C₂₁H₂₀N₄O₃ | 376.42 | (1*R*) |
| **144** | 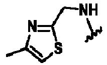 | Me | 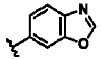 | A3 | C₁₇H₁₅N₅O₂S | 353.40 | N/A |
| **145** | 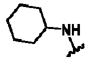 | Me | 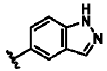 | A1 | C₁₈H₂₁N₅O | 323.40 | N/A |
| **146** | 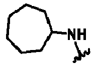 | Me | 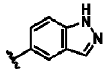 | A1 | C₁₉H₂₃N₅O | 337.43 | N/A |
| **147** | 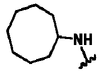 | Me | 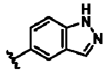 | A1 | C₂₀H₂₅N₅O | 351.45 | N/A |
| **148** | 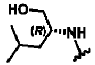 | Me | 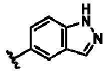 | A1 | C₁₈H₂₃N₅O₂ | 341.42 | (1*R*) |
| **149** | 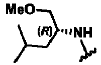 | Me | 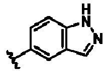 | A1 | C₁₉H₂₅N₅O₂ | 355.44 | (1*R*) |
| **150** | 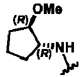 | Me | 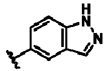 | A1 | C₁₈H₂₁N₅O₂ | 339.40 | (1*R*,2*R*) |
| **151** | 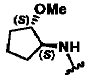 | Me | 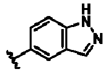 | A1 | C₁₈H₂₁N₅O₂ | 339.40 | (1*S*,2*S*) |
| **152** | 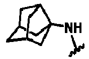 | Me | 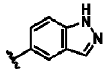 | A1 | C₂₁H₂₃N₅O | 361.45 | N/A |
| **153** | 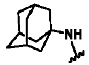 | Me | 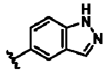 | A1 | C₂₂H₂₅N₅O | 375.48 | N/A |
| **154** | 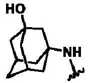 | Me | 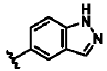 | A1 | C₂₂H₂₅N₅O₂ | 391.48 | N/A |
| **155** | 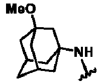 | Me | 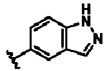 | A1 | C₂₃H₂₇N₅O₂ | 405.50 | N/A |
| **156** | 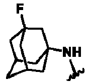 | Me | 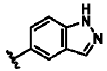 | A1 | C₂₂H₂₄FN₅O | 393.47 | N/A |
| **157** | 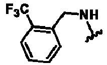 | Me | 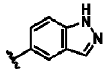 | A2 | C₂₀H₁₆F₃N₅O | 399.38 | N/A |
| **158** | 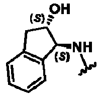 | Me | 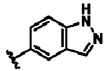 | A2 | C₂₁H₁₉N₅O₂ | 373.42 | (1*S*,2*S*) |
| **159** | 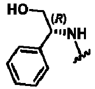 | Me | 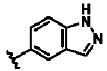 | A2 | C₂₀H₁₉N₅O₂ | 361.41 | (1*R*) |
| **160** | 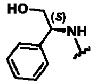 | Me | 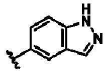 | A2 | C₂₀H₁₉N₅O₂ | 361.41 | (1*R*) |
| **161** | 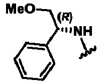 | Me | 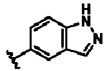 | A2 | C₂₁H₂₁N₅O₂ | 375.43 | (1*R*) |
| **162** | 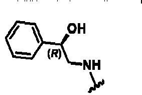 | Me | 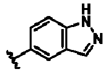 | A2 | C₂₀H₁₉N₅O₂ | 361.41 | (2*R*) |
| **163** | 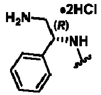 | Me | 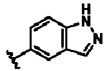 | A2 | C₂₀H₂₂Cl₂N₆O | 433.34 | (1*R*) |
| **164** | 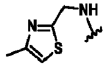 | Me | 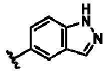 | A3 | C₁₇H₁₆N₆OS | 352.42 | N/A |
| **165** | 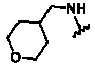 | Me | 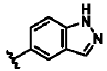 | A4 | C₁₈H₂₁N₅O₂ | 339.40 | N/A |
| **166** | 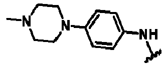 | Me | 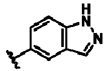 | A5 | C₂₃H₂₅N₇O | 415.50 | N/A |
| **167** | 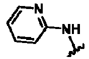 | Me | 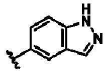 | A6 | C₁₇H₁₄N₆O | 318.34 | N/A |
| **168** | 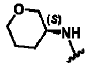 | Me | 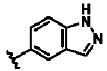 | A7 | C₁₇H₁₉N₅O₂ | 325.37 | (3*S*) |
| **169** | 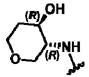 | Me | 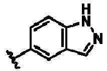 | A7 | C₁₇H₁₉N₅O₃ | 341.37 | (3*R*,4*R*) |
| **170** | 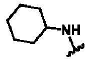 | Me | 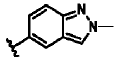 | A1 | C₁₉H₂₃N₅O | 337.43 | N/A |
| **171** | 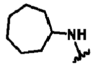 | Me | 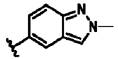 | A1 | C₂₀H₂₅N₅O | 351.45 | N/A |
| **172** | 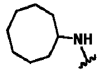 | Me | 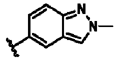 | A1 | C₂₁H₇N₅O | 365.48 | N/A |
| **173** | 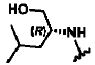 | Me | 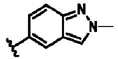 | A1 | C₁₉H₂₅N₅O₂ | 355.44 | (1*R*) |
| **174** | 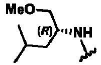 | Me | 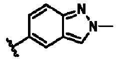 | A1 | C₂₀H₂₇N₅O₂ | 369.47 | (1*R*) |
| **175** | 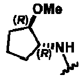 | Me | 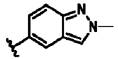 | A1 | C₁₉H₂₃N₅O₂ | 353.43 | (1*R*,2*R*) |
| **176** | 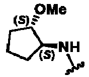 | Me | 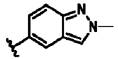 | A1 | C₁₉H₂₃N₅O₂ | 353.43 | (1*S*,2*S*) |
| **177** | 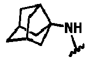 | Me | 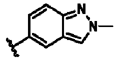 | A1 | C₂₂H₂₅N₅O | 375.48 | N/A |
| **178** | 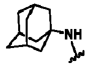 | Me | 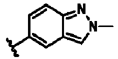 | A1 | C₂₃H₂₇N₅O | 389.50 | N/A |
| **179** | 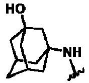 | Me | 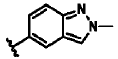 | A1 | C₂₃H₂₇N₅O₂ | 405.50 | N/A |
| **180** | 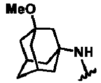 | Me | 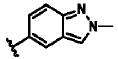 | A1 | C₂₄H₂₉N₅O₂ | 419.53 | N/A |
| **181** | 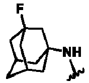 | Me | 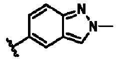 | A1 | C₂₃H₂₆FN₅O | 407.49 | N/A |
| **182** | 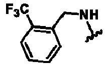 | Me | 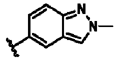 | A2 | C₂₁H₁₈F₃N₅O | 413.40 | N/A |
| **183** | 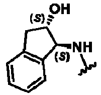 | Me | 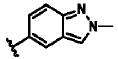 | A2 | C₂₂H₂₁N₅O₂ | 387.44 | (1*S*,2*S*) |
| **184** | 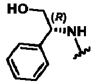 | Me | 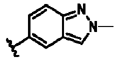 | A2 | C₂₁H₂₁N₅O₂ | 375.43 | (1*R*) |
| **185** | 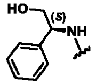 | Me | 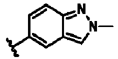 | A2 | C₂₁H₂₁N₅O₂ | 375.43 | (1*R*) |
| **186** | 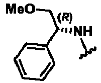 | Me | 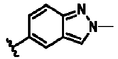 | A2 | C₂₂H₂₃N₅O₂ | 389.46 | (1*R*) |
| **187** | 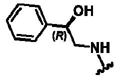 | Me | 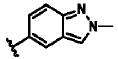 | A2 | C₂₁H₂₁N₅O₂ | 375.43 | (2*R*) |
| **188** | 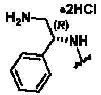 | Me | 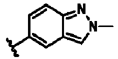 | A2 | C₂₁H₂₄CI₂N₆O | 447.36 | (1*R*) |
| **189** | 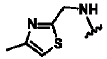 | Me | 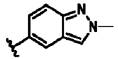 | A3 | C₁₈H₁₈N₆OS | 366.44 | N/A |
| **190** | 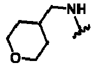 | Me | 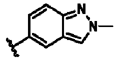 | A4 | C₁₉H₂₃N₅O₂ | 353.43 | N/A |
| **191** | 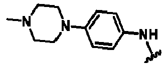 | Me | 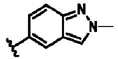 | A5 | C₂₄H₂₇N₇O | 429.53 | N/A |
| **192** | 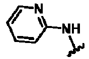 | Me | 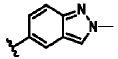 | A6 | C₁₈H₁₆N₆O | 332.37 | N/A |
| **193** | 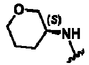 | Me | 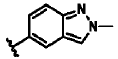 | A7 | C₁₈H₂₁N₅O₂ | 339.40 | (3*S*) |
| **194** | 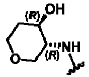 | Me | 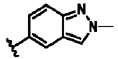 | A7 | C₁₈H₂₁N₅O₃ | 355.40 | (3*R*,4*R*) |
| **195** | 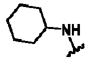 | Me | 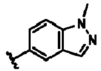 | A1 | C₁₉H₂₃N₅O | 337.43 | N/A |
| 196 | 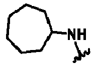 | Me | 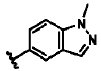 | A1 | C₂₀H₂₅N₅O | 351.45 | N/A |
| **197** | 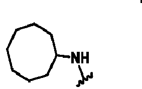 | Me | 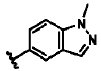 | A1 | C₂₁H₂₇N₅O | 365.48 | N/A |
| **198** | 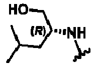 | Me | 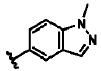 | A1 | C₁₉H₂₅N₅O₂ | 355.44 | (1*R*) |
| **199** | 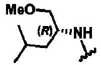 | Me | 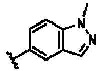 | A1 | C₂₀H₂₇N₅O₂ | 369.47 | (1*R*) |
| **200** | 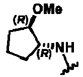 | Me | 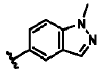 | A1 | C₁₉H₂₃N₅O₂ | 353.43 | (1*R*,2*R*) |
| **201** | 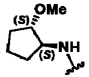 | Me | 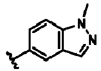 | A1 | C₁₉H₂₃N₅O₂ | 353.43 | (1*S*,2*S*) |
| **202** | 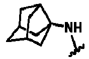 | Me | 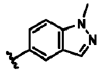 | A1 | C₂₂H₂₅N₅O | 375.48 | N/A |
| **203** | 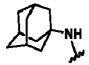 | Me | 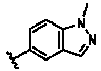 | A1 | C₂₃H₂₇N₅O | 389.50 | N/A |
| **204** | 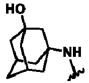 | Me | 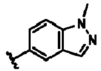 | A1 | C₂₃H₂₇N₅O₂ | 405.50 | N/A |
| **205** | 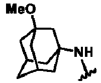 | Me | 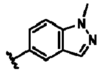 | A1 | C₂₄H₂₉N₅O₂ | 419.53 | N/A |
| **206** | 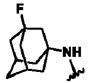 | Me | 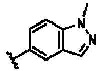 | A1 | C₂₃H₂₆FN₅O | 407.49 | N/A |
| **207** | 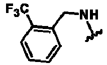 | Me | 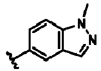 | A2 | C₂₁H₁₈F₃N₅O | 413.40 | N/A |
| **208** | 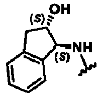 | Me | 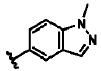 | A2 | C₂₂H₂₁N₅O₂ | 387.44 | (1*S*,2*S*) |
| **209** | 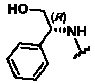 | Me | 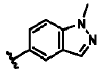 | A2 | C₂₁H₂₁N₅O₂ | 375.43 | (1*R*) |
| **210** | 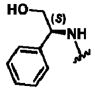 | Me | 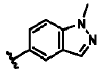 | A2 | C₂₁H₂₁N₅O₂ | 375.43 | (1*R*) |
| **211** | 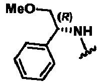 | Me | 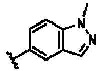 | A2 | C₂₂H₂₃N₅O₂ | 389.46 | (1*R*) |
| **212** | 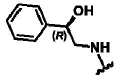 | Me | 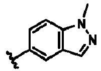 | A2 | C₂₁H₂₁N₅O₂ | 375.43 | (2*R*) |
| **213** | 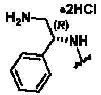 | Me | 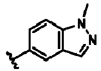 | A2 | C₂₁H₂₄Cl₂N₆O | 447.36 | (1*R*) |
| **214** | 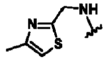 | Me | 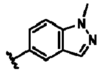 | A3 | C₁₈H₁₈N₆OS | 366.44 | N/A |
| **215** | 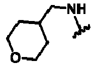 | Me | 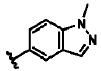 | A4 | C₁₉H₂₃N₅O₂ | 353.43 | N/A |
| **216** | 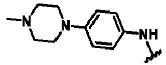 | Me | 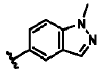 | A5 | C₂₄H₂₇N₇O | 429.53 | N/A |
| **217** | 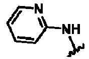 | Me | 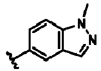 | A6 | C₁₈H₁₆N₆O | 332.37 | N/A |
| **218** | 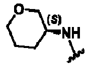 | Me | 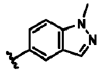 | A7 | C₁₈H₂₁N₅O₂ | 339.40 | (3*S*) |
| **219** | 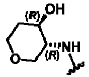 | Me | 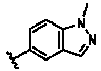 | A7 | C₁₈H₂₁N₅O₃ | 355.40 | (3*R*,4*R*) |
| **220** | 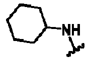 | Me | 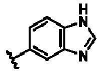 | A1 | C₁₈H₂₁N₅O | 323.40 | N/A |
| **221** | 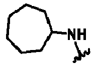 | Me | 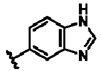 | A1 | C₁₉H₂₃N₅O | 337.43 | N/A |
| **222** | 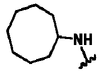 | Me | 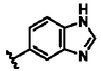 | A1 | C₂₀H₂₅N₅O | 351.45 | N/A |
| **223** | 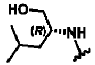 | Me | 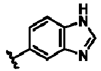 | A1 | C₁₈H₂₃N₅O₂ | 341.42 | (1*R*) |
| **224** | 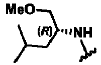 | Me | 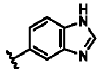 | A1 | C₁₉H₂₅N₅O₂ | 355.44 | (1*R*) |
| **225** | 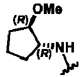 | Me | 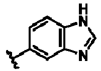 | A1 | C₁₈H₂₁N₅O₂ | 339.40 | (1*R*,2*R*) |
| **226** | 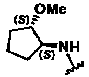 | Me | 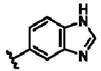 | A1 | C₁₈H₂₁N₅O₂ | 339.40 | (1*S*,2*S*) |
| **227** | 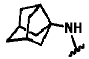 | Me | 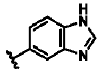 | A1 | C₂₁H₂₃N₅O | 361.45 | N/A |
| **228** | 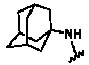 | Me | 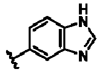 | A1 | C₂₂H₂₅N₅O | 375.48 | N/A |
| **229** | 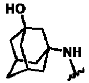 | Me | 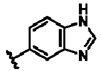 | A1 | C₂₂H₂₅N₅O₂ | 391.48 | N/A |
| **230** | 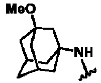 | Me | 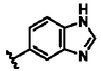 | A1 | C₂₃H₂₇N₅O₂ | 405.50 | N/A |
| **231** | 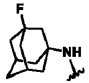 | Me | 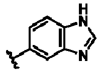 | A1 | C₂₂H₂₄FN₅O | 393.47 | N/A |
| **232** | 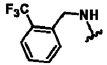 | Me | 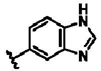 | A2 | C₂₀H₁₆F₃N₅O | 399.38 | N/A |
| **233** | 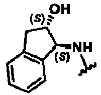 | Me | 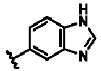 | A2 | C₂₁H₁₉N₅O₂ | 373.42 | (1*S*,2*S*) |
| **234** | 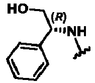 | Me | 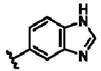 | A2 | C₂₀H₁₉N₅O₂ | 361.41 | (1*R*) |
| **235** | 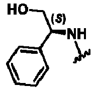 | Me | 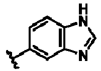 | A2 | C₂₀H₁₉N₅O₂ | 361.41 | (1*R*) |
| **236** | 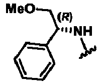 | Me | 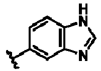 | A2 | C₂₁H₂₁N₅O₂ | 375.43 | (1*R*) |
| **237** | 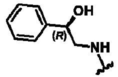 | Me | 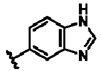 | A2 | C₂₀H₁₉N₅O₂ | 361.41 | (2*R*) |
| **238** | 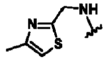 | Me | 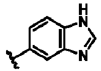 | A3 | C₁₇H₁₆N₆OS | 352.42 | N/A |
| **239** | 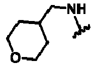 | Me | 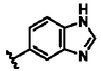 | A4 | C₁₈H₂₁N₅O₂ | 339.40 | N/A |
| **240** | 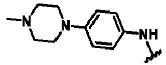 | Me | 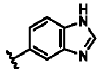 | A5 | C₂₃H₂₅N₇O | 415.50 | N/A |
| **241** | 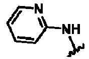 | Me | 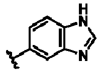 | A6 | C₁₇H₁₄N₆O | 318.34 | N/A |
| **242** | 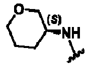 | Me | 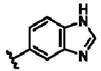 | A7 | C₁₇H₁₉N₅O₂ | 325.37 | (3*S*) |
| **243** | 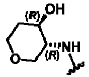 | Me | 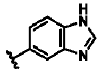 | A7 | C₁₇H₁₉N₅O₃ | 341.37 | (3*R*,4*R*) |
| **244** | 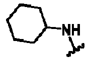 | Me | 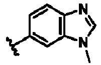 | A1 | C₁₉H₂₃N₅O | 337.43 | N/A |
| **245** | 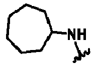 | Me | 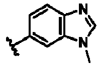 | A1 | C₂₀H₂₅N₅O | 351.45 | N/A |
| **246** | 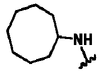 | Me | 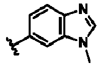 | A1 | C₂₁H₂₇N₅O | 365.48 | N/A |
| **247** | 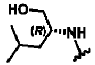 | Me | 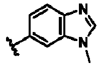 | A1 | C₁₉H₂₅N₅O₂ | 355.44 | (1*R*) |
| **248** | 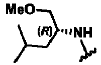 | Me | 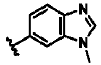 | A1 | C₂₀H₂₇N₅O₂ | 369.47 | (1*R*) |
| **249** | 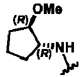 | Me | 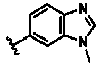 | A1 | C₁₉H₂₃N₅O₂ | 353.43 | (1*R*,2*R*) |
| **250** | 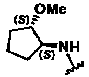 | Me | 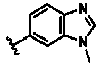 | A1 | C₁₉H₂₃N₅O₂ | 353.43 | (1*S*,2*S*) |
| **251** | 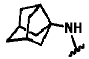 | Me | 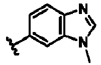 | A1 | C₂₂H₂₅N₅O | 375.48 | N/A |
| **252** | 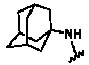 | Me | 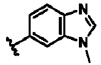 | A1 | C₂₃H₂₇N₅O | 389.50 | N/A |
| **253** | 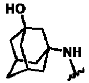 | Me | 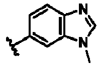 | A1 | C₂₃H₂₇N₅O₂ | 405.50 | N/A |
| **254** | 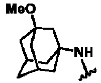 | Me | 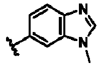 | A1 | C₂₄H₂₉N₅O₂ | 419.53 | N/A |
| **255** | 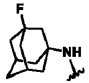 | Me | 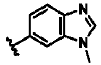 | A1 | C₂₃H₂₆FN₅O | 407.49 | N/A |
| **256** | 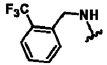 | Me | 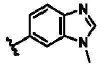 | A2 | C₂₁H₁₈F₃N₅O | 413.40 | N/A |
| **257** | 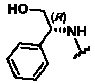 | Me | 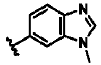 | A2 | C₂₁H₂₁N₅O₂ | 375.43 | (1*R*) |
| **258** | 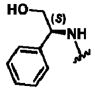 | Me | 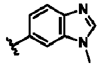 | A2 | C₂₁H₂₁N₅O₂ | 375.43 | (1*R*) |
| **259** | 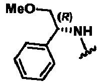 | Me | 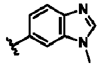 | A2 | C₂₂H₂₃N₅O₂ | 389.46 | (1*R*) |
| **260** | 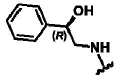 | Me | 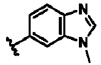 | A2 | C₂₁H₂₁N₅O₂ | 375.43 | (2*R*) |
| **261** | 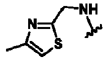 | Me | 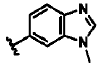 | A3 | C₁₈H₁₈N₆OS | 366.44 | N/A |
| **262** | 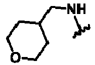 | Me | 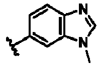 | A4 | C₁₉H₂₃N₅O₂ | 353.43 | N/A |
| **263** | 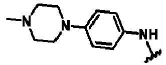 | Me | 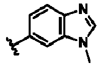 | A5 | C₂₄H₂₇N₇O | 429.53 | N/A |
| **264** | 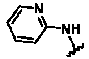 | Me | 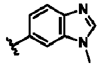 | A6 | C₁₈H₁₆N₆O | 332.37 | N/A |
| **265** | 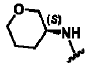 | Me | 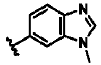 | A7 | C₁₈H₂₁N₅O₂ | 339.40 | (3*S*) |
| **266** | 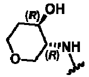 | Me | 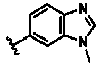 | A7 | C₁₈H₂₁N₅O₃ | 355.40 | (3*R*,4*R*) |

The below Table 3 describes the analytical and spectroscopic data of the compounds introduced in Table 2.
¹H NMR analyses (400 or 500 MHz) and ¹³C NMR spectra (101 MHz) were recorded with a Bruker ULTRASHIELD 500 or 400 spectrometer. Processing and analyses of the spectra were performed with MestReNova. Data appear in the following order: chemical shifts in ppm which were referenced to the internal solvent signal, multiplicity, number of protons, and coupling constant *J* in Hertz.

Reversed-phase HPLC/MS analyses were carried out with a Waters Alliance 2795 HPLC equipped with an autosampler, an inline membrane degasser, a column oven (T° = 45 °C), a UV detector, and a ZQ quadrupole mass detector working in ionization electrospray mode. Compounds (0.1 to 0.3 mg) were solubilized in a minimum amount of DMSO completed with acetonitrile (Vₜₒₜₐₗ: 1 mL). Standard analytical parameters: flow rate: 1mL/min, V_{inj.} : 5µL .
- Acidic conditions: Waters XSelect CSH C18 column (3.5µm, 2.1×50 mm). Gradient: (H₂O + 0.04% v/v HCOOH (10mM))/ACN from 95/5 to 0/100 in 18.5 min.
- Alkaline conditions: Waters Xbridge C18 column (3.5µm, 2.1× 50 mm). Gradient: (H₂O + 0.06% v/v NH_{3(aq)} (10mM))/ACN from 95/5 to 0/100 in 18.5 min.

**Table 3. Spectroscopic and analytical characterization of compounds (1) to (266)**

| **Cpd N°** | **Structure** | **Formula** | **MW** | **HPLC** | **Description** |
|---|---|---|---|---|---|
| **1** | 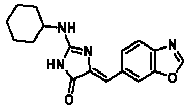 | C₁₇H₁₈N₄O₂ | 310.36 | 97% | Beige solid. ¹H NMR (400 MHz, DMSO-*d*₆, 343K) of major tautomer δ_{H} 10.39 (br s, 1H, NH, D₂O exchanged), 8.79 - 8.45 (m, 2H), 7.92 (d, *J* = 8.3 Hz, 1H), 7.71 (d, *J* = 8.3 Hz, 1H), 7.34 (br s, 1H, NH, D₂O exchanged), 6.41 (s, 1H), 3.73 (s, 1H), 1.94 (s, 2H), 1.83 - 1.66 (m, 2H), 1.68 - 1.55 (m, 1H), 1.48 - 1.28 (m, 4H), 1.28 - 1.11 (m, 1H). MS (ESI⁺) : [M+H]⁺ 311.2. |
| **2** | 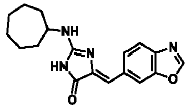 | C₁₈H₂₀N₄O₂ | 324.38 | >95% | Beige solid. ¹H NMR (400 MHz, DMSO-*d*₆, 343K) of major tautomer δ_{H} ¹H NMR 10.29 (br s, 1H, NH, D₂O exchanged), 8.63 (s, 2H), 7.92 (d, *J* = 8.3 Hz, 1H), 7.71 (d, *J* = 8.3 Hz, 1H), 7.33 (br s, 1H, NH, D₂O exchanged), 6.42 (s, 1H), 3.96 (s, 1H), 2.09 - 1.86 (m, 2H), 1.85 - 1.35 (m, 10H). MS (ESI⁺) : [M+H]⁺ 325.2. |
| **3** | 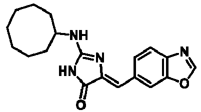 | C₁₉H₂₂N₄O₂ | 338.41 | >98% | Beige solid. ¹H NMR (400 MHz, DMSO-*d*₆, 323K) of major tautomer δ_{H} 10.37 (br s, 1H, NH, D₂O exchanged), 8.67 (br s, 2H), 7.91 (br s, 1H), 7.72 (d, *J* = 8.2 Hz, 1H), 7.44 (br s, 1H, NH, D₂O exchanged), 6.42 (s, 1H), 4.02 (br s, 1H), 1.95 - 1.80 (m, 2H), 1.78 - 1.45 (m, 12H). MS (ESI⁺) : [M+H]⁺ 339.3. |
| **4** | 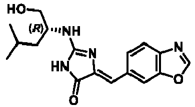 | C₁₇H₂₀N₄O₃ | 328.37 | 96% | Yellow solid. ¹H NMR (400 MHz, DMSO-*d*₆, 323K) of major tautomer δ_{H} 10.35 (br s, 1H, NH, D₂O exchanged), 8.67 (s, 1H), 8.65 (s, 1H), 7.90 (br s, 1H), 7.72 (d, *J* = 8.2 Hz, 1H), 7.20 (br s, 1H, NH, D₂O exchanged), 6.42 (s, 1H), 4.77 (br s, 1H, OH, D₂O exchanged), 4.03 (br s, 1H), 3.55 - 3.45 (m, 2H), 1.78 - 1.59 (m, 1H), 1.56 - 1.36 (m, 2H), 1.04 - 0.90 (m, 6H). MS (ESI⁺) : [M+H]+ 329.3. |
| **5** | 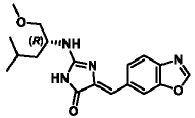 | C₁₈H₂₂N₄O₃ | 342.40 | >98% | Yellow solid. ¹H NMR (400 MHz, DMSO-*d*₆, 300K) of major tautomer δ_{H} 10.40 (br s, 1H, NH, D₂O exchanged), 8.67 (s, 1H), 8.65 (s, 1H), 7.92 (d, *J* = 8.3 Hz, 1H), 7.71 (d, *J* = 8.2 Hz, 1H), 7.31 (br s, 1H, NH, D₂O exchanged), 6.43 (s, 1H), 4.20 (br s, 1H), 3.52 - 3.36 (m, 2H), 3.32 (s, 3H), 1.77 - 1.63 (m, 1H), 1.59 -1.46 (m, 1H), 1.46 - 1.32 (m, 1H), 1.02 - 0.88 (m, 6H). MS (ESI⁺) : [M+H]+ 343.3. |
| **6** | 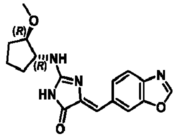 | C₁₇H₁₈N₄O₃ | 326.36 | >98% | Yellow solid. ¹H NMR (400 MHz, DMSO-*d*₆, 323K) of major tautomer δ_{H} 10.51 (br s, 1H, NH, D₂O exchanged), 8.68 (s, 1H), 8.63 (s, 1H), 7.93 (br s, 1H), 7.73 (d, *J* = 8.3 Hz, 1H), 7.64 (br s, 1H, NH, D₂O exchanged), 6.45 (s, 1H), 4.16 (br s, 1H), 3.84 - 3.75 (m, 1H), 3.36 (s, 3H), 2.15 - 2.02 (m, 1H), 1.98 - 1.85 (m, 1H), 1.80 -1.50 (m, 4H). MS (ESI⁺) : [M+H]⁺ 327.3. |
| **7** | 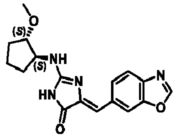 | C₁₇H₁₈N₄O₃ | 326.36 | >98% | Yellow solid. ¹H NMR (400 MHz, DMSO-*d*₆, 323K) of major tautomer δ_{H} 10.51 (br s, 1H, NH, D₂O exchanged), 8.68 (s, 1H), 8.63 (s, 1H), 7.93 (br s, 1H), 7.73 (d, *J* = 8.3 Hz, 1H), 7.64 (br s, 1H, NH, D₂O exchanged), 6.45 (s, 1H), 4.16 (br s, 1H), 3.84 - 3.75 (m, 1H), 3.36 (s, 3H), 2.15 - 2.02 (m, 1H), 1.98 - 1.85 (m, 1H), 1.80 -1.50 (m, 4H). MS (ESI⁺) : [M+H]⁺ 327.3. |
| **8** | 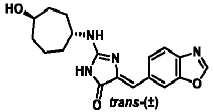 | C₁₈H₂₀N₄O₃ | 340.38 | 98% | Beige solid. ¹H NMR (400 MHz, DMSO-*d*₆, 323K) of major tautomer δ_{H} 10.35 (br s, 1H, NH, D₂O exchanged), 8.67 (s, 1H), 8.63 (br s, 1H), 7.89 (br s, 1H), 7.73 (d, *J* = 8.2 Hz, 1H), 7.46 (br s, 1H, NH, D₂O exchanged), 6.41 (s, 1H), 4.33 (d, *J* = 4.1 Hz, 1H, OH, D₂O exchanged), 3.96 (br s, 1H), 3.70 (br s, 1H), 2.05 ― 1.73 (m, 4H), 1.67 - 1.40 (m, 6H). MS (ESI⁺) : [M+H]+ 341.3. |
| **9** | 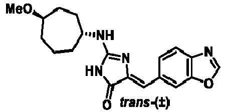 | C₁₉H₂₂N₄O₃ | 354.41 | >98% | Yellow solid. ¹H NMR (400 MHz, DMSO-*d*₆, 373K) of major tautomer δ_{H} 10.07 (br s, 1H, NH, D₂O exchanged), 8.80 - 8.38 (m, 2H), 7.90 (br s, 1H), 7.70 (d, *J* = 8.2 Hz, 1H), 7.11 (br s, 1H, NH, D₂O exchanged), 6.43 (s, 1H), 3.98 (br s, 1H), 3.45 - 3.33 (m, 1H), 3.26 (s, 3H), 2.09 - 1.89 (m, 3H), 1.89 - 1.75 (m, 1H), 1.73 - 1.48 (m, 6H). MS (ESI⁺) : [M+H]⁺ 355.3. |
| **10** | 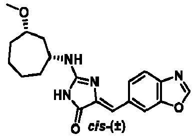 | C₁₉H₂₂N₄O₃ | 354.41 | >98% | Yellow solid. ¹H NMR (400 MHz, DMSO-*d*₆, 323K) of major tautomer δ_{H} 10.48 (br s, 1H, NH, D₂O exchanged), 8.67 (br s, 2H), 7.92 (br s, 1H), 7.71 (d, *J* = 8.2 Hz, 1H), 7.43 (br s, 1H, NH, D₂O exchanged), 6.43 (s, 1H), 3.93 (br s, 1H), 3.52 - 3.40 (m, 1H), 3.26 (s, 3H), 2.40 - 2.20 (m, 1H), 2.00 - 1.83 (m, 2H), 1.77 - 1.45 (m, 7H). MS (ESI⁺) : [M+H]+ 355.2. |
| **11** | 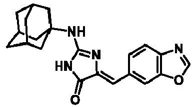 | C₂₁H₂₂N₄O₂ | 362.43 | >98% | Beige solid. ¹H NMR (400 MHz, DMSO-*d*₆, 323K) of major tautomer δ_{H} 9.84 (br s, 1H, NH, D₂O exchanged), 8.75 (s, 1H), 8.67 (s, 1H), 7.87 (d, *J* = 8.4 Hz, 1H), 7.72 (d, *J* = 8.3 Hz, 1H), 6.88 (br s, 1H, NH, D₂O exchanged), 6.43 (s, 1H), 2.23 ― 2.00 (m, 9H), 1.80 ― 1.60 (m, 6H). MS (ESI⁺) : [M+H]⁺ 363.3. |
| **12** | 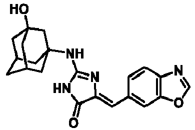 | C₂₁H₂₂N₄O₃ | 378.43 | 97% | Beige solid. ¹H NMR (400 MHz, DMSO-*d*₆, 323K) of major tautomer δ_{H} 9.85 (br s, 1H, NH, D₂O exchanged), 8.72 (s, 1H), 8.67 (s, 1H), 7.87 (d, *J* = 8.5 Hz, 1H), 7.72 (d, *J* = 8.2 Hz, 1H), 6.96 (br s, 1H, NH, D₂O exchanged), 6.43 (s, 1H), 4.49 (s, 1H, OH, D₂O exchanged), 2.25 (br s, 2H), 2.15 - 1.87 (m, 6H), 1.72 - 1.45 (m, 6H). MS (ESI⁺) : [M+H]+ 379.2. |
| **13** | 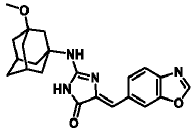 | C₂₂H₂₄N₄O₃ | 392.46 | >98% | Beige solid. ¹H NMR (400 MHz, DMSO-*d*₆, 323K) of major tautomer δ_{H} 9.89 (br s, 1H, NH, D₂O exchanged), 8.75 (s, 1H), 8.67 (s, 1H), 7.88 (d, *J* = 8.4 Hz, 1H), 7.72 (d, *J* = 8.2 Hz, 1H), 7.05 (br s, 1H, NH, D₂O exchanged), 6.45 (s, 1H), 3.19 (s, 3H), 2.31 (br s, 2H), 2.21 (br s, 2H), 2.18 - 1.95 (m, 4H), 1.82 - 1.50 (m, 6H). MS (ESI⁺) : [M+H]+ 393.3. |
| **14** | 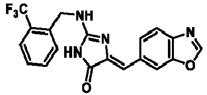 | C₁₉H₁₃F₃N₄O₂ | 386.33 | >98% | Beige solid. ¹H NMR (400 MHz, DMSO-*d*₆, 323K) of major tautomer δ_{H} 10.78 (br s, 1H, NH, D₂O exchanged), 8.67 (br s, 1H), 8.57 (br s, 1H), 8.04 (br s, 1H, NH, D₂O exchanged), 7.87 (br s, 1H), 7.80 ― 7.60 (m, 4H), 7.55 ― 7.42 (m,, 1H), 6.47(s, 1H), 4.82 (s, 2H). MS (ESI⁺) : [M+H]⁺ 387.2. |
| **15** | 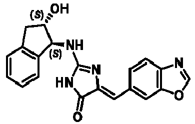 | C₂₀H₁₆N₄O₃ | 360.37 | 93% | Beige solid. ¹H NMR (400 MHz, DMSO-*d*₆, 323K) of major tautomer δ_{H} 10.69 (br s, 1H, NH, D₂O exchanged), 8.67 (s, 1H), 8.58 (br s, 1H), 7.96 (br s, 1H + NH, D₂O exchanged), 7.73 (d, *J* = 8.3 Hz, 1H), 7.35 - 7.15 (m, 4H), 6.50 (s, 1H), 5.45 (br s, 1H, OH, D₂O exchanged), 5.25 (br s, 1H), 4.45 (app q, *J* = 7.1 Hz, 1H), 3.28 - 3.16 (m, 1H), 2.81 (dd, *J* = 15.6, 7.5 Hz, 1H). MS (ESI⁺) : [M+H]+ 361.2. |
| **16** | 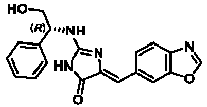 | C₁₉H₁₆N₄O₃ | 348.36 | >98% | Beige solid. ¹H NMR (400 MHz, DMSO-*d*₆, 323K) of major tautomer δ_{H} 10.51 (br s, 1H, NH, D₂O exchanged), 8.68 (s, 1H), 8.57 (br s, 1H), 8.05 ― 7.75 (br s, 1H, NH, D₂O exchanged), 7.91 (d, *J* = 8.4 Hz, 1H), 7.71 (d, *J* = 8.4 Hz, 1H), 7.50 ― 7.42 (m, 2H), 7.40 ― 7.32 (m, 2H), 7.30 ― 7.22 (m, 1H), 6.42 (s, 1H), 5.16 - 4.94 (m, 1H + OH, D₂O exchanged), 3.84 - 3.67 (m, 2H). MS (ESI⁺) : [M+H]+ 349.2. |
| **17** | 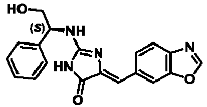 | C₁₉H₁₆N₄O₃ | 348.36 | 96% | Beige solid. ¹H NMR (400 MHz, DMSO-*d*₆, 323K) of major tautomer δ_{H} 10.51 (br s, 1H, NH, D₂O exchanged), 8.68 (s, 1H), 8.57 (br s, 1H), 8.05 ― 7.75 (br s, 1H, NH, D₂O exchanged), 7.91 (d, *J* = 8.4 Hz, 1H), 7.71 (d, *J* = 8.4 Hz, 1H), 7.50 ― 7.42 (m, 2H), 7.40 ― 7.32 (m, 2H), 7.30 ― 7.22 (m, 1H), 6.42 (s, 1H), 5.16 - 4.94 (m, 1H + OH, D₂O exchanged), 3.84 - 3.67 (m, 2H). MS (ESI⁺) : [M+H]+ 349.2. |
| 18 | 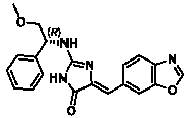 | C₂₀H₁₈N₄O₃ | 362.39 | >98% | Yellow solid. ¹H NMR (400 MHz, DMSO-*d*₆, 373K) of major tautomer δ_{H} 10.18 (br s, 1H, NH, D₂O exchanged), 8.59 (s, 1H), 8.53 (br s, 1H), 7.89 (br s, 1H), 7.75 - 7.52 (m, 1H + NH, D₂O exchanged), 7.51 ― 1.42 (m, 2H), 7.41 ― 7.33 (m, 2H), 1.32 ― 7.23 (m, 1H), 6.45 (s, 1H), 5.27 - 5.17 (m, 1H), 3.80 (dd, *J* = 10.2 and 7.0 Hz, 1H), 3.72 (dd, *J* = 10.2 and 5.2 Hz, 1H), 3.36 (s, 3H). MS (ESI⁺) : [M+H]+ 363.2. |
| **19** | 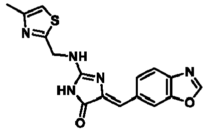 | C₁₆H₁₃N₅O₂S | 339.37 | >98% | Beige solid. ¹H NMR (400 MHz, DMSO-*d*₆, 323K) of major tautomer δ_{H} 10.87 (br s, 1H, NH, D₂O exchanged), 8.69 (br s, 1H), 8.62 (br s, 1H), 8.20 (br s, 1H, NH, D₂O exchanged), 7.96 (d, *J* = 8.4 Hz, 1H), 7.73 (d, *J* = 8.2 Hz, 1H), 7.16 (s, 1H), 6.51 (s, 1H), 4.84 (s, 2H), 2.37 (s, 3H). MS (ESI⁺) : [M+H]+ 340.2. |
| **20** | 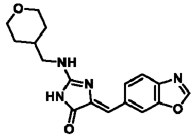 | C₁₇H₁₈N₄O₃ | 326.36 | >98% | Beige solid. ¹H NMR (400 MHz, DMSO-*d*₆, 323K) of major tautomer δ_{H} 10.64 (br s, 1H, NH, D₂O exchanged), 8.67 (br s, 1H), 8.62 (br s, 1H), 7.94 (br s, 1H), 7.73 (d, *J* = 8.2 Hz, 1H), 7.55 (br s, 1H, NH, D₂O exchanged), 6.42 (s, 1H), 3.92 - 3.83 (m, 2H), 3.36 - 3.24 (m, 4H), 1.88 (br s, 1H), 1.70 - 1.57 (m, 2H), 1.40 - 1.16 (m, 2H). MS (ESI⁺) : [M+H]⁺ 327.2. |
| **21** | 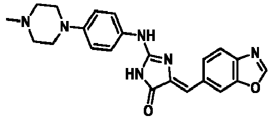 | C₂₂H₂₂N₆O₂ | 402.46 | >98% (¹H NMR) | Brown solid. ¹H NMR (400 MHz, DMSO-*d*₆, 323K) of major tautomer δ_{H} 10.54 (br s, 1H, NH, D₂O exchanged), 9.56 (br s, 1H, NH, D₂O exchanged), 8.71 (s, 1H), 8.61 (s, 1H), 8.03 (d, *J* = 8.3 Hz, 1H), 7.78 (d, *J* = 8.3 Hz, 1H), 7.63 (d, *J* = 8.4 Hz, 2H), 6.99 (d, *J* = 8.6 Hz, 2H), 6.59 (s, 1H), 3.17 (br s, 4H), 2.55 (br s, 4H), 2.29 (s, 3H). MS (ESI⁺) : [M+H]⁺ 403.3. |
| **22** | 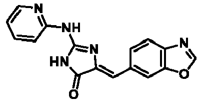 | C₁₆H₁₁N₅O₂ | 305.30 | >95% | Yellow solid. ¹H NMR (400 MHz, DMSO-*d*₆, 343K) of major tautomer δ_{H} 10.89 (br s, 2H, NH, D₂O exchanged), 8.71 (s, 1H), 8.56 (br s, 1H), 8.34 (d, *J* = 5.1 Hz, 1H), 8.02 (br s, 1H), 7.91 - 7.74 (m, 2H), 7.52 (br s, 1H), 7.19 - 6.97 (m, 1H), 6.75 (s, 1H). MS (ESI⁺) : [M+H]+ 306.2. |
| **23** | 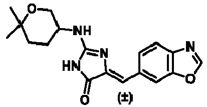 | C₁₈H₂₀N₄O₃ | 340.38 | >98% | Beige solid. ¹H NMR (400 MHz, DMSO-*d*₆, 323K) of major tautomer δ_{H} 10.48 (br s, 1H, NH, D₂O exchanged), 8.68 (s, 1H), 8.59 (s, 1H), 7.94 (s, 1H), 7.74 (d, *J* = 8.2 Hz, 1H), 7.43 (br s, 1H, NH, D₂O exchanged), 6.45 (s, 1H), 3.85 (br s, 1H), 3.78 (br d, *J* = 11.0 Hz, 1H), 3.51 (dd, *J* = 11.3, 7.8 Hz, 1H), 2.00 ― 1.75 (m, 2H), 1.70 - 1.58 (m, 1H), 1.56 - 1.44 (m, 1H), 1.22 (s, 3H), 1.21 (s, 3H). MS (ESI⁺) : [M+H]+ 341.3. |
| **24** | 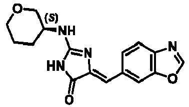 | C₁₆H₁₆N₄O₃ | 312.33 | >98% | Orange solid. ¹H NMR (400 MHz, DMSO-*d*₆, 323K) of major tautomer δ_{H} 10.33 (br s, 1H, NH, D₂O exchanged), 8.68 (s, 1H), 8.59 (s, 1H), 7.94 (s, 1H), 7.74 (d, *J* = 8.2 Hz, 1H), 7.51 (br s, 1H, NH, D₂O exchanged), 6.46 (s, 1H), 4.05 - 3.81 (m, 2H), 3.79 - 3.63 (m, 1H), 3.52 - 3.25 (m, 2H), 2.10 - 1.93 (m, 1H), 1.85 - 1.50 (m, 3H). MS (ESI⁺) : [M+H]+ 313.3. |
| **25** | 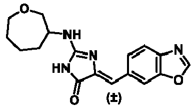 | C₁₇H₁₈N₄O₃ | 326.36 | >98% | Orange solid. ¹H NMR (400 MHz, DMSO-*d*₆, 323K) of major tautomer δ_{H} 10.32 (br s, 1H, NH, D₂O exchanged), 8.67 (s, 1H), 8.62 (s, 1H), 7.95 (d, *J* = 8.4 Hz, 1H), 7.73 (d, *J* = 8.2 Hz, 1H), 7.40 (br s, 1H, NH, D₂O exchanged), 6.44 (s, 1H), 4.12 (br s, 1H), 3.90 - 3.78 (m, 1H), 3.77 - 3.55 (m, 3H), 2.00 - 1.87 (m, 1H), 1.86 - 1.68 (m, 4H), 1.67 - 1.50 (m, 1H). MS (ESI⁺) : [M+H]⁺ 327.2. |
| **26** | 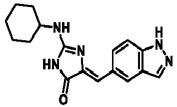 | C₁₇H₁₉N₅O | 309.37 | >98% | Pale yellow solid. ¹H NMR (400 MHz, DMSO-*d*₆, 343K) of major tautomer δ_{H} 13.08 (br s, 1H, NH, D₂O exchanged), 10.51 (br s, 1H, NH, D₂O exchanged), 8.41 (br s, 1H), 8.13 (br s, 1H), 8.06 (s, 1H), 7.49 (d, *J* = 8.6 Hz, 1H), 7.31 (br s, 1H, NH, D₂O exchanged), 6.40 (s, 1H), 3.81 - 3.54 (m, 1H), 2.00 - 1.83 (m, 2H), 1.80 - 1.66 (m, 2H), 1.66 - 1.53 (m, 1H), 1.47 - 1.06 (m, 5H). MS (ESI⁺) : [M+H]+ 310.2. |
| **27** | 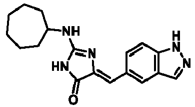 | C₁₈H₂₁N₅O | 323.40 | >98% | Pale yellow solid. ¹H NMR (400 MHz, DMSO-*d*₆, 343K) of major tautomer δ_{H} 12.91 (br s, 1H, NH, D₂O exchanged), 10.03 (br s, 1H, NH, D₂O exchanged), 8.42 (br s, 1H), 8.13 (br s, 1H), 8.03 (s, 1H), 7.49 (d, *J* = 8.6 Hz, 1H), 7.11 (br s, 1H, NH, D₂O exchanged), 6.42 (s, 1H), 4.03 - 3.84 (m, 1H), 2.12 - 1.91 (m, 2H), 1.82 - 1.34 (m, 10H). MS (ESI⁺) : [M+H]⁺ 324.4. |
| **28** | 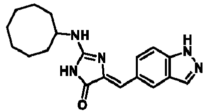 | C₁₉H₂₃N₅O | 337.43 | >98% | Pale yellow solid. ¹H NMR (400 MHz, DMSO-*d*₆, 343K) of major tautomer δ_{H} 12.91 (br s, 1H, NH, D₂O exchanged), 10.03 (br s, 1H, NH, D₂O exchanged), 8.47 (br s, 1H), 8.12 (br s, 1H), 8.02 (s, 1H), 7.48 (d, *J* = 8.7 Hz, 1H), 7.09 (br s, 1H, NH, D₂O exchanged), 6.42 (s, 1H), 4.11 - 3.89 (m, 1H), 1.95 - 1.82 (m, 2H), 1.82 ― 1.41 (m, 12H). MS (ESI⁺) : [M+H]⁺ 338.4. |
| **29** | 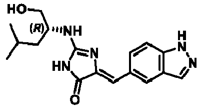 | C₁₇H₂₁N₅O₂ | 327.39 | >98% | Pale yellow solid. ¹H NMR (400 MHz, DMSO-*d*₆, 343K) of major tautomer δ_{H} 12.89 (br s, 1H, NH, D₂O exchanged), 10.17 (br s, 1H, NH, D₂O exchanged), 8.44 (s, 1H), 8.13 (d, *J* = 8.8 Hz, 1H), 8.00 (s, 1H), 7.46 (d, *J* = 8.7 Hz, 1H), 6.82 (br s, 1H, NH, D₂O exchanged), 6.42 (s, 1H), 4.72 - 4.60 (m, 1H, OH, D₂O exchanged), 4.00 (br s, 1H), 3.62 - 3.43 (m, 2H), 1.81 - 1.60 (m, 1H), 1.57 - 1.38 (m, 2H), 1.10 ― 0.81 (m, 6H). MS (ESI⁺) : [M+H]+ 328.4. |
| **30** | 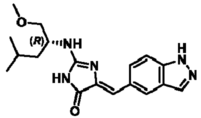 | C₁₈H₂₃N₅O₂ | 341.42 | >98% | Pale yellow solid. ¹H NMR (400 MHz, DMSO-*d*₆, 343K) of major tautomer δ_{H} 12.89 (br s, 1H, NH, D₂O exchanged), 10.20 (br s, 1H, NH, D₂O exchanged), 8.45 (br s, 1H), 8.12 (br s, 1H), 8.01 (s, 1H), 7.61 - 7.32 (m, 1H), 7.00 (br s, 1H, NH, D₂O exchanged), 6.43 (s, 1H), 4.15 (s, 1H), 3.55 - 3.38 (m, 2H), 3.33 (s, 3H), 1.86 - 1.62 (m, 1H), 1.61 - 1.36 (m, 2H), 1.12 - 0.82 (m, 6H). MS (ESI⁺) : [M+H]+ 342.3. |
| **31** | 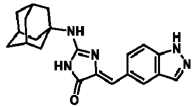 | C₂₁H₂₃N₅O | 361.45 | 97% | Pale yellow solid. ¹H NMR (400 MHz, DMSO-*d*₆, 323K) of major tautomer δ_{H} 12.99 (br s, 1H, NH, D₂O exchanged), 9.72 (br s, 1H, NH, D₂O exchanged), 8.48 (s, 1H), 8.16 (d, *J* = 8.7 Hz, 1H), 7.99 (s, 1H), 7.48 (d, *J* = 8.8 Hz, 1H), 6.64 (br s, 1H, NH, D₂O exchanged), 6.42 (s, 1H), 2.32 - 1.99 (m, 9H), 1.88 - 1.59 (m, 6H). MS (ESI⁺) : [M+H]+ 362.4. |
| **32** | 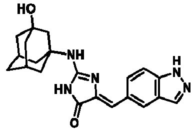 | C₂₁H₂₃N₅O₂ | 377.45 | >98% | Pale yellow solid. ¹H NMR (400 MHz, DMSO-*d*₆, 323K) of major tautomer δ_{H} 12.99 (br s, 1H, NH, D₂O exchanged), 9.73 (br s, 1H, NH, D₂O exchanged), 8.49 (s, 1H), 8.15 (d, *J* = 8.8 Hz, 1H), 8.00 (s, 1H), 7.48 (d, *J* = 8.9 Hz, 1H), 6.73 (br s, 1H, NH, D₂O exchanged), 6.43 (s, 1H), 4.49 (s, 1H, OH, D₂O exchanged), 2.30 - 2.16 (m, 2H), 2.14 - 1.88 (m, 6H), 1.78 - 1.43 (m, 6H). MS (ESI⁺) : [M+H]+ 378.4. |
| **33** | 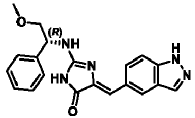 | C₂₀H₁₉N₅O₂ | 361.41 | 97% | Pale yellow solid. ¹H NMR (400 MHz, DMSO-*d*₆, 343K) of major tautomer δ_{H} 12.98 (br s, 1H, NH, D₂O exchanged), 10.42 (br s, 1H, NH, D₂O exchanged), 8.41 (s, 1H), 8.23 - 8.00 (m, 2H), 7.85 (br s, 1H, NH, D₂O exchanged), 7.63 - 7.44 (m, 3H), 7.43 - 7.33 (m, 2H), 7.32 - 7.23 (m, 1H), 6.43 (s, 1H), 5.19 (s, 1H), 3.84 - 3.59 (m, 2H), 3.33 (s, 3H). MS (ESI⁺) : [M+H]+ 362.4. |
| **34** | 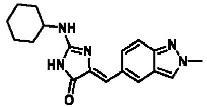 | C₁₈H₂₁N₅O | 323.40 | >98% | Yellow solid. ¹H NMR (400 MHz, DMSO-*d*₆, 373K) of major tautomer δ_{H} 10.24 (br s, 1H, NH, D₂O exchanged), 8.43 - 8.19 (m, 2H), 8.04 (d, *J* = 9.1 Hz, 1H), 7.51 (d, *J* = 9.0 Hz, 1H), 7.10 (br s, 1H, NH, D₂O exchanged), 6.37 (s, 1H), 4.15 (s, 3H), 3.71 (br s, 1H), 1.94 (br s, 2H), 1.84 - 1.68 (m, 2H), 1.66 - 1.52 (m, 1H), 1.47 - 1.13 (m, 5H). MS (ESI⁺) : [M+H]⁺ 324.3. |
| **35** | 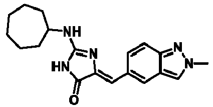 | C₁₉H₂₃N₅O | 337.43 | 98% | Pale yellow solid. ¹H NMR (400 MHz, DMSO-*d*₆, 343K) of major tautomer δ_{H} 10.15 (br s, 1H, NH, D₂O exchanged), 8.32 (s, 1H), 8.28 (s, 1H), 8.16 ― 7.96 (m, 1H), 7.67 ― 7.45 (m, 1H), 7.09 (br s, 1H, NH, D₂O exchanged), 6.37 (s, 1H), 4.16 (s, 3H), 3.94 (s, 1H), 1.96 (q, *J* = 6.9, 5.0 Hz, 2H), 1.82 - 1.39 (m, 10H). MS (ESI⁺) : [M+H]+ 338.4. |
| **36** | 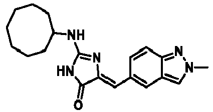 | C₂₀H₂₅N₅O | 351.45 | >98% | Pale yellow solid. ¹H NMR (400 MHz, DMSO-*d*₆, 343K) of major tautomer δ_{H} 10.11 (br s, 1H, NH, D₂O exchanged), 8.35 (s, 1H), 8.26 (s, 1H), 8.13 ― 7.99 (m, 1H), 7.67 ― 7.44 (m, 1H), 7.07 (br s, 1H, NH, D₂O exchanged), 6.37 (s, 1H), 4.16 (s, 3H), 4.07 - 3.91 (m, 1H), 1.96 - 1.81 (m, 2H), 1.81 - 1.42 (m, 12H). MS (ESI⁺) : [M+H]+ 352.4. |
| **37** | 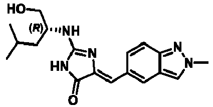 | C₁₈H₂₃N₅O₂ | 341.42 | >98% | Pale yellow solid. ¹H NMR (400 MHz, DMSO-*d*₆, 343K) of major tautomer δ_{H} 10.11 (br s, 1H, NH, D₂O exchanged), 8.58 - 8.17 (m, 2H), 8.03 (br s, 1H), 7.52 (d, *J* = 8.8 Hz, 1H), 6.87 (br s, 1H, NH, D₂O exchanged), 6.37 (s, 1H), 4.68 (br s, 1H, OH, D₂O exchanged), 4.16 (s, 3H), 3.98 (br s, 1H), 3.51 (d, *J* = 5.0 Hz, 2H), 1.82 - 1.63 (m, 1H), 1.59 - 1.37 (m, 2H), 1.12 - 0.79 (m, 6H). MS (ESI⁺) : [M+H]+342.4. |
| **38** | 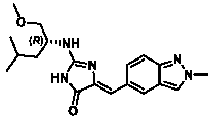 | C₁₉H₂₅N₅O₂ | 355.44 | >98% | Pale yellow solid. ¹H NMR (400 MHz, DMSO-*d*₆, 343K) of major tautomer δ_{H} 10.19 (br s, 1H, NH, D₂O exchanged), 8.33 (s, 1H), 8.25 (s, 1H), 8.04 (d, *J* = 9.1 Hz, 1H), 7.50 (d, *J* = 8.9 Hz, 1H), 6.97 (br s, 1H, NH, D₂O exchanged), 6.38 (s, 1H), 4.15 (s, 4H), 3.52 ― 3.38 (m, 2H), 3.32 (s, 3H), 1.80 ― 1.63 (m, 1H), 1.59 ― 1.47 (m, 1H), 1.46 - 1.36 (m, 1H), 0.96 (d, *J* = 6.5 Hz, 6H). MS (ESI⁺) [M+H]+ 356.4. : |
| **39** | 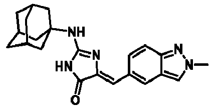 | C₂₂H₂₅N₅O | 375.48 | >98% | Pale yellow solid. ¹H NMR (400 MHz, DMSO-*d*₆, 323K) of major tautomer δ_{H} 9.70 (br s, 1H, NH, D₂O exchanged), 8.38 (s, 1H), 8.28 (s, 1H), 8.04 (d, *J* = 9.1 Hz, 1H), 7.51 (d, *J* = 9.0 Hz, 1H). 6.63 (br s, 1H, NH, D₂O exchanged), 6.37 (s, 1H), 4.15 (s, 3H), 2.33 - 1.96 (m, 9H), 1.71 (d, *J* = 23.1 Hz, 6H). MS (ESI⁺) [M+H]+ 376.4. : |
| **40** | 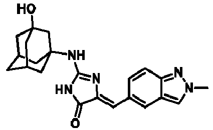 | C₂₂H₂₅N₅O₂ | 391.48 | >98% | Pale yellow solid. ¹H NMR (400 MHz, DMSO-*d*₆, 343K) of major tautomer δ_{H} 9.63 (br s, 1H, NH, D₂O exchanged), 8.37 (s, 1H), 8.24 (s, 1H), 8.04 (d, *J* = 9.1 Hz, 1H), 7.51 (d, *J* = 9.1 Hz, 1H), 6.58 (br s, 1H, NH, D₂O exchanged), 6.38 (s, 1H), 4.37 (s, 1H, OH, D₂O exchanged), 4.16 (s, 3H), 2.32 - 2.17 (m, 2H), 2.15 ― 1.86 (m, 6H), 1.76 - 1.42 (m, 6H). MS (ESI⁺) : [M+H]+ 392.4. |
| **41** | 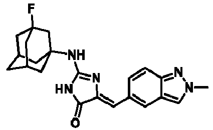 | C₂₂H₂₄FN₅O | 393.47 | >98% | Yellow solid. ¹H NMR (400 MHz, DMSO-*d*₆, 300K) of major tautomer δ_{H} 9.96 (br s, 1H, NH, D₂O exchanged), 8.37 (s, 1H), 8.31 (s, 1H), 8.04 (d, *J* = 9.1 Hz, 1H), 7.52 (d, *J* = 9.1 Hz, 1H), 7.07 (br s, 1H, NH, D₂O exchanged), 6.39 (s, 1H), 4.15 (s, 3H), 2.46 - 2.27 (m, 4H), 2.27 - 1.97 (m, 4H), 1.96 - 1.77 (m, 4H), 1.67 - 1.46 (m, 2H). MS (ESI⁺) : [M+H]⁺ 394.4. |
| **42** | 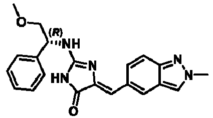 | C₂₁H₂₁N₅O₂ | 375.43 | >98% | Yellow solid. ¹H NMR (400 MHz, DMSO-*d*₆, 343K) of major tautomer δ_{H} 10.27 (br s, 1H, NH, D₂O exchanged), 8.31 - 8.24 (m, 2H), 8.00 (d, *J* = 9.0 Hz, 1H), 7.68 (br s, 1H, NH, D₂O exchanged), 7.56 - 7.45 (m, 3H), 7.45 - 7.34 (m, 2H), 7.34 - 7.23 (m, 1H), 6.39 (s, 1H), 5.27 - 5.11 (m, 1H), 4.16 (s, 3H), 3.83 - 3.61 (m, 2H), 3.34 (s, 3H). MS (ESI⁺) : [M+H]+ 376.3. |
| **43** | 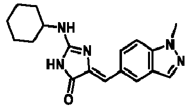 | C₁₈H₂₁N₅O | 323.40 | >98% | Colorless solid. ¹H NMR (400 MHz, DMSO-*d*₆, 373K) of major tautomer δ_{H} 10.19 (br s, 1H, NH, D₂O exchanged), 8.37 (br s, 1H), 8.18 (br s, 1H), 8.01 (s, 1H), 7.58 (d, *J* = 8.7 Hz, 1H), 7.13 (br s, 1H, NH, D₂O exchanged), 6.42 (s, 1H), 4.04 (s, 3H), 3.72 (br s, 1H), 2.03 - 1.85 (m, 2H), 1.82 - 1.68 (m, 2H), 1.66 - 1.56 (m, 1H), 1.48 - 1.29 (m, 4H), 1.29 - 1.15 (m, 1H). MS (ESI⁺) : [M+H]+ 324.3. |
| **44** | 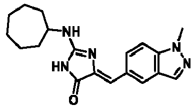 | C₁₉H₂₃N₅O | 337.43 | >98% | Offwhite solid. ¹H NMR (400 MHz, DMSO-*d*₆, 323K) of major tautomer δ_{H} 10.30 (br s, 1H, NH, D₂O exchanged), 8.40 (br s, 1H), 8.23 (d, *J* = 8.9 Hz, 1H), 8.01 (br s, 1H), 7.59 (d, *J* = 8.8 Hz, 1H), 7.26 (br s, 1H, NH, D₂O exchanged), 6.42 (br s, 1H), 4.04 (s, 3H), 3.93 (br s, 1H), 2.05 ― 1.95 (m, 2H), 1.75 ― 1.40 (m, 10H). MS (ESI⁺) : [M+H]+ 338.3. |
| **45** | 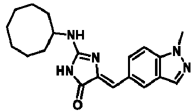 | C₂₀H₂₅N₅O | 351.45 | 98% | Offwhite solid. ¹H NMR (400 MHz, DMSO-*d*₆, 323K) of major tautomer δ_{H} 10.25 (br s, 1H, NH, D₂O exchanged), 8.43 (br s, 1H), 8.22 (d, *J* = 8.8 Hz, 1H), 8.03 (br s, 1H), 7.58 (d, *J* = 8.7 Hz, 1H), 7.25 (br s, 1H, NH, D₂O exchanged), 6.42 (br s, 1H), 4.04 (s, 3H), 3.99 (br s, 1H), 1.95 ― 1.81 (m, 2H), 1.79 ― 1.40 (m, 12H). MS (ESI⁺) : [M+H]+ 352.3. |
| **46** | 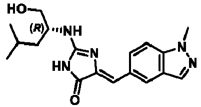 | C₁₈H₂₃N₅O₂ | 341.42 | >98% | Beige solid. ¹H NMR (400 MHz, DMSO-*d*₆, 323K) of major tautomer δ_{H} 10.27 (br s, 1H, NH, D₂O exchanged), 8.42 (br s, 1H), 8.20 (d, *J* = 8.8 Hz, 1H), 7.98 (br s, 1H), 7.56 (d, *J* = 8.8 Hz, 1H), 6.99 (br s, 1H, NH, D₂O exchanged), 6.42 (br s, 1H), 4.76 (br s, 1H, OH, D₂O exchanged), 4.04 (s, 3H), 4.04 - 3.90 (m, 1H), 3.55 - 3.45 (br s, 2H), 1.80 - 1.65 (m, 1H), 1.58 - 1.35 (m, 2H), 1.05 - 0.85 (m, 6H). MS (ESI⁺) : [M+H]⁺ 342.3. |
| **47** | 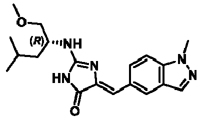 | C₁₉H₂₅N₅O₂ | 355.44 | >98% | Beige solid. ¹H NMR (400 MHz, DMSO-*d*₆, 323K) of major tautomer δ_{H} 10.33 (br s, 1H, NH, D₂O exchanged), 8.42 (br s, 1H), 8.21 (d, *J* = 8.8 Hz, 1H), 7.99 (br s, 1H), 7.57 (d, *J* = 8.8 Hz, 1H), 7.25 (br s, 1H, NH, D₂O exchanged), 6.43 (br s, 1H), 4.16 (br s, 1H), 4.04 (s, 3H), 3.50 - 3.35 (m, 2H), 3.32 (s, 3H), 1.80 - 1.61 (m, 1H), 1.58 - 1.45 (m, 1H), 1.45 - 1.32 (m, 1H), 1.02 - 0.85 (m, 6H). MS (ESI⁺) : [M+H]+ 356.3. |
| **48** | 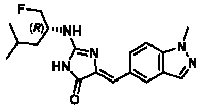 | C₁₈H₂₂FN₅O | 343.41 | 97% | Beige solid. ¹H NMR (400 MHz, DMSO-*d*₆, 323K) of major tautomer δ_{H} 10.49 (br s, 1H, NH, D₂O exchanged), 8.41 (br s, 1H), 8.21 (d, *J* = 8.9 Hz, 1H), 8.00 (br s, 1H), 7.58 (d, *J* = 8.8 Hz, 1H), 7.36 (br s, 1H, NH, D₂O exchanged), 6.47 (br s, 1H), 4.65 - 4.50 (m, 1H), 4.50 - 4.37 (m, 1H), 4.35 - 4.10 (m, 1H), 4.04 (s, 3H), 1.80 - 1.65 (m, 1H), 1.63 - 1.50 (m, 1H), 1.45 - 1.33 (m, 1H), 1.02 - 0.91 (m, 6H). MS (ESI⁺) : [M+H]⁺ 344.2. |
| **49** | 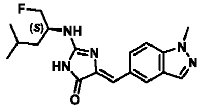 | C₁₈H₂₂FN₅O | 343.41 | 97% | Beige solid. ¹H NMR (400 MHz, DMSO-*d*₆, 323K) of major tautomer δ_{H} 10.49 (br s, 1H, NH, D₂O exchanged), 8.41 (br s, 1H), 8.21 (d, *J* = 8.9 Hz, 1H), 8.00 (br s, 1H), 7.58 (d, *J* = 8.8 Hz, 1H), 7.36 (br s, 1H, NH, D₂O exchanged), 6.47 (br s, 1H), 4.65 - 4.50 (m, 1H), 4.50 - 4.37 (m, 1H), 4.35 - 4.10 (m, 1H), 4.04 (s, 3H), 1.80 - 1.65 (m, 1H), 1.63 - 1.50 (m, 1H), 1.45 - 1.33 (m, 1H), 1.02 - 0.91 (m, 6H). MS (ESI⁺) : [M+H]⁺ 344.3. |
| **50** | 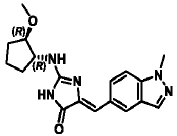 | C₁₈H₂₁N₅O₂ | 339.40 | 98% | Yellow solid. ¹H NMR (400 MHz, DMSO-*d*₆, 323K) of major tautomer δ_{H} 10.35 (br s, 1H, NH, D₂O exchanged), 8.44 (s, 1H), 8.20 (d, *J* = 8.8 Hz, 1H), 8.00 (s, 1H), 7.57 (d, *J* = 8.8 Hz, 1H), 7.41 (br s, 1H, NH, D₂O exchanged), 6.45 (br s, 1H), 4.14 (br s, 1H), 4.04 (s, 3H), 3.83 - 3.70 (m, 1H), 3.38 (s, 3H), 2.15 - 2.00 (m, 1H), 1.99 - 1.84 (m, 1H), 1.80 - 1.50 (m, 4H). MS (ESI⁺) : [M+H]+ 340.3. |
| **51** | 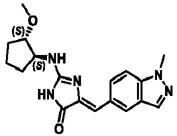 | C₁₈H₂₁N₅O₂ | 339.40 | >98% | Yellow solid. ¹H NMR (400 MHz, DMSO-*d*₆, 323K) of major tautomer δ_{H} 10.35 (br s, 1H, NH, D₂O exchanged), 8.44 (s, 1H), 8.20 (d, *J* = 8.8 Hz, 1H), 8.00 (s, 1H), 7.57 (d, *J* = 8.8 Hz, 1H), 7.41 (br s, 1H, NH, D₂O exchanged), 6.45 (br s, 1H), 4.14 (br s, 1H), 4.04 (s, 3H), 3.83 - 3.70 (m, 1H), 3.38 (s, 3H), 2.15 - 2.00 (m, 1H), 1.99 - 1.84 (m, 1H), 1.80 - 1.50 (m, 4H). MS (ESI⁺) : [M+H]+ 340.3. |
| **52** | 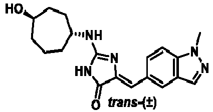 | C₁₉H₂₃N₅O₂ | 353.43 | >98% | Yellow solid. ¹H NMR (400 MHz, DMSO-*d*₆, 323K) of major tautomer δ_{H} 10.34 (br s, 1H, NH, D₂O exchanged), 8.41 (s, 1H), 8.22 (d, *J* = 8.8 Hz, 1H), 8.01 (s, 1H), 7.58 (d, *J* = 8.8 Hz, 1H), 7.25 (br s, 1H, NH, D₂O exchanged), 6.42 (s, 1H), 4.33 (d, *J* = 4.0 Hz, 1H, OH, D₂O exchanged), 4.04 (s, 3H), 3.93 (br s, 1H), 3.70 (br s, 1H), 2.07 - 1.72 (m, 4H), 1.66 - 1.40 (m, 6H). MS (ESI⁺) : [M+H]+ 354.3. |
| **53** | 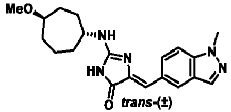 | C₂₀H₂₅N₅O₂ | 367.45 | >98% | Beige solid. ¹H NMR (400 MHz, DMSO-*d*₆, 323K) of major tautomer δ_{H} 10.35 (br s, 1H, NH, D₂O exchanged), 8.39 (s, 1H), 8.21 (d, *J* = 8.9 Hz, 1H), 8.00 (s, 1H), 7.58 (d, *J* = 8.8 Hz, 1H), 7.27 (br s, 1H, NH, D₂O exchanged), 6.42 (s, 1H), 4.03 (s, 3H), 3.92 (s, 1H), 3.39 - 3.34 (m, 1H), 3.24 (s, 3H), 2.06 - 1.87 (m, 3H), 1.85 - 1.72 (m, 1H), 1.71 - 1.43 (m, 6H). MS (ESI⁺) : [M+H]+ 368.3. |
| **54** | 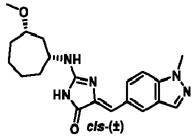 | C₂₀H₂₅N₅O₂ | 367.45 | >98% | Beige solid. ¹H NMR (400 MHz, DMSO-*d*₆, 323K) of major tautomer δ_{H} 10.38 (br s, 1H, NH, D₂O exchanged), 8.43 (br s, 1H), 8.23 (d, *J* = 8.9 Hz, 1H), 8.01 (s, 1H), 7.58 (d, *J* = 8.8 Hz, 1H), 7.24 (br s, 1H, NH, D₂O exchanged), 6.44 (s, 1H), 4.04 (s, 3H), 3.92 (br s, 1H), 3.55 - 3.36 (m, 1H), 3.26 (s, 3H), 2.40 - 2.20 (m, 1H), 2.00 - 1.85 (m, 2H), 1.77 - 1.40 (m, 7H). MS (ESI⁺): [M+H]⁺ 368.3. |
| **55** | 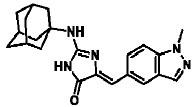 | C₂₂H₂₅N₅O | 375.48 | >98% | Yellow solid. ¹H NMR (400 MHz, DMSO-*d*₆, 323K) of major tautomer δ_{H} 9.72 (br s, 1H, NH, D₂O exchanged), 8.38 (s, 1H), 8.30 (d, *J* = 8.8 Hz, 1H), 7.97 (s, 1H), 7.60 (d, *J* = 8.8 Hz, 1H), 6.65 (br s, 1H, NH, D₂O exchanged), 6.43 (s, 1H), 4.04 (s, 3H), 2.26 - 2.00 (m, 9H), 1.81 - 1.61 (m, 6H). MS (ESI⁺) : [M+H]⁺ 376.3. |
| **56** | 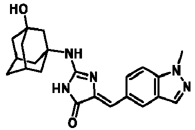 | C₂₂H₂₅N₅O₂ | 391.48 | >98% | Pale yellow solid. ¹H NMR (400 MHz, DMSO-*d*₆, 323K) of major tautomer δ_{H} 9.73 (br s, 1H, NH, D₂O exchanged), 8.41 (br s, 1H), 8.28 (d, *J* = 9.0 Hz, 1H), 7.98 (s, 1H), 7.60 (d, *J* = 8.8 Hz, 1H), 6.73 (br s, 1H, NH, D₂O exchanged), 6.44 (s, 1H), 4.49 (s, 1H, OH, D₂O exchanged), 4.05 (s, 3H), 2.26 (br s, 2H), 2.15 - 1.90 (m, 6H), 1.75 - 1.40 (m, 6H). MS (ESI⁺) : [M+H]+ 392.2. |
| **57** | 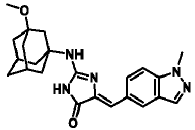 | C₂₃H₂₇N₅O₂ | 405.50 | >98% | Yellow solid. ¹H NMR (400 MHz, DMSO-*d*₆, 323K) of major tautomer δ_{H} 9.75 (br s, 1H, NH, D₂O exchanged), 8.41 (br s, 1H), 8.27 (d, *J* = 8.8 Hz, 1H), 7.96 (s, 1H), 7.59 (d, *J* = 8.8 Hz, 1H), 6.83 (br s, 1H, NH, D₂O exchanged), 6.45 (s, 1H), 4.05 (s, 3H), 3.19 (s, 3H), 2.32 (br s, 2H), 2.22 - 1.90 (m, 6H), 1.80 - 1.45 (m, 6H). MS (ESI⁺) : [M+H]+ 406.4. |
| **58** | 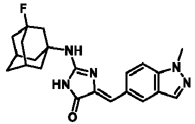 | C₂₂H₂₄FN₅O | 393.47 | >98% | Beige solid. ¹H NMR (400 MHz, DMSO-*d*₆, 323K) of major tautomer δ_{H} 9.84 (br s, 1H, NH, D₂O exchanged), 8.37 (br s, 1H), 8.27 (d, *J* = 8.9 Hz, 1H), 7.98 (s, 1H), 7. 61 (d, *J* = 8.8 Hz, 1H), 6.92 (br s, 1H, NH, D₂O exchanged), 6.47 (s, 1H), 4.05 (s, 3H), 2.45 - 2.25 (m, 4H), 2.20 - 2.10 (m, 2H), 2.10 - 2.00 (m, 2H), 2.00 - 1.80 (m, 4H), 1.70 - 1.50 (m, 2H). MS (ESI⁺) : [M+H]⁺ 394.2. |
| **59** | 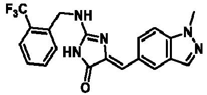 | C₂₀H₁₆F₃N₅O | 399.38 | >98% | Yellow solid. ¹H NMR (400 MHz, DMSO-*d*₆, 323K) of major tautomer δ_{H} 10.66 (br s, 1H, NH, D₂O exchanged), 8.39 (br s, 1H), 8.23 (d, *J* = 8.6 Hz, 1H), 7.95 (s, 1H), 7.85 (br s, 1H, NH, D₂O exchanged), 7.77 (d, *J* = 7.8 Hz, 1H), 7.74 - 7.62 (m, 2H), 7.56 - 7.42 (m, 2H), 6.48 (s, 1H), 4.81 (s, 2H), 4.03 (s, 3H). MS (ESI⁺) : [M+H]⁺ 400.2. |
| **60** | 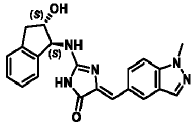 | C₂₁H₁₉N₅O₂ | 373.42 | >98% | Beige solid. ¹H NMR (400 MHz, DMSO-*d*₆, 323K) of major tautomer δ_{H} 10.53 (br s, 1H, NH, D₂O exchanged), 8.36 (br s, 1H), 8.21 (br s, 1H), 8.01 (s, 1H), 7.79 (br s, 1H, NH, D₂O exchanged), 7.58 (d, *J* = 8.7 Hz, 1H), 7.35 - 7.15 (m, 4H), 6.50 (s, 1H), 5.48 (br s, 1H, OH, D₂O exchanged), 5.21 (br s, 1H), 4.45 (app q, *J* = 7.1 Hz, 1H), 4.03 (s, 3H), 3.27 - 3.18 (m, 1H), 2.79 (dd, *J* = 15.6, 7.4 Hz, 1H). MS (ESI⁺) : [M+H]⁺ 374.3. |
| **61** | 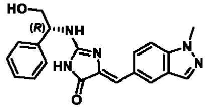 | C₂₀H₁₉N₅O₂ | 361.41 | >98% | Beige solid. ¹H NMR (400 MHz, DMSO-*d*₆, 323K) of major tautomer δ_{H} 10.39 (br s, 1H, NH, D₂O exchanged), 8.38 (br s, 1H), 8.13 (br s, 1H), 8.04 (s, 1H), 7.72 (br s, 1H, NH, D₂O exchanged), 7.58 (d, *J* = 8.6 Hz, 1H), 7.50 - 7.40 (m, 2H), 7.37 (t, *J* = 7.5 Hz, 2H), 7.30 ― 7.20 (m, 1H), 6.43 (s, 1H), 5.02 (br s, 1H + OH, D₂O exchanged), 4.04 (s, 3H), 3.76 (d, *J* = 6.0 Hz, 2H). MS (ESI⁺) : [M+H]+ 362.2. |
| **62** | 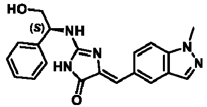 | C₂₀H₁₉N₅O₂ | 361.41 | >98% | Beige solid. ¹H NMR (400 MHz, DMSO-*d*₆, 323K) of major tautomer δ_{H} 10.39 (br s, 1H, NH, D₂O exchanged), 8.38 (br s, 1H), 8.13 (br s, 1H), 8.04 (s, 1H), 7.72 (br s, 1H, NH, D₂O exchanged), 7.58 (d, *J* = 8.6 Hz, 1H), 7.50 - 7.40 (m, 2H), 7.37 (t, *J* = 7.5 Hz, 2H), 7.30 ― 7.20 (m, 1H), 6.43 (s, 1H), 5.02 (br s, 1H + OH, D₂O exchanged), 4.04 (s, 3H), 3.76 (d, *J* = 6.0 Hz, 2H). MS (ESI⁺) : [M+H]+ 362.2. |
| **63** | 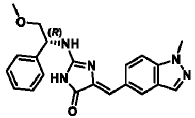 | C₂₁H₂₁N₅O₂ | 375.43 | >98% | Yellow solid. ¹H NMR (400 MHz, DMSO-*d*₆, 323K) of major tautomer δ_{H} 10.31 (br s, 1H, NH, D₂O exchanged), 8.39 (br s, 1H), 8.15 (d, *J* = 8.8 Hz, 1H), 8.04 (s, 1H), 7.87 (br s, 1H, NH, D₂O exchanged), 7.57 (d, *J* = 8.8 Hz, 1H), 7.53 - 7.42 (m, 2H), 7.38 (t, *J* = 7.5 Hz, 2H), 7.32 - 7.22 (m, 1H), 6.44 (s, 1H), 5.20 (br s, 1H), 4.04 (s, 3H), 3.80 - 3.71 (m, 1H), 3.69 - 3.60 (m, 1H), 3.34 (s, 3H). MS (ESI⁺) : [M+H]⁺ 376.3. |
| **64** | 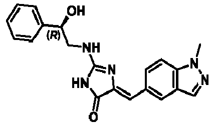 | C₂₀H₁₉N₅O₂ | 361.41 | 95% | Yellow solid. ¹H NMR (400 MHz, DMSO-*d*₆, 373K) of major tautomer δ_{H} 10.08 (br s, 1H, NH, D₂O exchanged), 8.40 (s, 1H), 8.18 (br s, 1H), 8.00 (s, 1H), 7.57 (d, *J* = 8.6 Hz, 1H), 7.51 - 7.42 (m, 2H), 7.38 (t, *J* = 7.6 Hz, 2H), 7.32 ― 7.24 (m, 1H), 6.89 (br s, 1H, NH, D₂O exchanged), 6.48 (br s, 1H), 5.42 (br s, 1H, OH, D₂O exchanged), 4.92 (s, 1H), 4.05 (s, 3H), 3.71 (dd, *J* = 13.4, 4.1 Hz, 1H), 3.51 (dd, *J* = 13.4, 7.6 Hz, 1H). MS (ESI⁺) : [M+H]+ 362.2. |
| **65** | 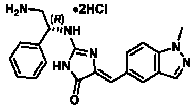 | C₂₀H₂₂Cl₂N₆O | 433.34 | 95% | Yellow foam. ¹H NMR (400 MHz, D₂O, 323K) of major tautomer δ_{H} 7.89 (br s, 1H), 7.86 - 7.70 (m, 5H), 7.54 (br s, 1H), 7.34 - 7.26 (m, 2H), 6.51 (s, 1H), 5.48 - 5.40 (m, 1H), 3.97 (s, 3H), 3.88 - 3.72 (m, 2H), MS (ESI⁺) : [M+H]⁺ 361.3 (+2HCl). |
| **66** | 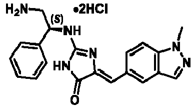 | C₂₀H₂₂Cl₂N₆O | 433.34 | 94% | Yellow foam. ¹H NMR (400 MHz, D₂O, 323K) of major tautomer δ_{H} 7.89 (br s, 1H), 7.86 - 7.70 (m, 5H), 7.54 (br s, 1H), 7.34 - 7.26 (m, 2H), 6.51 (s, 1H), 5.48 - 5.40 (m, 1H), 3.97 (s, 3H), 3.88 - 3.72 (m, 2H), MS (ESI⁺) : [M+H]⁺ 361.3 (+2HCl). |
| **67** | 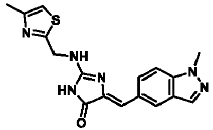 | C₁₇H₁₆N₆OS | 352.42 | >98% | Yellow solid. ¹H NMR (400 MHz, DMSO-*d*₆, 323K) of major tautomer δ_{H} 10.74 (br s, 1H, NH, D₂O exchanged), 8.42 (br s, 1H), 8.22 (d, *J* = 8.8 Hz, 1H), 8.03 (br s, 1H + NH, D₂O exchanged), 8.02 (br s, 1H), 7.58 (d, *J* = 8.8 Hz, 1H), 7.16 (s, 1H), 6.52 (s, 1H), 4.84 (s, 2H), 4.04 (s, 3H), 2.37 (s, 3H). MS (ESI⁺) : [M+H]+ 353.2. |
| **68** | 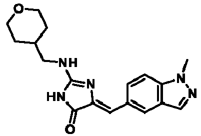 | C₁₈H₂₁N₅O₂ | 339.40 | >98% | Beige solid. ¹H NMR (400 MHz, DMSO-*d*₆, 323K) of major tautomer δ_{H} 10.53 (br s, 1H, NH, D₂O exchanged), 8.37 (br s, 1H), 8.19 (br s, 1H), 8.03 (s, 1H), 7.60 (d, *J* = 8.8 Hz, 1H), 7.40 (br s, 1H, NH, D₂O exchanged), 6.43 (s, 1H), 4.04 (s, 3H), 3.93 - 3.80 (m, 2H), 3.40 - 3.10 (m, 4H), 1.86 (br s, 1H), 1.71 - 1.59 (m, 2H), 1.34 - 1.19 (m, 2H). MS (ESI⁺) : [M+H]+ 340.2. |
| **69** | 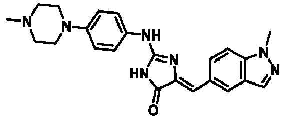 | C₂₃H₂₅N₇O | 415.50 | >98% | Dark yellow solid. ¹H NMR (400 MHz, DMSO-*d*₆, 323K) of major tautomer δ_{H} 10.57 (br s, 1H, NH, D₂O exchanged), 9.56 (br s, 1H, NH, D₂O exchanged), 8.47 (s, 1H), 8.28 (d, *J* = 8.8 Hz, 1H), 8.14 (s, 1H), 7.76 ― 7.55 (m, 3H), 7.02 (d, *J* = 8.7 Hz, 2H), 6.60 (s, 1H), 4.06 (s, 3H), 3.17 - 3.10 (m, 4H), 2.50 - 2.46 (m, 4H), 2.24 (s, 3H). MS (ESI⁺) : [M+H]⁺ 416.2. |
| **70** | 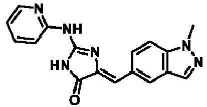 | C₁₇H₁₄N₆O | 318.34 | >98% | Yellow solid. ¹H NMR (400 MHz, DMSO-*d*₆, 373K) of major tautomer δ_{H} 10.62 (br s, 2H, NH, D₂O exchanged), 8.40 (br s, 1H), 8.35 (d, *J* = 5.1 Hz, 1H), 8.08 (s, 1H), 8.07 (br s, 1H), 7.83 (t, *J* = 7.9 Hz, 1H), 7.65 (d, *J* = 8.9 Hz, 1H), 7.54 (br s, 1H), 7.10 - 7.04 (m, 1H), 6.75 (s, 1H), 4.07 (s, 3H). MS (ESI⁺) : [M+H]⁺ 319.2. |
| **71** | 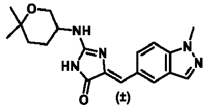 | C₁₉H₂₃N₅O₂ | 353.43 | >98% | Beige solid. ¹H NMR (400 MHz, DMSO-*d*₆, 323K) of major tautomer δ_{H} 10.38 (br s, 1H, NH, D₂O exchanged), 8.36 (br s, 1H), 8.23 (d, *J* = 8.8 Hz, 1H), 8.03 (s, 1H), 7.60 (d, *J* = 8.8 Hz, 1H), 7.23 (br s, 1H, NH, D₂O exchanged), 6.45 (s, 1H), 4.04 (s, 3H), 3.83 (br s, 1H), 3.76 (dd, *J* = 11.4, 4.0 Hz, 1H), 3.51 (dd, *J* = 11.4, 7.8 Hz, 1H), 1.96 - 1.74 (m, 2H), 1.70 - 1.58 (m, 1H), 1.56 - 1.42 (m, 1H), 1.23 (s, 3H), 1.21 (s, 3H). MS (ESI⁺) : [M+H]+ 354.3. |
| **72** | 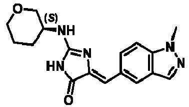 | C₁₇H₁₉N₅O₂ | 325.37 | >98% | Beige solid. ¹H NMR (400 MHz, DMSO-*d*₆, 323K) of major tautomer δ_{H} 10.33 (br s, 1H, NH, D₂O exchanged), 8.36 (br s, 1H), 8.22 (br s, 1H), 8.04 (s, 1H), 7.61 (d, *J* = 8.7 Hz, 1H), 7.31 (br s, 1H, NH, D₂O exchanged), 6.46 (s, 1H), 4.04 (s, 3H), 3.97 - 3.82 (m, 2H), 3.79 - 3.65 (m, 1H), 3.52 - 3.25 (m, 2H), 2.08 - 1.90 (m, 1H), 1.80 - 1.50 (m, 3H). MS (ESI⁺) : [M+H]+ 326.3. |
| **73** | 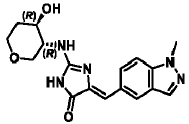 | C₁₇H₁₉N₅O₃ | 341.37 | >98% | Beige solid. ¹H NMR (400 MHz, DMSO-*d*₆, 323K) of major tautomer δ_{H} 10.37 (br s, 1H, NH, D₂O exchanged), 8.37 (br s, 1H), 8.23 (d, *J* = 8.8 Hz, 1H), 8.03 (s, 1H), 7.61 (d, *J* = 8.7 Hz, 1H), 7.23 (br s, 1H, NH, D₂O exchanged), 6.46 (s, 1H), 5.02 (br s, 1H, OH, D₂O exchanged), 4.04 (s, 3H), 4.04 - 3.95 (m, 1H), 3.90 - 3.80 (m, 1H), 3.75 - 3.58 (br s, 2H), 3.50 - 3.33 (m, 1H), 3.30 - 3.15 (m, 1H), 2.00 - 1.87 (m, 1H), 1.62 - 1.45 (m, 1H). MS (ESI⁺) : [M+H]+ 342.3. |
| **74** | 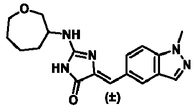 | C₁₈H₂₁N₅O₂ | 339.40 | 97% | Beige solid. ¹H NMR (400 MHz, DMSO-*d*₆, 323K) of major tautomer δ_{H} 10.21 (br s, 1H, NH, D₂O exchanged), 8.37 (br s, 1H), 8.22 (br s, 1H), 8.03 (s, 1H), 7.60 (d, *J* = 8.6 Hz, 1H), 7.20 (br s, 1H, NH, D₂O exchanged), 6.45 (s, 1H), 4.11 (br s, 1H), 4.04 (s, 3H), 3.90 - 3.55 (m, 4H). 2.00 - 1.50 (m, 6H). MS (ESI⁺) : [M+H]+ 340.2. |
| **75** | 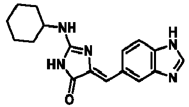 | C₁₇H₁₉N₅O | 309.37 | >98% | Pale yellow solid. ¹H NMR (400 MHz, DMSO-*d*₆, 343K) of major tautomer δ_{H} 12.28 (br s, 1H, NH, D₂O exchanged), 10.21 (br s, 1H, NH, D₂O exchanged), 8.32 (br s, 1H), 8.16 (s, 1H), 7.77 (br s, 1H), 7.54 (d, *J* = 8.3 Hz, 1H), 7.08 (br s, 1H, NH, D₂O exchanged), 6.42 (s, 1H), 3.73 (br s, 1H), 2.09 - 1.83 (m, 2H), 1.80 - 1.49 (m, 3H), 1.47 - 1.12 (m, 5H). MS (ESI⁺) : [M+H]⁺ 310.2. |
| **76** | 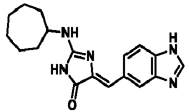 | C₁₈H₂₁N₅O | 323.4 | >98% | Pale yellow solid. ¹H NMR (400 MHz, DMSO-*d*₆, 343K) of major tautomer δ_{H} 12.28 (br s, 1H, NH, D₂O exchanged), 10.13 (br s, 1H, NH, D₂O exchanged), 8.35 (br s, 1H), 8.16 (s, 1H), 7.81 (br s, 1H), 7.54 (d, *J* = 8.2 Hz, 1H), 7.07 (br s, 1H, NH, D₂O exchanged), 6.43 (s, 1H), 3.96 (br s, 1H), 2.15 - 1.81 (m, 2H), 1.77 - 1.33 (m, 10H). MS (ESI⁺) : [M+H]+ 324.3. |
| **77** | 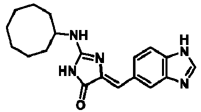 | C₁₉H₂₃N₅O | 337.43 | >98% | Pale yellow solid. ¹H NMR (400 MHz, DMSO-*d*₆, 343K) of major tautomer δ_{H} 12.34 (br s, 1H, NH, D₂O exchanged), 10.24 (br s, 1H, NH, D₂O exchanged), 8.59 - 8.26 (m, 1H), 8.17 (s, 1H), 7.88 (d, *J* = 8.4 Hz, 1H), 7.70 ― 7.45 (m, 1H), 7.19 (br s, 1H, NH, D₂O exchanged), 6.42 (s, 1H), 3.99 (br s, 1H), 2.03 - 1.34 (m, 14H). MS (ESI⁺) : [M+H]+ 338.3. |
| **78** | 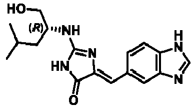 | C₁₇H₂₁N₅O₂ | 327.39 | 95% | Pale yellow solid. ¹H NMR (400 MHz, DMSO-*d*₆, 343K) of major tautomer δ_{H} 10.81 (br s, 2H, NH, D₂O exchanged), 8.37 - 8.07 (m, 2H), 7.76 (br s, 1H), 7.54 (d, *J* = 8.4 Hz, 1H), 6.90 (br s, 1H, NH, D₂O exchanged), 6.43 (s, 1H), 4.01 (s, 1H), 3.52 (d, *J* = 5.1 Hz, 2H), 1.80 - 1.64 (m, 1H), 1.57 - 1.38 (m, 2H), 0.96 (d, *J* = 6.6 Hz, 6H). MS (ESI⁺) : [M+H]+ 328.3. |
| **79** | 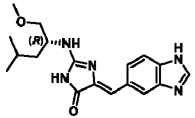 | C₁₈H₂₃N₅O₂ | 341.42 | 97% | Pale yellow solid. ¹H NMR (400 MHz, DMSO-*d*₆, 343K) of major tautomer δ_{H} 12.28 (br s, 1H, NH, D₂O exchanged), 10.18 (br s, 1H, NH, D₂O exchanged), 8.58 - 8.26 (m, 1H), 8.14 (s, 1H), 7.88 (d, *J* = 8.3 Hz, 1H), 7.54 (s, 1H), 6.95 (br s, 1H, NH, D₂O exchanged), 6.44 (s, 1H), 4.18 (s, 1H), 3.51 - 3.39 (m, 2H), 3.33 (s, 3H), 1.72 (s, 1H), 1.59 - 1.35 (m, 2H), 1.01 - 0.85 (m, 6H). MS (ESI⁺) : [M+H]+ 342.3. |
| **80** | 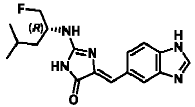 | C₁₇H₂₀FN₅O | 329.38 | 95% | Pale yellow solid. ¹H NMR (400 MHz, DMSO-*d*₆, 343K) of major tautomer δ_{H} 12.30 (br s, 1H, NH, D₂O exchanged), 10.33 (br s, 1H, NH, D₂O exchanged), 8.35 (br s, 1H), 8.15 (s, 1H), 7.98 - 7.82 (m, 1H), 7.66 - 7.47 (m, 1H), 7.18 (br s, 1H, NH, D₂O exchanged), 6.47 (s, 1H), 4.66 - 4.51 (m, 1H), 4.51 - 4.38 (m, 1H), 4.35 - 4.17 (m, 1H), 1.82 - 1.65 (m, 1H), 1.65 - 1.52 (m, 1H), 1.50 - 1.36 (m, 1H), 1.03 - 0.87 (m, 6H). MS (ESI⁺): [M+H]+ 330.3. |
| **81** | 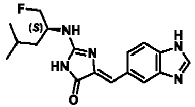 | C₁₇H₂₀FN₅O | 329.38 | 90% | Pale yellow solid. ¹H NMR (400 MHz, DMSO-*d*₆, 343K) of major tautomer δ_{H} 12.30 (br s, 1H, NH, D₂O exchanged), 10.33 (br s, 1H, NH, D₂O exchanged), 8.35 (br s, 1H), 8.15 (s, 1H), 7.98 - 7.82 (m, 1H), 7.66 - 7.47 (m, 1H), 7.18 (br s, 1H, NH, D₂O exchanged), 6.47 (s, 1H), 4.66 - 4.51 (m, 1H), 4.51 - 4.38 (m, 1H), 4.35 - 4.17 (m, 1H), 1.82 - 1.65 (m, 1H), 1.65 - 1.52 (m, 1H), 1.50 - 1.36 (m, 1H), 1.03 - 0.87 (m, 6H). MS (ESI⁺): [M+H]+ 330.3. |
| **82** | 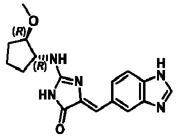 | C₁₇H₁₉N₅O₂ | 325.37 | 95% | Pale yellow solid. ¹H NMR (400 MHz, DMSO-*d*₆, 343K) of major tautomer δ_{H} 12.29 (br s, 1H, NH, D₂O exchanged), 10.22 (br s, 1H, NH, D₂O exchanged), 8.38 (br s, 1H), 8.15 (s, 1H), 7.98 - 7.72 (m, 1H), 7.52 (br s, 1H), 7.24 (br s, 1H, NH, D₂O exchanged), 6.46 (s, 1H), 4.16 (s, 1H), 3.78 (s, 1H), 3.36 (s, 3H), 2.15 - 2.03 (m, 1H), 2.01 - 1.87 (m, 1H), 1.80 - 1.51 (m, 4H). MS (ESI⁺) : [M+H]+ 326.3. |
| **83** | 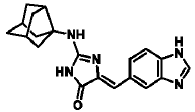 | C₂₀H₂₁N₅O | 347.42 | >98% | Pale yellow solid. ¹H NMR (400 MHz, DMSO-*d*₆, 343K) of major tautomer δ_{H} 12.28 (br s, 1H, NH, D₂O exchanged), 9.78 (br s, 1H, NH, D₂O exchanged), 8.35 (br s, 1H), 8.14 (br s, 1H), 7.93 ― 7.88 (m, 1H), 7.56 - 7.45 (m, 1H), 7.02 (br s, 1H, NH, D₂O exchanged), 6.44 (s, 1H), 2.67 (s, 1H), 2.44 - 1.90 (m, 8H), 1.79 - 1.41 (m, 4H). MS (ESI⁺) : [M+H]+ 348.4. |
| **84** | 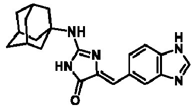 | C₂₁H₂₃N₅O | 361.45 | >98% | Pale yellow solid. ¹H NMR (400 MHz, DMSO-*d*₆, 323K) of major tautomer δ_{H} 12.40 (br s, 1H, NH, D₂O exchanged), 9.69 (br s, 1H, NH, D₂O exchanged), 8.40 (br s, 1H), 8.17 (s, 1H), 7.91 - 7.81 (m, 1H), 7.65 - 7.46 (m, 1H), 6.60 (br s, 1H, NH, D₂O exchanged), 6.42 (s, 1H), 2.37 - 1.94 (m, 9H), 1.92 - 1.52 (m, 6H). MS (ESI⁺) : [M+H]+ 362.3. |
| **85** | 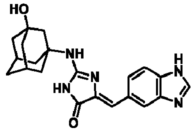 | C₂₁H₂₃N₅O₂ | 377.45 | 97% | Beige solid. ¹H NMR (400 MHz, DMSO-*d*₆, 323K) of major tautomer δ_{H} 12.38 (br s, 1H, NH, D₂O exchanged), 9.69 (br s, 1H, NH, D₂O exchanged), 8.67 - 8.09 (m, 2H), 7.90 (d, *J* = 8.3 Hz, 1H), 7.71 - 7.42 (m, 1H), 6.70 (br s, 1H, NH, D₂O exchanged), 6.43 (s, 1H), 4.46 (br s, 1H, OH, D₂O exchanged), 2.32 - 1.86 (m, 8H), 1.79 - 1.42 (m, 6H). MS (ESI⁺) : [M+H]⁺ 378.3. |
| **86** | 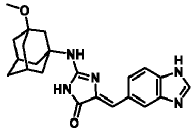 | C₂₂H₂₅N₅O₂ | 391.48 | 97% | Beige solid. ¹H NMR (400 MHz, DMSO-*d*₆, 343K) of major tautomer δ_{H} 12.28 (br s, 1H, NH, D₂O exchanged), 9.66 (br s, 1H, NH, D₂O exchanged), 8.67 - 8.19 (m, 1H), 8.15 (s, 1H), 7.91 (br s, 1H), 7.68 ― 7.41 (m, 1H), 6.64 (br s, 1H, NH, D₂O exchanged), 6.45 (s, 1H), 3.19 (s, 3H), 2.36 - 1.93 (m, 8H), 1.83 - 1.48 (m, 6H). MS (ESI⁺) : [M+H]+ 392.4. |
| **87** | 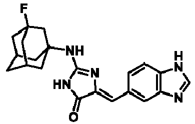 | C₂₁H₂₂FN₅O | 379.44 | >98% | Pale yellow solid. ¹H NMR (400 MHz, DMSO-*d*₆, 343K) of major tautomer δ_{H} 12.31 (br s, 1H, NH, D₂O exchanged), 9.72 (br s, 1H, NH, D₂O exchanged), 8.44 (br s, 1H), 8.15 (s, 1H), 7.88 (d, *J* = 8.5 Hz, 1H), 7.52 (br s, 1H), 6.74 (br s, 1H, NH, D₂O exchanged), 6.47 (s, 1H), 2.44 - 2.23 (m, 4H), 2.23 ― 1.77 (m, 8H), 1.71 ― 1.49 (m, 2H). MS (ESI⁺) : [M+H]+ 380.3. |
| **88** | 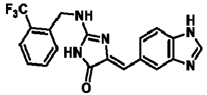 | C₁₉H₁₄F₃N₅O | 385.35 | >98% | Pale yellow solid. ¹H NMR (400 MHz, DMSO-*d*₆, 343K) of major tautomer δ_{H} 12.25 (br s, 1H, NH, D₂O exchanged), 10.47 (br s, 1H, NH, D₂O exchanged), 8.54 ― 8.07 (m, 2H), 8.07 - 7.26 (m, 6H + 1H, NH, D₂O exchanged), 6.49 (s, 1H), 4.82 (s, 2H). MS (ESI⁺) : [M+H]⁺ 386.2. |
| **89** | 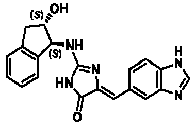 | C₂₀H₁₇N₅O₂ | 359.39 | 96% | Pale yellow solid. ¹H NMR (400 MHz, DMSO-*d*₆, 343K) of major tautomer δ_{H} 12.30 (br s, 1H, NH, D₂O exchanged), 10.42 (br s, 1H, NH, D₂O exchanged), 8.55 ― 8.24 (m, 1H), 8.13 (s, 1H), 8.00 ― 7.76 (m, 1H), 7.74 ― 7.43 (m, 1H + NH, D₂O exchanged), 7.41 ― 7.08 (m, 4H), 6.51 (s, 1H), 5.41 (br s, 1H, OH, D₂O exchanged), 5.26 (br s, 1H), 4.44 (br s, 1H), 3.23 (dd, *J* = 15.7, 7.2 Hz, 1H), 2.80 (dd, *J* = 15.8, 7.3 Hz, 1H). MS (ESI⁺) : [M+H]⁺ 360.3. |
| **90** | 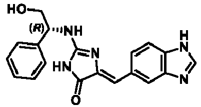 | C₁₉H₁₇N₅O₂ | 347.38 | >98% | Pale yellow solid. ¹H NMR (400 MHz, DMSO-*d*₆, 343K) of major tautomer δ_{H} 12.30 (br s, 1H, NH, D₂O exchanged), 10.32 (br s, 1H, NH, D₂O exchanged), 8.35 (br s, 1H), 8.17 (s, 1H), 7.82 (br s, 1H), 7.72 ― 7.05 (m, 6H + NH, D₂O exchanged), 6.44 (br s, 1H), 5.06 (br s, 1H), 4.94 (br s, 1H, OH, D₂O exchanged), 3.79 (br s, 2H). MS (ESI⁺) : [M+H]⁺ 348.3. |
| **91** | 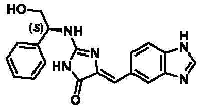 | C₁₉H₁₇N₅O₂ | 347.38 | >98% | Pale yellow solid. ¹H NMR (400 MHz, DMSO-*d*₆, 343K) of major tautomer δ_{H} 12.30 (br s, 1H, NH, D₂O exchanged), 10.32 (br s, 1H, NH, D₂O exchanged), 8.35 (br s, 1H), 8.17 (s, 1H), 7.82 (br s, 1H), 7.72 ― 7.05 (m, 6H + NH, D₂O exchanged), 6.44 (br s, 1H), 5.06 (br s, 1H), 4.94 (br s, 1H, OH, D₂O exchanged), 3.79 (br s, 2H). MS (ESI⁺) : [M+H]⁺ 348.3. |
| **92** | 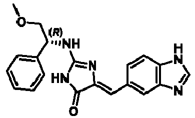 | C₂₀H₁₉N₅O₂ | 361.41 | >98% | Pale yellow solid. ¹H NMR (400 MHz, DMSO-*d*₆, 373K) of major tautomer δ_{H} 12.30 (br s, 1H, NH, D₂O exchanged), 10.26 (br s, 1H, NH, D₂O exchanged), 8.36 (br s, 1H), 8.16 (s, 1H), 7.82 (br s, 1H), 7.68 (br s, 1H, NH, D₂O exchanged), 7.61 ― 7.20 (m, 6H), 6.45 (s, 1H), 5.23 (br s, 1H), 3.85 - 3.64 (m, 2H), 3.34 (s, 3H). MS (ESI⁺) : [M+H]⁺ 362.3. |
| **93** | 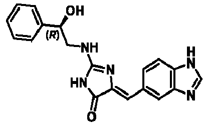 | C₁₉H₁₇N₅O₂ | 347.38 | 96% | Pale yellow solid. ¹H NMR (400 MHz, DMSO-*d*₆, 373K) of major tautomer δ_{H} 10.92 (br s, 2H, NH, D₂O exchanged), 8.24 (br s, 1H), 8.18 (s, 1H), 7.77 (br s, 1H), 7.56 (d, *J* = 8.4 Hz, 1H), 7.50 ― 7.44 (m, 2H), 7.41 ― 7.34 (m, 2H), 7.32 ― 7.24 (m, 1H), 7.06 (br s, 1H, NH, D₂O exchanged), 6.46 (s, 1H), 5.61 (br s, 1H, OH, D₂O exchanged), 4.88 (dd, *J* = 7.9, 4.1 Hz, 1H), 3.69 (dd, *J* = 13.5, 4.2 Hz, 1H), 3.48 (dd, *J* = 13.5, 7.8 Hz, 1H). MS (ESI⁺) : [M+H]+ 348.3. |
| **94** | 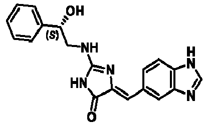 | C₁₉H₁₇N₅O₂ | 347.38 | >98% | Pale yellow solid. ¹H NMR (400 MHz, DMSO-*d*₆, 373K) of major tautomer δ_{H} 10.92 (br s, 2H, NH, D₂O exchanged), 8.24 (br s, 1H), 8.18 (s, 1H), 7.77 (br s, 1H), 7.56 (d, *J* = 8.4 Hz, 1H), 7.50 ― 7.44 (m, 2H), 7.41 ― 7.34 (m, 2H), 7.32 ― 7.24 (m, 1H), 7.06 (br s, 1H, NH, D₂O exchanged), 6.46 (s, 1H), 5.61 (br s, 1H, OH, D₂O exchanged), 4.88 (dd, *J* = 7.9, 4.1 Hz, 1H), 3.69 (dd, *J* = 13.5, 4.2 Hz, 1H), 3.48 (dd, *J* = 13.5, 7.8 Hz, 1H). MS (ESI⁺) : [M+H]+ 348.3. |
| **95** | 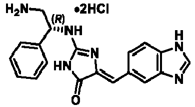 | C₁₉H₂₀Cl₂N₆O | 419.31 | 92% | Pale yellow solid. ¹H NMR (400 MHz, D₂O, 323K) of major tautomer δ_{H} 9.42 (s, 1H), 8.23 (s, 1H), 8.04 (d, *J* = 8.7 Hz, 1H), 7.92 (dd, *J* = 8.7, 1.5 Hz, 1H), 7.87 ― 7.71 (m, 5H), 7.08 (s, 1H), 5.65 ― 5.57 (m, 1H), 3.95 ― 3.81 (m, 2H). MS (ESI⁺) : [M+H]⁺ 347.2 (+2HCl). |
| **96** | 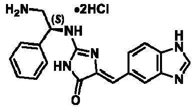 | C₁₉H₂₀Cl₂N₆O | 419.31 | 98% | Pale yellow solid. ¹H NMR (400 MHz, D₂O, 323K) of major tautomer δ_{H} 9.42 (s, 1H), 8.23 (s, 1H), 8.04 (d, *J* = 8.7 Hz, 1H), 7.92 (dd, *J* = 8.7, 1.5 Hz, 1H), 7.87 ― 7.71 (m, 5H), 7.08 (s, 1H), 5.65 ― 5.57 (m, 1H), 3.95 ― 3.81 (m, 2H). MS (ESI⁺) : [M+H]⁺ 347.2 (+2HCl). |
| **97** | 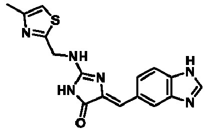 | C₁₆H₁₄N₆OS | 338.39 | 97% | Yellow solid. ¹H NMR (400 MHz, DMSO-*d*₆, 343K) of major tautomer δ_{H} 12.18 (br s, 1H, NH, D₂O exchanged), 10.09 (br s, 1H, NH, D₂O exchanged), 8.35 (br s, 1H), 8.16 (s, 1H), 7.83 (br s, 1H + NH, D₂O exchanged), 7.60 ― 7.39 (m, 1H), 7.14 (s, 1H), 6.52 (s, 1H), 4.86 (s, 2H), 2.37 (s, 3H). MS (ESI⁺) : [M+H]⁺ 339.2. |
| **98** | 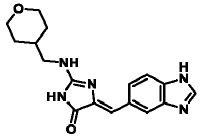 | C₁₇H₁₉N₅O₂ | 325.37 | >98% | Pale yellow solid. ¹H NMR (400 MHz, DMSO-*d*₆, 343K) of major tautomer δ_{H} 12.29 (br s, 1H, NH, D₂O exchanged), 10.35 (br s, 1H, NH, D₂O exchanged), 8.62 ― 8.27 (m, 1H), 8.15 (s, 1H), 7.84 (br s, 1H), 7.70 ― 7.43 (m, 1H), 7.20 (br s, 1H, NH, D₂O exchanged), 6.43 (s, 1H), 4.00 ― 3.79 (m, 2H), 3.52 ― 3.18 (m, 4H), 1.88 (br s, 1H), 1.76 ― 1.50 (m, 2H), 1.43 ― 1.14 (m, 2H). MS (ESI⁺) : [M+H]⁺ 326.3. |
| **99** | 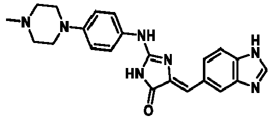 | C₂₂H₂₃N₇O | 401.47 | >98% | Yellow solid. ¹H NMR (400 MHz, DMSO-*d*₆, 343K) of major tautomer δ_{H} 12.38 (br s, 1H, NH, D₂O exchanged), 10.22 (br s, 1H, NH, D₂O exchanged), 9.23 (br s, 1H, NH, D₂O exchanged), 8.43 (br s, 1H), 8.20 (s, 1H), 8.07 ― 7.77 (m, 1H), 7.74 ― 7.36 (m, 3H), 6.99 (br s, 2H), 6.60 (s, 1H), 3.20 ― 3.12 (m, 4H), 2.50 ― 2.47 (m, 4H), 2.26 (s, 3H). MS (ESI⁺) : [M+H]⁺ 402.4. |
| **100** | 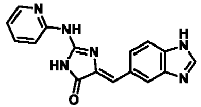 | C₁₆H₁₂N₆O | 304.31 | 95% | Yellow solid. ¹H NMR (400 MHz, DMSO-*d*₆, 343K) of major tautomer δ_{H} 12.41 (br s, 1H, NH, D₂O exchanged), 10.76 (br s, 2H, NH, D₂O exchanged), 8.75 ― 8.12 (m, 3H), 8.09 ― 7.73 (m, 2H), 7.69 ― 7.33 (m, 2H), 7.20 ― 6.96 (m, 1H), 6.76 (s, 1H). MS (ESI⁺) : [M+H]⁺ 305.3. |
| **101** | 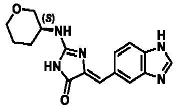 | C₁₆H₁₇N₅O₂ | 311.35 | >98% | Yellow solid. ¹H NMR (400 MHz, DMSO-*d*₆, 343K) of major tautomer δ_{H} 12.16 (br s, 1H, NH, D₂O exchanged), 10.24 (br s, 1H, NH, D₂O exchanged), 8.27 (br s, 1H), 8.17 (s, 1H), 7.76 (br s, 1H), 7.56 (d, *J* = 8.3 Hz, 1H), 7.17 (br s, 1H, NH, D₂O exchanged), 6.47 (s, 1H), 3.99 ― 3.81 (m, 2H), 3.78 ― 3.64 (m, 1H), 3.55 ― 3.33 (m, 2H), 2.10 ― 1.93 (m, 1H), 1.82 ― 1.52 (m, 3H). MS (ESI⁺) : [M+H]⁺ 312.3. |
| **102** | 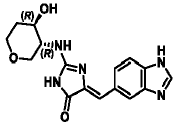 | C₁₆H₁₇N₅O₃ | 327.34 | >98% | Pale yellow solid. ¹H NMR (400 MHz, DMSO-*d*₆, 343K) of major tautomer δ_{H} 12.37 (br s, 1H, NH, D₂O exchanged), 10.24 (br s, 1H, NH, D₂O exchanged), 8.66 ― 8.24 (m, 1H), 8.15 (s, 1H), 7.84 (br s, 1H), 7.66 ― 7.45 (m, 1H), 7.07 (br s, 1H, NH, D₂O exchanged), 6.46 (s, 1H), 4.97 (br s, 1H, OH, D₂O exchanged), 4.10 ― 3.95 (m, 1H), 3.95 ― 3.79 (m, 1H), 3.69 (br s, 2H), 3.56 ― 3.35 (m, 1H), 3.32 ― 3.18 (m, 1H), 2.04 ― 1.87 (m, 1H), 1.68 ― 1.44 (m, 1H). MS (ESI⁺) : [M+H]⁺ 328.3. |
| **103** | 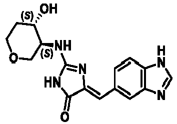 | C₁₆H₁₇N₅O₃ | 327.34 | >98% | Pale yellow solid. ¹H NMR (400 MHz, DMSO-*d*₆, 343K) of major tautomer δ_{H} 12.37 (br s, 1H, NH, D₂O exchanged), 10.24 (br s, 1H, NH, D₂O exchanged), 8.66 ― 8.24 (m, 1H), 8.15 (s, 1H), 7.84 (br s, 1H), 7.66 ― 7.45 (m, 1H), 7.07 (br s, 1H, NH, D₂O exchanged), 6.46 (s, 1H), 4.97 (br s, 1H, OH, D₂O exchanged), 4.10 ― 3.95 (m, 1H), 3.95 ― 3.79 (m, 1H), 3.69 (br s, 2H), 3.56 ― 3.35 (m, 1H), 3.32 ― 3.18 (m, 1H), 2.04 ― 1.87 (m, 1H), 1.68 ― 1.44 (m, 1H). MS (ESI⁺) : [M+H]⁺ 328.3. |
| **104** | 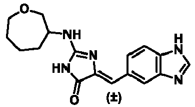 | C₁₇H₁₉N₅O₂ | 325.37 | >98% | Pale yellow solid. ¹H NMR (400 MHz, DMSO-*d*₆, 343K) of major tautomer δ_{H} 12.31 (br s, 1H, NH, D₂O exchanged), 10.08 (br s, 1H, NH, D₂O exchanged), 8.37 (br s, 1H), 8.15 (s, 1H), 7.83 (br s, 1H), 7.53 (br s, 1H), 7.02 (br s, 1H, NH, D₂O exchanged), 6.45 (s, 1H), 4.14 (br s, 1H), 3.91 ― 3.59 (m, 4H), 2.03 ― 1.52 (m, 6H). MS (ESI⁺) : [M+H]⁺ 326.3. |
| **105** | 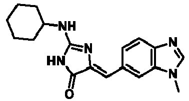 | C₁₈H₂₁N₅O | 323.40 | 97% | Pale yellow solid. ¹H NMR (400 MHz, DMSO-*d*₆, 343K) of major tautomer δ_{H} 10.23 (br s, 1H, NH, D₂O exchanged), 8.43 (br s, 1H), 8.14 (s, 1H), 7.79 (br s, 1H), 7.59 (d, *J* = 8.2 Hz, 1H), 7.13 (br s, 1H, NH, D₂O exchanged), 6.45 (s, 1H), 3.84 (s, 3H), 3.74 (br s, 1H), 1.99 (br s, 2H), 1.83 ― 1.68 (m, 2H), 1.67 ― 1.56 (m, 1H), 1.47 ― 1.13 (m, 5H). MS (ESI⁺) : [M+H]⁺ 324.2. |
| **106** | 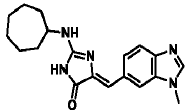 | C₁₉H₂₃N₅O | 337.43 | >98% | Pale yellow solid. ¹H NMR (400 MHz, DMSO-*d*₆, 343K) of major tautomer δ_{H} 10.15 (br s, 1H, NH, D₂O exchanged), 8.45 (s, 1H), 8.12 (s, 1H), 7.80 (d, *J* = 8.5 Hz, 1H), 7.57 (d, *J* = 8.4 Hz, 1H), 7.10 (br s, 1H, NH, D₂O exchanged), 6.44 (s, 1H), 3.97 (br s, 1H), 3.83 (s, 3H), 2.11 ― 1.85 (m, 2H), 1.82 ― 1.40 (m, 10H). MS (ESI⁺) : [M+H]⁺ 338.3. |
| **107** | 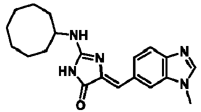 | C₂₀H₂₅N₅O | 351.45 | 95% | Pale yellow solid. ¹H NMR (400 MHz, DMSO-*d*₆, 343K) of major tautomer δ_{H} 10.12 (br s, 1H, NH, D₂O exchanged), 8.48 (s, 1H), 8.12 (s, 1H), 7.77 (d, *J* = 8.5 Hz, 1H), 7.57 (d, *J* = 8.4 Hz, 1H), 7.08 (br s, 1H, NH, D₂O exchanged), 6.44 (s, 1H), 4.07 (br s, 1H), 3.83 (s, 3H), 1.99 - 1.82 (m, 2H), 1.82 - 1.44 (m, 12H). MS (ESI⁺) : [M+H]⁺ 352.3. |
| **108** | 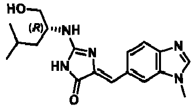 | C₁₈H₂₃N₅O₂ | 341.42 | >98% | Yellow solid. ¹H NMR (400 MHz, DMSO-*d*₆, 343K) of major tautomer δ_{H} 10.05 (br s, 1H, NH, D₂O exchanged), 8.37 (s, 1H), 8.25 ― 8.04 (m, 1H), 7.84 (d, *J* = 8.4 Hz, 1H), 7.56 (d, *J* = 8.3 Hz, 1H), 6.87 (br s, 1H, NH, D₂O exchanged), 6.45 (s, 1H), 4.85 ― 4.64 (m, 1H, OH, D₂O exchanged), 4.15 ― 3.95 (m, 1H), 3.82 (s, 3H), 3.53 (s, 2H), 1.81 ― 1.63 (m, 1H), 1.59 ― 1.41 (m, 2H), 1.03 ― 0.85 (m, 6H). MS (ESI⁺) : [M+H]⁺ 342.3. |
| **109** | 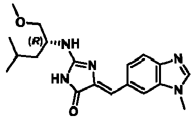 | C₁₉H₂₅N₅O₂ | 355.44 | >98% | Yellow solid. ¹H NMR (400 MHz, DMSO-*d*₆, 373K) of major tautomer δ_{H} 10.20 (br s, 1H, NH, D₂O exchanged), 8.38 (s, 1H), 8.12 (s, 1H), 7.83 (d, *J* = 8.5 Hz, 1H), 7.56 (d, *J* = 8.4 Hz, 1H), 6.99 (br s, 1H, NH, D₂O exchanged), 6.45 (s, 1H), 4.22 (s, 1H), 3.81 (s, 3H), 3.54 ― 3.40 (m, 2H), 3.33 (s, 3H), 1.79 ― 1.62 (m, 1H), 1.58 ― 1.36 (m, 2H), 1.12 ― 0.83 (m, 6H). MS (ESI⁺) : [M+H]⁺ 356.4. |
| **110** | 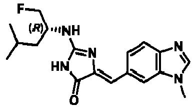 | C₁₈H₂₂FN₅O | 343.41 | 98% | Yellow solid. ¹H NMR (400 MHz, DMSO-*d*₆, 373K) of major tautomer δ_{H} 10.04 (br s, 1H, NH, D₂O exchanged), 8.36 (s, 1H), 8.13 (s, 1H), 7.84 (d, *J* = 8.5 Hz, 1H), 7.57 (d, *J* = 8.3 Hz, 1H), 7.23 (br s, 1H, NH, D₂O exchanged), 6.49 (s, 1H), 4.63 ― 4.52 (m, 1H), 4.51 ― 4.41 (m, 1H), 4.30 (br s, 1H), 3.82 (s, 3H), 1.83 ― 1.66 (m, 1H), 1.65 ― 1.52 (m, 1H), 1.50 ― 1.37 (m, 1H), 1.07 ― 0.81 (m, 6H). MS (ESI⁺) : [M+H]⁺ 344.3. |
| **111** | 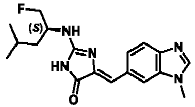 | C₁₈H₂₂FN₅O | 343.41 | >98% | Yellow solid. ¹H NMR (400 MHz, DMSO-*d*₆, 373K) of major tautomer δ_{H} 10.04 (br s, 1H, NH, D₂O exchanged), 8.36 (s, 1H), 8.13 (s, 1H), 7.84 (d, *J* = 8.5 Hz, 1H), 7.57 (d, *J* = 8.3 Hz, 1H), 7.23 (br s, 1H, NH, D₂O exchanged), 6.49 (s, 1H), 4.63 ― 4.52 (m, 1H), 4.51 ― 4.41 (m, 1H), 4.30 (br s, 1H), 3.82 (s, 3H), 1.83 ― 1.66 (m, 1H), 1.65 ― 1.52 (m, 1H), 1.50 ― 1.37 (m, 1H), 1.07 ― 0.81 (m, 6H). MS (ESI⁺) : [M+H]⁺ 344.3. |
| **112** | 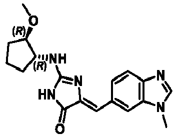 | C₁₈H₂₁N₅O₂ | 339.40 | 95% | Pale yellow solid. ¹H NMR (400 MHz, DMSO-*d*₆, 343K) of major tautomer δ_{H} 10.24 (br s, 1H, NH, D₂O exchanged), 8.33 (s, 1H), 8.13 (s, 1H), 7.90 (d, *J* = 8.5 Hz, 1H), 7.57 (d, *J* = 8.5 Hz, 1H), 7.27 (br s, 1H, NH, D₂O exchanged), 6.47 (s, 1H), 4.16 (br s, 1H), 3.93 ― 3.74 (m, 4H), 3.36 (s, 3H), 2.15 ― 2.04 (m, 1H), 1.99 ― 1.87 (m, 1H), 1.83 ― 1.48 (m, 4H). MS (ESI⁺) : [M+H]⁺ 340.3. |
| **113** | 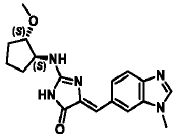 | C₁₈H₂₁N₅O₂ | 339.40 | 95% | Pale yellow solid. ¹H NMR (400 MHz, DMSO-*d*₆, 343K) of major tautomer δ_{H} 10.24 (br s, 1H, NH, D₂O exchanged), 8.33 (s, 1H), 8.13 (s, 1H), 7.90 (d, *J* = 8.5 Hz, 1H), 7.57 (d, *J* = 8.5 Hz, 1H), 7.27 (br s, 1H, NH, D₂O exchanged), 6.47 (s, 1H), 4.16 (br s, 1H), 3.93 ― 3.74 (m, 4H), 3.36 (s, 3H), 2.15 ― 2.04 (m, 1H), 1.99 ― 1.87 (m, 1H), 1.83 ― 1.48 (m, 4H). MS (ESI⁺) : [M+H]⁺ 340.3. |
| **114** | 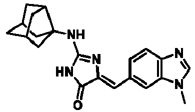 | C₂₁H₂₃N₅O | 361.45 | 95% | Yellow solid. ¹H NMR (400 MHz, DMSO-*d*₆, 373K) of major tautomer δ_{H} 9.84 (br s, 1H, NH, D₂O exchanged), 8.50 (s, 1H), 8.12 (s, 1H), 7.68 (d, *J* = 8.6 Hz, 1H), 7.56 (d, *J* = 8.4 Hz, 1H), 7.10 (br s, 1H, NH, D₂O exchanged), 6.46 (s, 1H), 3.81 (s, 3H), 2.66 (d, *J* = 7.1 Hz, 1H), 2.35 (d, *J* = 10.3 Hz, 4H), 2.19 (t, *J* = 8.6 Hz, 2H), 2.03 (d, *J* = 10.1 Hz, 2H), 1.77 ― 1.46 (m, 4H). MS (ESI⁺) : [M+H]⁺ 362.4. |
| **115** | 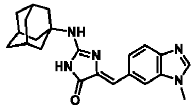 | C₂₂H₂₅N₅O | 375.48 | >98% | Yellow solid. ¹H NMR (400 MHz, DMSO-*d*₆, 323K) of major tautomer δ_{H} 9.71 (br s, 1H, NH, D₂O exchanged), 8.57 (s, 1H), 8.15 (s, 1H), 7.67 (d, *J* = 8.4 Hz, 1H), 7.57 (d, *J* = 8.4 Hz, 1H), 6.66 (br s, 1H, NH, D₂O exchanged), 6.44 (s, 1H), 3.82 (s, 3H), 2.29 ― 2.02 (m, 9H), 1.81 ― 1.61 (m, 6H). MS (ESI⁺) : [M+H]⁺ 376.3. |
| **116** | 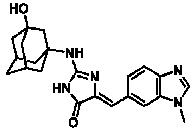 | C₂₂H₂₅N₅O₂ | 391.48 | 98% | Beige solid. ¹H NMR (400 MHz, DMSO-*d*₆, 300K) of major tautomer δ_{H} 9.85 (br s, 1H, NH, D₂O exchanged), 8.61 (s, 1H), 8.18 (s, 1H), 7.72 ― 7.52 (m, 2H), 6.92 (s, 1H), 6.44 (s, 1H), 4.60 (s, 1H, OH, D₂O exchanged), 3.84 (s, 3H), 2.29 ― 2.17 (m, 2H), 2.16 ― 1.87 (m, 6H), 1.72 ― 1.38 (m, 6H). MS (ESI⁺) : [M+H]⁺ 392.3. |
| **117** | 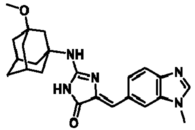 | C₂₃H₂₇N₅O₂ | 405.50 | 98% (¹H NMR) | Pale yellow solid. ¹H NMR (400 MHz, DMSO-*d*₆, 323K) of major tautomer δ_{H} 9.72 (br s, 1H, NH, D₂O exchanged), 8.53 (s, 1H), 8.15 (s, 1H), 7.68 (d, *J* = 8.6 Hz, 1H), 7.57 (d, *J* = 8.3 Hz, 1H), 6.82 (br s, 1H, NH, D₂O exchanged), 6.46 (s, 1H), 3.84 (s, 3H), 3.21 (s, 3H), 2.40 ― 1.91 (m, 8H), 1.81 ― 1.43 (m, 6H). MS (ESI⁺) : [M+H]⁺ 406.3. |
| **118** | 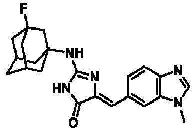 | C₂₂H₂₄FN₅O | 393.47 | 96% | Pale yellow solid. ¹H NMR (400 MHz, DMSO-*d*₆, 343K) of major tautomer δ_{H} 9.73 (br s, 1H, NH, D₂O exchanged), 8.52 (s, 1H), 8.14 (s, 1H), 7.67 (d, *J* = 8.5 Hz, 1H), 7.58 (d, *J* = 8.4 Hz, 1H), 6.80 (br s, 1H, NH, D₂O exchanged), 6.48 (s, 1H), 3.83 (s, 3H), 2.45 ― 2.27 (m, 4H), 2.23 ― 1.97 (m, 4H), 1.96 ― 1.79 (m, 4H), 1.68 ― 1.52 (m, 2H). MS (ESI⁺) : [M+H]⁺ 394.4. |
| **119** | 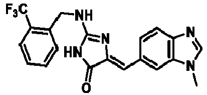 | C₂₀H₁₆F₃N₅O | 399.38 | 96% | Pale yellow solid. ¹H NMR (400 MHz, DMSO-*d*₆, 373K) of major tautomer δ_{H} 10.53 (br s, 1H, NH, D₂O exchanged), 8.32 (br s, 1H), 8.11 (br s, 1H), 7.98 ― 7.30 (m, 6H + 1H, NH, D₂O exchanged), 6.50 (s, 1H), 4.84 (s, 2H), 3.74 (s, 3H). MS (ESI⁺) : [M+H]+ 400.2. |
| **120** | 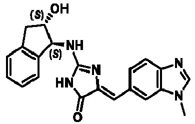 | C₂₁H₁₉N₅O₂ | 373.42 | 89% | Yellow solid. ¹H NMR (400 MHz, DMSO-*d*₆, 343K) of major tautomer δ_{H} 10.42 (br s, 1H, NH, D₂O exchanged), 8.32 (s, 1H), 8.11 (s, 1H), 7.96 ― 7.84 (m, 1H), 7.67 (br s, 1H, NH, D₂O exchanged), 7.62 ― 7.52 (m, 1H), 7.44 ― 7.10 (m, 4H), 6.53 (s, 1H), 5.44 (br s, 1H, OH, D₂O exchanged), 5.29 ― 5.14 (m, 1H), 4.53 ― 4.36 (m, 1H), 3.79 (s, 3H), 3.25 (dd, *J* = 15.8, 7.2 Hz, 1H), 2.80 (dd, *J* = 15.7, 7.1 Hz, 1H). MS (ESI⁺) : [M+H]⁺ 374.3. |
| **121** | 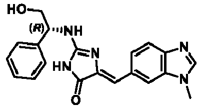 | C₂₀H₁₉N₅O₂ | 361.41 | 97% | Yellow solid. ¹H NMR (400 MHz, DMSO-*d*₆, 373K) of major tautomer δ_{H} 10.25 (br s, 1H, NH, D₂O exchanged), 8.27 (br s, 1H), 8.15 (s, 1H), 7.88 ― 7.51 (m, 2H + 1H, NH, D₂O exchanged), 7.50 ― 7.20 (m, 5H), 6.45 (s, 1H), 5.06 (br s, 1H), 4.94 (br s, 1H, OH, D₂O exchanged), 3.85 (s, 3H), 3.80 ― 3.71 (m, 2H). MS (ESI⁺) : [M+H]⁺ 362.3. |
| **122** | 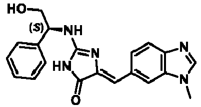 | C₂₀H₁₉N₅O₂ | 361.41 | 93% | Yellow solid. ¹H NMR (400 MHz, DMSO-*d*₆, 373K) of major tautomer δ_{H} 10.25 (br s, 1H, NH, D₂O exchanged), 8.27 (br s, 1H), 8.15 (s, 1H), 7.88 ― 7.51 (m, 2H + 1H, NH, D₂O exchanged), 7.50 ― 7.20 (m, 5H), 6.45 (s, 1H), 5.06 (br s, 1H), 4.94 (br s, 1H, OH, D₂O exchanged), 3.85 (s, 3H), 3.80 ― 3.71 (m, 2H). MS (ESI⁺) : [M+H]⁺ 362.3. |
| **123** | 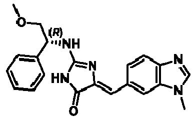 | C₂₁H₂₁N₅O₂ | 375.43 | 91% | Yellow solid. ¹H NMR (400 MHz, DMSO-*d*₆, 343K) of major tautomer δ_{H} 10.41 (br s, 1H, NH, D₂O exchanged), 8.35 (br s, 1H), 8.16 (s, 1H), 7.87 (br s, 1H, NH, D₂O exchanged), 7.78 (d, *J* = 8.3 Hz, 1H), 7.57 (d, *J* = 8.4 Hz, 1H), 7.49 (d, *J* = 7.6 Hz, 2H), 7.40 ― 7.32 (m, 2H), 7.32 ― 7.23 (m, 1H), 6.45 (s, 1H), 5.23 (br s, 1H), 3.85 (s, 3H), 3.78 ― 3.71 (m, 1H), 3.70 ― 3.62 (m, 1H), 3.34 (s, 3H). MS (ESI⁺) : [M+H]⁺ 376.3. |
| **124** | 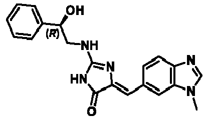 | C₂₀H₁₉N₅O₂ | 361.41 | >98% | Pale Yellow solid. ¹H NMR (400 MHz, DMSO-*d*₆, 343K) of major tautomer δ_{H} 10.30 (br s, 1H, NH, D₂O exchanged), 8.40 ― 8.31 (m, 1H), 8.15 (s, 1H), 7.93 ― 7.86 (m, 1H), 7.70 ― 7.55 (m, 1H), 7.46 (d, *J* = 7.5 Hz, 2H), 7.37 (t, *J* = 7.5 Hz, 2H), 7.28 (t, *J* = 7.2 Hz, 1H), 7.14 (br s, 1H, NH, D₂O exchanged), 6.49 (s, 1H), 5.62 (br s, 1H, OH, D₂O exchanged), 5.00 ― 4.81 (m, 1H), 3.84 (s, 3H), 3.77 ― 3.64 (m, 1H), 3.55 ― 3.40 (m, 1H). MS (ESI⁺) : [M+H]⁺ 362.3. |
| **125** | 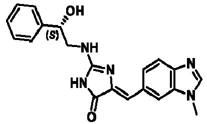 | C₂₀H₁₉N₅O₂ | 361.41 | >98% | Yellow solid. ¹H NMR (400 MHz, DMSO-*d*₆, 343K) of major tautomer δ_{H} 10.30 (br s, 1H, NH, D₂O exchanged), 8.40 ― 8.31 (m, 1H), 8.15 (s, 1H), 7.93 ― 7.86 (m, 1H), 7.70 ― 7.55 (m, 1H), 7.46 (d, *J* = 7.5 Hz, 2H), 7.37 (t, *J* = 7.5 Hz, 2H), 7.28 (t, *J* = 7.2 Hz, 1H), 7.14 (br s, 1H, NH, D₂O exch), 6.49 (s, 1H), 5.62 (br s, 1H, OH, D₂O exchanged), 5.00 ― 4.81 (m, 1H), 3.84 (s, 3H), 3.77 ― 3.64 (m, 1H), 3.55 ― 3.40 (m, 1H). MS (ESI⁺) : [M+H]⁺ 362.3. |
| **126** | 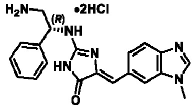 | C₂₀H₂₂Cl₂N₆O | 433.34 | 97% | Pale yellow solid. ¹H NMR (400 MHz, D₂O, 323K) of major tautomer δ_{H} 9.43 (s, 1H), 8.32 (s, 1H), 8.08 (s, 2H), 7.87 ― 7.74 (m, 5H), 7.07 (s, 1H), 5.65 (dd, *J* = 8.6, 6.1 Hz, 1H), 4.36 (s, 3H), 3.97 ― 3.79 (m, 2H). MS (ESI⁺) : [M+H]⁺ 361.3 (+2HCl). |
| **127** | 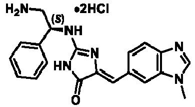 | C₂₀H₂₂Cl₂N₆O | 433.34 | 95% | Pale yellow solid. ¹H NMR (400 MHz, D₂O, 323K) of major tautomer δ_{H} 9.43 (s, 1H), 8.32 (s, 1H), 8.08 (s, 2H), 7.87 ― 7.74 (m, 5H), 7.07 (s, 1H), 5.65 (dd, *J* = 8.6, 6.1 Hz, 1H), 4.36 (s, 3H), 3.97 ― 3.79 (m, 2H). MS (ESI⁺) : [M+H]⁺ 361.2 (+2HCl). |
| **128** | 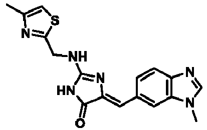 | C₁₇H₁₆N₆OS | 352.42 | 96% | Yellow solid. ¹H NMR (400 MHz, DMSO-*d*₆, 343K) of major tautomer δ_{H} 10.58 (br s, 1H, NH, D₂O exchanged), 8.52 ― 8.35 (m, 1H), 8.14 (s, 1H), 7.93 (br s, 1H, NH, D₂O exchanged), 7.86 ― 7.76 (m, 1H), 7.65 ― 7.51 (m, 1H), 7.13 (s, 1H), 6.53 (s, 1H), 4.85 (s, 2H), 3.82 (s, 3H), 2.36 (s, 3H). MS (ESI⁺) : [M+H]⁺ 353.3. |
| **129** | 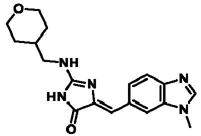 | C₁₈H₂₁N₅O₂ | 339.40 | >98% | Yellow solid. ¹H NMR (400 MHz, DMSO-*d*₆, 343K) of major tautomer δ_{H} 10.40 (br s, 1H, NH, D₂O exchanged), 8.41 (br s, 1H), 8.13 (br s, 1H), 7.93 ― 7.77 (m, 1H), 7.64 ― 7.49 (m, 1H), 7.26 (br s, 1H, NH, D₂O exchanged), 6.45 (s, 1H), 4.02 ― 3.71 (m, 5H), 3.43 ― 3.24 (m, 4H), 1.93 (s, 1H), 1.74 ― 1.56 (m, 2H), 1.39 ― 1.19 (m, 2H). MS (ESI⁺) : [M+H]⁺ 340.3. |
| **130** | 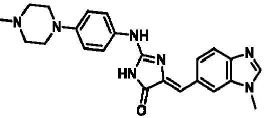 | C₂₃H₂₅N₇O | 415.50 | >98% | Yellow solid. ¹H NMR (400 MHz, DMSO-*d*₆, 300K) of major tautomer δ_{H} 10.61 (br s, 1H, NH, D₂O exchanged), 9.58 (br s, 1H, NH, D₂O exchanged), 8.64 (s, 1H), 8.22 (s, 1H), 7.86 ― 7.67 (m, 3H), 7.62 (d, *J* = 8.4 Hz, 1H), 6.98 (d, *J* = 8.7 Hz, 2H), 6.60 (s, 1H), 3.88 (s, 3H), 3.18 ― 3.06 (m, 4H), 2.47 (d, *J* = 4.9 Hz, 4H), 2.24 (s, 3H). MS (ESI⁺) : [M+H]⁺ 416.4. |
| **131** | 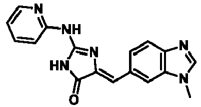 | C₁₇H₁₄N₆O | 318.34 | 96% | Yellow solid. ¹H NMR (400 MHz, DMSO-*d*₆, 300K) of major tautomer δ_{H} 10.93 (br s, 2H, NH, D₂O exchanged), 8.63 ― 8.14 (m, 3H), 7.98 (s, 1H), 7.90 ― 7.56 (m, 3H), 7.16 ― 7.01 (m, 1H), 6.77 (s, 1H), 3.89 (s, 3H). MS (ESI⁺) : [M+H]⁺ 319.3. |
| **132** | 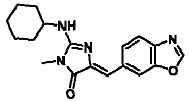 | C₁₈H₂₀N₄O₂ | 324.38 | >98% | Yellow solid. ¹H NMR (400 MHz, DMSO-*d*₆, 300K) δ_{H} 8.78 (s, 1H), 8.73 (s, 1H), 8.02 (dd, *J* = 8.4, 1.4 Hz, 1H), 7.80 (d, *J* = 8.3 Hz, 1H), 7.61 (d, *J* = 7.7 Hz, 1H, NH, D₂O exchanged), 6.59 (s, 1H), 3.97 (m, 1H), 3.14 (s, 3H), 2.15 ― 2.00 (m, 2H), 1.95 ― 1.80 (m, 2H), 1.78 ― 1.66 (m, 1H), 1.55 ― 1.35 (m, 4H), 1.32 ― 1.15 (m, 1H). MS (ESI⁺) : [M+H]⁺ 325.2. |
| **133** | 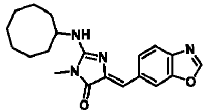 | C₂₀H₂₄N₄O₂ | 352.44 | >96% | Orange solid. ¹H NMR (400 MHz, DMSO-*d*₆, 300K) δ_{H} 8.77 (s, 1H), 8.73 (s, 1H), 7.95 (d, *J* = 8.4 Hz, 1H), 7.73 (d, *J* = 8.3 Hz, 1H), 7.59 (d, *J* = 7.3 Hz, 1H, NH, D₂O exchanged), 6.54 (s, 1H), 4.30 ― 4.15 (m, 1H), 3.08 (s, 3H), 1.97 ― 1.52 (m, 14H). MS (ESI⁺) : [M+H]⁺ 353.3. |
| **134** | 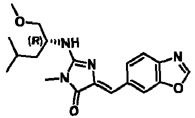 | C₁₉H₂₄N₄O₃ | 356.43 | >98% | Yellow solid. ¹H NMR (400 MHz, DMSO-*d*₆, 300K) δ_{H} 8.72 (s, 1H), 8.70 (s, 1H), 8.96 (dd, *J* = 8.4, 1.4 Hz, 1H), 7.73 (d, *J* = 8.3 Hz, 1H), 7.58 (d, *J* = 8.4 Hz, 1H, NH, D₂O exchanged), 6.55 (s, 1H), 4.50 ― 4.35 (m, 1H), 3.52 (dd, *J* = 9.8, 6.5 Hz, 1H), 3.44 (dd, *J* = 9.8, 5.5 Hz, 1H), 3.32 (s, 3H), 3.09 (s, 3H), 1.78 ― 1.66 (m, 1H), 1.64 ― 1.53 (m, 1H), 1.48 ― 1.37 (m, 1H), 1.00 ― 0.92 (m, 6H). MS (ESI⁺) : [M+H]⁺ 357.2. |
| **135** | 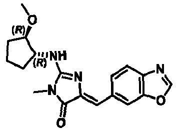 | C₁₈H₂₀N₄O₃ | 340.38 | >98% | Yellow solid. ¹H NMR (400 MHz, DMSO-*d*₆, 300K) δ_{H} 8.73 (s, 1H), 8.71 (s, 1H), 7.99 (dd, *J* = 8.4, 1.4 Hz, 1H), 7.74 (d, *J* = 8.3 Hz, 1H), 7.62 (d, *J* = 6.4 Hz, 1H, NH, D₂O exchanged), 6.57 (s, 1H), 4.40 ― 4.28 (m, 1H), 3.90 ― 3.84 (m, 1H), 3.39 (s, 3H), 3.09 (s, 3H), 2.21 ― 2.06 (m, 1H), 2.00 ― 1.86 (m, 1H), 1.85 ― 1.58 (m, 4H). MS (ESI⁺) : [M+H]⁺ 341.2. |
| **136** | 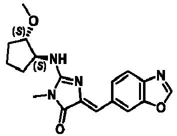 | C₁₈H₂₀N₄O₃ | 340.38 | >98% | Yellow solid. ¹H NMR (400 MHz, DMSO-*d*₆, 300K) δ_{H} 8.73 (s, 1H), 8.71 (s, 1H), 7.99 (dd, *J* = 8.4, 1.4 Hz, 1H), 7.74 (d, *J* = 8.3 Hz, 1H), 7.62 (d, *J* = 6.4 Hz, 1H, NH, D₂O exchanged), 6.57 (s, 1H), 4.40 ― 4.28 (m, 1H), 3.90 ― 3.84 (m, 1H), 3.39 (s, 3H), 3.09 (s, 3H), 2.21 ― 2.06 (m, 1H), 2.00 ― 1.86 (m, 1H), 1.85 ― 1.58 (m, 4H). MS (ESI⁺) : [M+H]⁺ 341.2. |
| **137** | 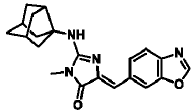 | C₂₁H₂₂N₄O₂ | 362.43 | 97% | Yellow solid. ¹H NMR (400 MHz, DMSO-*d*₆, 300K) δ_{H} 8.75 (s, 1H), 8.72 (s, 1H), 7.93 (d, *J* = 8.2 Hz, 1H), 7.72 (d, *J* = 8.3 Hz, 1H), 7.51 (s, 1H, NH, D₂O exchanged), 6.56 (s, 1H), 3.11 (s, 3H), 2.80 (t, *J* = 6.6 Hz, 1H), 2.41 ― 2.30 (m, 4H), 2.22 ― 2.13 (m, 2H), 2.07 ― 1.97 (m, 2H), 1.72 ― 1.52 (m, 4H). MS (ESI⁺) : [M+H]⁺ 363.2. |
| **138** | 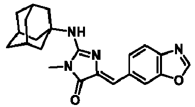 | C₂₂H₂₄N₄O₂ | 376.46 | >98% | Yellow solid. ¹H NMR (400 MHz, DMSO-*d*₆, 300K) δ_{H} 8.84 (s, 1H), 8.72 (s, 1H), 7.96 ― 7.66 (m, 2H), 6.90 (s, 1H, NH, D₂O exchanged), 6.55 (s, 1H), 3.08 (s, 3H), 2.28 (s, 6H), 2.16 (s, 3H), 1.75 (s, 6H). MS (ESI⁺) : [M+H]⁺ 377.3. |
| **139** | 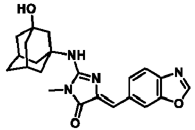 | C₂₂H₂₄N₄O₃ | 392.46 | >98% (*Z*)+(*E*) | Yellow solid. Isolated as an inseparable 71/29 mixture of (*Z*)/(*E*) isomers. ¹H NMR (400 MHz, DMSO-*d*₆, 300K) δ_{H} 8.83 & 8.80 (s, 1H), 8.74 & 8.73 (s, 1H), 7.93 & 7.89 (dd, *J* = 8.3, 1.5 Hz, 1H), 7.74 & 7.72 (d, *J* = 8.3 Hz, 1H), 6.97 & 6.84 (s, 1H, NH, D₂O exchanged), 6.56 & 6.52 (s, 1H), 4.62 & 4.56 (s, 1H, OH, D₂O exchanged), 3.08 (s, 3H), 2.30 ― 2.00 (m, 8H), 1.75 ― 1.45 (m, 6H). MS (ESI⁺) : [M+H]⁺ 393.2. |
| **140** | 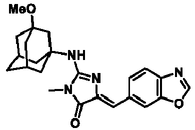 | C₂₃H₂₆N₄O₃ | 406.49 | >98% | Yellow solid. ¹H NMR (400 MHz, DMSO-*d*₆, 300K) δ_{H} 8.82 (s, 1H), 8.73 (s, 1H), 7.89 (dd, *J* = 8.3, 2.6 Hz, 1H), 7.73 (d, *J* = 8.3 Hz, 1H), 7.05 (s, 1H, NH, D₂O exchanged), 6.57 (s, 1H), 3.19 (s, 3H), 3.09 (s, 3H), 2.38 ― 2.25 (m, 4H), 2.23 ― 2.10 (m, 4H), 1.82 ― 1.53 (m, 6H). MS (ESI⁺) : [M+H]⁺ 407.2. |
| **141** | 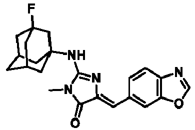 | C₂₂H₂₃FN₄O₂ | 394.45 | >98% | Yellow solid. ¹H NMR (400 MHz, DMSO-*d*₆, 300K) δ_{H} 8.80 (s, 1H), 8.74 (s, 1H), 7.89 (dd, *J* = 8.3, 1.4 Hz, 1H), 7.75 (d, *J* = 8.3 Hz, 1H), 7.14 (s, 1H, NH, D₂O exchanged), 6.59 (s, 1H), 3.09 (s, 3H), 2.50 ― 2.38 (m, 4H), 2.27 ― 2.12 (m, 4H), 1.91 (br s, 4H), 1.61 (s, 2H). ¹⁹F NMR (376 MHz, DMSO-*d*₆, 300K) δ_{F} -128.84. MS (ESI⁺) : [M+H]⁺ 395.2. |
| **142** | 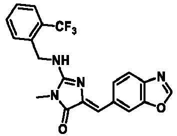 | C₂₀H₁₅F₃N₄O₂ | 400.36 | >98% | Yellow solid. ¹H NMR (400 MHz, DMSO-*d*₆, 300K) δ_{H} 8.71 (s, 1H), 8.62 ― 8.54 (m, 1H + NH, D₂O exchanged), 7.86 (dd, *J* = 8.5, 1.5 Hz, 1H), 7.81 ― 7.64 (m, 4H), 7.49 (t, *J* = 7.6 Hz, 1H), 6.57 (s, 1H), 4.88 (d, *J* = 5.6 Hz, 2H), 3.18 (s, 3H). ¹⁹F NMR (376 MHz, DMSO-*d*₆, 300K) δ_{F} -58.69. MS (ESI⁺) : [M+H]⁺ 401.2. |
| **143** | 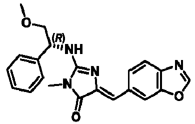 | C₂₁H₂₀N₄O₃ | 376.42 | >98% | Yellow solid. ¹H NMR (400 MHz, DMSO-*d*₆, 300K) δ_{H} 8.75 (s, 1H), 8.62 (s, 1H), 8.26 (d, *J* = 7.9 Hz, 1H, NH, D₂O exchanged), 7.98 (dd, *J* = 8.4, 1.4 Hz, 1H), 7.74 (d, *J* = 8.3 Hz, 1H), 7.57 ― 7.50 (m, 2H), 7.43 ― 7.36 (m, 2H), 7.32 ― 7.25 (m, 1H), 6.55 (s, 1H), 5.57 ― 5.38 (m, 1H), 3.84 (dd, *J* = 10.2, 8.7 Hz, 1H), 3.68 (dd, *J* = 10.2, 5.3 Hz, 1H), 3.36 (s, 3H), 3.16 (s, 3H). MS (ESI⁺) : [M+H]⁺ 377.2. |
| **144** | 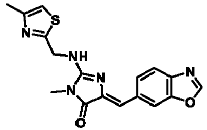 | C₁₇H₁₅N₅O₂S | 353.40 | >98% | Yellow solid. ¹H NMR (400 MHz, DMSO-*d*₆, 300K) δ_{H} 8.80 ― 8.74 (m, 1H + NH, D₂O exchanged), 8.70 (s, 1H), 8.00 (dd, *J* = 8.4, 1.5 Hz, 1H), 7.74 (d, *J* = 8.3 Hz, 1H), 7.19 (d, *J* = 1.2 Hz, 1H), 6.63 (s, 1H), 4.90 (s, 2H), 3.11 (s, 3H), 2.40 ― 2.33 (m, 3H). MS (ESI⁺) : [M+H]⁺ 354.2. |
| **145** | 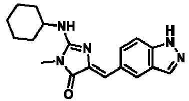 | C₁₈H₂₁N₅O | 323.40 | 95% | Yellow solid. ¹H NMR (400 MHz, DMSO-*d*₆, 300K) δ_{H} 13.09 (s, 1H, NH, D₂O exchanged), 8.44 (s, 1H), 8.21 (dd, *J* = 8.8, 1.5 Hz, 1H), 8.08 (s, 1H), 7.51 (d, *J* = 8.7 Hz, 1H), 7.35 (d, *J* = 7.8 Hz, 1H, NH, D₂O exchanged), 6.53 (s, 1H), 3.97 ― 3.86 (m, 1H), 3.07 (s, 3H), 2.10 ― 1.95 (m, 2H), 1.88 ― 1.75 (m, 2H), 1.72 ― 1.62 (m, 1H), 1.50 ― 1.32 (m, 4H), 1.26 ― 1.12 (m, 1H). MS (ESI⁺) : [M+H]⁺ 324.2. |
| **146** | 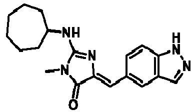 | C₁₉H₂₃N₅O | 337.43 | >98% | Yellow solid. ¹H NMR (400 MHz, DMSO-*d*₆, 300K) δ_{H} 13.09 (s, 1H, NH, D₂O exchanged), 8.47 (s, 1H), 8.23 (dd, *J* = 8.8, 1.5 Hz, 1H), 8.06 (s, 1H), 7.50 (d, *J* = 8.8 Hz, 1H), 7.37 (d, *J* = 7.7 Hz, 1H, NH, D₂O exchanged), 6.53 (s, 1H), 4.15 ― 4.06 (m, 1H), 3.07 (s, 3H), 2.08 ― 1.98 (m, 2H), 1.80 ― 1.46 (m, 10H). MS (ESI⁺) : [M+H]⁺ 338.3. |
| **147** | 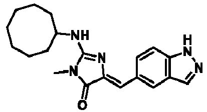 | C₂₀H₂₅N₅O | 351.45 | >98% | Yellow solid. ¹H NMR (400 MHz, DMSO-*d*₆, 300K) δ_{H} 13.09 (s, 1H, NH, D₂O exchanged), 8.55 (s, 1H), 8.20 (dd, *J* = 8.8, 1.5 Hz, 1H), 8.02 (s, 1H), 7.49 (d, *J* = 8.8 Hz, 1H), 7.40 (d, *J* = 7.8 Hz, 1H, NH, D₂O exchanged), 6.53 (s, 1H), 4.26 ― 4.17 (m, 1H), 3.07 (s, 3H), 1.95 ― 1.55 (m, 14H). MS (ESI⁺) : [M+H]⁺ 352.3. |
| **148** | 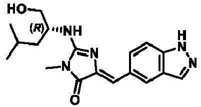 | C₁₈H₂₃N₅O₂ | 341.42 | >98% | Yellow solid. ¹H NMR (400 MHz, DMSO-*d*₆, 300K) δ_{H} 13.07 (s, 1H, NH, D₂O exchanged), 8.47 (s, 1H), 8.21 (dd, *J* = 8.8, 1.4 Hz, 1H), 8.03 (s, 1H), 7.48 (d, *J* = 8.8 Hz, 1H), 7.22 (d, *J* = 8.5 Hz, 1H, NH, D₂O exchanged), 6.52 (s, 1H), 4.82 (t, *J* = 5.8 Hz, 1H, OH, D₂O exchanged), 4.28 ― 4.16 (m, 1H), 3.60 ― 3.48 (m, 2H), 3.09 (s, 3H), 1.80 ― 1.65 (m, 1H), 1.56 (ddd, *J* = 14.1, 9.3, 5.2 Hz, 1H), 1.45 (ddd, *J* = 13.6, 8.8, 4.9 Hz, 1H), 0.99 (d, *J* = 4.3 Hz, 3H), 0.97 (d, *J* = 4.4 Hz, 3H). MS (ESI⁺) : [M+H]⁺ 342.2. |
| **149** | 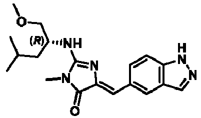 | C₁₉H₂₅N₅O₂ | 355.44 | >98% | Yellow solid. ¹H NMR (400 MHz, DMSO-*d*₆, 300K) δ_{H} 13.09 (s, 1H, NH, D₂O exchanged), 8.47 (s, 1H), 8.21 (d, *J* = 8.8 Hz, 1H), 8.03 (s, 1H), 7.49 (d, *J* = 8.8 Hz, 1H), 7.39 (d, *J* = 8.5 Hz, 1H, NH, D₂O exchanged), 6.54 (s, 1H), 4.45 ― 4.36 (m, 1H), 3.51 (dd, *J* = 9.8, 6.4 Hz, 1H), 3.44 (dd, *J* = 9.8, 4.6 Hz, 1H), 3.33 (s, 3H), 3.08 (s, 3H), 1.77 ― 1.67 (m, 1H), 1.61 ― 1.53 (m, 1H), 1.45 ― 1.37 (m, 1H), 1.00 ― 0.93 (m, 6H). MS (ESI⁺) : [M+H]⁺ 356.3. |
| **150** | 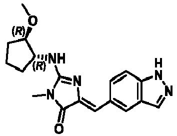 | C₁₈H₂₁N₅O₂ | 339.40 | >98% | Yellow solid. ¹H NMR (400 MHz, DMSO-*d*₆, 300K) δ_{H} 13.08 (s, 1H, NH, D₂O exchanged), 8.51 (s, 1H), 8.20 (dd, *J* = 8.8, 1.5 Hz, 1H), 8.05 (s, 1H), 7.49 (d, *J* = 8.7 Hz, 1H), 7.41 (d, *J* = 7.2 Hz, 1H, NH, D₂O exchanged), 6.57 (s, 1H), 4.37 ― 4.29 (m, 1H), 3.88 ― 3.82 (m, 1H), 3.32 (s, 3H), 3.08 (s, 3H), 2.22 ― 2.05 (m, 1H), 2.00 ― 1.95 (m, 1H), 1.95 ― 1.55 (m, 4H). MS (ESI⁺) : [M+H]⁺ 340.2. |
| **151** | 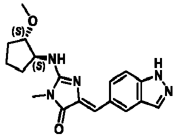 | C₁₈H₂₁N₅O₂ | 339.40 | 96% | Yellow solid. ¹H NMR (400 MHz, DMSO-*d*₆, 300K) δ_{H} 13.08 (s, 1H, NH, D₂O exchanged), 8.51 (s, 1H), 8.20 (dd, *J* = 8.8, 1.5 Hz, 1H), 8.05 (s, 1H), 7.49 (d, *J* = 8.7 Hz, 1H), 7.41 (d, *J* = 7.2 Hz, 1H, NH, D₂O exchanged), 6.57 (s, 1H), 4.37 ― 4.29 (m, 1H), 3.88 ― 3.82 (m, 1H), 3.32 (s, 3H), 3.08 (s, 3H), 2.22 ― 2.05 (m, 1H), 2.00 ― 1.95 (m, 1H), 1.95 ― 1.55 (m, 4H). MS (ESI⁺) : [M+H]⁺ 340.2. |
| **152** | 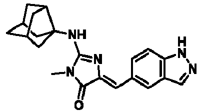 | C₂₁H₂₃N₅O | 361.45 | >98% | Orange solid. ¹H NMR (400 MHz, DMSO-*d*₆, 300K) δ_{H} 13.09 (s, 1H, NH, D₂O exchanged), 8.53 (s, 1H), 8.17 (d, *J* = 8.9 Hz, 1H), 8.01 (s, 1H), 7.49 (d, *J* = 8.8 Hz, 1H), 7.32 (s, 1H, NH, D₂O exchanged), 6.55 (s, 1H), 3.10 (s, 3H), 2.82 ― 2.74 (m, 1H), 2.42 ― 2.30 (m, 4H), 2.25 ― 2.15 (m, 2H), 2.05 ― 1.95 (m, 2H), 1.74 - 1.54 (m, 4H). MS (ESI⁺) : [M+H]⁺ 362.3. |
| **153** | 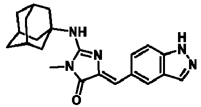 | C₂₂H₂₅N₅O | 375.48 | >98% | Yellow solid. ¹H NMR (400 MHz, DMSO-*d*₆, 300K) δ_{H} 13.10 (s, 1H, NH, D₂O exchanged), 8.54 (s, 1H), 8.20 (d, *J* = 8.8 Hz, 1H), 8.00 (s, 1H), 7.49 (d, *J* = 8.8 Hz, 1H), 6.71 (s, 1H, NH, D₂O exchanged), 6.55 (s, 1H), 3.07 (s, 3H), 2.32 ― 2.24 (m, 6H), 2.16 (s, 3H), 1.75 (s, 6H). MS (ESI⁺) : [M+H]⁺ 376.2. |
| **154** | 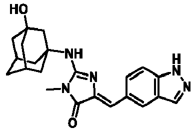 | C₂₂H₂₅N₅O₂ | 391.48 | >98% | Yellow solid. ¹H NMR (400 MHz, DMSO-*d*₆, 300K) δ_{H} 13.10 (s, 1H, NH, D₂O exchanged), 8.45 (s, 1H), 8.19 (d, *J* = 8.9 Hz, 1H), 8.01 (s, 1H), 7.49 (d, *J* = 8.8 Hz, 1H), 6.78 (s, 1H, NH, D₂O exchanged), 6.55 (s, 1H), 4.63 (s, 1H, OH, D₂O exchanged), 3.07 (s, 3H), 2.32 ― 2.06 (m, 8H), 1.73 ― 1.52 (m, 6H). MS (ESI⁺) : [M+H]⁺ 392.1. |
| **155** | 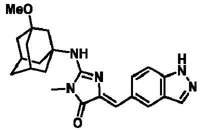 | C₂₃H₂₇N₅O₂ | 405.50 | >98% | Yellow solid. ¹H NMR (400 MHz, DMSO-*d*₆, 300K) δ_{H} 13.10 (s, 1H, NH, D₂O exchanged), 8.55 (s, 1H), 8.20 (d, *J* = 8.8 Hz, 1H), 7.99 (s, 1H), 7.48 (d, *J* = 8.7 Hz, 1H), 6.86 (s, 1H, NH, D₂O exchanged), 6.56 (s, 1H), 3.19 (s, 3H), 3.07 (s, 3H), 2.38 ― 2.26 (m, 4H), 2.24 ― 2.09 (m, 4H), 1.84 ― 1.54 (m, 6H). MS (ESI⁺) : [M+H]⁺ 406.2. |
| **156** | 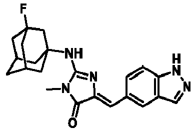 | C₂₂H₂₄FN₅O | 393.47 | >98% | Yellow solid. ¹H NMR (400 MHz, DMSO-*d*₆, 300K) δ_{H} 13.11 (s, 1H, NH, D₂O exchanged), 8.52 (s, 1H), 8.19 (d, *J* = 8.9 Hz, 1H), 8.01 (s, 1H), 7.50 (d, *J* = 8.8 Hz, 1H), 6.95 (s, 1H, NH, D₂O exchanged), 6.58 (s, 1H), 3.08 (s, 3H), 2.48 ― 2.35 (m, 4H), 2.27 ― 2.12 (m, 4H), 1.91 (s, 4H), 1.68 ― 1.56 (m, 2H). ¹⁹F NMR (376 MHz, DMSO-*d*₆, 300K) δ_{F} -128.72. MS (ESI⁺) : [M+H]⁺ 394.3. |
| **157** | 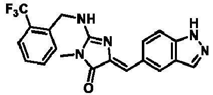 | C₂₀H₁₆F₃N₅O | 399.38 | 97% | Yellow solid. ¹H NMR (400 MHz, DMSO-*d*₆, 300K) δ_{H} 13.06 (s, 1H, NH, D₂O exchanged), 8.44 (s, 1H), 8.40 (m, 1H, NH, D₂O exchanged), 8.08 (dd, *J* = 8.8, 1.4 Hz, 1H), 7.97 (s, 1H), 7.83 ― 7.65 (m, 3H), 7.49 (t, *J* = 7.6 Hz, 1H), 7.42 (d, *J* = 8.7 Hz, 1H), 6.57 (s, 1H), 4.87 (s, 2H), 3.17 (s, 3H). ¹⁹F NMR (376 MHz, DMSO-*d*₆, 300K) δ_{F}-58.56. MS (ESI⁺) : [M+H]⁺ 400.1. |
| **158** | 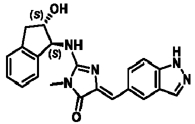 | C₂₁H₁₉N₅O₂ | 373.42 | >98% | Yellow solid. ¹H NMR (400 MHz, DMSO-*d*₆, 300K) δ_{H} 13.06 (s, 1H), 8.36 (s, 1H), 8.19 (d, *J* = 8.8 Hz, 1H), 8.05 (s, 1H), 7.98 (d, *J* = 8.4 Hz, 1H, NH, D₂O exchanged), 7.48 (d, *J* = 8.8 Hz, 1H), 7.32 ― 7.15 (m, 4H), 6.60 (s, 1H), 5.54 (d, *J* = 5.2 Hz, 1H, OH, D₂O exchanged), 5.45 (t, *J* = 7.7 Hz, 1H), 4.60 ― 4.48 (m, 1H), 3.25 (dd, *J* = 15.6, 7.4 Hz, 1H), 3.13 (s, 3H), 2.83 (dd, *J* = 15.6, 7.8 Hz, 1H). MS (ESI⁺) : [M+H]⁺ 374.2. |
| **159** | 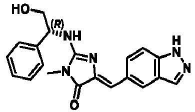 | C₂₀H₁₉N₅O₂ | 361.41 | >98% | Yellow solid. ¹H NMR (400 MHz, DMSO-*d*₆, 300K) δ_{H} 13.08 (s, 1H, NH, D₂O exchanged), 8.45 (s, 1H), 8.18 ― 8.08 (m, 2H), 7.94 (d, *J* = 7.9 Hz, 1H, NH, D₂O exchanged), 7.57 ― 7.46 (m, 3H), 7.38 (t, *J* = 7.6 Hz, 2H), 7.26 (t, *J* = 7.4 Hz, 1H), 6.52 (s, 1H), 5.28 ― 5.16 (m, 1H), 5.06 (t, *J* = 5.8 Hz, 1H, OH, D₂O exchanged), 3.90 ― 3.80 (m, 1H), 3.79 ― 3.70 (m, 1H), 3.16 (s, 3H). MS (ESI⁺) : [M+H]⁺ 362.2. |
| **160** | 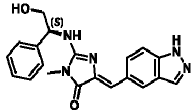 | C₂₀H₁₉N₅O₂ | 361.41 | >98% | Yellow solid. ¹H NMR (400 MHz, DMSO-*d*₆, 300K) δ_{H} 13.08 (s, 1H, NH, D₂O exchanged), 8.45 (s, 1H), 8.18 ― 8.08 (m, 2H), 7.94 (d, *J* = 7.9 Hz, 1H, NH, D₂O exchanged), 7.57 ― 7.46 (m, 3H), 7.38 (t, *J* = 7.6 Hz, 2H), 7.26 (t, *J* = 7.4 Hz, 1H), 6.52 (s, 1H), 5.28 ― 5.16 (m, 1H), 5.06 (t, *J* = 5.8 Hz, 1H, OH, D₂O exchanged), 3.90 ― 3.80 (m, 1H), 3.79 ― 3.70 (m, 1H), 3.16 (s, 3H). MS (ESI⁺) : [M+H]⁺ 362.2. |
| **161** | 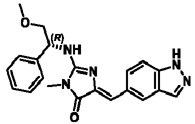 | C₂₁H₂₁N₅O₂ | 375.43 | >98% | Yellow solid. ¹H NMR (400 MHz, DMSO-*d*₆, 300K) δ_{H} 13.09 (s, 1H, NH, D₂O exchanged), 8.45 (s, 1H), 8.14 (dd, *J* = 8.8, 1.5 Hz, 1H), 8.12 ― 8.09 (m, 1H), 8.06 (d, *J* = 7.9 Hz, 1H, NH, D₂O exchanged), 7.60 ― 7.47 (m, 3H), 7.43 ― 7.34 (m, 2H), 7.30 ― 7.23 (m, 1H), 6.53 (s, 1H), 5.46 ― 5.36 (m, 1H), 3.88 ― 3.78 (m, 1H), 3.66 (dd, *J* = 10.1, 5.3 Hz, 1H), 3.32 (s, 3H), 3.14 (s, 3H). MS (ESI⁺) : [M+H]⁺ 376.1. |
| **162** | 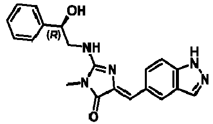 | C₂₀H₁₉N₅O₂ | 361.41 | >98% | Yellow solid. ¹H NMR (400 MHz, DMSO-*d*₆, 300K) δ_{H} 13.12 (s, 1H, NH, D₂O exchanged), 8.56 (s, 1H), 8.25 (d, *J* = 8.9 Hz, 1H), 8.07 (s, 1H), 7.96 (t, *J* = 5.6 Hz, 1H, NH, D₂O exchanged), 7.58 ― 7.48 (m, 3H), 7.44 (t, *J* = 7.5 Hz, 2H), 7.36 ― 7.28 (m, 1H), 6.59 (s, 1H), 5.70 (d, *J* = 4.3 Hz, 1H, OH, D₂O exchanged), 5.16 ― 5.06 (m, 1H), 3.80 ― 3.66 (m, 1H), 3.43 ― 3.32 (m, 1H), 3.10 (s, 3H). MS (ESI⁺) : [M+H]⁺ 362.2. |
| **163** | 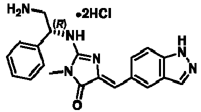 | C₂₀H₂₂Cl₂N₆O | 433.34 | 88% | Yellow solid. ¹H NMR (400 MHz, DMSO-*d*₆, 300K) δ_{H} 7.74 (s, 1H), 7.65 ― 7.50 (m, 5H), 7.38 (s, 1H), 7.25 ― 7.14 (m, 2H), 6.29 (s, 1H), 5.24 (dd, *J* = 8.6, 6.1 Hz, 1H), 3.62 (dd, *J* = 13.4, 8.7 Hz, 1H), 3.52 (dd, *J* = 13.4, 6.0 Hz, 1H), 2.97 (s, 3H). MS (ESI⁺) : [M+H]⁺ 361.2 (+2HCl). |
| **164** | 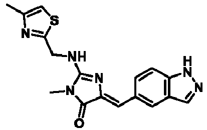 | C₁₇H₁₆N₆OS | 352.42 | >98% | Yellow solid. ¹H NMR (400 MHz, DMSO-*d*₆, 300K) δ_{H} 13.09 (s, 1H, NH, D₂O exchanged), 8.54 (t, *J* = 5.8 Hz, 1H, NH, D₂O exchanged), 8.49 (s, 1H), 8.22 (dd, *J* = 8.8, 1.5 Hz, 1H), 8.07 (s, 1H), 7.50 (d, *J* = 8.8 Hz, 1H), 7.20 - 7.18 (m, 1H), 6.63 (s, 1H), 4.90 (d, *J* = 5.6 Hz, 2H), 3.10 (s, 3H), 2.37 (s, 3H). MS (ESI⁺) : [M+H]⁺ 353.1. |
| **165** | 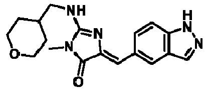 | C₁₈H₂₁N₅O₂ | 339.40 | >98% | Yellow solid. ¹H NMR (400 MHz, DMSO-*d*₆, 300K) δ_{H} 13.08 (s, 1H, NH, D₂O exchanged), 8.46 (s, 1H), 8.23 (d, *J* = 8.8 Hz, 1H), 8.07 (s, 1H), 7.63 (t, *J* = 5.8 Hz, 1H, NH, D₂O exchanged), 7.51 (d, *J* = 8.7 Hz, 1H), 6.54 (s, 1H), 3.95 - 3.85 (m, 2H), 3.0 - 3.25 (m, 4H), 3.08 (s, 3H), 2.06 - 1.92 (m, 1H), 1.76 - 1.65 (m, 2H), 1.38 - 1.22 (m, 2H). MS (ESI⁺) : [M+H]⁺ 340.1. |
| **166** | 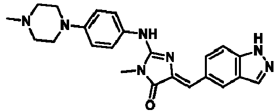 | C₂₃H₂₅N₇O | 415.50 | >98% | Yellow solid. ¹H NMR (400 MHz, DMSO-*d*₆, 300K) δ_{H} 13.15 (s, 1H, NH, D₂O exchanged), 9.21 (s, 1H, NH, D₂O exchanged), 8.53 (s, 1H), 8.21 (d, *J* = 8.8 Hz, 1H), 8.17 (s, 1H), 7.84 (d, *J* = 8.9 Hz, 2H), 7.56 (d, *J* = 8.8 Hz, 1H), 7.06 (d, *J* = 8.8 Hz, 2H), 6.71 (s, 1H), 3.23 (s, 3H), 3.20 - 3.13 (m, 4H), 2.52 - 2.45 (m, 4H), 2.25 (s, 3H). MS (ESI⁺) : [M+H]⁺ 416.1. |
| **167** | 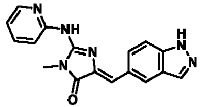 | C₁₇H₁₄N₆O | 318.34 | >98% | Yellow solid. ¹H NMR (400 MHz, CF₃COOD, 300K) δ_{H} 9.01 (s, 1H), 8.60 - 8.52 (m, 2H), 8.47 (s, 1H), 8.25 (d, *J* = 8.9 Hz, 1H), 8.16 (d, *J* = 9.1 Hz, 1H), 7.97 -7.90 (m, 1H), 7.71 (t, *J* = 6.8 Hz, 1H), 7.53 (s, 1H), 3.65 (s, 3H). MS (ESI⁺) : [M+H]⁺ 319.1. |
| **168** | 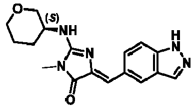 | C₁₇H₁₉N₅O₂ | 325.37 | >98% | Yellow solid. ¹H NMR (400 MHz, DMSO-*d*₆, 300K) δ_{H} 13.10 (s, 1H, NH, D₂O exchanged), 8.38 (s, 1H), 8.25 (d, *J* = 8.8 Hz, 1H), 8.09 (s, 1H), 7.53 (d, *J* = 8.8 Hz, 1H), 7.33 (d, *J* = 7.6 Hz, 1H, NH, D₂O exchanged), 6.58 (s, 1H), 4.08 (br s, 1H), 4.03 - 3.94 (m, 1H), 3.87 - 3.76 (m, 1H), 3.45 - 3.22 (m, 2H), 3.08 (s, 3H), 2.15 - 2.00 (m, 1H), 1.85 - 1.60 (m, 3H). MS (ESI⁺) : [M+H]⁺ 326.2. |
| **169** | 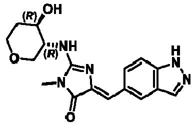 | C₁₇H₁₉N₅O₃ | 341.37 | >98% | Yellow solid. ¹H NMR (400 MHz, DMSO-*d*₆, 300K) δ_{H} 13.10 (s, 1H, NH, D₂O exchanged), 8.37 (s, 1H), 8.25 (d, *J* = 8.9 Hz, 1H), 8.09 (s, 1H), 7.54 (d, *J* = 8.8 Hz, 1H), 7.34 (d, *J* = 7.8 Hz, 1H, NH, D₂O exchanged), 6.57 (s, 1H), 5.10 (d, *J* = 4.7 Hz, 1H, OH, D₂O exchanged), 4.03 - 3.84 (m, 3H), 3.82 - 3.72 (m, 1H), 3.38 (t, *J* = 11.6 Hz, 1H), 3.20 (t, *J* = 10.4 Hz, 1H), 3.10 (s, 3H), 2.02 - 1.90 (m, 1H), 1.65-1.51 (m, 1H). MS (ESI⁺) : [M+H]⁺ 342.2. |
| **170** | 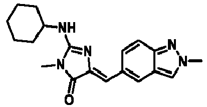 | C₁₉H₂₃N₅O | 337.43 | >98% | Yellow solid. ¹H NMR (400 MHz, DMSO-*d*₆, 300K) δ_{H} 8.49 - 8.33 (m, 2H), 8.11 (dd, *J* = 9.1, 1.5 Hz, 1H), 7.54 (d, *J* = 9.0 Hz, 1H), 7.33 (d*, J* = 7.7 Hz, 1H, NH, D₂O exchanged), 6.48 (s, 1H), 4.15 (s, 3H), 3.90 (m, 1H), 3.06 (s, 3H), 2.10 - 1.95 (m, 2H), 1.90 - 1.72 (m, 2H), 1.73 - 1.60 (m, 1H), 1.50 - 1.30 (m, 4H), 1.28 - 1.10 (m, 1H). MS (ESI⁺) : [M+H]⁺ 338.3. |
| **171** | 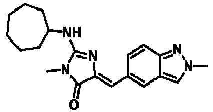 | C₂₀H₂₅N₅O | 351.45 | >98% | Yellow solid. ¹H NMR (400 MHz, DMSO-*d*₆, 300K) δ_{H} 8.38 (s, 1H), 8.35 (s, 1H), 8.11 (dd, *J* = 9.0, 1.5 Hz, 1H), 7.53 (d, *J* = 9.0 Hz, 1H), 7.36 (d, *J* = 7.6 Hz, 1H, NH, D₂O exchanged), 6.48 (s, 1H), 4.15 (s, 3H), 4.13 - 4.02 (m, 1H), 3.06 (s, 3H), 2.10 - 1.95 (m, 2H), 1.82 - 1.45 (m, 10H). MS (ESI⁺) : [M+H]⁺ 352.3. |
| **172** | 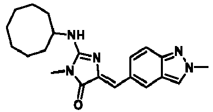 | C₂₁H₂₇N₅O | 365.48 | >98% | Yellow solid. ¹H NMR (400 MHz, DMSO-*d*₆, 300K) δ_{H} 8.43 (s, 1H), 8.31 (s, 1H), 8.10 (dd, *J* = 9.1, 1.5 Hz, 1H), 7.51 (d, *J* = 9.0 Hz, 1H), 7.37 (d, *J* = 7.6 Hz, 1H, NH, D₂O exchanged), 6.48 (s, 1H), 4.26 - 4.17 (m, 1H), 4.16 (s, 3H), 3.06 (s, 3H), 2.00 - 1.50 (m, 14H). MS (ESI⁺) : [M+H]⁺ 366.3. |
| **173** | 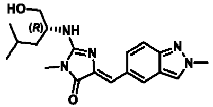 | C₁₉H₂₅N₅O₂ | 355.44 | 96% | Yellow solid. ¹H NMR (400 MHz, DMSO-*d*₆, 300K) δ_{H} 8.36 (s, 1H), 8.33 (s, 1H), 8.12 (d, *J* = 9.0 Hz, 1H), 7.51 (d, *J* = 9.0 Hz, 1H), 7.24 (d, *J* = 8.5 Hz, 1H, NH, D₂O exchanged), 6.47 (s, 1H), 4.83 (t, *J* = 5.8 Hz, 1H, OH, D₂O exchanged), 4.26 - 4.17 (m, 1H), 4.15 (s, 3H), 3.60 - 3.48 (m, 2H), 3.08 (s, 3H), 1.78 - 1.66 (m, 1H), 1.56 (ddd, *J* = 14.0, 9.5, 5.2 Hz, 1H), 1.44 (ddd, *J* = 13.7, 8.8, 4.7 Hz, 1H), 1.01 - 0.94 (m, 6H). MS (ESI⁺) : [M+H]⁺ 356.2. |
| **174** | 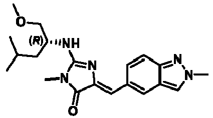 | C₂₀H₂₇N₅O₂ | 369.47 | >98% | Yellow solid. ¹H NMR (400 MHz, DMSO-*d*₆, 300K) δ_{H} 8.37 (s, 1H), 8.33 (s, 1H), 8.11 (dd, *J* = 9.1, 1.6 Hz, 1H), 7.52 (d, *J* = 9.1 Hz, 1H), 7.36 (d, *J* = 8.5 Hz, 1H, NH, D₂O exchanged), 6.49 (s, 1H), 4.47 - 4.32 (m, 1H), 4.15 (s, 3H), 3.51 (dd, *J* = 9.8, 6.3 Hz, 1H), 3.43 (dd, *J* = 9.8, 5.6 Hz, 1H), 3.32 (s, 3H), 3.07 (s, 3H), 1.80 - 1.66 (m, 1H), 1.62 - 1.52 (m, 1H), 1.46 - 1.36 (m, 1H), 1.02 - 0.91 (m, 6H). MS (ESI⁺) : [M+H]⁺ 370.3. |
| **175** | 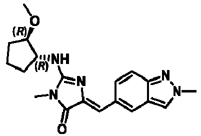 | C₁₉H₂₃N₅O₂ | 353.43 | >98% | Yellow solid. ¹H NMR (400 MHz, DMSO-*d*₆, 300K) δ_{H} 8.40 (s, 1H), 8.34 (s, 1H), 8.12 (dd, *J* = 9.1, 1.5 Hz, 1H), 7.52 (d, *J* = 9.0 Hz, 1H), 7.41 (d, *J* = 7.2 Hz, 1H, NH, D₂O exchanged), 6.52 (s, 1H), 4.39 - 4.24 (m, 1H), 4.15 (s, 3H), 3.88 - 3.80 (m, 1H), 3.41 (s, 3H), 3.07 (s, 3H), 2.20 - 2.06 (m, 1H), 1.98 - 1.86 (m, 1H), 1.84 - 1.56 (m, 4H). MS (ESI⁺): [M+H]⁺ 354.2. |
| **176** | 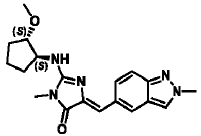 | C₁₉H₂₃N₅O₂ | 353.43 | >98% | Yellow solid. ¹H NMR (400 MHz, DMSO-*d*₆, 300K) δ_{H} 8.40 (s, 1H), 8.34 (s, 1H), 8.12 (dd, *J* = 9.1, 1.5 Hz, 1H), 7.52 (d, *J* = 9.0 Hz, 1H), 7.41 (d, *J* = 7.2 Hz, 1H, NH, D₂O exchanged), 6.52 (s, 1H), 4.39 - 4.24 (m, 1H), 4.15 (s, 3H), 3.88 - 3.80 (m, 1H), 3.41 (s, 3H), 3.07 (s, 3H), 2.20 - 2.06 (m, 1H), 1.98 - 1.86 (m, 1H), 1.84 - 1.56 (m, 4H). MS (ESI⁺): [M+H]⁺ 354.2. |
| **177** | 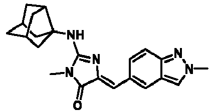 | C₂₂H₂₅N₅O | 375.48 | >98% | Pale yellow. ¹H NMR (400 MHz, DMSO-*d*₆, 300K) δ_{H} 8.47 (s, 1H), 8.32 (s, 1H), 8.04 (d, *J* = 9.1 Hz, 1H), 7.50 (d, *J* = 9.0 Hz, 1H), 7.32 (s, 1H, NH, D₂O exchanged), 6.50 (s, 1H), 4.15 (s, 3H), 3.09 (s, 3H), 2.78 (t, *J* = 6.7 Hz, 1H), 2.42 - 2.30 (m, 4H), 2.24 - 2.13 (m, 2H), 2.06 - 1.94 (m, 2H), 1.73 - 1.53 (m, 4H). MS (ESI⁺) : [M+H]⁺ 376.3. |
| **178** | 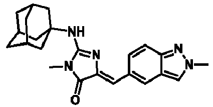 | C₂₃H₂₇N₅O | 389.50 | >98% | Yellow solid. ¹H NMR (400 MHz, DMSO-*d*₆, 300K) δ_{H} 8.44 (s, 1H), 8.31 (s, 1H), 8.09 (d, *J* = 9.1 Hz, 1H), 7.53 (d, *J* = 9.0 Hz, 1H), 6.69 (s, 1H, NH, D₂O exchanged), 6.49 (s, 1H), 4.16 (s, 3H), 3.07 (s, 3H), 2.27 (s, 6H), 2.16 (s, 3H), 1.75 (s, 6H). MS (ESI⁺) : [M+H]⁺ 390.1. |
| **179** | 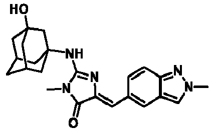 | C₂₃H₂₇N₅O₂ | 405.50 | >98% | Pale yellow solid. ¹H NMR (400 MHz, DMSO-*d*₆, 300K) δ_{H} 8.43 (s, 1H), 8.30 (s, 1H), 8.09 (d, *J* = 9.1 Hz, 1H), 7.53 (d, *J* = 9.0 Hz, 1H), 6.76 (s, 1H, NH, D₂O exchanged), 6.50 (s, 1H), 4.62 (s, 1H, OH, D₂O exchanged), 4.17 (s, 3H), 3.07 (s, 3H), 2.32 - 2.05 (m, 8H), 1.75 - 1.50 (m, 6H). MS (ESI⁺) : [M+H]⁺ 406.2. |
| **180** | 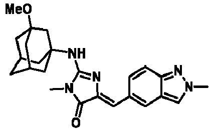 | C₂₄H₂₉N₅O₂ | 419.53 | >98% | Pale yellow solid. ¹H NMR (400 MHz, DMSO-*d*₆, 300K) δ_{H} 8.42 (s, 1H), 8.29 (s, 1H), 8.10 (d, *J* = 9.1 Hz, 1H), 7.51 (d, *J* = 9.0 Hz, 1H), 6.84 (s, 1H, NH, D₂O exchanged), 6.51 (s, 1H), 4.16 (s, 3H), 3.19 (s, 3H), 3.07 (s, 3H), 2.38 - 2.24 (m, 4H), 2.16 (s, 4H), 1.82 - 1.54 (m, 6H). MS (ESI⁺) : [M+H]⁺ 420.3. |
| **181** | 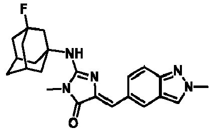 | C₂₃H₂₆FN₅O | 407.49 | >98% | Pale yellow solid. ¹H NMR (400 MHz, DMSO-*d*₆, 300K) δ_{H} 8.42 (s, 1H), 8.31 (s, 1H), 8.08 (d, *J* = 9.1 Hz, 1H), 7.53 (d, *J* = 9.1 Hz, 1H), 6.93 (s, 1H, NH, D₂O exchanged), 6.53 (s, 1H), 4.16 (s, 3H), 3.07 (s, 3H), 2.48 - 2.37 (m, 4H), 2.26 - 2.10 (m, 4H), 1.94 -1.86 (m, 4H), 1.61 (s, 2H). ¹⁹F NMR (376 MHz, DMSO-*d*₆, 300K) δ_{F} -128.67. MS (ESI⁺) : [M+H]⁺ 408.2. |
| **182** | 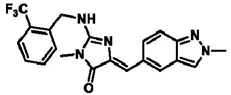 | C₂₁H₁₈F₃N₅O | 413.40 | >98% | Yellow solid. ¹H NMR (400 MHz, DMSO-*d*₆, 300K) δ_{H} 8.37 (t, *J* = 5.7 Hz, 1H, NH, D₂O exchanged), 8.32 (s, 1H), 8.25 (s, 1H), 7.99 (dd, *J* = 9.1, 1.5 Hz, 1H), 7.82 - 7.65 (m, 3H), 7.54 - 7.40 (m, 2H), 6.52 (s, 1H), 4.87 (d, *J* = 4.5 Hz, 2H), 4.15 (s, 3H), 3.17 (s, 3H). ¹⁹F NMR (376 MHz, DMSO-*d*₆, 300K) δ_{F} -58.61. MS (ESI⁺) : [M+H]⁺ 414.2. |
| **183** | 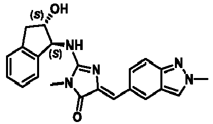 | C₂₂H₂₁N₅O₂ | 387.44 | 97% | Yellow solid. ¹H NMR (400 MHz, DMSO-*d*₆, 300K) δ_{H} 8.34 (s, 1H), 8.28 (s, 1H), 8.13 (d, *J* = 9.2 Hz, 1H), 7.99 (d, *J* = 8.3 Hz, 1H, NH D₂O exchanged), 7.51 (d, *J* = 9.1 Hz, 1H), 7.32 - 7.17 (m, 4H), 6.56 (s, 1H), 5.53 (d, *J* = 5.2 Hz, 1H, OH, D₂O exchanged), 5.44 (t, *J* = 7.6 Hz, 1H), 4.59 - 4.49 (m, 1H), 4.13 (s, 3H), 3.25 (dd, *J* = 15.6, 7.4 Hz, 1H), 3.13 (s, 3H), 2.84 (dd, *J* = 15.6, 7.9 Hz, 1H). MS (ESI⁺) : [M+H]⁺ 388.3. |
| **184** | 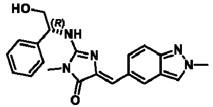 | C₂₁H₂₁N₅O₂ | 375.43 | >98% | Yellow solid. ¹H NMR (400 MHz, DMSO-*d*₆, 300K) δ_{H} 8.39 (s, 1H), 8.34 (s, 1H), 8.05 (dd, *J* = 9.1, 1.5 Hz, 1H), 7.93 (d, *J* = 7.8 Hz, 1H, NH, D₂O exchanged), 7.57 - 7.48 (m, 3H), 7.42 - 7.34 (m, 2H), 7.29 -7.22 (m, 1H), 6.47 (s, 1H), 5.25 - 5.16 (m, 1H), 5.02 (t, *J* = 5.8 Hz, 1H, OH, D₂O exchanged), 4.16 (s, 3H), 3.89 - 3.79 (m, 1H), 3.78 - 3.69 (m, 1H), 3.16 (s, 3H). MS (ESI⁺) : [M+H]+ 376.2. |
| **185** | 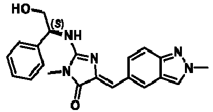 | C₂₁H₂₁N₅O₂ | 375.43 | >98% | Yellow solid. ¹H NMR (400 MHz, DMSO-*d*₆, 300K) δ_{H} 8.39 (s, 1H), 8.34 (s, 1H), 8.05 (dd, *J* = 9.1, 1.5 Hz, 1H), 7.93 (d, *J* = 7.8 Hz, 1H, NH, D₂O exchanged), 7.57 - 7.48 (m, 3H), 7.42 - 7.34 (m, 2H), 7.29 -7.22 (m, 1H), 6.47 (s, 1H), 5.25 - 5.16 (m, 1H), 5.02 (t, *J* = 5.8 Hz, 1H, OH, D₂O exchanged), 4.16 (s, 3H), 3.89 - 3.79 (m, 1H), 3.78 - 3.69 (m, 1H), 3.16 (s, 3H). MS (ESI⁺) : [M+H]+ 376.2. |
| 186 | 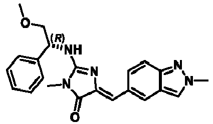 | C₂₂H₂₃N₅O₂ | 389.46 | >98% | Yellow solid. ¹H NMR (400 MHz, DMSO-*d*₆, 300K) δ_{H}. 8.38 (s, 1H), 8.34 (s, 1H), 8.05 (d, *J* = 8.6 Hz, 1H + NH, D₂O exchanged), 7.53 (d, *J =* 8.2 Hz, 3H), 7.38 (t, *J =* 7.5 Hz, 2H), 7.31-7.21 (m, 1H), 6.48 (s, 1H), 5.46 - 5.30 (m, 1H), 4.16 (s, 3H), 3.88 - 3.74 (m, 1H), 3.65 (dd, *J* = 10.2, 5.3 Hz, 1H), 3.34 (s, 3H), 3.13 (s, 3H) MS (ESI⁺) : [M+H]⁺ 390.1. |
| 187 | 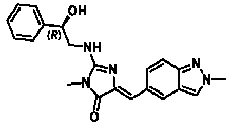 | C₂₁H₂₁N₅O₂ | 375.43 | >98% | Yellow solid. ¹H NMR (400 MHz, DMSO-*d*₆, 300K) δ_{H} 8.39 (s, 1H), 8.34 (s, 1H), 8.20 (d, *J* = 9.1 Hz, 1H), 7.93 (t, *J* = 5.7 Hz, 1H, NH, D₂O exchanged), 7.57 (d, *J* = 9.1 Hz, 1H), 7.53 - 7.40 (m, 4H), 7.36 - 7.27 (m, 1H), 6.53 (s, 1H), 5.68 (d, *J* = 4.3 Hz, 1H, OH, D₂O exchanged), 5.14 - 5.02 (m, 1H), 4.18 (s, 3H), 3.78 - 3.64 (m, 1H), 3.43 - 3.30 (m, 1H), 3.08 (s, 3H). MS (ESI⁺): [M+H]+ 376.2. |
| **188** | 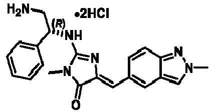 | C₂H₂₄Cl₂N₆O | 447.36 | 89% | Yellow solid. ¹H NMR (400 MHz, D₂O, 300K) δ_{H} 8.12 (s, 1H), 8.02 (s, 1H), 7.71 (dd, *J* = 9.1, 1.6 Hz, 1H), 7.53 (d, *J* = 9.0 Hz, 1H), 7.50 - 7.36 (m, 6H), 6.73 (s, 1H), 4.12 (s, 3H), 3.92 - 3.75 (m, 2H), 2.99 (s, 3H). MS (ESI⁺) : [M+H]⁺ 375.2 (+2HCl). |
| **189** | 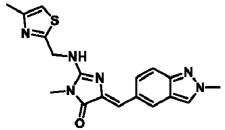 | C₁₈H₁₈N₆OS | 366.44 | >98% | Yellow solid. ¹H NMR (400 MHz, DMSO-*d*₆, 300K) δ_{H} 8.54 (br s, 1H, NH, D₂O exchanged), 8.38 (s, 1H), 8.36 (s, 1H), 8.15 (d, *J* = 9.1 Hz, 1H), 7.53 (d, *J* = 9.0 Hz, 1H), 7.19 (s, 1H), 6.58 (s, 1H), 4.89 (s, 2H), 4.15 (s, 3H), 3.10 (s, 3H), 2.37 (s, 3H). MS (ESI⁺) : [M+H]⁺ 367.1. |
| **190** | 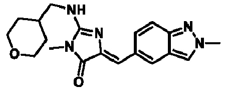 | C₁₉H₂₃N₅O₂ | 353.43 | >98% | Yellow solid. ¹H NMR (400 MHz, DMSO-*d*₆, 300K) δ_{H} 8.38 - 8.34 (m, 2H), 8.13 (dd, *J* = 9.1, 1.6 Hz, 1H), 7.63 (t, *J* = 5.7 Hz, 1H, NH, D₂O exchanged), 7.54 (d, *J* = 9.1 Hz, 1H), 6.49 (s, 1H), 4.16 (s, 3H), 3.94 - 3.86 (m, 2H), 3.38 (t, *J* = 6.3 Hz, 2H), 3.34 - 3.26 (m, 2H), 3.08 (s, 3H), 2.04 - 1.91 (m, 1H), 1.76 - 1.65 (m, 2H), 1.37 - 1.23 (m, 2H). MS (ESI⁺) : [M+H]⁺ 354.2. |
| **191** | 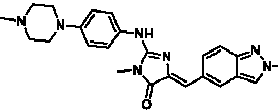 | C₂₄H₂₇N₇O | 429.53 | >98% | Yellow solid. ¹H NMR (400 MHz, DMSO-*d*₆, 300K) δ_{H} 9.20 (s, 1H, NH, D₂O exchanged), 8.43 (s, 1H), 8.41 (s, 1H), 8.13 (d, *J* = 9.2 Hz, 1H), 7.88 - 7.81 (m, 2H), 7.59 (d, *J* = 9.0 Hz, 1H), 7.09 - 7.01 (m, 2H), 6.66 (s, 1H), 4.17 (s, 3H), 3.23 (s, 3H), 3.20 - 3.13 (m, 4H), 2.51 - 2.45 (m, 4H), 2.25 (s, 3H). MS (ESI⁺) : [M+H]⁺ 430.1. |
| **192** | 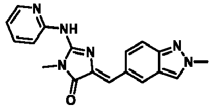 | C₁₈H₁₆N₆O | 332.37 | >98% | Orange solid. ¹H NMR (400 MHz, CDCl₃, 300K) δ_{H} 12.41 (br s, 1H, NH, D₂O exchanged), 8.37 (ddd, *J* = 5.1, 2.0, 0.8 Hz, 1H), 8.00 (s, 1H), 7.85 (s, 1H), 7.80 (d, *J* = 9.0 Hz, 1H), 7.70 (td, *J* = 7.3, 2.0 Hz, 1H), 7.50 (dd, *J* = 9.0, 1.6 Hz, 1H), 7.22 (d, *J* = 8.2 Hz, 1H), 7.00 (ddd, *J* = 7.3, 5.0, 1.1 Hz, 1H), 6.79 (s, 1H), 4.28 (s, 3H), 3.34 (s, 3H). MS (ESI⁺) : [M+H]⁺ 333.2. |
| **193** | 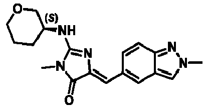 | C₁₈H₂₁N₅O₂ | 339.40 | 94% | Yellow solid. ¹H NMR (400 MHz, DMSO-*d*₆, 300K) δ_{H} 8.38 (s, 1H), 8.30 (s, 1H), 8.16 (dd, *J* = 9.2, 1.5 Hz, 1H), 7.56 (d, *J* = 9.1 Hz, 1H), 7.33 (d, *J* = 7.6 Hz, 1H, NH, D₂O exchanged), 6.53 (s, 1H), 4.15 (s, 3H), 4.12 - 3.96 (m, 2H), 3.87 - 3.77 (m, 1H), 3.40 - 3.22 (m, 2H), 3.07 (s, 3H), 2.13 - 2.00 (m, 1H), 1.82 - 1.62 (m, 3H). MS (ESI⁺) : [M+H]⁺ 340.2. |
| **194** | 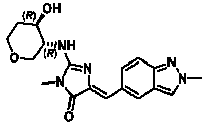 | C₁₈H₂₁N₅O₃ | 355.40 | >98% | Yellow solid. ¹H NMR (400 MHz, DMSO-*d*₆, 300K) δ_{H} 8.38 (s, 1H), 8.29 (s, 1H), 8.16 (dd, *J* = 9.1, 1.6 Hz, 1H), 7.57 (d, *J* = 9.1 Hz, 1H), 7.35 (d, *J* = 7.6 Hz, 1H, NH, D₂O echanged), 6.52 (s, 1H), 5.10 (d, *J* = 4.7 Hz, 1H, OH, D₂O exchanged), 4.15 (s, 3H), 3.99 (dd, *J* = 11.0, 4.7 Hz, 1H), 3.94 - 3.82 (m, 2H), 3.82 - 3.72 (m, 1H), 3.38 (td, *J* = 11.7, 2.2 Hz, 1H), 3.19 (t, *J* = 10.5 Hz, 1H), 3.09 (s, 3H), 2.02 - 1.90 (m, 1H), 1.65 - 1.50 (m, 1H). MS (ESI⁺) : [M+H]⁺ 356.2. |
| **195** | 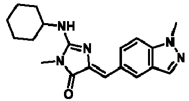 | C₁₉H₂₃N₅O | 337.43 | >98% | Orange solid. ¹H NMR (400 MHz, DMSO-*d*₆, 300K) δ_{H} 8.41 (s, 1H), 8.30 (d, *J* = 8.9 Hz, 1H), 8.06 (s, 1H), 7.63 (d, *J* = 8.8 Hz, 1H), 7.37 (d, *J* = 7.7 Hz, 1H, NH, D₂O exchanged), 6.54 (s, 1H), 4.04 (s, 3H), 3.92 (s, 1H), 3.07 (s, 3H), 2.04 (br s, 2H), 1.81 (br s, 2H), 1.73 - 1.65 (m, 1H), 1.50 - 1.31 (m, 4H), 1.28 - 1.12 (m, 1H). MS (ESI⁺) : [M+H]⁺ 338.2. |
| **196** | 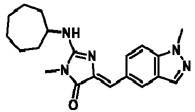 | C₂₀H₂₅N₅O | 351.45 | >98% | Orange solid. ¹H NMR (400 MHz, DMSO-*d*₆, 300K) δ_{H} 8.41 (s, 1H), 8.32 (d, *J* = 8.9 Hz, 1H), 8.03 (s, 1H), 7.62 (d, *J* = 8.8 Hz, 1H), 7.39 (d, *J* = 7.6 Hz, 1H, NH, D₂O exchanged), 6.53 (s, 1H), 4.16 - 4.06 (m, 1H), 4.04 (s, 3H), 3.06 (s, 3H), 2.10 - 1.94 (m, 2H), 1.85 ― 1.43 (m, 10H). MS (ESI⁺) : [M+H]⁺ 352.2. |
| **197** | 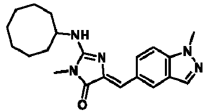 | C₂₁H₂₇N₅O | 365.48 | >98% | Orange solid. ¹H NMR (400 MHz, DMSO-*d*₆, 300K) δ_{H} 8.48 (s, 1H), 8.29 (d, *J* = 8.8 Hz, 1H), 7.99 (s, 1H), 7.60 (d, *J* = 8.9 Hz, 1H), 7.40 (d, *J* = 7.6 Hz, 1H, NH, D₂O exchanged), 6.53 (s, 1H), 4.27 - 4.15 (m, 1H), 4.04 (s, 3H), 3.06 (s, 3H), 2.00 - 1.41 (m, 14H). MS (ESI⁺) : [M+H]⁺ 366.2. |
| **198** | 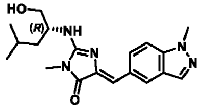 | C₁₉H₂₅N₅O₂ | 355.44 | 98% | Yellow solid. ¹H NMR (400 MHz, DMSO-*d*₆, 300K) δ_{H} 8.43 (s, 1H), 8.28 (dd, *J* = 8.9, 1.5 Hz, 1H), 8.01 (d, *J* = 1.3 Hz, 1H), 7.60 (d, *J* = 8.9 Hz, 1H), 7.25 (d, *J* = 8.5 Hz, 1H, NH, D₂O exchanged), 6.53 (s, 1H), 4.82 (t, *J* = 5.8 Hz, 1H, OH, D₂O exchanged), 4.23 (m, 1H), 4.04 (s, 3H), 3.60 - 3.48 (m, 2H), 3.09 (s, 3H), 1.80 - 1.65 (m, 1H), 1.56 (ddd, *J* = 14.0, 9.4, 5.2 Hz, 1H), 1.45 (ddd, *J* = 13.7, 8.8, 4.9 Hz, 1H), 0.99 (d, *J =* 5.2 Hz, 3H). 0.97 (d, *J =* 5.4 Hz, 3H). MS (ESI⁺) : [M+H]⁺ 356.2. |
| **199** | 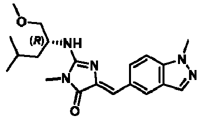 | C₂₀H₂₇N₅O₂ | 369.47 | >98% | Yellow solid. ¹H NMR (400 MHz, DMSO-*d*₆, 300K) δ_{H} 8.41 (s, 1H), 8.29 (d, *J* = 8.8 Hz, 1H), 8.00 (s, 1H), 7.60 (d, *J* = 8.9 Hz, 1H), 7.39 (d, *J* = 8.5 Hz, 1H, NH, D₂O exchanged), 6.54 (s, 1H), 4.48 - 4.34 (m, 1H), 4.03 (s, 3H), 3.51 (dd, *J* = 9.8, 6.4 Hz, 1H), 3.43 (dd, *J* = 9.8, 5.6 Hz, 1H), 3.32 (s, 3H), 3.07 (s, 3H), 1.78 - 1.66 (m, 1H), 1.63 - 1.52 (m, 1H), 1.46 - 1.34 (m, 1H), 1.01 - 0.89 (m, 6H). MS (ESI⁺) : [M+H]⁺ 370.2. |
| **200** | 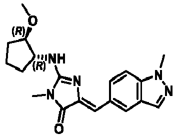 | C₁₉H₂₃N₅O₂ | 353.43 | >98% | Beige solid. ¹H NMR (400 MHz, DMSO-*d*₆, 300K) δ_{H} 8.48 (s, 1H), 8.27 (d, *J* = 8.4 Hz, 1H), 8.02 (s, 1H), 7.60 (d, *J* = 8.8 Hz, 1H), 7.43 (d, *J =* 7.2 Hz, 1H, NH, D₂O exchanged), 6.57 (s, 1H), 4.40 - 4.27 (m, 1H), 4.04 (s, 3H), 3.90 - 3.82 (m, 1H), 3.42 (s, 3H), 3.08 (s, 3H), 2.20 - 2.05 (m, 1H), 2.00 - 1.86 (m, 1H), 1.85 - 1.56 (m, 4H). MS (ESI⁺): [M+H]⁺ 354.3. |
| 201 | 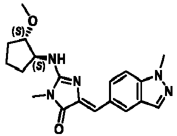 | C₁₉H₂₃N₅O₂ | 353.43 | >98% | Beige solid. ¹H NMR (400 MHz, DMSO-*d*₆, 300K) δ_{H} 8.48 (s, 1H), 8.27 (d, *J* = 8.4 Hz, 1H), 8.02 (s, 1H), 7.60 (d, *J* = 8.8 Hz, 1H), 7.43 (d, *J* = 7.2 Hz, 1H, NH, D₂O exchanged), 6.57 (s, 1H), 4.40 - 4.27 (m, 1H), 4.04 (s, 3H), 3.90 - 3.82 (m, 1H), 3.42 (s, 3H), 3.08 (s, 3H), 2.20 - 2.05 (m, 1H), 2.00 - 1.86 (m, 1H), 1.85 - 1.56 (m, 4H). MS (ESI⁺): [M+H]⁺ 354.3. |
| **202** | 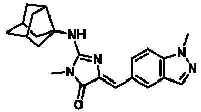 | C₂₂H₂₅N₅O | 375.48 | >98% | Orange solid. ¹H NMR (400 MHz, DMSO-*d*₆, 300K) δ_{H} 8.45 (s, 1H), 8.29 (d, *J* = 8.8 Hz, 1H), 7.99 (s, 1H), 7.59 (d, *J* = 8.8 Hz, 1H), 7.34 (s, 1H, NH, D₂O exchanged), 6.56 (s, 1H), 4.04 (s, 3H), 3.10 (s, 3H), 2.79 (t, *J* = 6.6 Hz, 1H), 2.42 - 2.30 (m, 4H), 2.26 - 2.14 (m, 2H), 2.08 - 1.96 (m, 2H), 1.74 - 1.54 (m, 4H). MS (ESI⁺) : [M+H]⁺ 376.2. |
| **203** | 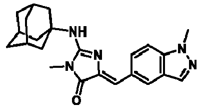 | C₂₃H₂₇N₅O | 389.50 | >98% | Pale Yellow solid. ¹H NMR (400 MHz, DMSO-*d*₆, 300K) δ_{H} 8.42 (s, 1H), 8.36 (d, *J* = 8.9 Hz, 1H), 7.98 (s, 1H), 7.63 (d, *J* = 8.8 Hz, 1H), 6.71 (s, 1H, NH, D₂O exchanged), 6.55 (s, 1H), 4.05 (s, 3H), 3.08 (s, 3H), 2.28 (s, 6H), 2.16 (s, 3H), 1.75 (s, 6H). MS (ESI⁺) : [M+H]⁺ 390.1. |
| **204** | 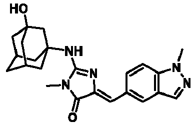 | C₂₃H₂₇N₅O₂ | 405.50 | >98% | Pale Yellow solid. ¹H NMR (400 MHz, DMSO-*d*₆, 300K) δ_{H} 8.45 (s, 1H), 8.33 (d, *J* = 8.9 Hz, 1H), 7.99 (s, 1H), 7.62 (d, *J* = 8.8 Hz, 1H), 6.78 (s, 1H, NH, D₂O exchanged), 6.56 (s, 1H), 4.63 (s, 1H, OH, D₂O exchanged), 4.05 (s, 3H), 3.07 (s, 3H), 2.32 - 2.06 (m, 8H), 1.74 - 1.50 (m, 6H). MS (ESI⁺) : [M+H]⁺ 406.1. |
| **205** | 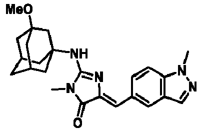 | C₂₄H₂₉N₅O₂ | 419.53 | >98% | Beige solid. ¹H NMR (400 MHz, DMSO-*d*₆, 300K) δ_{H} 8.45 (s, 1H), 8.33 (d, *J* = 8.9 Hz, 1H), 7.97 (s, 1H), 7.62 (d, *J* = 8.9 Hz, 1H), 6.89 (s, 1H, NH, D₂O exchanged), 6.57 (s, 1H), 4.05 (s, 3H), 3.19 (s, 3H), 3.07 (s, 3H), 2.33 (s, 2H), 2.28 (s, 2H), 2.17 (s, 4H), 1.85- 1.50 (m, 6H). MS (ESI⁺) : [M+H]⁺ 420.3. |
| **206** | 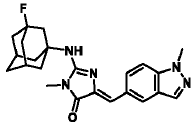 | C₂₃H₂₆FN₅O | 407.49 | >98% | Orange solid. ¹H NMR (400 MHz, DMSO-*d*₆, 300K) δ_{H} 8.41 (s, 1H), 8.35 (d, *J* = 8.9 Hz, 1H), 7.99 (s, 1H), 7.65 (d, *J* = 8.9 Hz, 1H), 6.96 (s, 1H, NH, D₂O exchanged), 6.59 (s, 1H), 4.06 (s, 3H), 3.08 (s, 3H), 2.49 - 2.37 (m, 4H), 2.26 - 2.10 (m, 4H), 1.91 (s, 4H), 1.62 (s, 2H). ¹⁹F NMR (376 MHz, DMSO-*d*₆, 300K) δ_{F} -128.73. MS (ESI⁺) : [M+H]⁺ 408.2. |
| **207** | 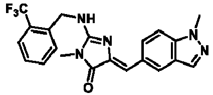 | C₂₁H₁₈F₃N₅O | 413.40 | >98% | Yellow solid. ¹H NMR (400 MHz, DMSO-*d*₆, 300K) δ_{H} 8.45 - 8.37 (m, 1H + NH, D₂O exchanged), 8.13 (dd, *J* = 9.0, 1.5 Hz, 1H), 7.94 (s, 1H), 7.85 -7.65 (m, 3H), 7.56 ― 7.45 (m, 2H), 6.57 (s, 1H), 4.87 (d, *J* = 5.2 Hz, 2H), 4.02 (s, 3H), 3.17 (s, 3H). ¹⁹F NMR (376 MHz, DMSO-*d*₆, 300K) δ_{F} -58.59. MS (ESI⁺): [M+H]+ 414.2. |
| **208** | 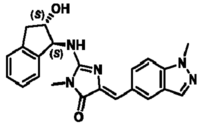 | C₂₂H₂₁N₅O₂ | 387.44 | >98% | Yellow solid. ¹H NMR (400 MHz, DMSO-*d*₆, 300K) δ_{H} 8.38 (s, 1H), 8.26 (dd, *J* = 8.9, 1.5 Hz, 1H), 8.06 - 7.98 (m, 1H + NH, D₂O exchanged), 7.59 (d, *J* = 8.9 Hz, 1H), 7.35 - 7.18 (m, 4H), 6.61 (s, 1H), 5.54 (d, *J* = 5.2 Hz, 1H, OH, D₂O exchanged), 5.45 (t, *J* = 4.6 Hz, 1H), 4.62 - 4.49 (m, 1H), 4.01 (s, 3H), 3.25 (dd, *J* = 15.6, 7.5 Hz, 1H), 3.14 (s, 3H), 2.85 (dd, *J* = 15.5, 7.8 Hz, 1H). MS (ESI⁺): [M+H]⁺ 388.2. |
| **209** | 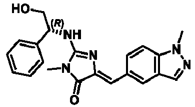 | C₂₁H₂₁N₅O₂ | 375.43 | >98% | Yellow solid. ¹H NMR (400 MHz, DMSO-*d*₆, 300K) δ_{H} 8.43 (s, 1H), 8.19 (dd, *J* = 8.9, 1.4 Hz, 1H), 8.08 (s, 1H), 7.97 (d, *J* = 7.8 Hz, 1H, NH, D₂O exchanged), 7.61 (d, *J* = 8.8 Hz, 1H), 7.55 - 7.50 (m, 2H), 7.42 - 7.34 (m, 2H), 7.29 - 7.22 (m, 1H), 6.53 (s, 1H), 5.28 - 5.16 (m, 1H), 5.08 (br s, 1H, OH, D₂O exchanged), 4.05 (s, 3H), 3.90 - 3.69 (m, 2H), 3.16 (s, 3H). MS (ESI⁺): [M+H]+ 376.2. |
| **210** | 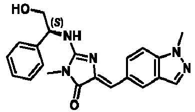 | C₂₁H₂₁N₅O₂ | 375.43 | >98% | Yellow solid. ¹H NMR (400 MHz, DMSO-*d*₆, 300K) δ_{H} 8.43 (s, 1H), 8.19 (dd, *J* = 8.9, 1.4 Hz, 1H), 8.08 (s, 1H), 7.97 (d, *J* = 7.8 Hz, 1H, NH, D₂O exchanged), 7.61 (d, *J* = 8.8 Hz, 1H), 7.55 - 7.50 (m, 2H), 7.42 - 7.34 (m, 2H), 7.29 - 7.22 (m, 1H), 6.53 (s, 1H), 5.28 - 5.16 (m, 1H), 5.08 (br s, 1H, OH, D₂O exchanged), 4.05 (s, 3H), 3.90 - 3.69 (m, 2H), 3.16 (s, 3H). MS (ESI⁺): [M+H]⁺ 376.2. |
| **211** | 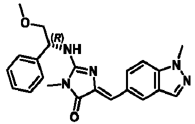 | C₂₂H₂₃N₅O₂ | 389.46 | >98% | Yellow solid. ¹H NMR (400 MHz, DMSO-*d*₆, 300K) δ_{H} 8.43 (s, 1H), 8.20 (d, *J* = 8.9 Hz, 1H), 8.14 - 8.02 (m, 1H + NH, D₂O exchanged), 7.61 (d, *J* = 8.8 Hz, 1H), 7.54 (d, *J* = 7.6 Hz, 2H), 7.38 (t, *J* = 7.5 Hz, 2H), 7.29 -7.23 (m, 1H), 6.54 (s, 1H), 5.46 - 5.34 (m, 1H), 4.04 (s, 3H), 3.86 - 3.79 (m, 1H), 3.65 (dd, *J* = 10.0, 5.3 Hz, 1H), 3.35 (s, 3H), 3.14 (s, 3H). MS (ESI⁺): [M+H]⁺ 390.1. |
| **212** | 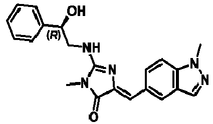 | C₂₁H₂₁N₅O₂ | 375.43 | >98% | Yellow solid. ¹H NMR (400 MHz, DMSO-*d*₆, 300K) δ_{H} 8.52 (s, 1H), 8.30 (dd, *J* = 8.9, 1.5 Hz, 1H), 8.04 (s, 1H), 7.97 (t, *J* = 5.7 Hz, 1H, NH, D₂O exchanged), 7.64 (d, *J* = 8.8 Hz, 1H), 7.53 - 7.48 (m, 2H), 7.48 - 7.41 (m, 2H), 7.35 - 7.28 (m, 1H), 6.59 (s, 1H), 5.69 (d, *J* = 4.2 Hz, 1H, OH, D₂O exchanged), 5.13 - 5.05 (m, 1H), 4.06 (s, 3H), 3.78 - 3.67 (m, 1H), 3.42 ― 3.31 (m, 1H), 3.09 (s, 3H). MS (ESI⁺): [M+H]⁺ 376.2. |
| **213** | 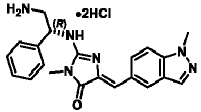 | C₂H₂₄Cl₂N₆O | 447.36 | 96% | Yellow solid. ¹H NMR (400 MHz, D₂O, 300K) δ_{H} 7.68 -7.50 (m, 6H), 7.37 (s, 1H), 7.29 (d, *J* = 9.2 Hz, 1H), 7.12 (d, *J* = 8.8 Hz, 1H), 6.21 (s, 1H), 5.27 (dd, *J* = 8.8, 5.9 Hz, 1H), 3.81 (s, 3H), 3.63 (dd, *J* = 13.4, 8.8 Hz, 1H), 3.54 (dd, *J* = 13.4, 6.0 Hz, 1H), 3.03 (s, 3H). MS (ESI⁺) : [M+H]⁺ 375.3 (+2HCl). |
| **214** | 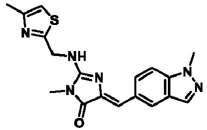 | C₁₈H₁₈N₆OS | 366.44 | >98% | Yellow solid. ¹H NMR (400 MHz, DMSO-*d*₆, 300K) δ_{H} 8.57 (t, *J* = 5.9 Hz, 1H, NH, D₂O exchanged), 8.48 (s, 1H), 8.28 (dd, *J* = 8.9, 1.6 Hz, 1H), 8.05 (s, 1H), 7.61 (d, *J* = 8.8 Hz, 1H), 7.21 - 7.19 (m, 1H), 6.64 (s, 1H), 4.90 (d, *J* = 5.9 Hz, 2H), 4.04 (s, 3H), 3.10 (s, 3H), 2.37 (d, *J* = 1.0 Hz, 3H). MS (ESI⁺) : [M+H]⁺ 367.1. |
| **215** | 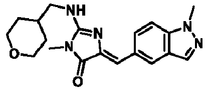 | C₁₉H₂₃N₅O₂ | 353.43 | >98% | Yellow solid. ¹H NMR (400 MHz, DMSO-*d*₆, 300K) δ_{H} 8.42 (s, 1H), 8.30 (dd, *J* = 9.0, 1.6 Hz, 1H), 8.05 (d, *J* = 0.9 Hz, 1H), 7.70 -7.60 (m, 1H + NH, D₂O exchanged), 6.55 (s, 1H), 4.04 (s, 3H), 3.94 - 3.86 (m, 2H), 3.38 (t, *J* = 6.3 Hz, 2H), 3.35 - 3.27 (m, 2H), 3.08 (s, 3H), 2.07 - 1.93 (m, 1H), 1.76- 1.66 (m, 2H), 1.39 -1.23 (m, 2H). MS (ESI⁺) : [M+H]⁺ 354.2. |
| **216** | 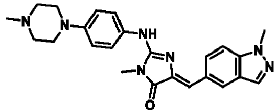 | C₂₄H₂₇N₇O | 429.53 | >98% | Yellow solid. ¹H NMR (400 MHz, DMSO-*d*₆, 300K) δ_{H} 9.22 (s, 1H, NH, D₂O exchanged), 8.49 (s, 1H), 8.31 (d, *J* = 8.9 Hz, 1H), 8.14 (s, 1H), 7.85 (d, *J* = 9.0 Hz, 2H), 7.67 (d, *J* = 8.9 Hz, 1H), 7.06 (d, *J* = 9.1 Hz, 2H), 6.72 (s, 1H), 4.06 (s, 3H), 3.23 (s, 3H), 3.20 - 3.14 (m, 4H), 2.51 - 2.47 (m, 4H), 2.25 (s, 3H). MS (ESI⁺) : [M+H]⁺ 430.1. |
| **217** | 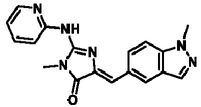 | C₁₈H₁₆N₆O | 332.37 | >98% | Yellow solid. ¹H NMR (400 MHz, CDCl₃, 300K) δ_{H} 12.45 (br s, 1H, NH, D₂O exchanged), 8.38 (dd, *J* = 5.0, 2.0 Hz, 1H), 8.07 (s, 1H), 7.94 (s, 1H), 7.70 (td, *J =* 8.2, 2.0 Hz, 1H), 7.58 (dd, *J =* 8.7, 1.6 Hz, 1H), 7.51 (d, *J* = 8.8 Hz, 1H), 7.23 (d, *J* = 8.2 Hz, 1H), 7.00 (ddd, *J* = 7.2, 5.0, 1.1 Hz, 1H), 6.82 (s, 1H), 4.15 (s, 3H), 3.34 (s, 3H). MS (ESI⁺) : [M+H]⁺ 333.1. |
| **218** | 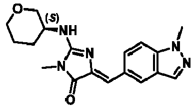 | C₁₈H₂₁N₅O₂ | 339.40 | >98% | Beige solid. ¹H NMR (400 MHz, DMSO-*d*₆, 300K) δ_{H} 8.37 (s, 1H), 8.31 (d, *J* = 8.9 Hz, 1H), 8.07 (s, 1H), 7.65 (d, *J* = 8.9 Hz, 1H), 7.36 (d, *J* = 7.4 Hz, 1H, NH, D₂O exchanged), 6.58 (s, 1H), 4.15 - 3.95 (m, 5H), 3.87 - 3.77 (m, 1H), 3.42 - 3.22 (m, 2H), 3.08 (s, 3H), 2.14 - 2.00 (m, 1H), 1.84 -1.58 (m, 3H). MS (ESI⁺): [M+H]+ 340.2. |
| **219** | 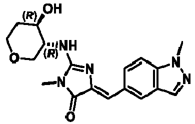 | C₁₈H₂₁N₅O₃ | 355.40 | >98% | Yellow solid. ¹H NMR (400 MHz, DMSO-*d*₆, 300K) δ_{H} 8.36 (s, 1H), 8.32 (dd, *J* = 8.8, 1.4 Hz, 1H), 8.07 (d, *J* = 0.9 Hz, 1H), 7.66 (d, *J* = 8.8 Hz, 1H), 7.38 (d, *J* = 7.8 Hz, 1H, NH, D₂O exchanged), 6.58 (s, 1H), 5.10 (d, *J* = 4.7 Hz, 1H, OH, D₂O exchanged), 4.04 (s, 3H), 3.99 (dd, *J* = 10.9, 4.8 Hz, 1H), 3.95 - 3.84 (m, 2H), 3.83 - 3.72 (m, 1H), 3.38 (td, *J* = 11.7; 2.3 Hz, 1H), 3.20 (t, *J* = 10.4 Hz, 1H), 3.10 (s, 3H), 2.02 - 1.92 (m, 1H), 1.65 ― 1.51 (m, 1H). MS (ESI⁺) : [M+H]⁺ 356.2. |
| **220** | 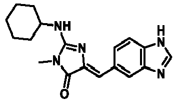 | C₁₈H₂₁N₅O | 323.40 | >98% | Yellow solid. ¹H NMR (400 MHz, DMSO-*d*₆, 300K) δ_{H} 12.54 (br s, 1H, NH, D₂O exchanged), 8.47 (s, 1H), 8.23 (s, 1H), 7.85 (d, *J* = 8.4 Hz, 1H), 7.54 (d, *J* = 8.4 Hz, 1H), 7.34 (d, *J* = 7.8 Hz, 1H, NH, D₂O exchanged), 6.53 (s, 1H), 3.93 (br s, 1H), 3.07 (s, 3H), 2.10 - 2.95 (m, 2H), 1.90 - 1.75 (m, 2H), 1.72 - 1.64 (m, 1H), 1.50 - 1.34 (m, 4H), 1.30 - 1.10 (m, 1H). MS (ESI⁺) : [M+H]⁺ 324.2. |
| **221** | 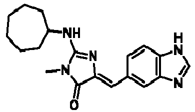 | C₁₉H₂₃N₅O | 337.43 | >98% | Yellow solid. ¹H NMR (400 MHz, DMSO-*d*₆, 300K, mixture of tautomers) δ_{H} 12.52 & 12.44 (br s, 1H, NH, D₂O exchanged), 8.52 & 8.42 (s, 1H), 8.22 & 8.20 (s, 1H), 7.92 & 7.82 (d, *J* = 8.5 Hz, 1H), 7.58 & 7.48 (d, *J* = 8.4 Hz, 1H), 7.37 & 7.33 (d, *J* = 7.6 Hz, 1H, NH, D₂O exchanged), 6.53 (s, 1H), 4.20 - 4.07 (m, 1H), 3.07 (s, 3H), 2.12 - 1.95 (m, 2H), 1.82 - 1.45 (m, 10H). MS (ESI⁺) : [M+H]⁺ 338.2. |
| **222** | 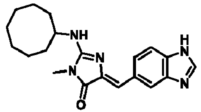 | C₂₀H₂₅N₅O | 351.45 | >98% | Yellow solid. ¹H NMR (400 MHz, DMSO-*d*₆, 300K, mixture of tautomers) δ_{H} 12.49 & 12.43 (br s, 1H, NH, D₂O exchanged), 8.53 & 8.33 (s, 1H), 8.22 & 8.20 (s, 1H), 8.00 -7.88 (m, 1H), 7.58 & 7.47 (d, *J* = 8.5 Hz, 1H), 7.40 - 7.27 (m, 1H, NH, D₂O exchanged), 6.54 (s, 1H), 4.27 - 4.14 (m, 1H), 3.07 (s, 3H), 2.00 - 1.45 (m, 14H). MS (ESI⁺) : [M+H]⁺ 352.2. |
| **223** | 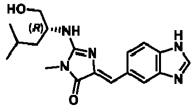 | C₁₈H₂₃N₅O₂ | 341.42 | >98 | Yellow solid. ¹H NMR (400 MHz, DMSO-*d*₆, 300K, mixture of tautomers) δ_{H} 12.51 & 12.43 (br s, 1H, NH, D₂O exchanged), 8.49 & 8.34 (s, 1H), 8.20 (s, 1H), 7.93 & 7.88 (d, *J* = 8.4 Hz, 1H), 7.58 & 7.46 (d, *J* = 8.4 Hz, 1H), 7.23 & 7.19 (d, *J* = 7.1 Hz, 1H, NH, D₂O exchanged), 6.53 (s, 1H), 4.83 (t, *J* = 5.7 Hz, 1H, OH, D₂O exchanged), 4.25 (br s, 1H), 3.60 - 3.48 (m, 2H), 3.09 (s, 3H), 1.80 - 1.65 (m, 1H), 1.64 - 1.37 (m, 2H), 1.01 - 0.92 (m, 6H). MS (ESI⁺) : [M+H]⁺ 342.2. |
| **224** | 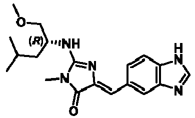 | C₁₉H₂₅N₅O₂ | 355.44 | 94% | Yellow solid. ¹H NMR (400 MHz, DMSO-*d*₆, 300K, mixture of tautomers) δ_{H} 12.52 & 12.43 (br s, 1H, NH, D₂O exchanged), 8.51 & 8.37 (s, 1H), 8.20 (s, 1H), 7.92 & 7.86 (d, *J* = 8.5 Hz, 1H), 7.58 & 7.46 (d, *J* = 8.4 Hz, 1H), 7.41 - 7.28 (m, 1H, NH, D₂O exchanged), 6.54 (s, 1H), 4.44 (br s, 1H), 3.51 (dd, *J* = 9.7, 6.3 Hz, 1H), 3.43 (dd, *J* = 9.7, 5.8 Hz, 1H), 3.33 (s, 3H), 3.08 (s, 3H),1.80 - 1.66 (m, 1H), 1.64 - 1.54 (m, 1H), 1.48 - 1.38 (m, 1H), 1.01- 0.92 (m, 6H). MS (ESI⁺) : [M+H]⁺ 356.2. |
| **225** | 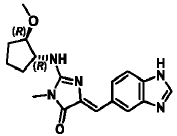 | C₁₈H₂₁N₅O₂ | 339.40 | 98% (¹H NMR) | Beige solid. ¹H NMR (400 MHz, DMSO-*d*₆, 300K, mixture of tautomers) δ_{H} 12.51 & 12.44 (br s, 1H, NH, D₂O exchanged), 8.53 & 8.39 (s, 1H), 8.21 (s, 1H), 7.94 & 7.86 (d, *J* = 8.4 Hz, 1H), 7.64 - 7.36 (m, 1H + NH, D₂O exchanged), 6.57 (s, 1H), 4.42 - 4.27 (m, 1H), 3.92 - 3.77 (m, 1H), 3.40 & 3.38 (s, 3H), 3.08 (s, 3H), 2.22 - 2.06 (m, 1H), 2.00 - 1.86 (m, 1H), 1.84 - 1.56 (m, 4H). MS (ESI⁺): [M+H]⁺ 340.2. |
| **226** | 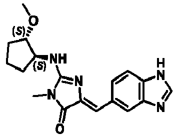 | C₁₈H₂₁N₅O₂ | 339.40 | 98% (¹H NMR) | Beige solid. ¹H NMR (400 MHz, DMSO-*d*₆, 300K, mixture of tautomers) δ_{H} 12.51 & 12.44 (br s, 1H, NH, D₂O exchanged), 8.53 & 8.39 (s, 1H), 8.21 (s, 1H), 7.94 & 7.86 (d, *J* = 8.4 Hz, 1H), 7.64 - 7.36 (m, 1H + NH, D₂O exchanged), 6.57 (s, 1H), 4.42 - 4.27 (m, 1H), 3.92 - 3.77 (m, 1H), 3.40 & 3.38 (s, 3H), 3.08 (s, 3H), 2.22 - 2.06 (m, 1H), 2.00 - 1.86 (m, 1H), 1.84 - 1.56 (m, 4H). MS (ESI⁺): [M+H]⁺ 340.2. |
| **227** | 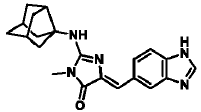 | C₂₁H₂₃N₅O | 361.45 | 97% | Pale yellow solid. ¹H NMR (400 MHz, DMSO-*d*₆, 300K, mixture of tautomers) δ_{H} 12.48 & 12.44 (br s, 1H, NH, D₂O exchanged), 8.50 & 8.30 (s, 1H), 8.20 (s, 1H), 7.93 (s, 1H), 7.56 & 7.45 (s, 1H), 7.33 & 7.28 (s, 1H, NH, D₂O exchanged), 6.55 (s, 1H), 3.09 (s, 3H), 2.76 (br s, 1H), 2.34 (br s, 4H), 2.28 - 1.98 (m, 4H), 1.74 - 1.52 (m, 4H). MS (ESI⁺) : [M+H]⁺ 362.3. |
| **228** | 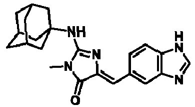 | C₂₂H₂₅N₅O | 375.48 | >98% | Pale yellow solid. ¹H NMR (400 MHz, DMSO-*d*₆, 300K, mixture of tautomers) δ_{H} 12.52 & 12.43 (br s, 1H, NH, D₂O exchanged), 8.24 (s, 1H), 8.23 & 8.20 (s, 1H), 7.96 & 7.87 (d, *J =* 8.5 Hz, 1H), 7.59 & 7.47 (d, *J* = 8.5 Hz, 1H), 6.66 & 6.64 (s, 1H, NH, D₂O exchanged), 6.55 (s, 1H), 3.07 (s, 3H), 2.32 - 2.22 (m, 6H), 2.15 (s, 3H), 1.84 - 1.66 (s, 6H). MS (ESI⁺) : [M+H]⁺ 376.2. |
| **229** | 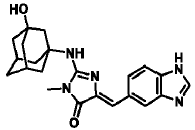 | C₂₂H₂₅N₅O₂ | 391.48 | >98% | Pale yellow solid. ¹H NMR (400 MHz, DMSO-*d*₆, 300K, mixture of tautomers) δ_{H} 12.43 & 12.37 (br s, 1H, NH, D₂O exchanged), 8.48 & 8.16 (s, 1H), 8.17 & 8.14 (s, 1H), 7.91 & 7.87 (d, *J* = 8.5 Hz, 1H), 7.52 & 7.39 (d, *J* = 8.5 Hz, 1H), 6.68 & 6.67 (s, 1H, NH, D₂O exchanged), 6.49 (s, 1H), 4.54 & 4.52 (s, 1H, OH, D₂O exchanged), 3.00 (s, 3H), 2.25 ― 2.11 (m, 4H), 2.07 - 1.98 (m, 4H), 1.67 ― 1.41 (s, 6H). MS (ESI⁺) : [M+H]⁺ 392.3. |
| **230** | 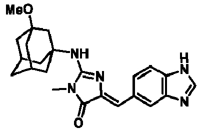 | C₂₃H₂₇N₅O₂ | 405.50 | >98% | Orange solid. ¹H NMR (400 MHz, DMSO-*d*₆, 300K, mixture of tautomers) δ_{H} 12.51 & 12.42 (s, 1H, NH, D₂O exchanged), 8.63 & 8.20 (s, 1H), 8.23 & 8.18 (s, 1H), 8.04 & 7.88 (d, *J* = 8.5 Hz, 1H), 7.58 & 7.46 (d, *J* = 8.5 Hz, 1H), 6.82 & 6.80 (s, 1H, NH, D₂O exchanged), 6.56 (s, 1H), 4.19 & 4.17 (s, 3H), 3.07 (s, 3H), 2.38 - 2.10 (m, 8H), 1.86 ― 1.51 (m, 6H). MS (ESI⁺): [M+H]⁺ 406.3. |
| **231** | 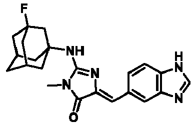 | C₂₂H₂₄FN₅O | 393.47 | >98% | Orange solid. ¹H NMR (400 MHz, DMSO-*d*₆, 300K, mixture of tautomers) δ_{H} 12.55 & 12.44 (br s, 1H, NH, D₂O exchanged), 8.61 & 8.22 (s, 1H), 8.24 & 8.21 (s, 1H), 7.96 & 7.86 (d, *J* = 8.6 Hz, 1H), 7.60 & 7.48 (d, *J* = 8.5 Hz, 1H), 6.91 & 6.89 (s, 1H, NH, D₂O exchanged), 6.58 (s, 1H), 3.08 (s, 3H), 2.50 - 2.36 (m, 4H), 2.30 - 2.12 (m, 4H), 2.00 ― 1.82 (m, 4H), 1.72 - 1.54 (m, 2H). ¹⁹F NMR (376 MHz, DMSO-*d*₆, 300K, mixture of tautomers) δ_{F} - 128.73. MS (ESI⁺) : [M+H]⁺ 394.3. |
| **232** | 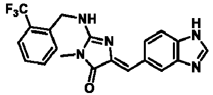 | C₂₀H₁₆F₃N₅O | 399.38 | >98% | Yellow solid. ¹H NMR (400 MHz, DMSO-*d*₆, 300K, mixture of tautomers) δ_{H} 12.43 & 12.41 (br s, 1H, NH, D₂O exchanged), 8.39 - 8.16 (m, 2H + NH, D₂O exchanged), 7.98 -7.87 (m, 1H), 7.81 -7.65 (m, 3H), 7.57 -7.37 (m, 2H), 6.60 & 6.59 (s, 1H), 4.89 (br s, 2H), 3.17 & 3.16 (s, 3H). ¹⁹F NMR (376 MHz, DMSO-*d*₆, 300K, mixture of tautomers) δ_{H} -58.51 & -58.68. MS (ESI⁺) : [M+H]⁺ 400.1. |
| **233** | 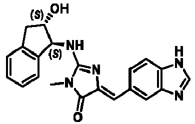 | C₂₁H₁₉N₅O₂ | 373.42 | 98% | Yellow solid. ¹H NMR (400 MHz, DMSO-*d*₆, 300K, mixture of tautomers) δ_{H} 12.49 & 12.44 (s, 1H, NH, D₂O exchanged), 8.46 & 8.41 (s, 1H), 8.18 (s, 1H), 8.05 - 7.78 (m, 1H + NH, D₂O exchanged), 7.58 & 7.47 (d, *J* = 8.5 Hz, 1H), 7.35 -7.15 (m, 4H), 6.62 (s, 1H), 5.62 - 5.43 (m, 1H + OH, D₂O exchanged), 4.60 - 4.48 (m, 1H), 3.25 (dd, *J* = 15.5, 7.5 Hz, 1H), 3.14 (s, 3H), 2.92 - 2.76 (m, 1H). MS (ESI⁺): [M+H]⁺ 374.2. |
| **234** | 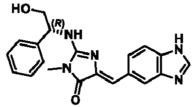 | C₂₀H₁₉N₅O₂ | 361.41 | >98% | Yellow solid. ¹H NMR (400 MHz, DMSO-*d*₆, 300K, mixture of tautomers) δ_{H} 12.56 & 12.45 (s, 1H, NH, D₂O exchanged), 8.47 & 8.46 (s, 1H), 8.24 & 8.22 (s, 1H), 8.00 - 7.72 (m, 1H + NH, D₂O exchanged), 7.62 - 7.45 (m, 3H), 7.41 -7.37 (m, 2H), 7.28 ― 7.21 (m, 1H), 6.54 & 6.53 (s, 1H), 5.32 - 5.20 (m, 1H), 5.11 - 5.02 (m, 1H, OH, D₂O exchanged), 3.92 ― 3.81 (m, 1H), 3.80 - 3.70 (m, 1H), 3.16 (s, 3H). MS (ESI⁺): [M+H]⁺ 362.2. |
| **235** | 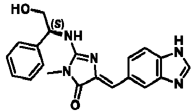 | C₂₀H₁₉N₅O₂ | 361.41 | >98% | Yellow solid. ¹H NMR (400 MHz, DMSO-*d*₆, 300K, mixture of tautomers) δ_{H} 12.56 & 12.45 (s, 1H, NH, D₂O exchanged), 8.47 & 8.46 (s, 1H), 8.24 & 8.22 (s, 1H), 8.00 - 7.72 (m, 1H + NH, D₂O exchanged), 7.62 - 7.45 (m, 3H), 7.41 ― 7.37 (m, 2H), 7.28 ― 7.21 (m, 1H), 6.54 & 6.53 (s, 1H), 5.32 - 5.20 (m, 1H), 5.11 - 5.02 (m, 1H, OH, D₂O exchanged), 3.92 - 3.81 (m, 1H), 3.80 - 3.70 (m, 1H), 3.16 (s, 3H). MS (ESI⁺): [M+H]⁺ 362.2. |
| **236** | 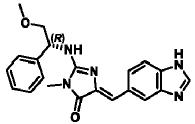 | C₂₁H₂₁N₅O₂ | 375.43 | >98% | Yellow solid. ¹H NMR (400 MHz, DMSO-*d*₆, 300K, mixture of tautomers) δ_{H} 12.55 & 12.45 (br s, 1H, NH, D₂O exchanged), 8.52 - 8.40 (m, 1H), 8.28 - 8.18 (m, 1H), 8.15 - 7.70 (m, 1H + NH, D₂O exchanged), 7.65 - 7.43 (m, 3H), 7.40 - 7.34 (m, 2H), 7.31 -7.24 (m, 1H), 6.55 (s, 1H), 5.45 (s, 1H), 3.84 (dd, *J =* 10.0, 8.5 Hz, 1H), 3.68 (dd, *J* = 10.0, 5.5 Hz, 1H), 3.36 (s, 3H), 3.14 (s, 3H). MS (ESI⁺) : [M+H]+ 376.2. |
| **237** | 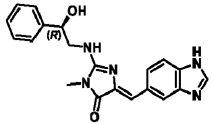 | C₂₀H₁₉N₅O₂ | 361.41 | 87% | Yellow solid. ¹H NMR (400 MHz, DMSO-*d*₆, 300K, mixture of tautomers) δ_{H} 12.53 & 12.46 (br s, 1H, NH, D₂O exchanged), 8.61 & 8.26 (s, 1H), 8.23 (s, 1H), 8.05 - 7.75 (m, 1H + NH, D₂O exchanged), 7.70 ― 7.25 (m, 6H), 6.59 (s, 1H), 5.69 (d, *J* = 4.2 Hz, 1H, OH, D₂O exchanged), 5.15 - 4.95 (m, 1H), 3.88 - 3.65 (m, 1H), 3.50 - 3.25 (m, 1H), 3.09 (s, 3H). MS (ESI⁺): [M+H]⁺ 362.3. |
| **238** | 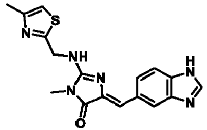 | C₁₇H₁₆N₆OS | 352.42 | >98% | Yellow solid. ¹H NMR (400 MHz, DMSO-*d*₆, 300K, mixture of tautomers) δ_{H} 12.52 & 12.46 (br s, 1H, NH, D₂O exchanged), 8.65 - 8.40 (m, 1H + NH, D₂O exchanged), 8.22 & 8.21 (s, 1H), 8.00 & 7.86 (d, *J* = 8.4 Hz, 1H), 7.59 & 7.48 (d, *J* = 8.4 Hz, 1H), 7.19 (s, 1H), 6.64 (s, 1H), 4.98 - 4.86 (m, 2H), 3.11 (s, 3H), 2.37 (s, 3H). MS (ESI⁺) : [M+H]⁺ 353.1. |
| **239** | 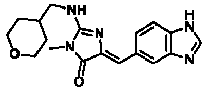 | C₁₈H₂₁N₅O₂ | 339.40 | >98% (¹H NMR) | Yellow solid. ¹H NMR (400 MHz, DMSO-*d*₆, 300K, mixture of tautomers) δ_{H} 12.53 & 12.45 (br s, 1H, NH, D₂O exchanged), 8.51 - 8.46 (s, 1H), 8.23 & 8.20 (s, 1H), 7.94 & 7.80 (d, *J* = 8.4 Hz, 1H), 7.70 - 7.45 (m, 1H + NH, D₂O exchanged), 6.55 (s, 1H), 3.94 - 3.80 (m, 2H), 3.43 - 3.36 (m, 2H), 3.35 - 3.26 (m, 2H), 3.08 (s, 3H), 2.05 - 1.89 (m, 1H), 1.77 - 1.65 (m, 2H), 1.38 ― 1.21 (m, 2H). MS (ESI⁺) : [M+H]⁺ 340.2. |
| **240** | 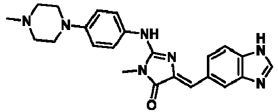 | C₂₃H₂₅N₇O | 415.50 | >98% (¹H NMR) | Yellow solid. ¹H NMR (400 MHz, DMSO-*d*₆, 300K, mixture of tautomers) δ_{H} 12.55 & 12.44 (br s, 1H, NH, D₂O exchanged), 9.14 & 9.11 (s, 1H, NH, D₂O exchanged), 8.42 & 8.40 (s, 1H), 8.21 & 8.16 (s, 1H), 8.00 ― 7.68 (m, 3H), 7.57 & 7.46 (d, *J* = 8.4 Hz, 1H), 7.00 & 6.94 (d, *J* = 8.5 Hz, 2H), 6.64 (s, 1H), 3.16 (s, 3H), 3.08 (br s, 4H), 2.42 (br s, 4H), 2.17 (s, 3H). MS (ESI⁺) : [M+H]⁺ 416.2. |
| **241** | 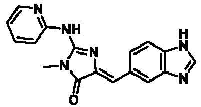 | C₁₇H₁₄N₆O | 318.34 | >98% | Yellow solid. ¹H NMR (400 MHz, CF₃COOD, 300K) δ_{H} 9.34 (s, 1H), 8.61 - 8.49 (m, 2H), 8.28 (s, 1H), 8.13 (d, *J* = 8.7 Hz, 1H), 8.03 (d, *J* = 8.8 Hz, 1H), 7.93 (d, *J* = 8.6 Hz, 1H), 7.71 (t, *J* = 6.8 Hz, 1H), 7.52 (s, 1H), 3.63 (s, 3H). MS (ESI⁺) : [M+H]⁺ 319.1. |
| **242** | 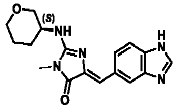 | C₁₇H₁₉N₅O₂ | 325.37 | 98% (¹H NMR) | Yellow solid. ¹H NMR (400 MHz, DMSO-*d*₆, 300K, mixture of tautomers) δ_{H} 12.58 & 12.48 (s, 1H, NH, D₂O exchanged), 8.49 & 8.39 (s, 1H), 8.22 (s, 1H), 7.93 & 7.82 (d, *J* = 8.5 Hz, 1H), 7.61 & 7.51 (d, *J* = 8.5 Hz, 1H), 7.41 - 7.28 (m, 1H, NH, D₂O exchanged), 6.58 (s, 1H), 4.20 - 3.96 (m, 2H), 3.89 - 3.78 (m, 1H), 3.42 - 3.22 (m, 2H), 3.08 (s, 3H), 2.14 - 2.02 (m, 1H), 1.82 - 1.60 (m, 3H). MS (ESI⁺): [M+H]⁺ 326.2. |
| **243** | 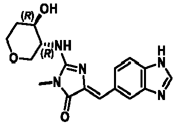 | C₁₇H₁₉N₅O₃ | 341.37 | 98% (¹H NMR) | Beige solid. ¹H NMR (400 MHz, DMSO-*d*₆, 300K, mixture of tautomers) δ_{H} 12.60 & 12.47 (s, 1H, NH, D₂O exchanged), 8.49 & 8.37 (s, 1H), 8.21 (s, 1H), 7.92 & 7.82 (d, *J* = 8.4 Hz, 1H), 7.61 & 7.51 (d, *J* = 8.4 Hz, 1H), 7.38 - 7.30 (m, 1H, NH, D₂O exchanged), 6.57 (s, 1H), 5.14 - 5.08 (m, 1H, OH, D₂O exchanged), 4.05 - 3.85 (m, 3H), 3.82 - 3.70 (m, 1H), 3.38 (t, *J* = 11.8 Hz, 1H), 3.18 (t, *J* = 10.4 Hz, 1H), 3.10 (s, 3H), 2.05 ― 1.93 (m, 1H), 1.67 - 1.50 (m, 1H). MS (ESI⁺): [M+H]⁺ 342.2. |
| **244** | 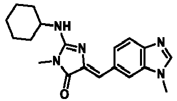 | C₁₉H₂₃N₅O | 337.43 | >98% | Yellow solid. ¹H NMR (400 MHz, DMSO-*d*₆, 300K) δ_{H} 8.61 (s, 1H), 8.19 (s, 1H), 7.76 (dd, *J* = 8.5, 1.5 Hz, 1H), 7.59 (d, *J* = 8.4 Hz, 1H), 7.43 (d, *J* = 7.4 Hz, 1H, NH, D₂O exchanged), 6.55 (s, 1H), 4.00 - 3.88 (m, 1H), 3.84 (s, 3H), 3.08 (s, 3H), 2.16 - 2.03 (m, 2H), 1.87 - 1.75 (m, 2H), 1.72 - 1.62 (m, 1H)), 1.50 - 1.30 (m, 4H), 1.27 - 1.10 (m, 1H). MS (ESI⁺) : [M+H]⁺ 338.3. |
| **245** | 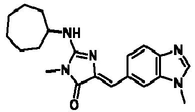 | C₂₀H₂₅N₅O | 351.45 | >98% | Yellow solid. ¹H NMR (400 MHz, DMSO-*d*₆, 300K) δ_{H} 8.60 (s, 1H), 8.19 (s, 1H), 7.73 (dd, *J* = 8.4, 1.4 Hz, 1H), 7.58 (d, *J* = 8.4 Hz, 1H), 7.46 (d, *J* = 7.6 Hz, 1H, NH, D₂O exchanged), 6.55 (s, 1H), 4.16 - 4.04 (m, 1H), 3.84 (s, 3H), 3.07 (s, 3H), 2.12 - 2.00 (m, 2H), 1.80 ― 1.48 (m, 10H). MS (ESI⁺) : [M+H]⁺ 352.3. |
| **246** | 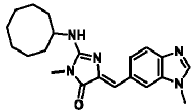 | C₂₁H₂₇N₅O | 365.48 | >98% | Orange solid. ¹H NMR (400 MHz, DMSO-*d*₆, 300K) δ_{H} 8.63 (s, 1H), 8.19 (s, 1H), 7.76 (dd, *J* = 8.5, 1.5 Hz, 1H), 7.58 (d, *J* = 8.4 Hz, 1H), 7.46 (d, *J* = 7.7 Hz, 1H, NH, D₂O exchanged), 6.55 (s, 1H), 4.33 - 4.19 (m, 1H), 3.83 (s, 3H), 3.08 (s, 3H), 1.97 - 1.50 (m, 14H). MS (ESI⁺) : [M+H]⁺ 366.3. |
| **247** | 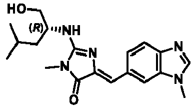 | C₁₉H₂₅N₅O₂ | 355.44 | >98% | Yellow solid. ¹H NMR (400 MHz, DMSO-*d*₆, 300K) δ_{H} 8.47 (s, 1H), 8.19 (s, 1H), 7.85 (d, *J* = 8.5 Hz, 1H), 7.58 (d, *J* = 8.5 Hz, 1H), 7.31 (d, *J* = 8.6 Hz, 1H, NH, D₂O exchanged), 6.54 (s, 1H), 4.84 (t, *J* = 5.7 Hz, 1H, OH, D₂O exchanged), 4.31 - 4.20 (m, 1H), 3.82 (s, 3H), 3.60 - 3.48 (m, 2H), 3.09 (s, 3H), 1.77 - 1.67 (m, 1H), 1.61 - 1.51 (m, 1H), 1.51 - 1.41 (m, 1H), 1.01 (d, *J* = 6.5 Hz, 3H), 0.96 (d, *J* = 6.6 Hz, 3H). MS (ESI⁺) : [M+H]⁺ 356.3. |
| **248** | 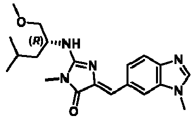 | C₂₀H₂₇N₅O₂ | 369.47 | >98% | Yellow solid. ¹H NMR (400 MHz, DMSO-*d*₆, 300K) δ_{H} 8.48 (s, 1H), 8.19 (s, 1H), 7.83 (d, *J* = 8.5 Hz, 1H), 7.58 (d, *J* = 8.7 Hz, 1H), 7.44 (d, *J* = 8.6 Hz, 1H, NH, D₂O exchanged), 6.56 (s, 1H), 4.51 - 4.40 (m, 1H), 3.81 (s, 3H), 3.52 (dd, *J* = 9.9, 6.5 Hz, 1H), 3.44 (dd, *J* = 9.9, 4.8 Hz, 1H), 3.32 (s, 3H), 3.09 (s, 3H), 1.78 - 1.66 (m, 1H), 1.63 - 1.53 (m, 1H), 1.47 - 1.38 (m, 1H), 0.99 (d, *J* = 6.5 Hz, 3H), 0.95 (d, *J* = 6.6 Hz, 3H). MS (ESI⁺) : [M+H]⁺ 370.3. |
| **249** | 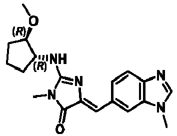 | C₁₉H₂₃N₅O₂ | 353.43 | >98% (¹H NMR) | Beige solid. ¹H NMR (400 MHz, DMSO-*d*₆, 300K) δ_{H} 8.40 (s, 1H), 8.20 (s, 1H), 7.94 (dd, *J* = 8.5, 1.5 Hz, 1H), 7.59 (d, *J* = 8.5 Hz, 1H), 7.49 (d, *J* = 7.3 Hz, 1H, NH, D₂O exchanged), 6.59 (s, 1H), 4.40 - 4.27 (m, 1H), 3.89 - 3.83 (m, 1H), 3.82 (s, 3H), 3.37 (s, 3H), 3.08 (s, 3H), 2.22 - 2.07 (m, 1H), 2.00 - 1.86 (m, 1H), 1.84 - 1.56 (m, 4H). MS (ESI⁺): [M+H]⁺ 354.3. |
| **250** | 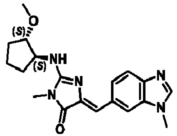 | C₁₉H₂₃N₅O₂ | 353.43 | >98% (¹H NMR) | Beige solid. ¹H NMR (400 MHz, DMSO-*d*₆, 300K) δ_{H} 8.40 (s, 1H), 8.20 (s, 1H), 7.94 (dd, *J* = 8.5, 1.5 Hz, 1H), 7.59 (d, *J* = 8.5 Hz, 1H), 7.49 (d, *J* = 7.3 Hz, 1H, NH, D₂O exchanged), 6.59 (s, 1H), 4.40 - 4.27 (m, 1H), 3.89 - 3.83 (m, 1H), 3.82 (s, 3H), 3.37 (s, 3H), 3.08 (s, 3H), 2.22 - 2.07 (m, 1H), 2.00 - 1.86 (m, 1H), 1.84 - 1.56 (m, 4H). MS (ESI⁺): [M+H]⁺ 354.3. |
| **251** | 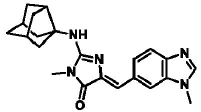 | C₂₂H₂₅N₅O | 375.48 | >98% | Yellow solid. ¹H NMR (400 MHz, DMSO-*d*₆, 300K) δ_{H} 8.54 (s, 1H), 8.19 (s, 1H), 7.71 (d, *J* = 8.4 Hz, 1H), 7.57 (d, *J* = 8.4 Hz, 1H), 7.37 (s, 1H, NH, D₂O exchanged), 6.56 (s, 1H), 3.82 (s, 3H), 3.10 (s, 3H), 2.79 - 2.69 (m, 1H), 2.46 - 2.29 (m, 4H), 2.26 - 2.00 (m, 4H), 1.74 - 1.52 (m, 4H). MS (ESI⁺) : [M+H]⁺ 376.2. |
| **252** | 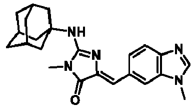 | C₂₃H₂₇N₅O | 389.50 | >98% | Pale yellow solid. ¹H NMR (400 MHz, DMSO-*d*₆, 300K) δ_{H} 8.55 (s, 1H), 8.19 (s, 1H), 7.73 (d, *J* = 8.4 Hz, 1H), 7.58 (d, *J* = 8.4 Hz, 1H), 6.68 (s, 1H, NH, D₂O exchanged), 6.56 (s, 1H), 3.83 (s, 3H), 3.08 (s, 3H), 2.29 (s, 6H), 2.14 (s, 3H), 1.73 (s, 6H). MS (ESI⁺) : [M+H]+ 390.2. |
| **253** | 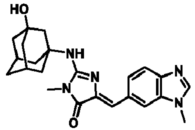 | C₂₃H₂₇N₅O₂ | 405.50 | >98% | Yellow solid. ¹H NMR (400 MHz, DMSO-*d*₆, 300K) δ_{H} 8.58 (s, 1H), 8.20 (s, 1H), 7.69 (d, *J* = 8.5 Hz, 1H), 7.59 (d, *J* = 8.4 Hz, 1H), 6.76 (s, 1H, NH, D₂O exchanged), 6.57 (s, 1H), 4.61 (s, 1H, OH, D₂O exchanged), 3.86 (s, 3H), 3.09 (s, 3H), 2.32 - 2.10 (m, 8H), 1.73 - 1.48 (m, 6H). MS (ESI⁺) : [M+H]⁺ 406.2. |
| **254** | 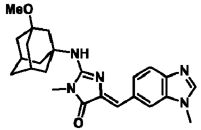 | C₂₄H₂₉N₅O₂ | 419.53 | >98% | Yellow solid. ¹H NMR (400 MHz, DMSO-*d*₆, 300K) δ_{H} 8.51 (s, 1H), 8.20 (s, 1H), 7.76 (d, *J* = 8.5 Hz, 1H), 7.59 (d, *J* = 8.4 Hz, 1H), 6.83 (s, 1H, NH, D₂O exchanged), 6.58 (s, 1H), 3.85 (s, 3H), 3.17 (s, 3H), 3.09 (s, 3H), 2.38 - 2.24 (m, 4H), 2.18 (br s, 4H), 1.72 (s, 4H), 1.66 - 1.51 (m, 2H). MS (ESI⁺) : [M+H]⁺ 420.3. |
| **255** | 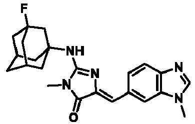 | C₂₃H₂₆FN₅O | 407.49 | >98% | Yellow solid. ¹H NMR (400 MHz, DMSO-*d*₆, 300K) δ_{H} 8.51 (s, 1H), 8.20 (s, 1H), 7.73 (d, *J* = 8.5 Hz, 1H), 7.59 (d, *J* = 8.4 Hz, 1H), 6.92 (s, 1H, NH, D₂O exchanged), 6.60 (s, 1H), 3.84 (s, 3H), 3.09 (s, 3H), 2.55 - 2.30 (m, 4H), 2.26 - 2.10 (m, 4H), 1.89 (s, 4H), 1.64 - 1.52 (m, 2H). ¹⁹F NMR (376 MHz, DMSO-d₆) δ_{F} - 129.02. MS (ESI⁺) : [M+H]⁺ 408.2. |
| **256** | 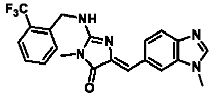 | C₂₁H₁₈F₃N₅O | 413.40 | >98% | Yellow solid. ¹H NMR (400 MHz, DMSO-*d*₆, 300K) δ_{H} 8.30 (t, *J* = 5.9 Hz, 1H, NH, D₂O exchanged), 8.25 (s, 1H), 8.09 (s, 1H), 7.74 ― 7.65 (m, 3H), 7.62 (t, *J* = 7.7 Hz, 1H), 7.48 ― 7.38 (m, 2H), 6.52 (s, 1H), 4.84 (d, *J* = 5.6 Hz, 2H), 3.64 (s, 3H), 3.11 (s, 3H). ¹⁹F NMR (376 MHz, DMSO-d₆) δ_{F} -58.93. MS (ESI⁺) : [M+H]⁺ 414.2. |
| **257** | 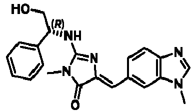 | C₂₁H₂₁N₅O₂ | 375.43 | 98% | Yellow solid. ¹H NMR (400 MHz, DMSO-*d*₆, 300K) δ_{H} 8.45 (s, 1H), 8.21 (s, 1H), 8.01 (d, *J* = 7.8 Hz, 1H, NH, D₂O exchanged), 7.77 (dd, *J* = 8.5, 1.5 Hz, 1H), 7.59 (d, *J* = 8.4 Hz, 1H), 7.55 - 7.50 (m, 2H), 7.40 - 7.34 (m, 2H), 7.29 - 7.22 (m, 1H), 6.55 (s, 1H), 5.28 - 5.19 (m, 1H), 5.07 (t, *J* = 5.8 Hz, 1H, OH, D₂O exchanged), 3.88 (s, 3H), 3.87 - 3.80 (m, 1H), 3.78 - 3.68 (m, 1H), 3.18 (s, 3H). MS (ESI⁺): [M+H]⁺ 376.2. |
| **258** | 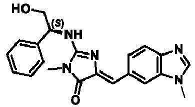 | C₂₁H₂₁N₅O₂ | 375.43 | 98% | Yellow solid. ¹H NMR (400 MHz, DMSO-*d*₆, 300K) δ_{H} 8.45 (s, 1H), 8.21 (s, 1H), 8.01 (d, *J* = 7.8 Hz, 1H, NH, D₂O exchanged), 7.77 (dd, *J* = 8.5, 1.5 Hz, 1H), 7.59 (d, *J* = 8.4 Hz, 1H), 7.55 - 7.50 (m, 2H), 7.40 - 7.34 (m, 2H), 7.29 - 7.22 (m, 1H), 6.55 (s, 1H), 5.28 - 5.19 (m, 1H), 5.07 (t, *J* = 5.8 Hz, 1H, OH, D₂O exchanged), 3.88 (s, 3H), 3.87 - 3.80 (m, 1H), 3.78 - 3.68 (m, 1H), 3.18 (s, 3H). MS (ESI⁺): [M+H]⁺ 376.2. |
| **259** | 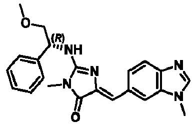 | C₂₂H₂₃N₅O₂ | 389.46 | >98% | Yellow solid. ¹H NMR (400 MHz, DMSO-*d*₆, 300K) δ_{H} 8.46 (s, 1H), 8.21 (s, 1H), 8.14 (d, *J* = 7.8 Hz, 1H, NH, D₂O exchanged), 7.76 (dd, *J* = 8.4, 1.5 Hz, 1H), 7.59 (d, *J* = 8.4 Hz, 1H), 7.56 - 7.52 (m, 2H), 7.42 - 7.35 (m, 2H), 7.30 - 7.25 (m, 1H), 6.55 (s, 1H), 5.47 - 5.37 (m, 1H), 3.88 (s, 3H), 3.82 (dd, *J* = 10.2, 8.8 Hz, 1H), 3.64 (dd, *J* = 10.2, 5.2 Hz, 1H), 3.35 (s, 3H), 3.16 (s, 3H). MS (ESI⁺) : [M+H]⁺ 390.2. |
| **260** | 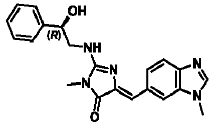 | C₂₁H₂₁N₅O₂ | 375.43 | 98% (¹H NMR) | Yellow solid. ¹H NMR (400 MHz, DMSO-*d*₆, 300K) δ_{H} 8.45 (s, 1H), 8.20 (s, 1H), 8.05 ― 7.98 (m, 1H, NH, D₂O exchanged), 7.94 (dd, *J* = 8.5, 1.5 Hz, 1H), 7.62 (d, *J* = 8.4 Hz, 1H), 7.51 ― 7.45 (m, 2H), 7.43 ― 7.37 (m, 2H), 7.33 ― 7.26 (m, 1H), 6.60 (s, 1H), 5.75 (d, *J* = 4.2 Hz, 1H, OH, D₂O exchanged), 5.10 - 5.03 (m, 1H), 3.84 (s, 3H), 3.81 - 3.72 (m, 1H), 3.45 - 3.34 (m, 1H), 3.10 (s, 3H). MS (ESI⁺): [M+H]⁺ 376.2. |
| **261** | 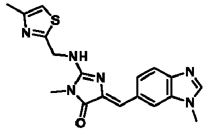 | C₁₈H₁₈N₆OS | 366.44 | >98% | Yellow solid. ¹H NMR (400 MHz, DMSO-*d*₆, 300K) δ_{H} 8.63 (br s, 1H, NH, D₂O exchanged), 8.57 (s, 1H), 8.20 (s, 1H), 7.84 (d, *J* = 8.5 Hz, 1H), 7.59 (d, *J* = 8.4 Hz, 1H), 7.18 (s, 1H), 6.65 (s, 1H), 4.90 (d, *J* = 5.9 Hz, 2H), 3.83 (s, 3H), 3.11 (s, 3H), 2.36 (s, 3H). MS (ESI⁺) : [M+H]⁺ 367.2. |
| **262** | 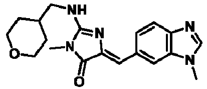 | C₁₉H₂₃N₅O₂ | 353.43 | >98% | Yellow solid. ¹H NMR (400 MHz, DMSO-*d*₆, 300K) δ_{H} 8.57 (s, 1H), 8.19 (s, 1H), 7.85 - 7.70 (m, 1H + NH, D₂O exchanged), 7.59 (d, *J* = 8.4Hz, 1H), 6.56 (s, 1H), 3.93 - 3.85 (m, 2H), 3.83 (s, 3H), 3.38 (t, *J* = 6.3 Hz, 2H), 3.35 - 3.26 (m, 2H), 3.08 (s, 3H), 2.13 - 1.97 (m, 1H), 1.76 - 1.64 (m, 2H), 1.38 - 1.22 (m, 2H). MS (ESI⁺) : [M+H]⁺ 354.2. |
| **263** | 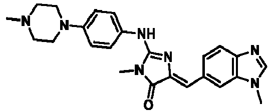 | C₂₄H₂₇N₇O | 429.53 | >98% (¹H NMR) | Yellow solid. ¹H NMR (400 MHz, DMSO-*d*₆, 300K) δ_{H} 9.30 (s, 1H, NH, D₂O exchanged), 8.71 (s, 1H), 8.23 (s, 1H), 7.88 ― 7.80 (m, 2H), 7.74 (d, *J* = 8.4 Hz, 1H), 7.62 (d, *J* = 8.4 Hz, 1H), 7.05 - 6.97 (m, 2H), 6.71 (s, 1H), 3.87 (s, 3H), 3.23 (s, 3H), 3.18 - 3.10 (m, 4H), 2.52 - 2.44 (m, 4H), 2.24 (s, 3H). MS (ESI⁺) : [M+H]+ 430.2. |
| **264** | 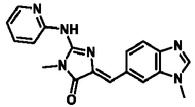 | C₁₈H₁₆N₆O | 332.37 | >98% | Yellow solid. ¹H NMR (400 MHz, CDCl₃, 300K) δ_{H} 12.41 (br s, 1H, NH, D₂O exchanged), 8.33 (dd, *J* = 5.0, 2.0 Hz, 1H), 7.97 (s, 1H), 7.90 (d, *J* = 8.4 Hz, 1H), 7.72 - 7.64 (m, 1H), 7.57 (s, 1H), 7.51 (dd, *J* = 8.4, 1.7 Hz, 1H), 7.23 (d, *J* = 8.1 Hz, 1H), 7.00 - 6.94 (m, 1H), 6.49 (s, 1H), 3.95 (s, 3H), 3.34 (s, 3H). MS (ESI⁺) : [M+H]+ 333.2. |
| **265** | 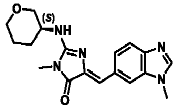 | C₁₈H₂₁N₅O₂ | 339.40 | >98% (¹H NMR) | Yellow solid. ¹H NMR (400 MHz, DMSO-*d*₆, 300K) δ_{H} 8.42 (s, 1H), 8.20 (s, 1H), 7.94 (d, *J* = 8.4 Hz, 1H), 7.61 (d, *J* = 8.4 Hz, 1H), 7.41 (d, *J* = 7.2 Hz, 1H, NH, D₂O exchanged), 6.60 (s, 1H), 4.14 - 3.98 (m, 2H), 3.86 - 3.77 (m, 4H), 3.42 - 3.26 (m, 2H), 3.08 (s, 3H), 2.17 - 2.05 (m, 1H), 1.82 ― 1.56 (m, 3H). MS (ESI⁺): [M+H]+ 340.2. |
| **266** | 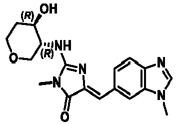 | C₁₈H₂₁N₅O₃ | 355.40 | >95% (¹H NMR) | Beige solid. ¹H NMR (400 MHz, DMSO-*d*₆, 300K) δ_{H} 8.36 (s, 1H), 8.20 (s, 1H), 7.99 (dd, *J* = 8.6, 1.5 Hz, 1H), 7.62 (d, *J* = 8.5 Hz, 1H), 7.45 (d, *J* = 6.9 Hz, 1H, NH, exchanged), 6.59 (s, 1H), 5.12 (d, *J* = 4.5 Hz, 1H, OH, D₂O exchanged), 4.03 (dd, *J* = 10.8, 4.4 Hz, 1H), 3.95 ― 3.75 (m, 6H), 3.39 (td, *J* = 11.6, 2.2 Hz, 1H), 3.23 (t, *J* = 10.3 Hz, 1H), 3.10 (s, 3H), 2.03 - 1.92 (m, 1H), 1.64 - 1.48 (m, 1H). MS (ESI⁺): [M+H]⁺ 356.3. |

### PATHOLOGIES

The compounds of formula (I) may be useful in the treatment and/or in the prevention of a disease selected from cognitive deficits and neuroinflammation associated with Down syndrome (Trisomy 21), Alzheimer's disease and related diseases, dementia; tauopathies; and other neurodegenerative diseases (Parkinson's disease; Pick disease, including Niemann-Pick Type C Disease); CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as malaria, Leishmaniasis, Chagas and sleeping sickness (*Trypanosoma* sp.), cattle diseases due to unicellular pathogens and for regulating body temperature.

According to a particular embodiment, the compounds of formula (I) of the present invention may be useful in the treatment and/or in the prevention of a disease selected from cognitive deficits and neuroinflammation associated with Down syndrome (Trisomy 21), Alzheimer's disease and related diseases, dementia or tauopathies; other neurodegenerative diseases (Parkinson's disease; Pick disease, including Niemann-Pick Type C Disease); CDKL5 Deficiency Disorder; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointerstinal cancer and breast cancer, such as Triple-negative breast cancer (TNBC) and viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature. Said diseases are more particularly associated with the abnormalities in DYRK1A and/or CLK1 dosage.

Still according to this particular embodiment, the compounds of formula (I) of the present invention may be useful in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions, more particularly due to high expression and activity of DYRK1A.

Still according to this particular embodiment, the compounds of formula (I) of the present invention may be useful in the treatment and/or prevention of a disease selected from Down syndrome, Alzheimer's disease and related Tauopathies, Parkinson's disease, the cognitive/motor disorders associated therewith, or one or more symptoms of such diseases. As a typical symptom of such diseases is a decline in learning and memory and social interactions.

Still according to this particular embodiment, the compounds of formula (I) of the present invention may be useful in combatting cognitive decline associated with Down syndrome (Trisomy 21), in learning and memory, in particular associated with the cognitive or neurodegenerative disorders as mentioned above.

Still according to this particular embodiment, the compounds of formula (I) of the present invention may be useful in the treatment and/or prevention of type 1 and type 2 diabetes.

The compounds of formula (I) of the present invention may be useful in the treatment and/or prevention of type 1 and type 2 diabetes either by direct treatment of the diabetic patient or by treating isolated/cultured pancreatic islets or β-cells *in vitro* or *ex vivo* prior to transplantation into diabetic patients.

Herein is further provided a method of treatment of type 1 and type 2 diabetes in a patient in need thereof comprising a step of administering a compound of formula (I) as defined above.

Herein is further provided a method of treatment of type 1 and type 2 diabetes in a patient in need thereof comprising a step of treating isolated or cultured pancreatic islets or β-cells *in vitro* or *ex vivo* prior to transplantation into said patient with a compound of formula (I) as defined above.

Still according to this particular embodiment, the compounds of formula (I) of thre present invention may be useful in the treatment and/or prevention of viral infections, in particular caused by such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV), and in particular by Herpes, Coronaviruses, Cytomegaloviruses and Influenzas. These infections may be associated with high expression and activity of DYRK1A and/or CLK1, and optionally additionally with dual inhibitors of CLKs/DYRKS.

Acute respiratory disease has recently been caused by a new coronavirus (SARS-CoV-2, previously known as 2019-nCoV), also known here as coronavirus 2019 (COVID-19), which belongs to *Coronaviridae.* The compounds of formula (I) according to the present invention can also treat said infection caused by SARS-CoV-2 virus.

Still according to this particular embodiment, the compounds of formula (I) of the present invention may be useful in the treatment and/or prevention of cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC). These cancers may be associated with high expression and activity of DYRK1A and/or CLK1, and optionally additionally with dual inhibitors of CLKs/DYRKS.

Still according to this particular embodiment, the compounds of formula (I) of the present invention may be useful in the treatment and/or prevention of tendinopathy and osteoarthritis. Tendinopathy and osteoarthritis may be associated with high expression and activity of DYRK1A and/or CLK2.

Still according to this particular embodiment, the compounds of formula (I) of the present invention may be useful in the treatment and/or prevention of infections caused by unicellular parasites, such as such as malaria, Leishmaniasis, Chagas and sleeping sickness (*Trypanosoma* sp.), and cattle diseases due to unicellular pathogens Said parasitic infections may be associated with expression and activity of DYRKs/CLKs.

Still according to this particular embodiment, the compounds of formula (I) of the present invention may be useful in the regulation of the body temperature. Said body temperature regulation may be associated with expression and activity of CLKs.

According to another particular embodiment, the compounds of formula (I) of the present invention may be useful in the treatment and/or in the prevention of a disease selected from Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as such as malaria, Leishmaniasis, Chagas and sleeping sickness (*Trypanosoma* sp.), and cattle diseases due to unicellular pathogens. Said diseases are more particularly associated with the abnormalities in other DYRKs (DYR1B, 2, 3, 4) and the closely related further cdc2-like kinases (CLKs) (CLK 2, 3, 4).

The following examples are provided as illustrations and in no way limit the scope of this invention.

The following examples illustrate in detail the preparation of some compounds according to the invention. The structures of the products obtained have been confirmed by NMR analyses and mass spectroscopy.

### Exemple 1: S-alkylation of 2-thiohydantoins

### Example 1.1: Synthesis of 2-methylsulfanyl-1,4-dihydroimidazol-5-one (1.1)

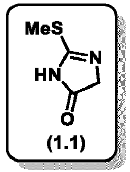

MeI (51.4 mL, 0.827 mol, 4 eq) was slowly added dropwise to a stirred suspension of 2-thiohydantoin (24 g, 206.6 mmol, 1 eq), DIPEA (72 mL, 413.2 mmol, 2 eq) and DMAP (10.096 g, 82.64 mmol, 0.4 eq) in DCM (413 mL) maintained at 0 °C. The resulting mixture was stirred 6h at 0 °C. A precipitate gradually appeared. Upon completion (TLC), the precipitate was filtered on a fritted glass funnel. The resulting solid was adsorbed on silica and purified by FC on silica gel (elution : DCM/MeOH : 99/1 to 9/1). The volume of the collected fractions was reduced to approximately an eighth of its initial volume, until yellow crystals started to appear. The mixture was stirred 30 min at 0 °C and the solid was collected by filtration on a fritted glass funnel to yield 2-methylsulfanyl-1,4-dihydroimidazol-5-one (15.368 g, 118.1 mmol, 57%) in analytically pure form. Pale yellow solid. ¹H NMR (400 MHz, DMSO-*d*₆, 300K) of major tautomer δ_{H} 11.24 (br s, 1H, NH, D₂O exchanged), 4.01 (s, 2H), 2.47 (s, 3H). MS (ESI⁺) : [M+H]⁺ 131.0.

### Example 1.2: Synthesis of 1-methyl-2-methylsulfanyl-4H-imidazol-5-one (1.2)

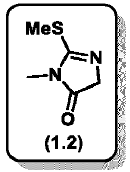

MeO₃BF₄ (6.818 g, 46.1 mmol, 1.5 eq) was added portionwise to a stirred solution of 3-methyl-2-thiohydantoin (4 g, 30.73 mmol, 1 eq) in DCM. The reaction medium was stirred at room temperature for 12h. Upon completion (TLC), the resulting mixture was quenched with sat. Na₂CO₃(aq). The aqueous layer was extracted three times with DCM. The combined organic layers were dried over MgSO₄, filtered, concentrated *in vacuo,* adsorbed on silica, and purified by FC on silica gel (elution : cyclohexane/AcOEt/DCM: 92/5/3 to 50/47/3) to give the desired isothiourea (3.797 g, 26.33 mmol, 85%) in analytically pure form. ¹H NMR (400 MHz, DMSO-*d*₆, 300K) δ_{H} 4.11 (s, 2H), 2.93 (s, 3H), 2.51 (s, 3H). ¹³C NMR (101 MHz, DMSO) δ_{C} 179.6, 162.6, 58.3, 25.9, 11.8. MS (ESI⁺) : [M+H]⁺ 145.0.

### Example 2: General Protocol 1 - Addition of aliphatic and aromatic amines on 2-alkylsulfanyl-1,4-dihydroimidazol-5-ones - ROUTE 1

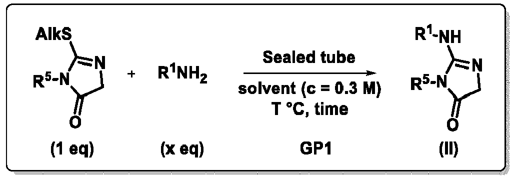

In the above scheme, R¹ and R⁵ are as defined above and Alk is a (C₁-C₅)alkyl.

**GP1:** the appropriate amine (x eq) was added to a stirred solution of the appropriate 2-alkylsulfanyl-1,4-dihydroimidazol-5-one^{(a)} (1 eq) in dry THF (C = 0.3 M/isothiourea) in a sealed tube or sealed round flask (heating block). The mixture was thoroughly purged with vacuum/argon cycles and heated at the appropriate temperature for the indicated time (see details below). Upon completion (followed by consumption of the isothiourea on TLC), the mixture was brought back to room temperature.
- **GP1-A: direct precipitation of the desired product:** The reaction medium was stirred 1h at 0 °C. The precipitated solid was filtered off on a fritted glass funnel. High purity may be achieved after filtration by washing, reprecipitation, trituration, or recrystallization.
- **GP1-B: the product failed to precipitate:** the reaction mixture was poured on Et₂O maintained at 0 °C. The precipitated solid was filtered off on a fritted glass funnel. High purity may be achieved after filtration by washing, reprecipitation, trituration, or recrystallization.
- **GP1-C: the product failed to precipitate:** the reaction mixture was concentrated *in vacuo*, adsorbed on silica, and purified by FC. High purity may be achieved after purification by washing, reprecipitation, trituration, or recrystallization.

- (a) May require activation with AcOH, depending on the amine (see details below).

### Example 2.1: Synthesis of 2-(cyclohexylamino)-1,4-dihydroimidazol-5-one (2.1)

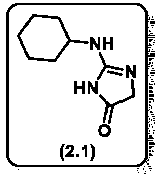

Compound (2.1) was synthesized according to **GP1-A** : reaction carried out in THF, with 2-methylsulfanyl-1,4-dihydroimidazol-5-one (1.1) (8.91 mmol) and 4 eq of cyclohexylamine, at 120 °C (heating block), for 12h. The product directly precipitated in the reaction medium: it was isolated after filtration, washing with cold THF, then pentane. Off-white solid, 35% (571 mg). ¹H NMR (400 MHz, DMSO-*d*₆, 300K) of major tautomer δ_{H} 7.41 (br s, 1H, NH, D₂O exchanged), 6.95 (br s, 1H, NH, D₂O exchanged), 3.61 (s, 2H), 3.39 - 3.23 (m, 1H), 1.88 - 1.75 (m, 2H), 1.74 - 1.62 (m, 2H), 1.61 - 1.51 (m, 1H), 1.33 - 1.03 (m, 5H). MS (ESI⁺) : [M+H]⁺ 182.0.

### Example 2.2: Synthesis of 2-(cycloheptylamino)-1,4-dihydroimidazol-5-one (2.2)

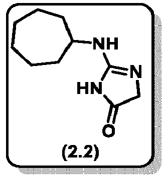

Compound (2.2) was synthesized according to **GP1-A** : reaction carried out in THF, with 2-methylsulfanyl-1,4-dihydroimidazol-5-one (1.1) (11.52 mmol) and 4 eq of cycloheptylamine, at 120 °C (sealed tube, heating bock), for 12h. The product directly precipitated in the reaction medium: it was isolated after filtration, washing with cold THF, then pentane. Off-white solid, 64% (1.429 g). ¹H NMR (400 MHz, DMSO-*d*₆, 300K) of major tautomer δ_{H} 7.42 (br s, 1H, NH, D₂O exchanged), 6.88 (br s, 1H, NH, D₂O exchanged), 3.86 - 3.68 (m, 1H), 3.59 (s, 2H), 1.89 - 1.76 (m, 2H), 1.70 - 1.32 (m, 10H). MS (ESI⁺) : [M+H]⁺ 196.0.

### Example 2.3 : Synthesis of 2-(cyclooctylamino)-1,4-dihydroimidazol-5-one (2.3)

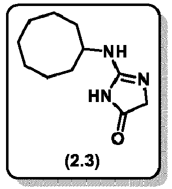

Compound (2.3) was synthesized according to **GP1-A** : reaction carried out in THF, with 2-methylsulfanyl-1,4-dihydroimidazol-5-one (1.1) (11.52 mmol) and 4 eq of cyclooctylamine, at 120 °C (sealed tube, heating bock), for 12h. The product directly precipitated in the reaction medium: it was isolated after filtration, washing with cold THF, then pentane. Off-white solid, 72% (1.739 g). ¹H NMR (400 MHz, DMSO-*d*₆, 300K) of major tautomer δ_{H} 7.43 (br s, 1H, NH, D₂O exchanged), 6.86 (br s, 1H, NH, D₂O exchanged), 3.89 - 3.74 (m, 1H), 3.59 (s, 2H), 1.90 - 1.27 (m, 14H). MS (ESI⁺) : [M+H]⁺ 210.0.

### Example 2.4: Synthesis of 2-[[(1R)-1-(hydroxymethyl)-3-methylbutyl]amino]-1,4-dihydroimidazol-5-one (2.4)

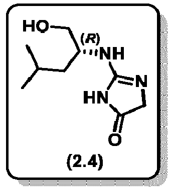

Compound (2.4) was synthesized according to **GP1-A:** reaction carried out in THF with 2-methylsulfanyl-1,4-dihydroimidazol-5-one (1.1) (16.90 mmol), 2.5 eq of (D)-leucinol, at 125 °C, for 4h. The product directly precipitated in the reaction medium: it was isolated after filtration, washing with cold THF, then pentane. Light beige solid, 52% (1.750 g). ¹H NMR (400 MHz, DMSO-*d*₆, 343K) of major tautomer δ_{H} 7.11 (br s, 2H, NH, D₂O exchanged), 4.61 (s, 1H, OH, D₂O exchanged), 3.91 - 3.70 (m, 1H), 3.61 (s, 2H), 3.46 - 3.28 (m, 2H), 1.70 - 1.56 (m, 1H), 1.37 (t, *J* = 7.0 Hz, 2H), 0.89 (t, *J* = 7.1 Hz, 6H). MS (ESI⁺) : [M+H]⁺ 200.1.

### Example 2.5: Synthesis of 2-[[(1R)-1-(methoxymethyl)-3-methylbutyl]amino]-1,4-dihydroimidazol-5-one (2.5)

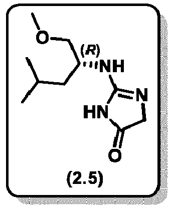

Compound (2.5) was synthesized according to **GP1-B** : reaction carried out in THF, with 2-methylsulfanyl-1,4-dihydroimidazol-5-one (1.1) (11.52 mmol), 2 eq of (2*R*)-1-methoxy-4-methyl-pentan-2-amine and 4 eq of AcOH. Light beige solid, 39% (948 mg). ¹H NMR (400 MHz, DMSO-*d*₆, 343K) of major tautomer δ_{H} 7.20 (br s, 1H, NH, D₂O exchanged), 6.98 (br s, 1H, NH, D₂O exchanged), 4.09 - 3.75 (m, 1H), 3.61 (s, 2H), 3.39 - 3.29 (m, 2H), 3.27 (s, 3H), 1.71 - 1.56 (m, 1H), 1.47 - 1.27 (m, 2H), 0.89 (t, *J* = 6.7 Hz, 6H). MS (ESI⁺) : [M+H]⁺ 214.3.

### Example 2.6: Preparation of 2-[[(1R)-1-(fluoromethyl)-3-methylbutyl]amino]-1,4-dihydroimidazol-5-one (2.6)

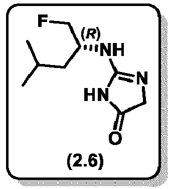

Compound (2.6) was synthesized according to **GP1-B** : reaction carried out in THF, with 2-methylsulfanyl-1,4-dihydroimidazol-5-one (1.1) (4.088 mmol), 1.6 eq of (2*R*)-1-fluoro-4-methyl-pentan-2-amine and 4 eq of AcOH, at 120 °C (heating block), for 4h. Beige solid, 32% (267 mg). ¹H NMR (400 MHz, DMSO-*d*₆, 343K) of major tautomer δ_{H} 7.39 (br s, 1H, NH, D₂O exchanged), 7.12 (br s, 1H, NH, D₂O exchanged), 4.47 - 4.38 (m, 1H), 4.36 ― 4.26 (m, 1H), 4.11 - 3.89 (m, 1H), 3.63 (s, 2H), 1.73 ― 1.59 (m, 1H), 1.52 ― 1.42 (m, 1H), 1.40 - 1.29 (m, 1H), 0.97 - 0.86 (m, 6H). MS (ESI⁺) : [M+H]⁺ 202.1.

### Example 2.7: Synthesis of 2-[[(1S)-1-(fluoromethyl)-3-methylbutyl]amino]-1,4-dihydroimidazol-5-one (2.7)

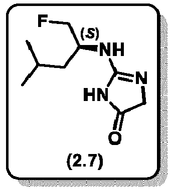

Compound (2.7) was synthesized according to **GP1-B** : reaction carried out in THF, with 2-methylsulfanyl-1,4-dihydroimidazol-5-one (1.1) (4.088 mmol), 1.6 eq of (2*S*)-1-fluoro-4-methyl-pentan-2-amine and 4 eq of AcOH, at 120 °C (heating block), for 4h. Beige solid, 44% (366 mg). ¹H NMR (400 MHz, DMSO-*d*₆, 343K) of major tautomer δ_{H} 7.39 (br s, 1H, NH, D₂O exchanged), 7.12 (br s, 1H, NH, D₂O exchanged), 4.47 - 4.38 (m, 1H), 4.36 ― 4.26 (m, 1H), 4.11 - 3.89 (m, 1H), 3.63 (s, 2H), 1.73 ― 1.59 (m, 1H), 1.52 - 1.42 (m, 1H), 1.40 - 1.29 (m, 1H), 0.97 - 0.86 (m, 6H). MS (ESI⁺) : [M+H]⁺ 202.1.

### Example 2.8: Synthesis of 2-[[(1R,2R)-2-methoxycyclopentyl]amino]-1,4-dihydroimidazol-5-one (2.8)

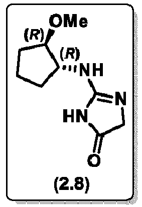

Compound (2.8) was synthesized according to **GP1-A:** reaction carried out in THF with 2-methylsulfanyl-1,4-dihydroimidazol-5-one (1.1) (868 µmol), 3 eq of (1*R*,2*R*)-2-methoxycyclopentanamine, at 115 °C, for 12h. The product directly precipitated in the reaction medium: it was isolated after filtration, washing with cold THF, then pentane. Colorless solid, 74% (127 mg). ¹H NMR (400 MHz, DMSO-*d*₆, 343K) of major tautomer δ_{H} 7.57 (br s, 1H, NH, D₂O exchanged), 7.13 (br s, 1H, NH, D₂O exchanged), 3.99 - 3.79 (m, 1H), 3.69 - 3.63 (m, 1H), 3.61 (s, 2H), 3.25 (s, 3H), 2.05 - 1.83 (m, 2H), 1.73 - 1.40 (m, 4H). MS (ESI⁺) : [M+H]⁺ 198.1.

### Example 2.9: Synthesis of 2-[[(1S,2S)-2-methoxycyclopentyl]amino]-1,4-dihydroimidazol-5-one (2.9)

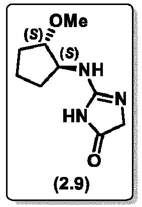

Compound (2.9) was synthesized according to **GP1-A:** reaction carried out in THF with 2-methylsulfanyl-1,4-dihydroimidazol-5-one (1.1) (2.89 mmol), 3 eq of (1S*R*,2*S*)-2-methoxycyclopentanamine, at 115 °C, for 12h. The product directly precipitated in the reaction medium: it was isolated after filtration, washing with cold THF, then pentane. Colorless solid, 71% (405 mg). ¹H NMR (400 MHz, DMSO-*d*₆, 343K) of major tautomer δ_{H} 7.57 (br s, 1H, NH, D₂O exchanged), 7.13 (br s, 1H, NH, D₂O exchanged), 3.99 - 3.79 (m, 1H), 3.69 - 3.63 (m, 1H), 3.61 (s, 2H), 3.25 (s, 3H), 2.05 - 1.83 (m, 2H), 1.73 - 1.40 (m, 4H). MS (ESI⁺) : [M+H]⁺ 198.1.

### Example 2.10: Synthesis of 2-(3-noradamantylamino)-1,4-dihydroimidazol-5-one (2.10)

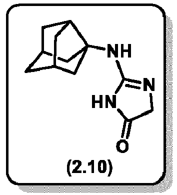

Compound (2.10) was synthesized according to **GP1-C** : reaction carried out in dioxane, with 2-methylsulfanyl-1,4-dihydroimidazol-5-one (1.1) (3.55 mmol), 3 eq of 3-noradamantanamine and 4 eq of AcOH, at 130 °C (heating block), for 12h. Purification by FC (elution: DCM/MeOH (7N NH₃): 99/1 to 9/1). The final product required a trituration in DCM at 0 °C. Light beige solid, 65% (502 mg). ¹H NMR (400 MHz, DMSO-*d*₆, 343K) δ_{H} 7.47 (br s, 1H, NH, D₂O exchanged), 6.64 (br s, 1H, NH, D₂O exchanged), 3.56 (s, 2H), 2.41 (t, *J* = 6.8 Hz, 1H), 2.29 - 2.20 (m, 2H), 2.10 - 1.90 (m, 6H), 1.64 - 1.45 (m, 4H). MS (ESI⁺) : [M+H]⁺ 220.2.

### Example 2.11: Synthesis of 2-(1-adamantylamino)-1,4-dihydroimidazol-5-one (2.11)

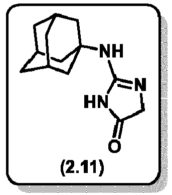

Compound (2.11) was synthesized according to **GP1-C** : reaction carried out in dioxane, with 2-methylsulfanyl-1,4-dihydroimidazol-5-one (1.1) (3.073 mmol), 3 eq of adamantan-1-amine and 4 eq of AcOH, at 150 °C (heating block), for 16h. Purification by FC (elution: DCM/MeOH (7N NH₃): 99/1 to 9/1). The final product required a trituration in DCM at 0 °C. Beige solid, 35% (254 mg). ¹H NMR (400 MHz, DMSO-*d*₆, 300K) δ_{H} 7.18 (br s, 1H, NH, D₂O exchanged), 6.73 (br s, 1H, NH, D₂O exchanged), 3.52 (s, 2H), 2.03 (s, 3H), 1.96 (s, 6H), 1.62 (s, 6H). MS (ESI⁺) : [M+H]⁺ 234.2.

### Example 2.12: Synthesis of 2-[(3-hydroxy-1-adamantyl)amino]-1,4-dihydroimidazol-5-one (2.12)

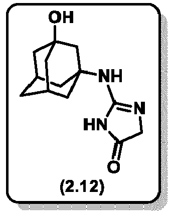

Compound (2.11) was synthesized according to **GP1-C** : reaction carried out in dioxane, with 2-methylsulfanyl-1,4-dihydroimidazol-5-one (1.1) (9.22 mmol), 3 eq of 3-aminoadamantan-1-ol and 4 eq of AcOH, at 145 °C (heating block), for 12h. Purification by FC (elution: DCM/MeOH (7N NH₃): 99/1 to 85/15). The final product required a trituration in refluxing DCM. Beige solid, 58% (1.336 g). ¹H NMR (400 MHz, DMSO-*d*₆, 300K) δ_{H} 7.25 (br s, 1H, NH, D₂O exchanged), 6.75 (br s, 1H, NH, D₂O exchanged), 4.53 (s, 1H, OH, D₂O exchanged), 3.53 (s, 2H), 2.15 (br s, 2H), 1.90 ― 1.78 (m, 6H), 1.60 ― 1.35 (m, 6H). MS (ESI⁺) : [M+H]⁺ 250.3.

### Example 2.13: Synthesis of 2-[(3-methoxy-1-adamantyl)amino]-1,4-dihydroimidazol-5-one (2.13)

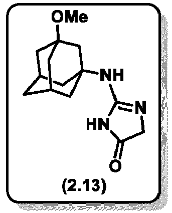

Compound (2.13) was synthesized according to **GP1-C** : reaction carried out in dioxane, with 2-methylsulfanyl-1,4-dihydroimidazol-5-one (1.1) (768 µmol), 3 eq of 3-methoxyadamantan-1-amine and 4 eq of AcOH, at 150 °C (heating block), for 16h. Purification by FC (elution: DCM/MeOH (7N NH₃): 99/1 to 9/1). Brown solid, 50% (102 mg). ¹H NMR (400 MHz, DMSO-*d*₆, 300K) δ_{H} 7.30 (br s, 1H, NH, D₂O exchanged), 6.77 (br s, 1H, NH, D₂O exchanged), 3.53 (s, 2H), 3.12 (s, 3H), 2.21 (br s, 2H), 1.95 - 1.80 (m, 6H), 1.67 - 1.56 (m, 4H), 1.55 - 1.40 (m, 2H). MS (ESI⁺) : [M+H]⁺ 264.3.

### Example 2.14: Synthesis of 2-[(3-fluoro-1-adamantyl)amino]-1,4-dihydroimidazol-5-one (2.14)

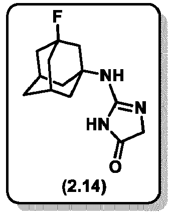

Compound (2.14) was synthesized according to **GP1-C** : reaction carried out in dioxane, with 2-methylsulfanyl-1,4-dihydroimidazol-5-one (1.1) (3.073 mmol), 3 eq of 3-fluoro-adamantan-1-amine and 4 eq of AcOH, at 130 °C (heating block), for 12h. Purification by FC (elution: DCM/MeOH (7N NH₃): 99/1 to 85/15). The final product required a trituration in Et₂O at 0 °C. Beige solid, 44% (343 mg). ¹H NMR (400 MHz, DMSO-*d*₆, 300K) δ_{H} 7.41 (br s, 1H, NH, D₂O exchanged), 6.83 (br s, 1H, NH, D₂O exchanged), 3.54 (s, 2H), 2.35 - 2.24 (m, 2H), 2.14 (d, *J* = 5.9 Hz, 2H), 2.01 ― 1.72 (m, 8H), 1.56 ― 1.43 (m, 2H). MS (ESI⁺) : [M+H]⁺ 252.3.

### Example 2.15 : Synthesis of 2-[[(1R)-2-methoxy-1-phenyl-ethyl]amino]-1,4-dihydroimidazol-5-one (2.15)

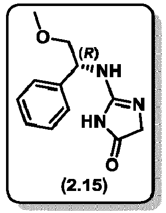

Compound (2.15) was synthesized according to **GP1-A** : reaction carried out in THF, with 2-methylsulfanyl-1,4-dihydroimidazol-5-one (1.1) (3.073 mmol), 3 eq of (1*R*)-2-methoxy-1-phenyl-ethanamine and 4 eq of AcOH, at 115 °C (sealed tube, heating bock), for 12h. The product directly precipitated in the reaction medium: it was isolated after filtration, washing with cold THF, then pentane. Beige solid, 58% (414 mg). ¹H NMR (400 MHz, DMSO-*d*₆, 343K) 7.95 (bs, 1H, NH, D₂O exchanged), 7.60 - 7.22 (m, 5H), 7.17 (bs, 1H, NH, D₂O exchanged), 5.09 - 4.85 (m, 1H), 3.67 - 3.63 (m, 1H), 3.62 (s, 2H), 3.56 (dd, *J* = 10.2, 5.2 Hz, 1H), 3.29 (s, 3H). MS (ESI⁺) : [M+H]⁺ 234.2.

### Example 2.16: Synthesis of tert-butyl N-[(2R)-2-[(5-oxo-1,4-dihydroimidazol-2-yl)amino]-2-phenyl-ethyl]carbamate (2.16)

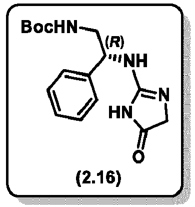

Compound (2.16) was synthesized according to **GP1-A** : reaction carried out in THF, with 2-methylsulfanyl-1,4-dihydroimidazol-5-one (1.1) (10.18 mmol), 1.5 eq of *tert-*butyl *N*-[(2*R*)-2-amino-2-phenyl-ethyl]carbamate and 2.3 eq of AcOH, at 115 °C (sealed tube, heating bock), for 6h. The product directly precipitated in the reaction medium: it was isolated after filtration, washing with cold THF, then pentane. Colorless solid, 40% (1.517 g). ¹H NMR (400 MHz, DMSO-*d*₆, 343K) 7.83 (br s, 1H, NH, D₂O exchanged), 7.56 ― 7.05 (m, 5H + NH, D₂O exchanged), 6.63 (br s, 1H, NH, D₂O exchanged), 4.91 (br s, 1H), 3.59 (s, 2H), 3.29 (t, *J* = 6.5 Hz, 2H), 1.36 (s, 9H). MS (ESI⁺) : [M+H]⁺ 319.2.

### Example 2.17: Synthesis of tert-butyl N-[(2S)-2-[(5-oxo-1,4-dihydroimidazol-2-yl)amino]-2-phenyl-ethyl]carbamate (2.17)

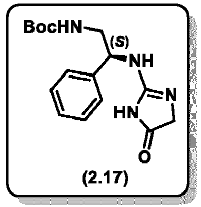

Compound (2.17) was synthesized according to **GP1-A** : reaction carried out in THF, with 2-methylsulfanyl-1,4-dihydroimidazol-5-one (1.1) (10.92 mmol), 1.5 eq of *tert-*butyl *N*-[(2*S*)-2-amino-2-phenyl-ethyl]carbamate and 2.3 eq of AcOH, at 115 °C (heating bock), for 6h. The product directly precipitated in the reaction medium: it was isolated after filtration, washing with cold THF, then pentane. Colorless solid, 38% (1.306 g). ¹H NMR (400 MHz, DMSO-*d*₆, 343K) 7.83 (br s, 1H, NH, D₂O exchanged), 7.56 - 7.05 (m, 5H + NH, D₂O exchanged), 6.63 (br s, 1H, NH, D₂O exchanged), 4.91 (br s, 1H), 3.59 (s, 2H), 3.29 (t, *J* = 6.5 Hz, 2H), 1.36 (s, 9H). MS (ESI⁺) : [M+H]⁺ 319.3.

### Example 2.18: Synthesis of 2-[(4-methylthiazol-2-yl)methylamino]-1,4-dihydroimidazol-5-one (2.18)

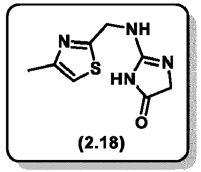

Compound (2.18) was synthesized according to **GP1-A** : reaction carried out in THF, with 2-methylsulfanyl-1,4-dihydroimidazol-5-one (1.1) (3.073 mmol), 2 eq of (4-methylthiazol-2-yl)methanamine and 3 eq of AcOH, at 110 °C (sealed tube, heating block), for 3h. The product directly precipitated in the reaction medium: it was isolated after filtration, washing with cold THF, then pentane. Beige solid, 35% (248 mg). ¹H NMR (400 MHz, DMSO-*d*₆, 300K) δ_{H} 8.21 (br s, 1H, NH, D₂O exchanged), 7.59 (br s, 1H, NH, D₂O exchanged), 7.17 (s, 1H), 4.65 (s, 2H), 3.69 (s, 2H), 2.33 (s, 3H). MS (ESI⁺) : [M+H]⁺ 211.1.

### Example 2.19: Synthesis of 2-(tetrahydropyran-4-ylmethylamino)-1,4-dihydroimidazol-5-one (2.19)

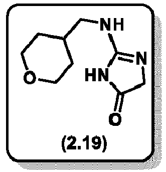

Compound (2.19) was synthesized according to **GP1-A** : reaction carried out in THF, with 2-methylsulfanyl-1,4-dihydroimidazol-5-one (1.1) (3.073 mmol) and 3 eq of tetrahydropyran-4-ylmethanamine, at 110 °C (sealed tube, heating block), for 16h. The product directly precipitated in the reaction medium: it was isolated after filtration, washing with cold THF, then pentane. Beige solid, 70% (424 mg). ¹H NMR (400 MHz, DMSO-*d*₆, 343K) δ_{H} 7.47 (br s, 1H, NH, D₂O exchanged), 7.22 (br s, 1H, NH, D₂O exchanged), 3.90 - 3.80 (m, 2H), 3.61 (s, 2H), 3.28 (td, *J* = 11.6, 2.0 Hz, 2H), 3.11 - 3.05 (m, 2H), 1.80 ― 1.73 (m, 1H), 1.64 ― 1.52 (m, 2H), 1.19 (qd, *J* = 11.8, 4.4 Hz, 2H). MS (ESI⁺) : [M+H]⁺ 198.1.

### Example 2.20: Synthesis of 2-[4-(4-methylpiperazin-1-yl)anilino]-1,4-dihydroimidazol-5-one (2.20)

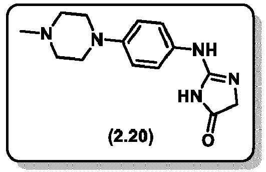

Compound (2.20) was synthesized according to **GP1-C** : reaction carried out in dioxane, with 2-methylsulfanyl-1,4-dihydroimidazol-5-one (1.1) (4.61 mmol), 2 eq of 4-(4-methylpiperazin-1-yl)aniline and 3 eq of AcOH, at 130 °C (heating block), for 3h. Purification by FC (elution: DCM/MeOH (7N NH₃): 99/1 to 85/15). The final product required two successive triturations in EtOH at 0 °C. Beige solid, 59% (749 mg). ¹H NMR (400 MHz, DMSO-*d*₆, 300K) δ_{H} 9.66 (br s, 1H, NH, D₂O exchanged), 7.43 (br s, 1H, NH, D₂O exchanged), 7.33 ― 7.19 (m, 2H), 6.91 (d, *J* = 8.9 Hz, 2H), 3.67 (s, 2H), 3.13 - 3.03 (m, 4H), 2.47 - 2.41 (m, 4H), 2.21 (s, 3H). MS (ESI⁺) : [M+H]⁺ 274.2.

### Example 2.21 : Synthesis of 2-(2-pyridylamino)-1,4-dihydroimidazol-5-one (2.21)

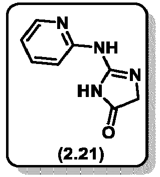

Compound (2.21) was synthesized according to **GP1-C** : reaction carried out in dioxane, with 2-methylsulfanyl-1,4-dihydroimidazol-5-one (1.1) (6.15 mmol), 2.5 eq of 2-aminopyridine and 3 eq of AcOH, at 130 °C (heating block), for 12h. Purification by FC (elution: DCM/MeOH (7N NH₃): 99/1 to 85/15). The final product required a trituration in DCM at room temperature. Light beige solid, 35% (384 mg). ¹H NMR (400 MHz, DMSO-*d*6, 300K) δ_{H} 10.99 (bs, 1H, NH, D₂O exchanged), 9.20 (bs, 1H, NH, D₂O exchanged), 8.30 - 8.22 (m, 1H), 7.79 ― 7.73 (m, 1H), 7.15 (d, *J* = 8.3 Hz, 1H), 7.05 (dd, *J* = 7.3, 5.0 Hz, 1H), 3.91 (s, 2H). MS (ESI⁺) : [M+H]⁺ 177.2.

### Example 2.22 : Synthesis of (±)-2-(oxepan-3-ylamino)-1,4-dihydroimidazol-5-one (2.22)

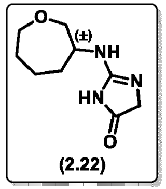

Compound 2.22 was synthesized according to **GP1-A** : reaction carried out in THF, with 2-methylsulfanyl-1,4-dihydroimidazol-5-one (1.1) (851 µmol), 2.5 eq of oxepan-3-amine, at 110 °C (heating block), for 12h The product directly precipitated in the reaction medium: it was isolated after filtration, washing with cold THF, then pentane. Light beige solid, 73% (122 mg). ¹H NMR (400 MHz, DMSO-*d*₆, 343K) δ_{H} 7.33 (br s, 1H, NH, D₂O exchanged), 7.05 (br s, 1H, NH, D₂O exchanged), 3.96 - 3.79 (m, 1H), 3.76 - 3.62 (m, 3H), 3.61 (s, 2H), 3.56 - 3.46 (m, 1H), 1.91 - 1.77 (m, 1H), 1.77 - 1.60 (m, 4H), 1.59 - 1.46 (m, 1H). MS (ESI⁺) : [M+H]⁺ 198.1.

### Example 2.23: Synthesis of 2-(cyclohexylamino)-1-methyl-4H-imidazol-5-one (2.23)

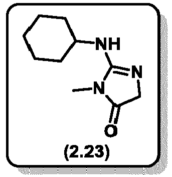

Compound (2.23) was synthesized according to **GP1-C** : reaction carried out in dioxane, with 1-methyl-2-methylsulfanyl-4*H*-imidazol-5-one (1.2) (1.04 mmol), 3 eq of cyclohexylamine and 5 eq of AcOH, at 150 °C (heating block), for 3h. Purification by FC (elution: DCM/MeOH: 99/1 to 9/1). Brown solid, 44% (89 mg). ¹H NMR (400 MHz, DMSO-*d*₆, 300K) δ_{H} 6.64 (br s, 1H, NH, D₂O exchanged), 3.81 (s, 2H), 3.48 - 3.37 (m, 1H), 2.88 (s, 3H), 1.87 - 1.55 (m, 4H), 1.34 - 1.07 (m, 6H). MS (ESI⁺) : [M+H]⁺ 196.3.

### Example 2.24: Synthesis of 2-(cyclooctylamino)-1-methyl-4H-imidazol-5-one (2.24)

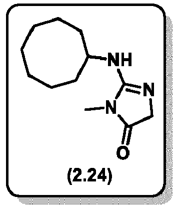

Compound (2.24) was synthesized according to **GP1-C** : reaction carried out in dioxane, with 1-methyl-2-methylsulfanyl-4*H*-imidazol-5-one (1.2) (1.04 mmol), 3 eq of cyclooctylamine and 5 eq of AcOH, at 150 °C (heating block), for 3h. Purification by FC (elution: DCM/MeOH: 99/1 to 9/1). Brown oil, 41% (105 mg). ¹H NMR (400 MHz, DMSO-*d*₆, 300K) δ_{H} 6.71 (br s, 1H, NH, D₂O exchanged), 3.84 (s, 2H), 3.70 (br s, 1H), 2.89 (s, 3H), 1.78 - 1.41 (m, 14H). MS (ESI⁺) : [M+H]⁺ 224.3.

### Example 2.25: Synthesis of 2-[[(1R)-1-(methoxymethyl)-3-methylbutyl]amino]-1-methyl-4H-imidazol-5-one (2.25)

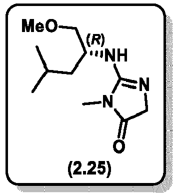

Compound (2.25) was synthesized according to **GP1-C** : reaction carried out in dioxane, with 1-methyl-2-methylsulfanyl-4*H*-imidazol-5-one (1.2) (1.04 mmol), 3 eq of (2*R*)-1-methoxy-4-methyl-pentan-2-amine and 3 eq of AcOH, at 150 °C (heating block), for 3h. Purification by FC (elution: DCM/MeOH: 99/1 to 9/1). Brown oil, 41% (97 mg). ¹H NMR (400 MHz, DMSO-*d*₆, 300K) δ_{H} n.d. MS (ESI⁺) : [M+H]⁺ 228.3.

### Example 2.26: Synthesis of 2-[[(1R,2R)-2-methoxycyclopentyl]amino]-1-methyl-4H-imidazol-5-one (2.26)

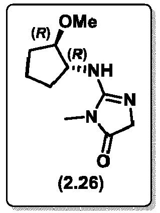

Compound (2.26) was synthesized according to **GP1-C** : reaction carried out in dioxane, with 1-methyl-2-methylsulfanyl-4*H*-imidazol-5-one (1.04 mmol), 3 eq of (1*R*,2*R*)-2-methoxycyclopentanamine and 5 eq of AcOH, at 130 °C (heating block), for 3h. Purification by FC (elution: DCM/MeOH: 99/1 to 9/1). Brown oil, 47% (130 mg). ¹H NMR (400 MHz, DMSO-*d*₆, 300K) δ_{H} 7.07 (br s, 1H, NH, D₂O exchanged), 3.83 (s, 2H), 3.76 (br s, 1H), 3.60 (br s, 1H), 3.22 (s, 3H), 2.86 (s, 3H), 1.98 - 1.82 (m, 2H), 1.69 - 1.47 (m, 3H), 1.43 ― 1.33 (m, 1H). MS (ESI⁺) : [M+H]⁺ 212.3.

### Example 2.27: Synthesis of 2-[[(1S,2S)-2-methoxycyclopentyl]amino]-1-methyl-4H-imidazol-5-one (2.27)

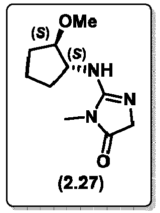

Compound (2.26) was synthesized according to **GP1-C** : reaction carried out in dioxane, with 1 -methyl-2-methylsulfanyl-4*H*-imidazol-5 -one (1.04 mmol), 3 eq of (15,25)-2-methoxycyclopentanamine and 5 eq of AcOH, at 130 °C (heating block), for 3h. Purification by FC (elution: DCM/MeOH: 99/1 to 9/1). Brown oil, 33% (96 mg). ¹H NMR (400 MHz, DMSO-*d*₆, 300K) δ_{H} 7.07 (br s, 1H, NH, D₂O exchanged), 3.83 (s, 2H), 3.76 (br s, 1H), 3.60 (br s, 1H), 3.22 (s, 3H), 2.86 (s, 3H), 1.98 - 1.82 (m, 2H), 1.69 - 1.47 (m, 3H), 1.43 ― 1.33 (m, 1H). MS (ESI⁺) : [M+H]⁺ 212.3.

### Example 2.28: Synthesis of 2-(3-noradamantylamino)-1-methyl-4H-imidazol-5-one (2.28)

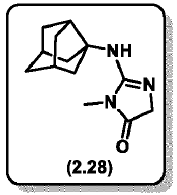

Compound (2.28) was synthesized according to **GP1-C** : reaction carried out in dioxane, with 1-methyl-2-methylsulfanyl-4*H*-imidazol-5-one (1.04 mmol), 3 eq of 3-noradamantanamine and 5 eq of AcOH, at 150 °C (heating block), for 6h. Purification by FC (elution: DCM/MeOH: 99/1 to 9/1). Beige solid, 37% (100 mg). ¹H NMR (400 MHz, DMSO-*d*₆, 300K) δ_{H} ¹H NMR (400 MHz, DMSO-*d*₆, 300K) δ 6.33 (s, 1H, NH, D₂O exchanged), 3.84 (s, 2H), 2.92 (s, 3H), 2.26 - 2.09 (m, 3H), 2.01 - 1.84 (m, 6H), 1.61 - 1.43 (m, 4H). MS (ESI⁺) : [M+H]⁺ 234.3.

### Example 2.29: Synthesis of 2-(1-adamantylamino)-1-methyl-4H-imidazol-5-one (2.29)

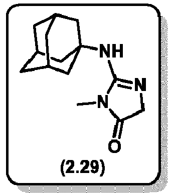

Compound (2.29) was synthesized according to **GP1-C** : reaction carried out in dioxane, with 1-methyl-2-methylsulfanyl-4*H*-imidazol-5-one (1.2) (1.04 mmol), 3 eq of adamantan-1-amine and 5 eq of AcOH, at 150 °C (heating block), for 24h. Purification by FC (elution: DCM/MeOH: 99/1 to 9/1). Brown solid, 7% (19 mg). ¹H NMR (400 MHz, DMSO-*d*₆, 300K) δ_{H} 5.58 (br s, 1H, NH, D₂O exchanged), 3.83 (s, 2H), 2.90 (s, 3H), 2.11 - 1.97 (m, 8H), 1.71 - 1.59 (m, 7H). MS (ESI⁺) : [M+H]⁺ 248.3.

### Example 2.30: Synthesis of 2-[(3-hydroxy-1-adamantyl)amino]-1-methyl-4H-imidazol-5-one (2.30)

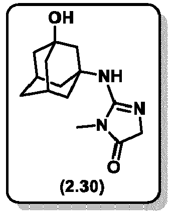

Compound (2.30) was synthesized according to **GP1-C** : reaction carried out in dioxane, with 1-methyl-2-methylsulfanyl-4*H*-imidazol-5-one (1.04 mmol), 3 eq of 3-hydroxyadamantan-1-amine and 5 eq of AcOH, at 150 °C (heating block), for 24h. Purification by FC (elution: DCM/MeOH: 99/1 to 9/1). Beige solid, 11% (52 mg). ¹H NMR (400 MHz, DMSO-*d*₆, 300K) δ_{H} 5.76 (br s, 1H, NH, D₂O exchanged), 4.46 (s, 1H, OH, D₂O exchanged), 3.85 (s, 2H), 2.90 (s, 3H), 2.15 (s, 2H), 2.03 - 1.91 (m, 6H), 1.60 - 1.39 (m, 6H). MS (ESI⁺) : [M+H]⁺ 264.2.

### Example 2.31 : Synthesis of 2-[(3-methoxy-1-adamantyl)amino]-1-methyl-4H-imidazol-5-one (2.31)

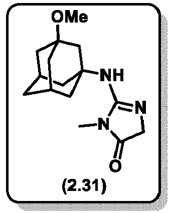

Compound (2.31) was synthesized according to **GP1-C** : reaction carried out in dioxane, with 1-methyl-2-methylsulfanyl-4*H*-imidazol-5-one (1.04 mmol), 3 eq of 3-methoxyadamantan-1-amine and 5 eq of AcOH, at 150 °C (heating block), for 24h. Purification by FC (elution: DCM/MeOH: 99/1 to 9/1). Beige solid, 10% (36 mg). ¹H NMR (400 MHz, DMSO-*d*₆, 300K) δ_{H} 5.75 (br s, 1H, NH, D₂O exchanged), 3.84 (s, 2H), 3.11 (s, 3H), 2.89 (br s, 3H), 2.20 (br s, 2H), 2.09 - 1.91 (m, 6H), 1.68 - 1.54 (m, 4H), 1.54 - 1.41 (m, 2H). MS (ESI⁺) : [M+H]⁺ 278.3.

### Example 2.32: Synthesis of 2-[(3-fluoro-1-adamantyl)amino]-1-methyl-4H-imidazol-5-one (2.32)

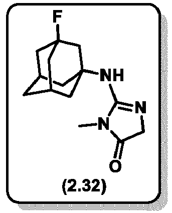

Compound (2.32) was synthesized according to **GP1-C** : reaction carried out in dioxane, with 1-methyl-2-methylsulfanyl-4*H*-imidazol-5-one (1.04 mmol), 3 eq of 3-fluoroadamantan-1-amine and 5 eq of AcOH, at 150 °C (heating block), for 24h. Purification by FC (elution: DCM/MeOH: 99/1 to 9/1). Beige solid, 14% (64 mg). ¹H NMR (400 MHz, DMSO-*d*₆, 300K) δ_{H} n.d. MS (ESI⁺) : [M+H]⁺ 266.2.

### Example 2.33: Synthesis of 1-methyl-2-[[2-(trifluoromethyl)phenyl]methylamino]-4H-imidazol-5-one (2.33)

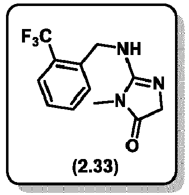

Compound (2.33) was synthesized according to **GP1-C** : reaction carried out in dioxane, with 1-methyl-2-methylsulfanyl-4*H*-imidazol-5-one (1.04 mmol), 3 eq of [2-(trifluoromethyl)phenyl]methanamine and 5 eq of AcOH, at 150 °C (heating block), for 1.5h. Purification by FC (elution: DCM/MeOH: 99/1 to 9/1). Brown oil, 33% (131 mg). ¹H NMR (400 MHz, DMSO-*d*₆, 300K) δ_{H} 7.87 ― 7.76 (m, 1H), 7.72 ― 7.61 (m, 2H), 7.47 ― 7.38 (m, 1H), 7.25 (br s, 1H, NH, D₂O exchanged), 4.49 (s, 2H), 3.86 (s, 2H), 2.96 (s, 3H). MS (ESI⁺) : [M+H]⁺ 272.2.

### Example 2.34: Synthesis of 2-[[(1R)-2-methoxy-1-phenyl-ethyl]amino]-1-methyl-4H-imidazol-5-one (2.34)

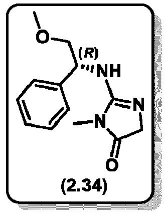

Compound (2.34) was synthesized according to **GP1-C** : reaction carried out in dioxane, with 1-methyl-2-methylsulfanyl-4*H*-imidazol-5-one (1.04 mmol), 3 eq of 3 eq of (1*R*)-2-methoxy-1-phenyl-ethanamine and 3 eq of AcOH, at 150 °C (heating block), for 1.5h. Purification by FC (elution: DCM/MeOH: 99/1 to 9/1). Beige oil, 45% (195 mg). ¹H NMR (400 MHz, DMSO-*d*₆, 300K) δ_{H} 7.40 ― 7.36 (m, 2H), 7.29 (t, *J* = 7.4 Hz, 2H), 7.23 - 7.17 (m, 1H), 6.96 (br s, 1H, NH, D₂O exchanged), 4.60 (br s, 1H), 3.88 - 3.70 (m, 2H), 3.46 (br s, 2H), 3.24 (s, 3H), 2.92 (br s, 3H). MS (ESI⁺) : [M+H]⁺ 248.2.

### Example 2.35: Synthesis of 1-methyl-2-[(4-methylthiazol-2-yl)methylamino]-4H-imidazol-5-one (2.35)

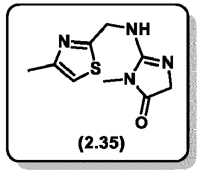

Compound (2.35) was synthesized according to **GP1-C** : reaction carried out in dioxane, with 1-methyl-2-methylsulfanyl-4*H*-imidazol-5-one (1.04 mmol), 3 eq of (4-methylthiazol-2-yl)methanamine and 5 eq of AcOH, at 150 °C (heating block), for 1.5h. Purification by FC (elution: DCM/MeOH: 99/1 to 9/1). Beige solid, 39% (130 mg). ¹H NMR (400 MHz, DMSO-*d*₆, 300K) δ_{H} 7.32 (bs, 1H, NH, D₂O exchanged), 7.06 (s, 1H), 4.46 (s, 2H), 3.89 (s, 2H), 2.92 (s, 3H), 2.32 (s, 3H). MS (ESI⁺) : [M+H]⁺ 225.2.

### Example 3: General Protocol 2: Synthesis of 4- and 5-substituted N-alkyl-2-nitro-aniline

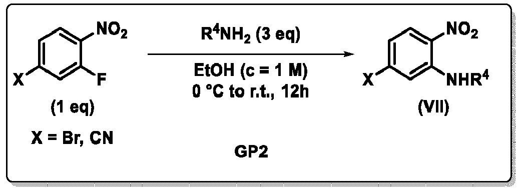

In the above scheme, X is either a cyano group or a halogen atom, in particular a bromine atom and R⁴ is a (C₁-C₃)alkyl.

**GP2**: The appropriate amine (3 eq) was added dropwise to a stirred solution of the suitably substituted o-fluoro-nitro-benzene (1 eq) in EtOH (c = 1M) maintained at 0 °C. The resulting mixture was stirred 1h at 0 °C, brought back to room temperature and stirred another 12h. Upon completion (TLC), the mixture was partially concentrated *in vacuo* and poured onto water. The precipitated solid was filtered off on a Büchner funnel and thoroughly dried *in vacuo.* The resulting bright red/orange solid was reprecipitated from DCM/pentane at 0 °C to yield the desired product in analytically pure form.

### Example 3.1: Synthesis of 5-bromo-N-methyl-2-nitro-aniline (3.1)

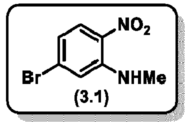

Compound (3.1) was synthesized according to **GP2** : reaction carried out with 4-bromo-2-fluoro-1-nitro-benzene (68.18 mmol) with MeNH₂ (2 M solution in MeOH or 33% w/w in EtOH, 3 eq). Bright orange solid, 87% (13.763 g). ¹H NMR (400 MHz, DMSO-*d*₆, 300K) δ_{H} 8.30 - 8.20 (m, 1H, NH D₂O exchanged), 7.98 (d, *J* = 9.1 Hz, 1H), 7.16 (s, 1H), 6.82 (d, *J* = 9.1 Hz, 1H), 2.94 (d, *J* = 5.0 Hz, 3H). ¹³C NMR (101 MHz, DMSO-*d*₆, 300K) δ_{C} 146.3, 130.9, 130.1, 127.9, 117.7, 116.4, 29.8. MS (ESI⁺): [M+H]⁺ 232.8.

### Example 3.2: Synthesis of 3-(methylamino)-4-nitro-benzonitrile (3.2)

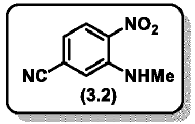

Compound (3.2) was synthesized according to **GP2** : reaction carried out with 3-fluoro-4-nitro-benzonitrile (150.5 mmol) and MeNH₂ (2 M solution in MeOH or 33% w/w in EtOH, 3 eq). Bright orange solid, 97% (25.917 g). ¹H NMR (400 MHz, DMSO-*d*₆, 300K) δ_{H} 8.25 (d, *J* = 5.3 Hz, 1H, NH, D₂O exchanged), 8.18 (d, *J* = 8.7 Hz, 1H), 7.51 (s, 1H), 7.01 (dd, *J* = 8.8, 1.7 Hz, 1H), 2.97 (d, *J* = 5.0 Hz, 3H). ¹³C NMR (101 MHz, DMSO-*d*₆, 300K) δ_{C} 145.2, 132.9, 127.4, 119.4, 118.1, 117.7, 116.4, 29.9. MS (ESI⁺) : [M+H]⁺ 178.0.

### Example 4: General Protocol 3 - Synthesis of substituted N1- and N2-alkylbenzene-1,2-diamine

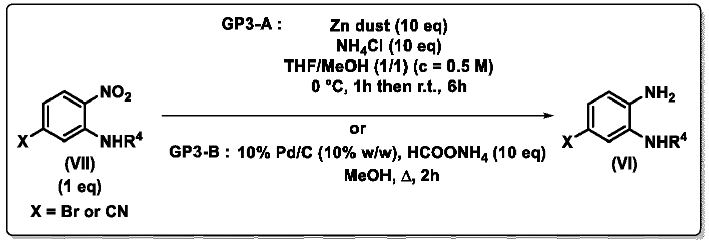

In the above scheme, X is either a cyano group or a halogen atom, in particular a bromine atom and R⁴ is a (C₁-C₃)alkyl.

**GP3-A (X = Br)**: NH₄Cl (10 eq) was carefully added portionwise over 30 min to a stirred solution of the appropriate bromo-*N*-alkyl-2-nitro-aniline (1 eq) and zinc dust (10 eq) in a THF/MeOH mixture (1/1) (c = 0.5 M) maintained at 0 °C. When the addition ended, the mixture was stirred another hour at 0 °C (until the orange color fully disappeared) and gradually brought back to room temperature. The reaction media was stirred another 6h at room temperature. Upon completion (TLC), the mixture was filtered off on a pad of celite. The celite was rinsed with MeOH. The filtrate was concentrated *in vacuo,* adsorbed on silica and purified by FC on silica gel (elution : cyclohexane/AcOEt: 8/2 to 1/1) to yield the desired bromo-*N*-alkyl-benzene-1,2-diamine in analytically pure form.

### Example 4.1: Synthesis of 4-bromo-N2-methyl-benzene-1,2-diamine (4.1)

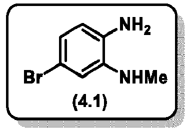

Compound (4.1) was synthesized according to **GP3-A** : reaction carried out with 5-bromo-*N*-methyl-2-nitro-aniline (3.1) (17.31 mmol). Brown oil, 81% (2.83 g). ¹H NMR (400 MHz, DMSO-*d*₆, 300K) δ_{H} 6.52 (dd, *J* = 8.1, 2.2 Hz, 1H), 6.44 (d, *J* = 8.1 Hz, 1H), 6.40 (d, *J* = 2.2 Hz, 1H), 4.88 (bs, 1H), 4.61 (bs, 2H), 2.68 (d, *J* = 3.8 Hz, 3H). ¹³C NMR (101 MHz, DMSO-*d*₆, 300K) δ_{C} 138.8, 134.4, 118.4, 114.6, 110.9, 108.8, 29.9. MS (ESI⁺) : [M+H]⁺ 201.1.

**GP3-B (X** = **CN)**: 10% Pd/C (10% w/w) was carefully added portionwise to a stirred solution of the appropriate cyano-substituted *N*-alkyl-2-nitro-aniline (1 eq) and ammonium formiate (10 eq) in MeOH (c = 0.3 M). The resulting mixture was refluxed 6h. Upon completion (TLC), the mixture was filtered off on pad of celite. The celite was rinsed with MeOH. The filtrate was concentrated *in vacuo,* the resulting crude was partitioned between AcOEt and sat. NaHCO_{3(aq)}. The aqueous layer was extracted three times with AcOEt. The combined organic layers were washed with water and brine, dried over MgSO₄, filtered, and concentrated *in vacuo.* The resulting solid was reprecipitated from DCM/pentane at 0 °C to yield the desired product in analytically pure form.

### Example 4.2: Synthesis of 4-amino-3-(methylamino)benzonitrile (4.2)

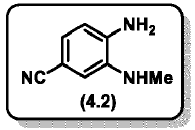

Compound (4.2) was synthesized according to **GP3-B** : reaction carried out with 3-(methylamino)-4-nitro-benzonitrile (3.2) (146.2 mmol). Beige solid, 87% (18.764 g). ¹H NMR (400 MHz, DMSO-*d*₆, 300K) δ_{H} 6.85 (dd, *J* = 8.0, 1.8 Hz, 1H), 6.61 - 6.52 (m, 2H), 5.48 (bs, 2H, NH, D₂O exchanged), 5.01 (q, *J* = 5.0 Hz, 1H, NH, D₂O exchanged), 2.72 (d, *J* = 4.9 Hz, 3H). ¹³C NMR (101 MHz, DMSO-*d*₆, 300K) δ_{C} 140.4, 136.4, 122.2, 121.4, 112.2, 110.4, 97.3, 29.9. MS (ESI⁺) : [M+H]⁺ 148.1.

### Example 5: General protocol 4: cyclization of brominated 2-aminophenols and N2-methylbenzene-1,2-diamines with triethyl orthoformate

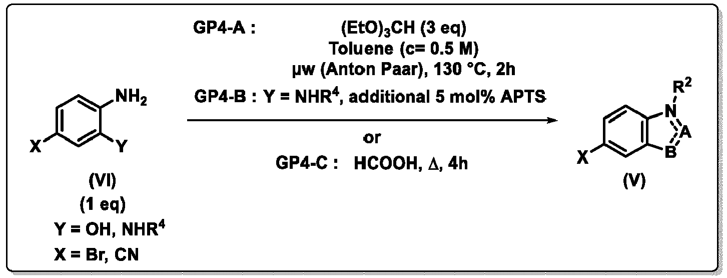

In the above scheme, Y is either a -OH group or a -NHR⁴, X is either a cyano group or a halogen atom, in particular a bromine atom and R⁴ is a (C₁-C₃)alkyl.

**GP4-A (Y = OH and X = Br)**: a solution of the appropriate 2-amino-bromophenol (1 eq) and (EtO)₃CH (2 eq) in toluene (c = 0.5 M) was irradiated at 130 °C for 2h. Upon completion (TLC), the mixture was cooled down room temperature, directly adsorbed on silica, and purified by FC on silica gel using the appropriate gradient of solvents (elution : cyclohexane/AcOEt : 1/0 to 6/4) to yield the desired bromo-1,3-benzoxazole in analytically pure form.

### Example 5.1: Synthesis of 6-bromo-1,3-benzoxazole (5.1)

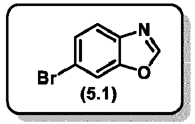

Compound (5.1) was synthesized according to **GP4-A** : reaction carried out with 2-amino-5-bromo-phenol (21.27 mmol). Light beige solid, 89% (3.745 g). ¹H NMR (400 MHz, DMSO-*d*₆, 300K) δ_{H} 8.79 (s, 1H), 8.12 (s, 1H), 7.77 (d, *J* = 8.4 Hz, 1H), 7.58 (d, *J* = 8.5 Hz, 1H). ¹³C NMR (101 MHz, DMSO-*d*₆, 300K) δ_{C} 154.8, 150.0, 139.1, 127.8, 121.5, 117.7, 114.5. MS (ESI⁺) : [M+H]⁺ 199.7.

**GP4-B (Y = NHR⁴ and X = Br):** a solution of the appropriate 4-bromo-*N-*methyl-benzene-1,2-diamine (1 eq), (EtO)₃CH (2 eq), and APTS.H₂O (5 mol%) in toluene (c = 0.5 M) was irradiated at 130 °C for 2h. Upon completion (TLC), the mixture was partitioned between AcOEt and sat. NaHCO_{3(aq)}. The aqueous layer was extracted three times with AcOEt. The combined organic layers were washed with brine and water, dried over MgSO₄, filtered, and concentrated *in vacuo.* The resulting brown solid was dissolved in DCM at 0 °C and precipitated from pentane. The precipitated light beige solid was filtered off on a Büchner funnel, thoroughly rinsed with pentane, and dried *in vacuo* to yield the desired bromo-1-methyl-benzimidazole in analytically pure form.

### Example 5.2.: Synthesis of 6-bromo-1-methyl-benzimidazole (5.2)

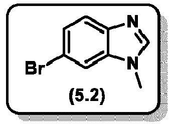

Compound (5.2) was synthesized according to **GP4-B** : reaction carried out with 4-bromo-*N*2-methyl-benzene-1,2-diamine (4.1) (10.94 mmol). Light beige solid, 79% (1.815 g). ¹H NMR (400 MHz, DMSO-*d*₆, 300K) δ_{H} 8.21 (s, 1H), 7.86 (s, 1H), 7.60 (d, *J* = 8.5 Hz, 1H), 7.33 (d, *J* = 8.4 Hz, 1H), 3.83 (s, 3H). ¹³C NMR (101 MHz, DMSO-*d*₆, 300K) δ_{C} 145.6, 142.4, 135.8, 124.3, 121.0, 114.7, 113.3, 30.8. MS (ESI⁺) : [M+H]⁺ 212.8.

**GP4-C (Y = NHR⁴ and** X = **CN)**: a solution of cyano-substituted N2-methylbenzene-1,2-diamine (1 eq) was refluxed in HCOOH (c = 0.4 M) for 4h. Upon completion (TLC), the mixture was brought back to room temperature, and concentrated *in vacuo.* The resulting crude was carefully treated with sat. NaHCO_{3(aq)} to neutral pH. The precipitated solid was filtered on a Büchner funnel. The solid was solubilized in DCM and washed with water, sat. NaHCO_{3(aq)}, dried over MgSO₄, filtered and concentrated *in vacuo* to yield the desired solid in analytically pure form. Higher purity may be achieved by reprecipitation from DCM/pentane at 0 °C.

### Example 5.3: Synthesis of 3-methylbenzimidazole-5-carbonitrile (5.3)

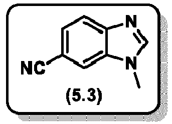

Compound (5.3) was synthesized according to **GP4-C** : reaction carried out with 4-amino-3-(methylamino)benzonitrile (4.2) (127.5 mmol). Beige solid, 94% (18.829 g). ¹H NMR (400 MHz, DMSO-*d*₆, 300K) δ_{H} 8.46 (s, 1H), 8.24 (s, 1H), 7.82 (d, *J* = 8.4 Hz, 1H), 7.59 (dd, *J* = 8.3, 1.6 Hz, 1H), 3.90 (s, 3H). ¹³C NMR (101 MHz, DMSO-*d*₆, 300K) δ_{C} 148.1, 146.2, 134.3, 124.8, 120.4, 119.9, 115.9, 104.0, 31.1. MS (ESI⁺) : [M+H]⁺ 158.1.

### Example 6: General protocol 5 - Palladium-catalyzed vinylation of heteroarylbromides

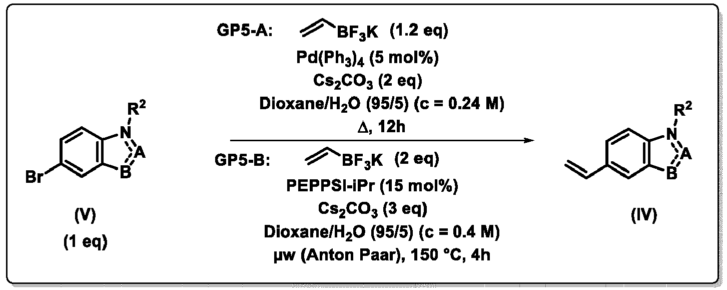

In the above scheme, A, B and R² are as defined above.

**GP5-A**: a mixture of the appropriate heteroarylbromide (1 eq), potassium vinyltrifluoroborate (1.2 eq), Cs₂CO₃ (2 eq), and Pd(PPh3)4 (5 mol%) in dioxane/H₂O (95/5) (C = 0.4 M) was thoroughly purged with vacuum/argon cycles. The mixture was brought to reflux and heated for 12h. Upon completion (¹H NMR), the mixture was partitioned between H₂O and AcOEt. The aqueous layer was extracted three times with AcOEt. The combined organic layers were dried over MgSO₄, filtered, concentrated *in vacuo,* adsorbed on silica, and purified by FC on silica gel using the appropriate gradient of solvents (see details below for each compound) to yield the desired vinylheteroaryl in analytically pure form.

### Example 6.1: Synthesis of 6-vinyl-1,3-benzoxazole (6.1)

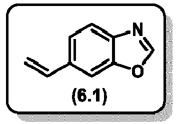

Compound (6.1) was synthesized according to **GP5-A** : reaction carried out with 6-bromo-1,3-benzoxazole (5.1) (40.70 mmol). Purification by FC : elution: cyclohexane/AcOEt: 99/1 to 8/2. Brown oil, 74% (4.345 g). ¹H NMR (400 MHz, DMSO-*d*₆, 300K) δ_{H} 8.73 (s, 1H), 7.90 (s, 1H), 7.75 (d, *J* = 8.3 Hz, 1H), 7.53 (d, *J* = 8.2 Hz, 2H), 6.86 (dd, *J* = 17.7, 11.0 Hz, 1H), 5.94 (d, *J* = 17.6 Hz, 1H), 5.33 (d, *J* = 10.9 Hz, 1H). ¹³C NMR (101 MHz, DMSO-*d*₆, 300K) δ_{C} 154.7, 149.9, 139.5, 136.2, 135.3, 123.2, 119.9, 115.0, 108.4. MS (ESI⁺) : [M+H]⁺ 145.9. (N.B. : (6.1) has nearly identical Rf as starting material (5.1) in cyclohexane/AcOEt : 8/2).

**GP5-B** : In a sealed tube thoroughly purged with vacuum/argon cycles, a mixture of the appropriate heteroarylbromide (1 eq), potassium vinyltrifluoroborate (2 eq), Cs₂CO₃ (3 eq), and PEPPSI-iPr (15 mol%) in dioxane/H₂O (95/5) (C = 0.4 M) was irradiated for 4h at the adequate temperature (see details below for each compound). Upon completion (¹H NMR), the mixture was cooled down room temperature, directly adsorbed on silica, and purified by FC on silica gel using the appropriate gradient of solvents (see details below for each compound) to yield the desired vinylheteroaryl in analytically pure form.

### Example 6.2: Synthesis of 1-methyl-6-vinyl-benzimidazole (6.2)

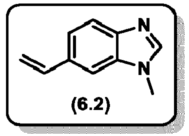

Compound (6.2) was synthesized according to **GP5-B** : reaction carried out with 6-bromo-1-methyl-benzimidazole (5.2) (4.26 mmol). Purification by FC : elution: DCM/MeOH : 99/1 to 94/6. Brown oil, 82% (551 mg). ¹H NMR (400 MHz, CDCl₃, 300K) δ_{H} 7.87 (s, 1H), 7.74 (d, *J* = 8.3 Hz, 1H), 7.49 ― 7.33 (m, 2H), 6.86 (dd, *J* = 17.5, 10.9 Hz, 1H), 5.79 (d, *J* = 17.5 Hz, 1H), 5.26 (d, *J* = 10.9 Hz, 1H), 3.85 (s, 3H). ¹³C NMR (101 MHz, CDCl₃) δ_{C} 144.2, 143.8, 137.3, 135.0, 133.2, 120.8, 120.2, 113.2, 107.3, 31.1. MS (ESI⁺) : [M+H]⁺ 159.1.

### Example 7: General protocol 6 - Synthesis of heteroarylcarbaldehydes from vinylheteroaryls - Osmium-catalyzed Lemieux-Johnson oxidation

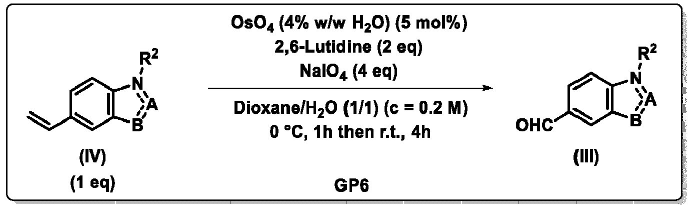

In the above scheme, A, B and R² are as defined above.

**GP6:** NaIO₄ (4 eq) was added portionwise to a stirred solution of the appropriate vinylheteroaryl (1 eq), 2,6-lutidine (2 eq), OsO₄ (4% w/w H₂O sol., 5 mol%), in dioxane/H₂O (1/1) (C = 0.2 M) maintained at 0 °C. The reaction mixture was vigorously stirred at 0 °C for 1h, brought back to room temperature and stirred another 4h. Upon completion (TLC), the mixture was partitioned between AcOEt and H₂O. The aqueous layer was extracted with AcOEt (x3). The combined organic layers were washed with sat. NH₄Cl_{(aq)}, sat. NaHCO_{3(aq)}, dried over MgSO₄, filtered, and concentrated *in vacuo* (trace amounts of 2,6-lutidine were removed by co-evaporation with toluene). The solid residue was adsorbed on silica and purified by FC on silica gel using the appropriate gradient of solvents (see details below for each compound).

### Example 7.1: Synthesis of 1,3-benzoxazole-6-carbaldehyde (7.1)

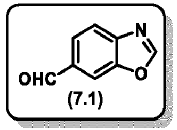

Compound (7.1) was synthesized according to **GP6** : reaction carried out with 6-vinyl-1,3-benzoxazole (6.1) (20.66 mmol). Purification by FC : elution: cyclohexane/AcOEt: 99/1 to 7/3. Light beige solid, 87% (2.654 g). ¹H NMR (400 MHz, DMSO-*d*₆, 300K) δ_{H} 10.12 (s, 1H), 9.01 (s, 1H), 8.34 (s, 1H), 8.16 ― 7.91 (m, 2H). ¹³C NMR (101 MHz, DMSO-*d*₆, 300K) δ_{C} 192.1, 157.5, 149.5, 144.6, 134.0, 126.0, 120.7, 112.6. MS (ESI⁺) : [M+H]⁺ 147.9.

### Example 7.2: Synthesis of 3-methylbenzimidazole-5-carbaldehyde (7.2)

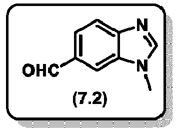

Compound (7.2) was synthesized according to **GP6** : reaction carried out with 1-methyl-6-vinyl-benzimidazole (6.2) (3.48 mmol). Purification by FC : elution: DCM/MeOH : 99/1 to 94/6. Colorless solid, 54% (300 mg). ¹H NMR (400 MHz, CDCl₃, 300K) δ_{H} 10.12 (s, 1H), 8.39 ― 8.22 (m, 1H), 8.02 (s, 1H), 7.95 (d, *J* = 8.4 Hz, 1H), 7.87 (d, *J* = 8.3 Hz, 1H), 3.98 (s, 3H). ¹³C NMR (101 MHz, CDCl_{3,} 300K) δ 192.1, 148.5, 147.0, 134.9, 132.1, 124.7, 120.9, 111.4, 31.5. MS (ESI⁺) : [M+H]⁺ 161.1.

### Example 8: General protocol 7 - Synthesis of heteroarylcarbaldehydes from heteroarylcarbonitriles ― Adam's catalyst-mediated oxidation

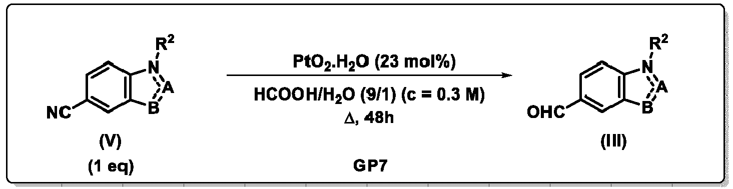

In the above scheme, A, B and R² are as defined above.

**GP7:** Adam's catalyst (23 mol%) was carefully added portionwise to a stirred solution of the appropriate carbonitrile (1 eq) in a HCOOH/H₂O solution (9/1) (C = 0.3 M). The resulting mixture was refluxed for 48h. Upon completion (TLC), the mixture was brought back to room temperature and filtered off on pad of celite. The celite was rinsed with HCOOH. The filtrate was concentrated *in vacuo* and partitioned between DCM and sat. NaHCO_{3(aq)}. The aqueous layer was extracted three times with DCM. The combined organic layers were washed with sat. NaHCO_{3(aq)}, water, dried over MgSO₄, filtered, concentrated *in vacuo,* adsorbed on silica, and purified by FC on silica gel (see details below).

### Example 8.1: Synthesis of 3-methylbenzimidazole-5-carbaldehyde (7.2)

Compound (7.2) was synthesized according to **GP7** from 3-methylbenzimidazole-5-carbonitrile (5.3) (30.32 mmol). Purification by FC : elution : DCM/MeOH : 99/1 to 94/6. Colorless solid, 61% (2.986 g). The final product required a reprecipitation from DCM/pentane at 0 °C. Spectroscopic and analytical data were identical to those described above.

### Example 9: General protocol 8 - Knoevenagel condensation between thiohydantoin and heteroarylcarbalhydes (ROUTE 2)

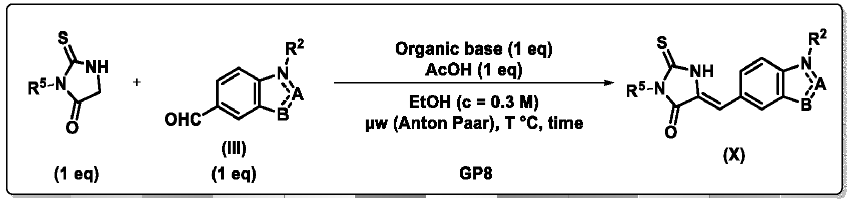

In the above scheme, A, B, R² and R⁵ are as defined above.

**GP8:** a stirred solution of thiohydantoin (1 eq), the appropriate heteroarylcarbaldehyde (1 eq), organic base (1 eq) and AcOH (1 eq) in EtOH (C = 0.3 M) was heated in a sealed tube in a microwave oven (Anton Paar) for the indicated time at the adequate temperature (see details below for each compound). Upon completion (followed by consumption of the heteroarylcarbaldehyde on TLC), the reaction media was cooled down and added onto stirred water. The precipitated solid was stirred for 30 min and filtered off on a fritted glass funnel, thoroughly dried, and could be used in the next step without further purification. Higher purity may be achieved by trituration in EtOH.

### Example 9.1: Synthesis of (5Z)-5-(1,3-benzoxazol-6-ylmethylene)-2-thioxo-imidazolidin-4-one (9.1)

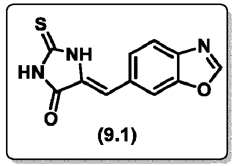

Compound 9.1 was synthesized according to **GP8** : reaction carried out with 2-thiohydantoin (2.72 mmol), 1,3-benzoxazole-6-carbaldehyde (7.1), AcOH, and ethanolamine as the organic base. Reaction temperature: 80 °C, time: 15 min. The final product required a trituration in EtOH. Yellow solid, 72% (483 mg). ¹H NMR (400 MHz, DMSO-*d*₆, 300K) δ_{H} 12.33 (bs, 2H, NH, D₂O exchanged), 8.84 (s, 1H), 8.25 (s, 1H), 7.82 (d, *J* = 8.3 Hz, 1H), 7.75 (d, *J* = 8.4 Hz, 1H), 6.64 (s, 1H). ¹³C NMR (101 MHz, DMSO-*d*₆, 300K) δ_{C} 179.4, 165.8, 155.7, 149.8, 140.3, 130.2, 128.0, 127.7, 120.2, 112.2, 111.1. MS (ESI⁺) : [M+H]⁺ 245.8.

### Example 9.2: Synthesis of (5Z)-5-(1H-indazol-5-ylmethylene)-2-thioxo-imidazolidin-4-one (9.2)

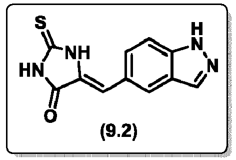

Compound (9.2) was synthesized according to **GP8** : reaction carried out with 2-thiohydantoin (4.42 mmol), indazole-5-carbaldehyde, AcOH, and piperidine as the organic base. Reaction temperature : 110 °C, time : 60 min. The final product required a trituration in EtOH. Yellow solid, 89% (742 mg). ¹H NMR (400 MHz, DMSO-*d*₆, 300K) δ_{H} 13.24 (bs, 1H, NH, D₂O exchanged), 12.33 (bs, 1H, NH, D₂O exchanged), 12.19 (bs, 1H, NH, D₂O exchanged), 8.27 (s, 1H), 8.14 (s, 1H), 7.70 (d, *J* = 8.7 Hz, 1H), 7.55 (d, *J* = 8.7 Hz, 1H), 6.64 (s, 1H). ¹³C NMR (101 MHz, DMSO-*d*₆, 300K) δ_{C} 179.2, 166.3, 140.1, 135.0, 129.0, 126.8, 125.2, 123.8, 113.6, 111.0. MS (ESI⁺) : [M+H]⁺ 244.9.

### Example 9.3: Synthesis of (5Z)-5-[(2-methylindazol-5-yl)methylene]-2-thioxo-imidazolidin-4-one (9.3)

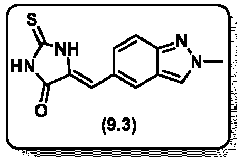

Compound (9.3) was synthesized according to **GP8** : reaction carried out with 2-thiohydantoin (2.12 mmol), 2-methylindazole-5-carbaldehyde, AcOH, with piperidine as the organic base, Reaction temperature : 110 °C, time : 60 min. The final product required a trituration in EtOH. Yellow solid, 89% (485 mg). ¹H NMR (400 MHz, DMSO-*d*₆, 300K) δ_{H} 12.31 (bs, 1H, NH, D₂O exchanged), 12.16 (bs, 1H, NH, D₂O exchanged), 8.44 (s, 1H), 8.22 (s, 1H), 7.62 ― 7.54 (m, 2H), 6.58 (s, 1H), 4.19 (s, 3H). ¹³C NMR (101 MHz, DMSO-*d*₆, 300K) δ_{C} 178.6, 165.8, 147.7, 127.5, 126.3, 126.1, 125.2, 123.9, 121.9, 117.0, 113.3, 39.9. MS (ESI⁺) : [M+H]⁺ 259.1.

### Example 9.4: Synthesis of (5Z)-5-[(1-methylindazol-5-yl)methylene]-2-thioxo-imidazolidin-4-one (9.4)

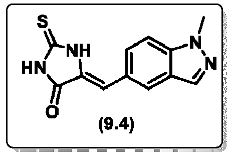

Compound (9.4) was synthesized according to **GP8** : reaction carried out with 2-thiohydantoin (1.72 mmol), 1-methylindazole-5-carbaldehyde, AcOH, and piperidine as the organic base. Reaction temperature: 110 °C, time: 60 min. The final product required a trituration in EtOH. Yellow solid, 92% (408 mg). ¹H NMR (400 MHz, DMSO-*d*₆, 300K) δ_{H} 12.33 (bs, 1H, NH, D₂O exchanged), 12.20 (bs, 1H, NH, D₂O exchanged), 8.24 (s, 1H), 8.11 (d, *J* = 0.9 Hz, 1H), 7.75 (dd, *J* = 8.9, 1.4 Hz, 1H), 7.67 (d, *J* = 8.8 Hz, 1H), 6.64 (s, 1H), 4.06 (s, 3H). ¹³C NMR (101 MHz, DMSO-*d*₆, 300K) δ_{C} 178.8, 165.8, 139.4, 133.5, 128.4, 126.4, 124.8, 123.9, 123.6, 112.9, 110.1, 35.5. MS (ESI⁺) : [M+H]+ 244.9.

### Example 9.5: Synthesis of (5Z)-5-(1H-benzimidazol-5-ylmethylene)-2-thioxo-imidazolidin-4-one (9.5)

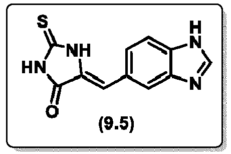

Compound (9.5) was synthesized according to **GP8** : reaction carried out with 2-thiohydantoin (4.2 mmol), 1*H*-benzimidazole-5-carbaldehyde, AcOH, and piperidine as the organic base. Reaction temperature : 110 °C, time : 60 min. The final product required a trituration in EtOH. Yellow solid, 95% (975 mg). ¹H NMR (400 MHz, DMSO-*d*₆, 343K) of major tautomer δ_{H} 12.42 (bs, 1H, NH, D₂O exchanged), 12.03 (bs, 2H, NH, D₂O exchanged), 8.25 (s, 1H), 8.03 (br s, 1H), 7.69 ― 7.51 (m, 2H), 6.64 (s, 1H). MS (ESI⁺) : [M+H]⁺ 245.8.

### Example 9.6: Synthesis of (5Z)-5-[(3-methylbenzimidazol-5-yl)methylene]-2-thioxo-imidazolidin-4-one (9.6)

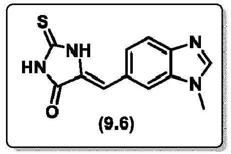

Compound (9.6) was synthesized according to **GP8** : reaction carried out with 2-thiohydantoin (4.37 mmol), 3-methylbenzimidazole-5-carbaldehyde (7.2), AcOH, and piperidine as the organic base. Reaction temperature : 110 °C, time : 60 min. The final product required a trituration in EtOH. Yellow solid, 76% (858 mg). ¹H NMR (400 MHz, DMSO-*d*₆, 300K) δ_{H} 12.38 (bs, 1H, NH, D₂O exchanged), 12.24 (bs, 1H, NH, D₂O exchanged), 8.29 (s, 1H), 8.02 (s, 1H), 7.66 (d, *J* = 8.4 Hz, 1H), 7.52 (d, *J* = 8.2 Hz, 1H), 6.65 (s, 1H), 3.92 (s, 3H). ¹³C NMR (101 MHz, DMSO-*d*₆, 300K) δ_{C} 178.8, 165.8, 146.6, 144.3, 135.1, 126.5, 126.4, 125.4, 119.6, 113.2, 111.6, 31.2. MS (ESI⁺) : [M+H]+ 259.1.

### Example 9.7: Synthesis of (5Z)-5-(1,3-benzoxazol-6-ylmethylene)-3-methyl-2-thioxo-imidazolidin-4-one (9.7)

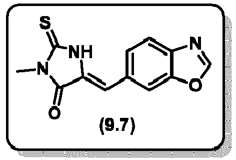

Compound (9.7) was synthesized according to **GP8** : reaction carried out with 3-methyl-2-thiohydantoin (13.83 mmol), 1,3-benzoxazole-6-carbaldehyde (7.1), AcOH, and ethanolamine as the organic base. Reaction temperature: 80 °C, time: 20 min. The final product required a trituration in EtOH. Yellow solid, 80% (2.90 g). ¹H NMR (400 MHz, DMSO-*d*₆, 300K) δ_{H} 12.46 (bs, 1H, NH, D₂O exchanged), 8.83 (s, 1H), 8.25 (s, 1H), 7.82 (d, *J* = 8.3 Hz, 1H), 7.78 ― 7.73 (m, 1H), 6.76 (s, 1H), 3.21 (s, 3H). ¹³C NMR (101 MHz, DMSO-*d*₆, 300K) δ_{C} 179.2, 164.1, 155.7, 149.8, 140.5, 130.0, 127.7, 126.5, 120.2, 112.3, 112.2, 27.3. MS (ESI⁺) : [M+H]⁺ 260.1.

### Example 9.8: Synthesis of (5Z)-5-(1H-indazol-5-ylmethylene)-3-methyl-2-thioxo-imidazolidin-4-one (9.8)

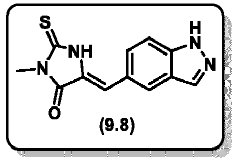

Compound (9.8) was synthesized according to **GP8** : reaction carried out with 3-methyl-2-thiohydantoin (13.67 mmol), indazole-5-carbaldehyde, AcOH, and piperidine as the organic base. Reaction temperature: 110 °C, time: 60 min. The final product required a trituration in EtOH. Yellow solid, 92% (3.25 g). ¹H NMR (400 MHz, DMSO-*d*₆, 300K) δ_{H} 13.25 (bs, 1H, NH, D₂O exchanged), 12.38 (bs, 1H, NH, D₂O exchanged), 8.30 (s, 1H), 8.15 (s, 1H), 7.72 (dd, *J* = 8.8, 1.7 Hz, 1H), 7.56 (d, *J* = 8.7 Hz, 1H), 6.78 (s, 1H), 3.21 (s, 3H). ¹³C NMR (101 MHz, DMSO-*d*₆, 300K) δ_{C} 178.7, 164.1, 139.7, 134.6, 128.6, 124.9, 124.7, 123.6, 123.4, 114.3, 110.5, 27.2. MS (ESI⁺) : [M+H]⁺ 259.1.

### Example 9.9: Synthesis of (5Z)-3-methyl-5-[(2-methylindazol-5-yl)methylene]-2-thioxo-imidazolidin-4-one (9.9)

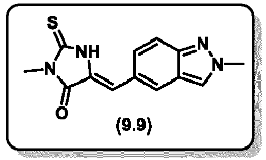

Compound (9.9) was synthesized according to **GP8** : reaction carried out with 3-methyl-2-thiohydantoin (8.07 mmol), 2-methylindazole-5-carbaldehyde, AcOH, and piperidine as the organic base. Reaction temperature: 110 °C, time: 60 min. The final product required a trituration in EtOH. Yellow solid, 92% (2.01 g). ¹H NMR (400 MHz, DMSO-*d*₆) δ_{H} ¹H NMR (400 MHz, DMSO-*d*₆, 300K) δ 12.35 (bs, 1H, NH, D₂O exchanged), 8.45 (s, 1H), 8.25 (s, 1H), 7.63 ― 7.57 (m, 2H), 6.72 (s, 1H), 4.19 (s, 3H), 3.21 (s, 3H). ¹³C NMR (101 MHz, DMSO-*d*₆, 300K) δ_{C} 178.5, 164.2, 147.8, 127.6, 126.2, 125.2, 124.8, 124.2, 121.9, 117.1, 114.5, 40.1, 27.2. MS (ESI⁺) : [M+H]⁺ 273.1.

### Example 9.10: Synthesis of (5Z)-3-methyl-5-[(2-methylindazol-5-yl)methylene]-2-thioxo-imidazolidin-4-one (9.10)

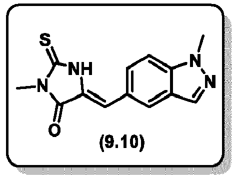

Compound (9.9) was synthesized according to **GP8** : reaction carried out with 3-methyl-2-thiohydantoin (14.90 mmol), 1-methylindazole-5-carbaldehyde, AcOH, and piperidine as the organic base. Reaction temperature: 110 °C, time: 60 min. The final product required a trituration in EtOH. Yellow solid, 96% (3.90 g). ¹H NMR (400 MHz, DMSO-*d*₆, 300K) δ_{H} 12.39 (bs, 1H, NH, D₂O exchanged), 8.27 (s, 1H), 8.12 (d, *J* = 0.9 Hz, 1H), 7.78 (dd, *J* = 8.8, 1.7 Hz, 1H), 7.67 (d, *J* = 8.9 Hz, 1H), 6.78 (s, 1H), 4.07 (s, 3H), 3.21 (s, 3H). ¹³C NMR (101 MHz, DMSO-*d*₆, 300K) δ_{C} 178.7, 164.1, 139.4, 133.5, 128.5, 124.9, 124.8, 123.9, 123.8, 114.1, 110.1, 35.5, 27.2. MS (ESI⁺) : [M+H]⁺ 273.1.

### Example 9.11: Synthesis of (5Z)-5-(1H-benzimidazol-5-ylmethylene)-3-methyl-2-thioxo-imidazolidin-4-one (9.11)

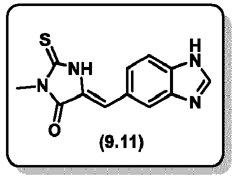

Compound (9.11) was synthesized according to **GP8** : reaction carried out with 3-methyl-2-thiohydantoin (8.07 mmol), 1*H*-benzimidazole-5-carbaldehyde, AcOH, and piperidine as the organic base. Reaction temperature: 110 °C, time: 60 min. The final product required a trituration in EtOH. Yellow solid, 90% (1.88 g). ¹H NMR (400 MHz, DMSO-*d*₆, 300K) of major tautomer δ_{H} 12.63 (bs, 1H, NH, D₂O exchanged), 12.42 (bs, 1H, NH, D₂O exchanged), 8.32 (s, 1H), 8.08 (s, 1H), 7.62 (s, 2H), 6.80 (s, 1H), 3.21 (s, 3H). MS (ESI⁺) : [M+H]⁺ 259.1.

### Example 9.12: Synthesis of (5Z)-5-(1H-benzimidazol-5-ylmethylene)-3-methyl-2-thioxo-imidazolidin-4-one (9.11)

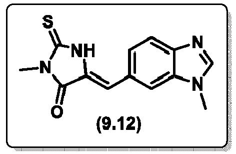

Compound (9.12) was synthesized according to **GP8** : reaction carried out with 3-methyl-2-thiohydantoin (8.53 mmol), 3-methylbenzimidazole-5-carbaldehyde (7.2), AcOH, and piperidine as the organic base. Reaction temperature: 110 °C, time: 60 min. The final product required a trituration in EtOH. Yellow solid, 95% (2.21 g). ¹H NMR (400 MHz, CF₃COOD, 300K) δ_{H} 9.03 (s, 1H), 8.03 (d, *J* = 1.5 Hz, 1H), 7.89 (d, *J* = 8.7 Hz, 1H), 7.82 (dd, *J* = 8.8, 1.5 Hz, 1H), 7.10 (s, 1H), 4.22 (s, 3H), 3.40 (s, 3H). ¹³C NMR (101 MHz, CF₃COOD, 300K) δ_{C} 182.5, 168.1, 143.2, 134.6, 134.1, 133.2, 132.7, 129.4, 117.9, 117.4, 114.8, 35.0, 29.1. MS (ESI⁺) : [M+H]⁺ 273.1.

### Example 10: General protocol 9 - S-Alkylation of (5Z)-5-heteroarylmethylene-2-thioxo-imidazolidin-4-ones (ROUTE 2)

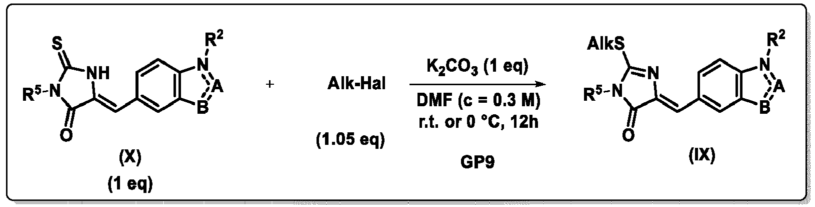

In the above scheme, A, B, R² and R⁵ are as defined above and Alk is a (C₁-C₅)alkyl.

**GP9** : The appropriate alkyliodide (1.05 eq) was added dropwise to a stirred solution of the adequate 5-heteroaryl-2-thioxo-imidazolidin-4-one (1 eq) and K₂CO₃ (1 eq) in DMF (C = 0.3 M) at the appropriate temperature (see details below). The resulting mixture was stirred at the appropriate temperature, for the indicated time. Upon completion (TLC), the mixture was poured into water. The precipitated solid was stirred for 30 min and filtered off on a fritted glass funnel, thoroughly dried, and could be used in the next step without further purification. Trace impurities resulting from *N*3-alkylation may be removed by trituration.

### Example 10.1: Synthesis of (4Z)-4-(1,3-benzoxazol-6-ylmethylene)-2-ethylsulfanyl-1H-imidazol-5-one (10.1)

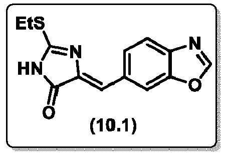

Compound (10.1) was synthesized according to **GP9** : reaction carried out with (5Z)-5-(1,3-benzoxazol-6-ylmethylene)-2-thioxo-imidazolidin-4-one (9.1) (1.97 mmol) and EtI, at room temperature for 12h. Yellow solid, 76% (411 mg). ¹H NMR (400 MHz, DMSO-*d*₆, 300K) δ_{H} 11.85 (bs, 1H, NH, D₂O exchanged), 8.82 (s, 1H), 8.69 (s, 1H), 8.16 (d, *J* = 8.4 Hz, 1H), 7.83 (d, *J* = 8.3 Hz, 1H), 6.90 (s, 1H), 3.33 - 3.27 (m, 2H), 1.45 (t, *J* = 7.3 Hz, 3H). ¹³C NMR (101 MHz, DMSO-*d*₆, 300K) δ_{C} 170.4, 165.0, 155.7, 149.6, 140.6, 139.4, 132.3, 128.6, 120.1, 120.0, 113.1, 24.4, 14.5. MS (ESI⁺) : [M+H]⁺ 273.9.

### Example 10.2: Synthesis of (4Z)-4-(1,3-benzoxazol-6-ylmethylene)-2-methylsulfanyl-1H-imidazol-5-one (10.2)

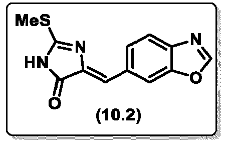

Compound (10.2) was synthesized according to **GP9** : reaction carried out with (5Z)-5-(1,3-benzoxazol-6-ylmethylene)-2-thioxo-imidazolidin-4-one (9.1) (11.89 mmol) and MeI, from 0 °C to room temperature, for 12h. The final product required a trituration in DCM. Yellow solid, 91% (2.805 g). ¹H NMR (400 MHz, DMSO-*d*₆, 300K) δ_{H} 11.88 (bs, 1H, NH, D₂O exchanged), 8.82 (s, 1H), 8.69 (s, 1H), 8.19 (d, *J* = 8.4 Hz, 1H), 7.83 (d, *J* = 8.3 Hz, 1H), 6.91 (s, 1H), 2.71 (s, 3H). ¹³C NMR (101 MHz, DMSO-*d*₆, 300K) δ_{C} 170.5, 165.7, 155.7, 149.6, 140.6, 139.4, 132.3, 128.6, 120.2, 120.0, 113.3, 12.3. MS (ESI⁺): [M+H]⁺ 260.2.

### Example 10.3: Synthesis of (4Z)-2-ethylsulfanyl-4-(1H-indazol-5-ylmethylene)-1H-imidazol-5-one (10.3)

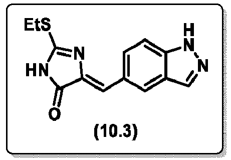

Compound (10.3) was synthesized according to **GP9** : reaction carried out with (5*Z*)-5-(1*H*-indazol-5-ylmethylene)-2-thioxo-imidazolidin-4-one (9.2) (2.05 mmol) and EtI, at room temperature. Yellow solid, 86% (481 mg). ¹H NMR (400 MHz, DMSO-*d*₆, 300K) δ_{H} 13.24 (bs, 1H, NH, D₂O exchanged), 11.71 (bs, 1H, NH, D₂O exchanged), 8.52 (s, 1H), 8.36 (d, *J* = 8.9 Hz, 1H), 8.16 (s, 1H), 7.58 (d, *J* = 8.8 Hz, 1H), 6.88 (s, 1H), 3.58 - 3.03 (m, 2H), 1.44 (t, *J* = 7.3 Hz, 3H). ¹³C NMR (101 MHz, DMSO-*d*₆, 300K) δ_{C} 170.6, 162.8, 139.9, 137.8, 134.8, 129.0, 127.1, 125.2, 123.3, 122.2, 110.4, 24.2, 14.6. MS (ESI⁺) : [M+H]⁺ 272.9.

### Example 10.4: Synthesis of (4Z)-2-ethylsulfanyl-4-[(2-methylindazol-5-yl)methylene]-1H-imidazol-5-one (10.4)

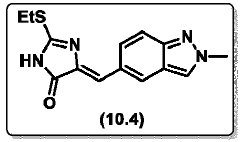

Compound (10.4) was synthesized according to **GP9** : reaction carried out with (5Z)-5-[(2-methylindazol-5-yl)methylene]-2-thioxo-imidazolidin-4-one (9.3) (1.87 mmol) and EtI, at room temperature for 12h. Yellow solid, 88% (471 mg). ¹H NMR (400 MHz, DMSO-*d*₆, 300K) δ_{H} 11.70 (bs, 1H, NH, D₂O exchanged), 8.46 (s, 1H), 8.40 (s, 1H), 8.31 (d, *J* = 9.1 Hz, 1H), 7.60 (d, *J* = 9.1 Hz, 1H), 6.83 (s, 1H), 4.17 (s, 3H), 3.32 ― 3.26 (m, 2H), 1.44 (t, *J* = 7.3 Hz, 3H). ¹³C NMR (101 MHz, DMSO-*d*₆, 300K) δ_{C} 170.6, 162.6, 148.0, 137.7, 127.8, 127.7, 126.4, 126.0, 122.4, 121.9, 116.9, 39.9, 24.2, 14.6. MS (ESI⁺) : [M+H]⁺ 287.9.

### Example 10.5: (4Z)-2-ethylsulfanyl-4-[(1-methylindazol-5-yl)methylene]-1H-imidazol-5-one (10.5)

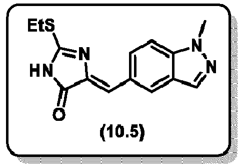

Compound (10.5) was synthesized according to **GP9** : reaction carried out with (5*Z*)-5-[(1-methylindazol-5-yl)methylene]-2-thioxo-imidazolidin-4-one (9.4) (1.58 mmol) and EtI at room temperature for 12h. Yellow solid, 76% (344 mg). ¹H NMR (400 MHz, DMSO-*d*₆, 300K) δ_{H} 11.73 (bs, 1H, NH, D₂O exchanged), 8.50 (s, 1H), 8.41 (d, *J* = 8.9 Hz, 1H), 8.14 (s, 1H), 7.69 (d, *J* = 9.0 Hz, 1H), 6.89 (s, 1H), 4.05 (s, 3H), 3.31 (q, *J* = 7.3 Hz, 2H), 1.45 (t, *J* = 7.3 Hz, 3H). ¹³C NMR (101 MHz, DMSO-*d*₆, 300K) δ_{C} 170.6, 163.0, 139.5, 137.9, 133.7, 128.9, 127.1, 125.3, 123.8, 122.0, 110.0, 35.5, 24.3, 14.6. MS (ESI⁺) : [M+H]⁺ 287.9.

### Example 10.6: (4Z)-4-[(1-methylindazol-5-yl)methylene]-2-methylsulfanyl-1H-imidazol-5-one (10.6)

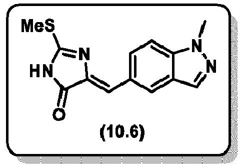

Compound (10.6) was synthesized according to **GP9** : reaction carried out with (5*Z*)-5-[(1-methylindazol-5-yl)methylene]-2-thioxo-imidazolidin-4-one (9.4) (12.00 mmol) and MeI, from 0 °C to room temperature, for 12h. Yellow solid, 94% (3.060 g). ¹H NMR (400 MHz, DMSO-*d*₆, 300K) δ_{H} 11.75 (bs, 1H, NH, D₂O exchanged), 8.53 (s, 1H), 8.39 (d, *J* = 8.9 Hz, 1H), 8.14 (s, 1H), 7.69 (d, *J* = 8.9 Hz, 1H), 6.89 (s, 1H), 4.06 (s, 3H), 2.70 (s, 3H). ¹³C NMR (101 MHz, DMSO-*d*₆, 300K) δ_{C} 170.7, 163.7, 139.5, 137.9, 133.7, 129.1, 127.1, 125.3, 123.8, 122.0, 109.9, 35.4, 12.2. MS (ESI⁺): [M+H]⁺ 273.2.

### Example 10.7: Synthesis of (4Z)-4-(1H-Benzimidazol-5-ylmethylene)-2-ethylsulfanyl-1H-imidazol-5-one (10.7)

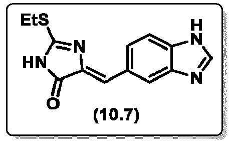

Compound (10.7) was synthesized according to **GP9** : reaction carried out with (5*Z*)-5-(1*H*-benzimidazol-5-ylmethylene)-2-thioxo-imidazolidin-4-one (9.5) (1.64 mmol) and EtI, at room temperature for 12h. Yellow solid, 61% (274 mg). ¹H NMR (400 MHz, DMSO-*d*₆, 300K) δ_{H} 12.85 - 12.51 (br m, 1H, NH, D₂O exchanged), 11.71 (br s, 1H, NH, D₂O exchanged), 8.52 (s, 1H), 8.29 (s, 1H), 8.17 - 7.90 (m, 1H), 7.74 ― 7.48 (m, 1H), 6.89 (s, 1H), 3.38 - 3.29 (m, 2H), 1.45 (t, *J* = 7.3 Hz, 3H). MS (ESI⁺) : [M+H]+ 272.9.

### Example 10.8: Synthesis of (4Z)-4-(1H-Benzimidazol-5-ylmethylene)-2-methylsulfanyl-1H-imidazol-5-one (10.8)

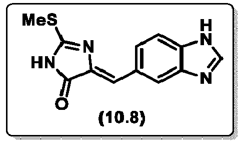

Compound (10.8) was synthesized according to **GP9** : reaction carried out with (5*Z*)-5-(1*H*-benzimidazol-5-ylmethylene)-2-thioxo-imidazolidin-4-one (9.5) (16.04 mmol) and MeI, from 0 °C to room temperature, for 12h. Yellow solid, 92% (3.827 g). ¹H NMR (400 MHz, DMSO-*d*₆, 300K) δ_{H} 12.64 (br s, 1H, NH, D₂O exchanged), 11.75 (br s, 1H, NH, D₂O exchanged), 8.53 (s, 1H), 8.29 (s, 1H), 8.05 (br s, 1H), 7.72 ― 7.53 (m, 1H), 6.89 (s, 1H), 2.71 (s, 3H). MS (ESI⁺) : [M+H]+ 259.1.

### Example 10.9: Synthesis of (4Z)-4-[(3-methylbenzimidazol-5-yl)methylene]-2-methylsulfanyl-1H-imidazol-5-one (10.9)

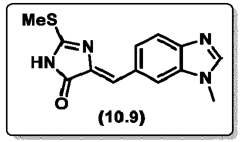

Compound (10.9) was synthesized according to **GP9** : reaction carried out with (5Z)-5-[(3-methylbenzimidazol-5-yl)methylene]-2-thioxo-imidazolidin-4-one (9.6) (12.99 mmol) and MeI, from 0 °C to room temperature, for 12h. Yellow solid, 96% (3.395 g). ¹H NMR (400 MHz, DMSO-*d*₆, 300K) δ_{H} 11.79 (br s, 1H, NH, D₂O exchanged), 8.52 (s, 1H), 8.28 (s, 1H), 8.07 (d, *J* = 8.5 Hz, 1H), 7.67 (d, *J* = 8.5 Hz, 1H), 6.90 (s, 1H), 3.86 (s, 3H), 2.71 (s, 3H). MS (ESI⁺) : [M+H]+ 273.1.

### Example 10.10: Synthesis of (5Z)-5-(1,3-benzoxazol-6-ylmethylene)-3-methyl-2-methylsulfanyl-imidazol-4-one (10.10)

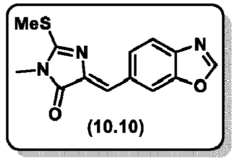

Compound (10.10) was synthesized according to **GP9** : reaction carried out with (5*Z*)-5-(1,3-benzoxazol-6-ylmethylene)-3-methyl-2-thioxo-imidazolidin-4-one (9.7) (11.18 mmol) and MeI, from 0 °C to room temperature, for 12h. Yellow solid, 96% (2.92 g). ¹H NMR (400 MHz, CDCl_{3,} 300K) δ_{H} 8.66 (s, 1H), 8.15 (s, 1H), 7.98 (dd, *J* = 8.4, 1.5 Hz, 1H), 7.78 (d, *J* = 8.3 Hz, 1H), 7.04 (s, 1H), 3.19 (s, 3H), 2.78 (s, 3H). ¹³C NMR (101 MHz, CDCl₃, 300K) δ_{C} 170.0, 166.3, 154.1, 150.5, 141.3, 138.9, 132.7, 129.3, 123.1, 120.5, 114.0, 26.7, 13.2. MS (ESI⁺) : [M+H]⁺ 274.1.

### Example 10.11: Synthesis of (5Z)-5-(1H-indazol-5-ylmethylene)-3-methyl-2-methylsulfanyl-imidazol-4-one (10.11)

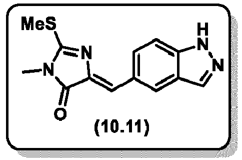

Compound (10.11) was synthesized according to **GP9** : reaction carried out with (5*Z*)-5-(1*H*-indazol-5-ylmethylene)-3-methyl-2-thioxo-imidazolidin-4-one (9.8) (12.27 mmol) and MeI, from 0 °C to room temperature, for 12h. Yellow solid, 99% (3.30 g). ¹H NMR (400 MHz, DMSO-*d*₆, 300K) δ_{H} 13.25 (br s, 1H, NH, D₂O exchanged), 8.57 (s, 1H), 8.40 (dd, *J* = 8.9, 1.5 Hz, 1H), 8.17 (s, 1H), 7.59 (d, *J* = 8.8 Hz, 1H), 7.01 (s, 1H), 3.09 (s, 3H), 2.76 (s, 3H). ¹³C NMR (101 MHz, DMSO-*d*₆, 300K) δ_{C} 169.0, 164.6, 139.9, 136.7, 134.8, 129.3, 127.0, 125.6, 123.5, 123.3, 110.4, 26.3, 12.5. MS (ESI⁺) : [M+H]⁺ 273.1.

### Example 10.12: Synthesis of (5Z)-3-methyl-5-[(2-methylindazol-5-yl)methylene]-2-methylsulfanyl-imidazol-4-one (10.12)

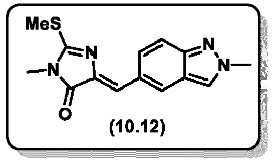

Compound (10.12) was synthesized according to **GP9** : reaction carried out with (5*Z*)-3-methyl-5-[(2-methylindazol-5-yl)methylene]-2-thioxo-imidazolidin-4-one (9.9) (7.12 mmol) and MeI, from 0 °C to room temperature, for 12h. Yellow solid, 95% (1.93 g). ¹H NMR (400 MHz, DMSO-*d*₆, 300K) δ_{H} 8.47 (s, 1H), 8.44 (s, 1H), 8.35 (d, *J* = 1.7 Hz, 1H), 7.61 (d, *J* = 9.1 Hz, 1H), 6.96 (s, 1H), 4.17 (s, 3H), 3.09 (s, 3H), 2.75 (s, 3H). ¹³C NMR (101 MHz, DMSO-*d*₆, 300K) δ_{C} 169.0, 164.3, 148.0, 136.7, 127.9, 127.6, 126.5, 126.4, 123.7, 121.9, 116.9, 40.1, 26.3, 12.5. MS (ESI⁺) : [M+H]⁺ 287.1.

### Example 10.13: Synthesis of (5Z)-3-methyl-5-[(1-methylindazol-5-yl)methylene]-2-methylsulfanyl-imidazol-4-one (10.13)

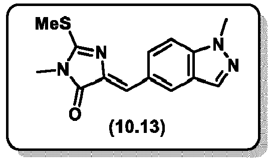

Compound (10.13) was synthesized according to **GP9** : reaction carried out with (5*Z*)-3-methyl-5-[(2-methylindazol-5-yl)methylene]-2-thioxo-imidazolidin-4-one (9.10) (14.14 mmol) and MeI, from 0 °C to room temperature, for 12h. Yellow solid, 100% (4.05 g). ¹H NMR (400 MHz, CF3COOD, 300K) δ_{H} 8.77 (s, 1H), 8.30 (s, 1H), 7.97 (dd, *J* = 9.2, 1.6 Hz, 1H), 7.78 (d, *J* = 9.1 Hz, 1H), 7.67 (s, 1H), 4.31 (s, 3H), 3.40 (s, 3H), 2.97 (s, 3H). ¹³C NMR (101 MHz, CF₃COOD, 300K) δ_{C} 177.0, 164.0, 142.6, 136.8, 133.4, 130.6, 129.7, 129.5, 127.5, 122.9, 114.0, 36.8, 29.4, 15.0. MS (ESI⁺) : [M+H]⁺ 287.1.

### Example 10.14: Synthesis of (5Z)-5-(1H-benzimidazol-5-ylmethylene)-3-methyl-2-methylsulfanyl-imidazol-4-one (10.14)

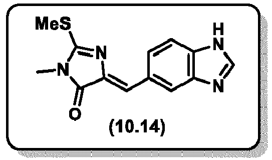

Compound (10.14) was synthesized according to **GP9** : reaction carried out with (5*Z*)-5-(1*H*-benzimidazol-5-ylmethylene)-3-methyl-2-thioxo-imidazolidin-4-one (9.11) (7.24 mmol) and MeI, from 0 °C to room temperature, for 12h. Yellow solid, 81% (1.59 g). ¹H NMR (400 MHz, DMSO-*d*₆, 300K) δ_{H} 12.65 (br s, 1H, NH, D₂O exchanged), 8.56 (s, 1H), 8.30 (s, 1H), 8.09 (d, *J* = 8.4 Hz, 1H), 7.62 (d, *J* = 8.4 Hz, 1H), 7.03 (s, 1H), 3.10 (s, 3H), 2.77 (s, 3H). MS (ESI⁺) : [M+H]+ 273.1.

### Example 10.15: Synthesis of (5Z)-3-methyl-5-[(3-methylbenzimidazol-5-yl)methylene]-2-methylsulfanyl-imidazol-4-one (10.15)

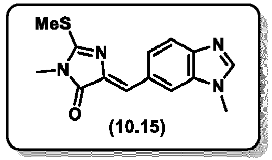

Compound (10.15) was synthesized according to **GP9** : reaction carried out with (5*Z*)-5-(1*H*-benzimidazol-5-ylmethylene)-3-methyl-2-thioxo-imidazolidin-4-one (9.11) (7.93 mmol) and MeI, from 0 °C to room temperature, for 12h. Yellow solid, 95% (2.15 g). ¹H NMR (400 MHz, DMSO-*d*₆, 300K) δ_{H} 8.55 (s, 1H), 8.29 (s, 1H), 8.09 (dd, *J* = 8.5, 1.5 Hz, 1H), 7.67 (d, *J* = 8.5 Hz, 1H), 7.02 (s, 1H), 3.86 (s, 3H), 3.10 (s, 3H), 2.77 (s, 3H). ¹³C NMR (101 MHz, DMSO-*d*₆, 300K) δ_{C} 168.9, 165.0, 146.6, 144.6, 137.1, 134.9, 128.6, 125.8, 123.3, 119.3, 113.9, 30.6, 26.3, 12.5. MS (ESI⁺) : [M+H]⁺ 287.1.

### Example 11: Synthesis of compounds of formula (I) according to general protocols 10 (GP10), 11 (GP11) and 12 (GP12)

### General protocol 10 - Knoevenagel condensation between N2-functionalized 2-amino-1,4-dihydroimidazol-5-ones and heteroarylcarbalhydes (ROUTE 1)

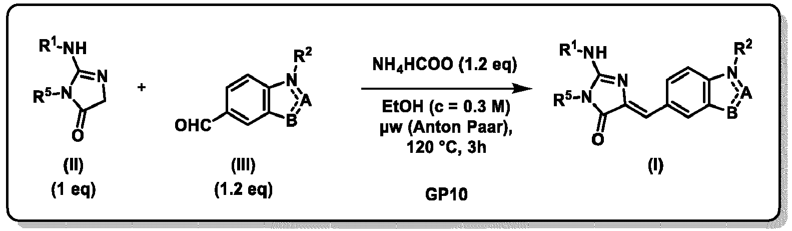

In the above scheme, A, B, R¹, R² and R⁵ are as defined above.

**GP10:** a stirred solution of the appropriate *N*2-functionalized 2-amino-1,4-dihydroimidazol-5-one (1 eq), heteroarylcarboxaldehyde (1.2 eq) and NH₄HCOO (1.2 eq) in EtOH (C = 0.3 M) was heated in a sealed tube in a microwave oven (Anton Paar) at 120 °C for 3h. Upon completion (followed by consumption of the heteroarylcarboxaldehyde on TLC), the mixture was brought back to room temperature, adsorbed on silica and purified by FC (see details below). After FC, higher purity may be achieved by reprecipitation, trituration, or recrystallization (see details below).

### General protocol 11 - Addition of amines on (Z)-heteroarylmethylene-2-alkylsulfanyl-1H-imidazol-5-ones (ROUTE 2)

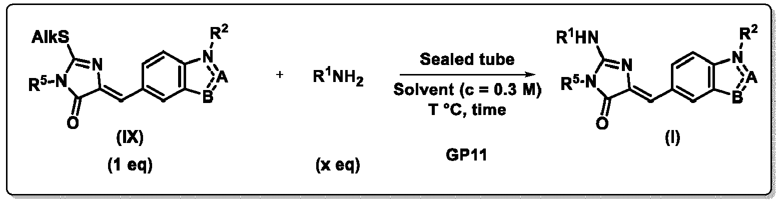

In the above scheme, A, B, R¹, R² and R⁵ are as defined above and Alk is a (C₁-C₅)alkyl.

**GP11:** A stirred solution of the appropriate amine (x eq), (4*Z*)-4-heteroaryl-2-alkylsulfanyl-1*H*-imidazol-5-one^{(a)} (1 eq) in the appropriate solvent (C = 0.3 M) was heated in a sealed tube (heating block). Upon completion (followed by consumption of the isothiourea on TLC), the mixture was brought back to room temperature.
- **GP11-A : direct precipitation of the desired product:** The reaction medium was stirred 1h at 0 °C. The precipitated solid was filtered off on a fritted-glass funnel. High purity may be achieved after filtration by washing, reprecipitation, trituration, or recrystallization (see Table 3 for details).
- **GP11-B : the product failed to precipitate :** the reaction mixture was concentrated *in vacuo,* adsorbed on silica, and purified by FC. High purity may be achieved after filtration by reprecipitation, trituration, or recrystallization.
- **GP11-C: the product failed to precipitate:** the reaction mixture was concentrated *in vacuo.* The resulting crude was triturated in EtOH (at r.t. or reflux), filtered off on a fritted-glass funnel.
- (a) May require activation with AcOH, depending on the amine (see details below).

### General protocol 12 ― Addition of amines on (4Z)-4-(1,3-benzoxazol-6-ylmethylene)-2-alkylsulfanyl-1H-imidazol-5-ones (ROUTE 2')

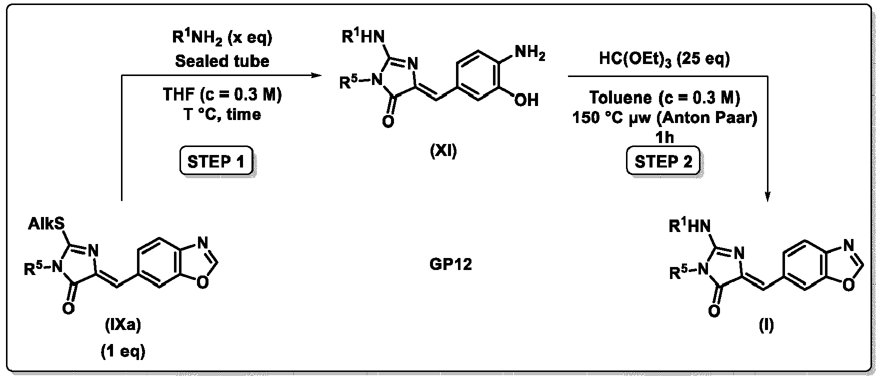

In the above scheme, R¹ and R⁵ are as defined above and Alk is a (C₁-C₅)alkyl.

**GP12** - **Step 1:** a stirred solution of the appropriate amine (x eq), (4*Z*)-4-(1,3-benzoxazol-6-ylmethylene)-2-alkylsulfanyl-1*H*-imidazol-5-one (1 eq) in THF was heated in a sealed tube (heating block). Upon completion (followed by consumption of the isothiourea on TLC), the mixture was brought back to room temperature. The precipitated red/brown solid was isolated by filtration, washed with ice-cold THF or dioxane and dried.

**GP12** - **Step 2:** a stirred solution of the previously isolated solid (1 eq) and HC(OEt)3 (25 eq) in toluene (C = 0.3 M) was heated in a sealed tube in a microwave oven (Anton Paar) at 150 °C for 1h. Upon completion, the mixture was directly adsorbed on silica and purified by FC (see details below). Higher purity may be achieved by reprecipitation, trituration, or recrystallization (see details below).

### Selected examples from benzoxazole sub-series:

### Example 11.1: Synthesis of (4Z)-4-(1,3-benzoxazol-6-ylmethylene)-2-(cyclohexylamino)-1H-imidazol-5-one (1)

Reaction was carried out according to GP12 ― step 1, in THF (0.3M), on a 915 µmol scale of (10.1), with 12 eq of cyclohexylamine, at 110 °C (sealed tube, heating block), for 12h. The isolated aminophenol intermediate was cyclized according to GP12 ― step 2. Purification by FC (elution: DCM/MeOH: 99/1 to 9/1). The final product required a reprecipitation from DCM/pentane at 0 °C. Isolated yield: 34% (2 steps).

### Example 11.2: Synthesis of (4Z)-4-(1,3-benzoxazol-6-ylmethylene)-2-(cycloheptylamino)-1H-imidazol-5-one (2)

Reaction was carried out according to GP12 ― step 1, in THF (0.3M), on a 695 µmol scale of (10.1), with 12 eq of cycloheptylamine, at 110 °C (sealed tube, heating block), for 12h. The isolated aminophenol intermediate was cyclized according to GP12 ― step 2. Purification by FC (elution: DCM/MeOH: 99/1 to 9/1). The final product required a reprecipitation from DCM/pentane at 0 °C. Isolated yield: 38% (2 steps).

### Example 11.3: Synthesis of (4Z)-4-(1,3-benzoxazol-6-ylmethylene)-2-[[(1R)-1-(methoxymethyl)-3-methyl-butyl]amino]-1H-imidazol-5-one (5)

Reaction was carried out according to GP12 ― step 1, in THF (0.3M), on a 352 µmol scale of (10.2), with 5 eq of (2*R*)-1-methoxy-4-methyl-pentan-2-amine, at 120 °C (sealed tube, heating block), for 72h. The isolated aminophenol intermediate was cyclized according to GP12 ― step 2. Purification by FC (elution: DCM/MeOH: 99/1 to 9/1). Isolated yield: 39% (2 steps).

### Example 11.4: Synthesis of (4Z)-4-(1,3-benzoxazol-6-ylmethylene)-2-[[(1R,2R)-2-methoxycyclopentyl]amino]-1H-imidazol-5-one (6)

Reaction was carried out according to GP12 ― step 1, in THF (0.3M), on a 386 µmol scale of (10.2), with 5 eq of (1*R*,2*R*)-2-methoxycyclopentan-1-amine, at 120 °C (sealed tube, heating block), for 28h. The isolated aminophenol intermediate was cyclized according to GP12 ― step 2. Purification by PTLC (elution: DCM/MeOH: 97/3). Isolated yield: 39% (2 steps).

### Example 11.5: Synthesis of (4Z)-2-(1-adamantylamino)-4-(1,3-benzoxazol-6-ylmethylene)-1H-imidazol-5-one (11)

Reaction carried out according to GP10, on a 429 µmol scale of (2.11), with 1,3-benzoxazole-6-carbaldehyde (7.1). Purification by FC (elution: DCM/MeOH: 99/1 to 93/7). Isolated yield: 19%.

### Example 11.6: Synthesis of (4Z)-4-(1,3-benzoxazol-6-ylmethylene)-2-[(3-hydroxy-1-adamantyl)amino]-1H-imidazol-5-one (12)

Reaction carried out according to GP10, on a 401 µmol scale of (2.12), with 1,3-benzoxazole-6-carbaldehyde (7.1). Purification by FC (elution: DCM/MeOH: 99/1 to 93/7). Isolated yield: 5%.

### Example 11.7: Synthesis of (4Z)-4-(1,3-benzoxazol-6-ylmethylene)-2-[(3-methoxy-1-adamantyl)amino]-1H-imidazol-5-one (13)

Reaction carried out according to GP10, on a 379 µmol scale of (2.13), with 1,3-benzoxazole-6-carbaldehyde (7.1). Purification by FC (elution: DCM/MeOH: 99/1 to 93/7). Isolated yield: 20%.

### Example 11.8: Synthesis of (4Z)-4-(1,3-benzoxazol-6-ylmethylene)-2-[[(1R)-2-methoxy-1-phenyl-ethyl]amino]-1H-imidazol-5-one (18)

Reaction was carried out according to GP12 ― step 1, in THF (0.3M), on a 386 µmol scale of (10.2), with 5 eq of (1*R*)-2-methoxy-1-phenyl-ethanamine, at 120 °C (sealed tube, heating block), for 96h. The isolated aminophenol intermediate was cyclized according to GP12 ― step 2. Purification by PTLC (elution: DCM/MeOH: 96/4). Isolated yield: 23% (2 steps).

### Example 11.9: Synthesis of (4Z)-4-(1,3-benzoxazol-6-ylmethylene)-2-[[(3S)-tetrahydropyran-3-yl]amino]-1H-imidazol-5-one (24)

Reaction was carried out according to GP12 ― step 1, in THF (0.3M), on a 386 µmol scale of (10.2), with 5 eq of (3*S*)-tetrahydropyran-3-amine, at 120 °C (sealed tube, heating block), for 24h. The isolated aminophenol intermediate was cyclized according to GP12 ― step 2. Purification by FC (elution: DCM/MeOH: 99/1 to 93/7). Isolated yield: 33% (2 steps).

### Selected examples from indazole sub-series:

### Example 11.10: Synthesis of (4Z)-2-(cyclohexylamino)-4-(1H-indazol-5-ylmethylene)-1H-imidazol-5-one (26)

Reaction was carried out according to GP11-A, in THF (0.3M), on a 734 µmol scale of (10.3), with 6 eq of cyclohexylamine, at 110 °C (sealed tube, heating block), for 24h. The product directly precipitated in the reaction medium: it was isolated after filtration, washing with cold THF, then pentane. Isolated yield: 54%.

### Example 11.11: Synthesis of (4Z)-2-(cycloheptylamino)-4-(1H-indazol-5-ylmethylene)-1H-imidazol-5-one (27)

Reaction was carried out according to GP11-A, in THF (0.3M), on a 734 µmol scale of (10.3), with 6 eq of cycloheptylamine, at 110 °C (sealed tube, heating block), for 24h. The product directly precipitated in the reaction medium: it was isolated after filtration, washing with cold THF, then pentane. Isolated yield: 57%.

### Example 11.12: Synthesis of (4Z)-2-[[(1R)-1-(hydroxymethyl)-3-methyl-butyl]amino]-4-(1H-indazol-5-ylmethylene)-1H-imidazol-5-one (29)

Reaction carried out according to GP10, on a 427 µmol scale of (2.4), with 1H-indazole-5-carbaldehyde. Purification by FC (elution: DCM/MeOH (7N NH₃): 99/1 to 93/7). The final product required a trituration in ACN at 0 °C. Isolated yield: 32%.

### Example 11.13: Synthesis of (4Z)-4-(1H-Indazol-5-ylmethylene)-2-[[(1R)-1-(methoxymethyl)-3-methyl-butyl]amino]-1H-imidazol-5-one (30)

Reaction carried out according to GP10, on a 427 µmol scale of (2.5), with 1*H-*indazole-5-carbaldehyde. Purification by FC (elution: DCM/MeOH (7N NH₃): 99/1 to 93/7). The final product required a reprecipitation from DCM/Et₂O/pentane at 0 °C. Isolated yield: 28%.

### Example 11.14: Synthesis of (4Z)-2-(1-adamantylamino)-4-(1H-indazol-5-ylmethylene)-1H-imidazol-5-one (31)

Reaction carried out according to GP10, on a 427 µmol scale of (2.11), with 1*H-*indazole-5-carbaldehyde. Purification by FC (elution: DCM/MeOH (7N NH₃): 99/1 to 93/7). The final product required a trituration in EtOH at 0 °C. Isolated yield: 36%.

### Example 11.15: Synthesis of (4Z)-2-[(3-hydroxy-1-adamantyl)amino]-4-(1H-indazol-5-ylmethylene)-1H-imidazol-5-one (32)

Reaction carried out according to GP10, on a 427 µmol scale of (2.11), with 1*H-*indazole-5-carbaldehyde. Purification by FC (elution: DCM/MeOH (7N NH₃): 99/1 to 93/7). The final product required a trituration in EtOH at 0 °C. Isolated yield: 50%.

### Example 11.16: Synthesis of (4Z)-4-(1H-indazol-5-ylmethylene)-2-[[(1R)-2-methoxy-1-phenyl-ethyl]amino]-1H-imidazol-5-one (33)

Reaction carried out according to GP10, on a 427 µmol scale of (2.15), with 1*H-*indazole-5-carbaldehyde. Purification by FC (elution: DCM/MeOH (7N NH₃): 99/1 to 94/6). The final product required a reprecipitation from DCM/pentane at 0 °C. Isolated yield: 38%.

### Selected examples from N2-methylindazole sub-series:

### Example 11.17: Synthesis of (4Z)-2-(cyclohexylamino)-4-[(2-methylindazol-5-yl)methylene]-1H-imidazol-5-one (34)

Reaction was carried out according to GP11-A, in THF (0.3M), on a 873 µmol scale of (10.4), with 6 eq of cyclohexylamine, at 110 °C (sealed tube, heating block), for 24h. The product directly precipitated in the reaction medium: it was isolated after filtration, washing with cold THF, then pentane. Isolated yield: 50%.

### Example 11.18: Synthesis of (4Z)-2-(cycloheptylamino)-4-[(2-methylindazol-5-yl)methylene]-1H-imidazol-5-one (35)

Reaction was carried out according to GP11-A, in THF (0.3M), on a 873 µmol scale of (10.4), with 6 eq of cycloheptylamine, at 110 °C (sealed tube, heating block), for 24h. The product directly precipitated in the reaction medium: it was isolated after filtration, washing with cold THF, then pentane. Isolated yield: 49%.

### Example 11.19: Synthesis of (4Z)-2-[[(1R)-1-(hydroxymethyl)-3-methyl-butyl]amino]-4-[(2-methylindazol-5-yl)methylene]-1H-imidazol-5-one (37)

Reaction carried out according to GP10, on a 427 µmol scale of (2.4), with 2-methylindazole-5-carbaldehyde. Purification by FC (elution: DCM/MeOH: 99/1 to 93/7). The final product required a trituration in ACN at 0 °C. Isolated yield: 38%.

### Example 11.20: Synthesis of (4Z)-2-[[(1R)-1-(methoxymethyl)-3-methyl-butyl]amino]-4-[(2-methylindazol-5-yl)methylene]-1H-imidazol-5-one (38)

Reaction carried out according to GP10, on a 427 µmol scale of (2.5), with 2-methylindazole-5-carbaldehyde. Purification by FC (elution: DCM/MeOH: 99/1 to 93/7). The final product required a trituration in ACN at 0 °C. Isolated yield: 28%.

### Example 11.21: Synthesis of (4Z)-2-(1-adamantylamino)-4-[(2-methylindazol-5-yl)methylene]-1H-imidazol-5-one (39)

Reaction carried out according to GP10, on a 321 µmol scale of (2.11), with 2-methylindazole-5-carbaldehyde. Purification by FC (elution: DCM/MeOH: 99/1 to 93/7). The final product required a trituration in EtOH at 0 °C. Isolated yield: 27%.

### Example 11.22: Synthesis of (4Z)-2-[(3-fluoro-1-adamantyl)amino]-4-[(2-methylindazol-5-yl)methylene]-1H-imidazol-5-one (41)

Reaction carried out according to GP10, on a 321 µmol scale of (2.14), with 2-methylindazole-5-carbaldehyde. Purification by FC (elution: DCM/MeOH: 99/1 to 93/7). The final product required a trituration in EtOH at 0 °C. Isolated yield: 61%.

### Example 11.23: Synthesis of (4Z)-2-[[(1R)-2-methoxy-1-phenyl-ethyl]amino]-4-[(2-methylindazol-5-yl)methylene]-1H-imidazol-5-one (42)

Reaction carried out according to GP10, on a 321 µmol scale of (2.15), with 2-methylindazole-5-carbaldehyde. Purification by FC (elution: DCM/MeOH: 99/1 to 94/6). The final product required a reprecipitation from DCM/pentane at 0 °C. Isolated yield: 45%.

### Selected examples from N1-methylindazole sub-series:

### Example 11.24: Synthesis of (4Z)-2-(cyclohexylamino)-4-[(1-methylindazol-5-yl)methylene]-1H-imidazol-5-one (43)

Reaction was carried out according to GP11-A, in THF (0.3M), on a 698 µmol scale of (10.5), with 6 eq of cyclohexylamine, at 110 °C (sealed tube, heating block), for 24h. The product directly precipitated in the reaction medium: it was isolated after filtration, washing with cold THF, then pentane. Isolated yield: 39%.

### Example 11.25: Synthesis of (4Z)-2-(cyclooctylamino)-4-[(1-methylindazol-5-yl)methylene]-1H-imidazol-5-one (45)

Reaction was carried out according to GP11-A, in THF (0.3M), on a 275 µmol scale of (10.6), with 3 eq of cyclooctylamine, at 110 °C (sealed tube, heating block), for 24h. The product directly precipitated in the reaction medium: it was isolated after filtration, washing with cold THF, then pentane. Isolated yield: 46%.

### Example 11.26: Synthesis of (4Z)-2-[[(1R)-1-(hydroxymethyl)-3-methyl-butyl]amino]-4-[(1-methylindazol-5-yl)methylene]-1H-imidazol-5-one (46)

Reaction was carried out according to GP11-B, in THF (0.3M), on a 275 µmol scale of (10.6), with 3 eq of (*R*)-Leucinol, at 120 °C (sealed tube, heating block), for 30h. Purification by FC (elution: DCM/MeOH: 99/1 to 88/12). Isolated yield: 55%.

### Example 11.27: Synthesis of (4Z)-2-[[(1R)-1-(methoxymethyl)-3-methyl-butyl]amino]-4-[(1-methylindazol-5-yl)methylene]-1H-imidazol-5-one (47)

Reaction was carried out according to GP11-B, in THF (0.3M), on a 275 µmol scale of (10.6), with 3 eq of (2*R*)-1-methoxy-4-methyl-pentan-2-amine, at 120 °C (sealed tube, heating block), for 72h. Purification by FC (elution: DCM/MeOH: 99/1 to 88/12). Isolated yield: 52%.

### Example 11.28: Synthesis of (4Z)-2-[[(1R)-1-(fluoromethyl)-3-methyl-butyl]amino]-4-[(1-methylindazol-5-yl)methylene]-1H-imidazol-5-one (48)

Reaction was carried out according to GP11-B, in THF (0.3M), on a 275 µmol scale of (10.6), with 3 eq of (2*R*)-1-fluoro-4-methyl-pentan-2-amine, at 120 °C (sealed tube, heating block), for 120h. Purification by PTLC (elution: DCM/MeOH: 98/2). Isolated yield: 32%.

### Example 11.29: Synthesis of (4Z)-2-[[(1S,2S)-2-methoxycyclopentyl]amino]-4-[(1-methylindazol-5-yl)methylene]-1H-imidazol-5-one (51)

Reaction was carried out according to GP11-A, in THF (0.3M), on a 273 µmol scale of (10.6), with 3 eq of (1*S*,2*S*)-2-methoxycyclopentanamine, at 120 °C (sealed tube, heating block), for 24h. The product directly precipitated in the reaction medium: it was isolated after filtration, washing with cold EtOH, then pentane. Isolated yield: 71%.

### Example 11.30: Synthesis of (±)-(4Z)-2-[[cis-3-methoxycycloheptyl]amino]-4-[(1-methylindazol-5-yl)methylene]-1H-imidazol-5-one (54)

Reaction was carried out according to GP11-B, in a THF/dioxane mixture (2/1) (0.3M), on a 273 µmol scale of (10.6), with 3 eq of (±)-*cis*-3-methoxycycloheptanamine, at 120 °C (sealed tube, heating block), for 44h. Purification by FC (elution: DCM/MeOH: 99/1 to 9/1). Isolated yield: 77%.

### Example 11.31: Synthesis of (4Z)-2-(1-adamantylamino)-4-[(1-methylindazol-5-yl)methylene]-1H-imidazol-5-one (55)

Reaction was carried out according to GP11-B, in dioxane (0.3M), on a 273 µmol scale of (10.6), with 3 eq of adamantan-1-amine and 9 eq of AcOH, at 150 °C (sealed tube, heating block), for 72h. Purification by FC (elution: DCM/MeOH: 99/1 to 9/1). The final product required at trituration in EtOH. Isolated yield: 52%.

### Example 11.32: Synthesis of (4Z)-2-[[(1S,2S)-2-hydroxyindan-1-yl]amino]-4-[(1-methylindazol-5-yl)methylene]-1H-imidazol-5-one (60)

Reaction was carried out according to GP11-A, in THF (0.3M), on a 275 µmol scale of (10.6), with 3 eq of (1*S*,2*S*)-1-aminoindan-2-ol, at 120 °C (sealed tube, heating block), for 52h. The product directly precipitated in the reaction medium: it was isolated after filtration, washing with cold EtOH, then pentane. The final product required at trituration in EtOH. Isolated yield: 62%.

### Example 11.33: Synthesis of (4Z)-2-[[(1R)-2-hydroxy-1-phenyl-ethyl]amino]-4-[(1-methylindazol-5-yl)methylene]-1H-imidazol-5-one (61)

Reaction was carried out according to GP11-A, in THF (0.3M), on a 275 µmol scale of (10.6), with 3 eq of (2*R*)-2-amino-2-phenyl-ethanol, at 120 °C (sealed tube, heating block), for 52h. The product directly precipitated in the reaction medium: it was isolated after filtration, washing with cold EtOH, then pentane. The final product required at trituration in EtOH. Isolated yield: 59%.

### Example 11.34: Synthesis of ((4Z)-2-[[(1R)-2-methoxy-1-phenyl-ethyl]amino]-4-[(1-methylindazol-5-yl)methylene]-1H-imidazol-5-one (63)

Reaction was carried out according to GP11-B, in THF (0.3M), on a 275 µmol scale of (10.6), with 3 eq of (1*R*)-2-methoxy-1-phenyl-ethanamine, at 120 °C (sealed tube, heating block), for 44h. Purification by FC (elution: DCM/MeOH: 99/1 to 9/1). Isolated yield: 47%.

### Example 11.35: Synthesis of (4Z)-2-[[(2R)-2-hydroxy-2-phenyl-ethyl]amino]-4-[(1-methylindazol-5-yl)methylene]-1H-imidazol-5-one (64)

Reaction was carried out according to GP11-A, in THF (0.3M), on a 273 µmol scale of (10.6), with 3 eq of (1*R*)-2-amino-1-phenyl-ethanol, at 120 °C (sealed tube, heating block), for 24h. The product directly precipitated in the reaction medium: it was isolated after filtration, washing with cold EtOH, then pentane. The final product required at trituration in EtOH. Isolated yield: 94%.

### Example 11.36: Synthesis of (4Z)-2-[[(1R)-2-amino-1-phenyl-ethyl]amino]-4-[(1-methylindazol-5-yl)methylene]-1H-imidazol-5-one dihydrochloride (65)

Reaction was carried out according to GP11-B, in THF (0.3M), on a 275 µmol scale of (10.6), with 3 eq of *tert*-butyl *N*-[(1*R*)-2-amino-1-phenyl-ethyl]carbamate, at 120 °C (sealed tube, heating block), for 48h. Purification by FC (elution: DCM/MeOH: 99/1 to 9/1). The isolated carbamate was engaged in a final deprotection step with HCl in dioxane (4M) at 40 °C. Isolated yield: 56% (2 steps).

### Example 11.37: Synthesis of (4Z)-2-[[(4S)-2-amino-1-phenyl-ethyl]amino]-4-[(1-methylindazol-5-yl)methylene]-1H-imidazol-5-one dihydrochloride (65)

Reaction was carried out according to GP11-B, in THF (0.3M), on a 275 µmol scale of (10.6), with 3 eq of *tert*-butyl *N*-[(1*S*)-2-amino-1-phenyl-ethyl]carbamate, at 120 °C (sealed tube, heating block), for 48h. Purification by FC (elution: DCM/MeOH: 99/1 to 9/1). The isolated carbamate was engaged in a final deprotection step with HCl in dioxane (4M) at 40 °C. Isolated yield: 46% (2 steps).

### Example 11.38: Synthesis of (4Z)-4-[(1-methylindazol-5-yl)methylene]-2-(2-pyridylamino)-1H-imidazol-5-one (70)

Reaction was carried out according to GP11-A, in dioxane (0.3M), on a 275 µmol scale of (10.6), with 5 eq of 2-aminopyridine and 15 eq of AcOH, at 150 °C (sealed tube, heating block), for 50h. The product directly precipitated in the reaction medium: it was isolated after filtration, washing with cold EtOH, then pentane. The final product required at trituration in EtOH. Isolated yield: 88%.

### Example 11.39: Synthesis of (4Z)-2-[[(3R,4R)-4-hydroxytetrahydropyran-3-yl]amino]-4-[(1-methylindazol-5-yl)methylene]-1H-imidazol-5-one (73)

Reaction was carried out according to GP11-B, in THF (0.3M), on a 275 µmol scale of (10.6), with 3 eq of (3*R*,4*R*)-3-aminotetrahydropyran-4-ol and 9 eq of AcOH, at 120 °C (sealed tube, heating block), for 24h. Purification by FC (elution: DCM/MeOH: 99/1 to 9/1). The final product required a trituration in EtOH. Isolated yield: 53%.

### Selected examples from benzimidazole sub-series:

### Example 11.40: Synthesis of (4Z)-4-(1H-benzimidazol-5-ylmethylene)-2-(cyclooctylamino)-1H-imidazol-5-one (77)

Reaction was carried out according to GP11-B, in THF (0.3M), on a 275 µmol scale of (10.8), with 3 eq of cyclooctylamine and 15 eq of AcOH, at 130 °C (sealed tube, heating block), for 12h. Purification by FC (elution: DCM/MeOH (7N NH3): 99/1 to 88/12). The final product required a trituration in EtOH at 0 °C. Isolated yield: 21%.

### Example 11.41: Synthesis of (4Z)-4-(1H-benzimidazol-5-ylmethylene)-2-[[(1R)-1-(hydroxymethyl)-3-methyl-butyl]amino]-1H-imidazol-5-one (78)

Reaction was carried out according to GP11-B, in THF (0.3M), on a 523 µmol scale of (10.8), with 3 eq of (*R*)-leucinol and 6 eq of AcOH, at 130 °C (sealed tube, heating block), for 12h. Purification by FC (elution: DCM/MeOH (7N NH₃): 99/1 to 80/20). The final product required a reprecipitation from DCM/pentane at 0 °C. Isolated yield: 18%.

### Example 11.42: Synthesis of (4Z)-4-(1H-benzimidazol-5-ylmethylene)-2-[[(1R)-1-(methoxymethyl)-3-methyl-butyl]amino]-1H-imidazol-5-one (79)

Reaction was carried out according to GP11-B, in THF (0.3M), on a 523 µmol scale of (10.8), with 3 eq of (2*R*)-1-methoxy-4-methyl-pentan-2-amine, at 130 °C (sealed tube, heating block), for 12h. Purification by FC (elution: DCM/MeOH (7N NH3): 99/1 to 90/10). The final product required a reprecipitation from DCM/pentane at 0 °C. Isolated yield: 30%.

### Example 11.43: Synthesis of (4Z)-4-(1H-benzimidazol-5-ylmethylene)-2-[[(1R)-1-(fluoromethyl)-3-methyl-butyl]amino]-1H-imidazol-5-one (80)

Reaction carried out according to GP10, on a 398 µmol scale of (2.6), with 1.2 eq of 1*H*-benzimidazole-5-carbaldehyde. Purification by FC (elution: DCM/MeOH (7N NH3): 99/1 to 85/15). The final product required a reprecipitation from DCM/pentane at 0 °C. Isolated yield: 47%.

### Example 11.44: Synthesis of (4Z)-4-(1H-benzimidazol-5-ylmethylene)-2-[[(1S)-1-(fluoromethyl)-3-methyl-butyl]amino]-1H-imidazol-5-one (81)

Reaction carried out according to GP10, on a 398 µmol scale of (2.7), with 1.2 eq of 1*H*-benzimidazole-5-carbaldehyde. Purification by FC (elution: DCM/MeOH (7N NH3): 99/1 to 85/15). The final product required a reprecipitation from DCM/pentane at 0 °C. Isolated yield: 40%.

### Example 11.45: Synthesis of (4Z)-4-(1H-benzimidazol-5-ylmethylene)-2-[[(1R,2R)-2-methoxycyclopentyl]amino]-1H-imidazol-5-one (82)

Reaction was carried out according to GP11-B, in THF (0.3M), on a 430 µmol scale of (10.8), with 3 eq of (1*R*,2*R*)-2-methoxycyclopentan-1-amine and 6 eq of AcOH, at 120 °C (sealed tube, heating block), for 12h. Purification by FC (elution: DCM/MeOH (7N NH3): 99/1 to 90/10). The final product required a reprecipitation from DCM/pentane at 0 °C. Isolated yield: 36%.

### Example 11.46: Synthesis of (4Z)-2-(3-noradamantylamino)-4-(1H-benzimidazol-5-ylmethylene)-1H-imidazol-5-one (83)

Reaction carried out according to GP10, on a 321 µmol scale of (2.10), with 1.2 eq of 1*H*-benzimidazole-5-carbaldehyde. Purification by FC (elution: DCM/MeOH (7N NH₃): 99/1 to 90/10). The final product required a trituration in EtOH at 0 °C. Isolated yield: 37%.

### Example 11.47: Synthesis of (4Z)-2-(1-Adamantylamino)-4-(1H-benzimidazol-5-ylmethylene)-1H-imidazol-5-one (84)

Reaction carried out according to GP10, on a 343 µmol scale of (2.11), with 1.2 eq of 1*H*-benzimidazole-5-carbaldehyde. Purification by FC (elution: DCM/MeOH (7N NH₃): 99/1 to 90/10). The final product required a trituration in EtOH at 0 °C. Isolated yield: 19%.

### Example 11.48: Synthesis of (4Z)-4-(1H-benzimidazol-5-ylmethylene)-2-[(3-hydroxy-1-adamantyl)amino]-1H-imidazol-5-one (85)

Reaction was carried out according to GP11-A, in dioxane (0.3M), on a 542 µmol scale of (10.8), with 3 eq of 3-aminoadamantan-1-ol and 6 eq of AcOH, at 150 °C (sealed tube, heating block), for 24h. The product directly precipitated in the reaction medium: it was isolated after filtration, washing with EtOH, then pentane. The final product required a trituration in refluxing EtOH. Isolated yield: 46%.

### Example 11.49: Synthesis of (4Z)-4-(1H-benzimidazol-5-ylmethylene)-2-[(3-fluoro-1-adamantyl)amino]-1H-imidazol-5-one (87)

Reaction carried out according to GP10, on a 321 µmol scale of (2.14), with 1.2 eq of 1*H*-benzimidazole-5-carbaldehyde. Purification by FC (elution: DCM/MeOH (7N NH₃): 99/1 to 90/10). The final product required a trituration in EtOH at 0 °C. Isolated yield: 48%.

### Example 11.50: Synthesis of (4Z)-4-(1H-benzimidazol-5-ylmethylene)-2-[[2-(trifluoromethyl)phenyl]methylamino]-1H-imidazol-5-one (88)

Reaction was carried out according to GP11-B, in THF (0.3M), on a 523 µmol scale of (10.8), with 3 eq of [2-(trifluoromethyl)phenyl]methanamine and 6 eq of AcOH, at 160 °C (sealed tube, heating block), for 12h. Purification by FC (elution: DCM/MeOH (7N NH3): 99/1 to 85/15). The final product required a reprecipitation from EtOH/Et₂O/pentane at 0 °C. Isolated yield: 40%.

### Example 11.51: Synthesis of (4Z)-4-(1H-benzimidazol-5-ylmethylene)-2-[[(1S,2S)-2-hydroxyindan-1-yl]amino]-1H-imidazol-5-one (89)

Reaction was carried out according to GP11-A, in THF (0.3M), on a 523 µmol scale of (10.8), with 3 eq of (1*S*,2*S*)-1-aminoindan-2-ol, at 130 °C (sealed tube, heating block), for 40h. The product directly precipitated in the reaction medium: it was isolated after filtration, washing with EtOH, then pentane. The final product required a trituration in ACN. Isolated yield: 16%.

### Example 11.51: Synthesis of (4Z)-4-(1H-benzimidazol-5-ylmethylene)-2-[[(1R)-2-hydroxy-1-phenyl-ethyl]amino]-1H-imidazol-5-one (90)

Reaction was carried out according to GP11-A, in THF (0.3M), on a 542 µmol scale of (10.8), with 3 eq of (2*R*)-2-amino-2-phenyl-ethanol and 6 eq of AcOH, at 130 °C (sealed tube, heating block), for 24h. The product directly precipitated in the reaction medium: it was isolated after filtration, washing with EtOH, then pentane. The final product required two successive triturations in MeOH. Isolated yield: 49%.

### Example 11.52: Synthesis of (4Z)-4-(1H-benzimidazol-5-ylmethylene)-2-[[(1R)-2-methoxy-1-phenyl-ethyl]amino]-1H-imidazol-5-one (92)

Reaction carried out according to GP10, on a 429 µmol scale of (2.15), with 1.2 eq of 1*H*-benzimidazole-5-carbaldehyde. Purification by FC (elution: DCM/MeOH (7N NH3): 99/1 to 90/10). The final product required a reprecipitation from DCM/pentane at 0 °C. Isolated yield: 44%.

### Example 11.53: Synthesis of (4Z)-4-(1H-benzimidazol-5-ylmethylene)-2-[[(2R)-2-hydroxy-2-phenyl-ethyl]amino]-1H-imidazol-5-one (93)

Reaction was carried out according to GP11-B, in THF (0.3M), on a 542 µmol scale of (10.8), with 3 eq of (1*R*)-2-amino-1-phenyl-ethanol and 6 eq of AcOH, at 130 °C (sealed tube, heating block), for 24h. Purification by FC (elution: DCM/MeOH (7N NH₃): 99/1 to 85/15). The final product required a reprecipitation from DCM/pentane at 0 °C. Isolated yield: 29%.

### Example 11.54: Synthesis of (4Z)-2-[[(1S)-2-Amino-1-phenyl-ethyl]amino]-4-(1H-benzimidazol-5-ylmethylene)-1H-imidazol-5-one dihydrochloride (96)

Reaction carried out according to GP10, on a 855 µmol scale of (2.17), with 1.2 eq of 1*H*-benzimidazole-5-carbaldehyde. Purification by FC (elution: DCM/MeOH (7N NH₃): 99/1 to 90/10). The isolated carbamate was engaged in a final deprotection step with HCl in dioxane (4M) at 40 °C. Isolated yield: 40% (2 steps).

### Example 11.55: Synthesis of (4Z)-4-(1H-benzimidazol-5-ylmethylene)-2-(tetrahydropyran-4-ylmethylamino)-1H-imidazol-5-one (98)

Reaction carried out according to GP10, on a 429 µmol scale of (2.19), with 1.2 eq of 1*H*-benzimidazole-5-carbaldehyde. Purification by FC (elution: DCM/MeOH (7N NH3): 99/1 to 90/10). The final product required a trituration in ACN. Isolated yield: 32%.

### Example 11.56: Synthesis of (4Z)-4-(1H-benzimidazol-5-ylmethylene)-2-[4-(4-methylpiperazin-1-yl)anilino]-1H-imidazol-5-one (99)°

Reaction carried out according to GP10, on a 366 µmol scale of (2.20), with 1.2 eq of 1*H*-benzimidazole-5-carbaldehyde. Purification by FC (elution: DCM/MeOH (7N NH₃): 99/1 to 85/15). The final product required a trituration in EtOH at 0 °C. Isolated yield: 12%.

### Example 11.57: Synthesis of (4Z)-4-(1H-benzimidazol-5-ylmethylene)-2-[[(3R,4R)-4-hydroxytetrahydropyran-3-yl]amino]-1H-imidazol-5-one (102)

Reaction was carried out according to GP11-A, in THF (0.3M), on a 542 µmol scale of (10.8), with 3 eq of (3*R*,4*R*)-3-aminotetrahydropyran-4-ol and 6 eq of AcOH, at 130 °C (sealed tube, heating block), for 24h. The product directly precipitated in the reaction medium: it was isolated after filtration, washing with EtOH, then pentane. The final product required a trituration in refluxing EtOH. Isolated yield: 35%.

### Example 11.58: Synthesis of (4Z)-4-(1H-benzimidazol-5-ylmethylene)-2-[[(3S,4S)-4-hydroxytetrahydropyran-3-yl]amino]-1H-imidazol-5-one (103)

Reaction was carried out according to GP11-A, in THF (0.3M), on a 542 µmol scale of (10.8), with 3 eq of (3*S*,4*S*)-3-aminotetrahydropyran-4-ol and 6 eq of AcOH, at 130 °C (sealed tube, heating block), for 24h. The product directly precipitated in the reaction medium: it was isolated after filtration, washing with EtOH, then pentane. The final product required a trituration in refluxing EtOH. Isolated yield: 40%.

### Example 11.59: Synthesis of (4Z)-4-(1H-benzimidazol-5-ylmethylene)-2-(oxepan-3-ylamino)-1H-imidazol-5-one (104)

Reaction was carried out according to GP11-B, in a dioxane/EtOH mixture (3/1) (0.3M), on a 239 µmol scale of (10.8), with 4 eq of oxepan-3-amine and 6 eq of AcOH, at 140 °C (sealed tube, heating block), for 24h. Purification by FC (elution: DCM/MeOH (7N NH3): 99/1 to 88/12). The final product required a reprecipitation from DCM/pentane at 0 °C. Isolated yield: 31%.

### Selected examples from N-methylbenzimidazole sub-series:

### Example 11.60: Synthesis of (4Z)-2-(cycloheptylamino)-4-[(3-methylbenzimidazol-5-yl)methylene]-1H-imidazol-5-one (106)

Reaction carried out according to GP10, on a 562 µmol scale of (2.2), with 1.2 eq of 3-methylbenzimidazole-5-carbaldehyde (7.2). Purification by FC (elution: DCM/MeOH (7N NH₃): 99/1 to 90/10). The final product required a reprecipitation from DCM/pentane at 0 °C. Isolated yield: 31%.

### Example 11.61: Synthesis of (4Z)-2-(cyclooctylamino)-4-[(3-methylbenzimidazol-5-yl)methylene]-1H-imidazol-5-one (107)

Reaction carried out according to GP10, on a 1.12 mmol scale of (2.3), with 1.2 eq of 3-methylbenzimidazole-5-carbaldehyde (7.2). Purification by FC (elution: DCM/MeOH (7N NH₃): 99/1 to 90/10). The final product required a reprecipitation from DCM/pentane at 0 °C. Isolated yield: 64%.

### Example 11.62: Synthesis of (4Z)-2-[[(1R)-1-(hydroxymethyl)-3-methyl-butyl]amino]-4-[(3-methylbenzimidazol-5-yl)methylene]-1H-imidazol-5-one (108)

Reaction carried out according to GP10, on a 376 µmol scale of (2.4), with 1.2 eq of 3-methylbenzimidazole-5-carbaldehyde (7.2). Purification by FC (elution: DCM/MeOH (7N NH3): 99/1 to 90/10). The final product required a reprecipitation from DCM/pentane at 0 °C. The final product required a trituration in EtOH at 0 °C. Isolated yield: 31%.

### Example 11.63: Synthesis of (4Z)-2-[[(1R)-1-(methoxymethyl)-3-methyl-butyl]amino]-4-[(3-methylbenzimidazol-5-yl)methylene]-1H-imidazol-5-one (109)

Reaction carried out according to GP10, on a 469 µmol scale of (2.5), with 1.2 eq of 3-methylbenzimidazole-5-carbaldehyde (7.2). Purification by FC (elution: DCM/MeOH (7N NH3): 99/1 to 90/10). The final product required a reprecipitation from EtOH/Et₂O/pentane at 0 °C. Isolated yield: 27%.

### Example 11.64: Synthesis of (4Z)-2-[[(1R)-1-(fluoromethyl)-3-methyl-butyl]amino]-4-[(3-methylbenzimidazol-5-yl)methylene]-1H-imidazol-5-one (110)

Reaction carried out according to GP10, on a 398 µmol scale of (2.6), with 1.2 eq of 3-methylbenzimidazole-5-carbaldehyde (7.2). Purification by FC (elution: DCM/MeOH (7N NH3): 99/1 to 90/10). The final product required a reprecipitation from DCM/pentane at 0 °C. Isolated yield: 40%.

### Example 11.65: Synthesis of (4Z)-2-[[(1S)-1-(fluoromethyl)-3-methyl-butyl]amino]-4-[(3-methylbenzimidazol-5-yl)methylene]-1H-imidazol-5-one (111)

Reaction carried out according to GP10, on a 398 µmol scale of (2.7), with 1.2 eq of 3-methylbenzimidazole-5-carbaldehyde (7.2). Purification by FC (elution: DCM/MeOH (7N NH₃): 99/1 to 90/10). The final product required a reprecipitation from DCM/pentane at 0 °C. Isolated yield: 36%.

### Example 11.66: Synthesis of (4Z)-2-[[(1R,2R)-2-methoxycyclopentyl]amino]-4-[(3-methylbenzimidazol-5-yl)methylene]-1H-imidazol-5-one (112)

Reaction carried out according to GP10, on a 355 µmol scale of (2.8), with 1.2 eq of 3-methylbenzimidazole-5-carbaldehyde (7.2). Purification by FC (elution: DCM/MeOH (7N NH3): 99/1 to 90/10). The final product required a reprecipitation from DCM/pentane at 0 °C. Isolated yield: 15%.

### Example 11.67: Synthesis of (4Z)-2-[[(1S,2S)-2-methoxycyclopentyl]amino]-4-[(3-methylbenzimidazol-5-yl)methylene]-1H-imidazol-5-one (113)

Reaction carried out according to GP10, on a 355 µmol scale of (2.9), with 1.2 eq of 3-methylbenzimidazole-5-carbaldehyde (7.2). Purification by FC (elution: DCM/MeOH (7N NH3): 99/1 to 90/10). The final product required a reprecipitation from DCM/pentane at 0 °C. Isolated yield: 26%.

### Example 11.68: Synthesis of (4Z)-2-(3-noradamantylamino)-4-[(3-methylbenzimidazol-5-yl)methylene]-1H-imidazol-5-one (114)

Reaction carried out according to GP10, on a 321 µmol scale of (2.10), with 1.2 eq of 3-methylbenzimidazole-5-carbaldehyde (7.2). Purification by FC (elution: DCM/MeOH (7N NH3): 99/1 to 93/7). The final product required a trituration in EtOH at 0 °C. Isolated yield: 20%.

### Example 11.69: Synthesis of (4Z)-2-(1-adamantylamino)-4-[(3-methylbenzimidazol-5-yl)methylene]-1H-imidazol-5-one (115)

Reaction was carried out according to GP11-A, in a THF/dioxane mixture (1/1) (0.3M), on a 551 µmol scale of (10.9), with 3 eq of 1-adamantylamine and 10 eq of AcOH, at 160 °C (sealed tube, heating block), for 24h. The product directly precipitated in the reaction medium: it was isolated after filtration, washing with cold THF, then pentane. The final product required a trituration in EtOH at 0 °C. Isolated yield: 33%.

### Example 11.70: Synthesis of (4Z)-2-[(3-hydroxy-1-adamantyl)amino]-4-[(3-methylbenzimidazol-5-yl)methylene]-1H-imidazol-5-one (116)

Reaction was carried out according to GP11-A, in a THF/dioxane mixture (1/1) (0.3M), on a 551 µmol scale of (10.9), with 3 eq of 3-aminoadamantan-1-ol and 15 eq of AcOH, at 160 °C (sealed tube, heating block), for 6h. The product directly precipitated in the reaction medium: it was isolated after filtration, washing with cold THF, then pentane. The final product required two successive triturations in refluxing EtOH. Isolated yield: 44%.

### Example 11.71: Synthesis of (4Z)-2-[(3-fluoro-1-adamantyl)amino]-4-[(3-methylbenzimidazol-5-yl)methylene]-1H-imidazol-5-one (118)

Reaction carried out according to GP10, on a 321 µmol scale of (2.10), with 1.2 eq of aldehyde (7.2). Purification by FC (elution: DCM/MeOH (7N NH₃): 99/1 to 93/7). The final product required a trituration in EtOH at 0 °C. Isolated yield: 29%.

### Example 11.72: Synthesis of (4Z)-2-[[(2R)-2-hydroxy-2-phenyl-ethyl]amino]-4-[(3-methylbenzimidazol-5-yl)methylene]-1H-imidazol-5-one (124)

Reaction was carried out according to GP11-A, in dioxane (0.3M), on a 367 µmol scale of (10.9), with 3 eq of (1*R*)-2-amino-1-phenyl-ethanol, at 135 °C (sealed tube, heating block), for 48h. The product directly precipitated in the reaction medium: it was isolated after filtration, washing with cold THF, then pentane. The final product required a trituration in EtOH at 0 °C. Isolated yield: 44%.

### Example 11.73: Synthesis of (4Z)-2-[[(1R)-2-amino-1-phenyl-ethyl]amino]-4-[(3-methylbenzimidazol-5-yl)methylene]-1H-imidazol-5-one dihydrochloride (126)

Reaction carried out according to GP10, on a 503 µmol scale of (2.16), with 1.2 eq of 3-methylbenzimidazole-5-carbaldehyde (7.2). Purification by FC (elution: DCM/MeOH (7N NH₃): 99/1 to 93/7). The isolated carbamate was engaged in a final deprotection step with HCl in dioxane (4M) at 40 °C. Isolated yield: 39% (2 steps).

### Example 11.74: Synthesis of (4Z)-2-[[(1S)-2-amino-1-phenyl-ethyl]amino]-4-[(3-methylbenzimidazol-5-yl)methylene]-1H-imidazol-5-one dihydrochloride (127)

Reaction carried out according to GP10, on a 251 µmol scale of (2.17), with 1.2 eq of 3-methylbenzimidazole-5-carbaldehyde (7.2). Purification by FC (elution: DCM/MeOH (7N NH₃): 99/1 to 93/7). The isolated carbamate was engaged in a final deprotection step with HCl in dioxane (4M) at 40 °C. Isolated yield: 26% (2 steps).

### Example 11.75: Synthesis of (4Z)-4-[(3-methylbenzimidazol-5-yl)methylene]-2-[(4-methylthiazol-2-yl)methylaminol-1H-imidazol-5-one (128)

Reaction carried out according to GP10, on a 428 µmol scale of (2.18), with 1.2 eq of 3-methylbenzimidazole-5-carbaldehyde (7.2). Purification by FC (elution: DCM/MeOH (7N NH₃): 99/1 to 93/7). The final product required a trituration in EtOH at 0 °C. Isolated yield: 17%.

### Example 11.76: Synthesis of (4Z)-4-[(3-methylbenzimidazol-5-yl)methylene]-2-[4-(4-methylpiperazin-1-yl)anilino]-1H-imidazol-5-one (130)

Reaction carried out according to GP10, on a 549 µmol scale of (2.20), with 1.2 eq of 3-methylbenzimidazole-5-carbaldehyde (7.2). Purification by FC (elution: DCM/MeOH (7N NH₃): 99/1 to 93/7). Isolated yield: 37%.

### Example 11.77: Synthesis of (4Z)-4-[(3-methylbenzimidazol-5-yl)methylene]-2-(2-pyridylamino)-1H-imidazol-5-one (131)

Reaction carried out according to GP10, on a 454 µmol scale of (2.21), with 1.2 eq of 3-methylbenzimidazole-5-carbaldehyde (7.2). Purification by FC (elution: DCM/MeOH (7N NH3): 99/1 to 93/7). The final product required two successive triturations in refluxing MeOH. Isolated yield: 69%.

Compounds of formula (I) wherein R⁵ is methyl group (compounds (132) to (266)) were isolated using similar procedures as described above.

### Example 12: Biological activity

### MATERIAL AND METHODS

### PROTEIN KINASE ASSAYS

### 1. Overview

Assays were performed by ProQinase GmbH (Engesserstr. 4, D-79108 Freiburg, Germany, www.proqinase.com).The IC₅₀ profile of all compounds was determined using 12 protein kinases (CDK2/cyclin E, CK1ε, CLK1, 2, 3, 4, DYRK1A, 1B, 2, 3, 4, GSK3β). IC₅₀ values were measured by testing 10 concentrations (10 µM to 30 nM) of each compound in singlicate.

### 2. Test Compounds

The compounds were provided as 1µM stock solutions in 100% DMSO. Prior to testing, the 1 µM stock solutions were subjected to a serial, semi-logarithmic dilution using 100 % DMSO as a solvent. This resulted in 10 distinct concentrations, with a dilution endpoint of 3 x 10 nM/100 % DMSO, with 100 % DMSO as controls. In the process, 90 µl H₂O were added to each well of each compound dilution plate. To minimize potential precipitation, the H₂O was added to each plate only a few minutes before the transfer of the compound solutions into the assay plates. The plate was shaken thoroughly, resulting in a compound dilution plate/ 10 % DMSO.

For the assays (see below), 5 µL solution from each well of the compound dilution plates/10 % DMSO were transferred into the assay plates. The final volume of the assay was 50 µL. All compounds were tested at 10 final assay concentrations in the range from 10 µM to 30 nM. The final DMSO concentration in the reaction cocktails was 1 % in all cases.

### 3. Recombinant Protein Kinases

All protein kinases provided by ProQinase were expressed in Sf9 insect cells or in *E. coli* as recombinant GST-fusion proteins or His-tagged proteins, either as full-length or enzymatically active fragments. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy.

### 4. Protein Kinase Assay

A radiometric protein kinase assay (33PanQinase^{®} Activity Assay) was used for measuring the kinase activity of the 12 protein kinases. All kinase assays were performed in 96-well FlashPlatesTM from PerkinElmer (Boston, MA, USA) in a 50 µL reaction volume. The reaction cocktail was pipetted in four steps in the following order:
- 25 µL of assay buffer (standard buffer/[γ-³³P]-ATP),
- 10 µL of ATP solution (in H₂O),
- 5 µL of test compound (in 10 % DMSO),
- 10 µL of enzyme/substrate mixture.

The assay for all protein kinases contained 70 mM HEPES-NaOH pH 7.5, 3 mM MgCl₂, 3 mM MnCl₂, 3 µM Na-orthovanadate, 1.2 mM DTT, 50 µg/ml PEG20000, ATP (variable concentrations, corresponding to the apparent ATP-Km of the respective kinase), [γ-³³P]-ATP (approx. 6.5 × 10-05 cpm per well), protein kinase (variable amounts), and substrate (variable amounts). The reaction cocktails were incubated at 30°C for 60 minutes. The reaction was stopped with 50 µL of 2% (v/v) H₃PO₄, plates were aspirated and washed two times with 200 µL 0.9% (w/v) NaCl. Incorporation of ³³Pi was determined with a microplate scintillation counter (Microbeta, Wallac). The IC50 values for all compounds were calculated from the dose response curves.

### 5. Quality controls

As a parameter for assay quality, the Z'-factor (Zhang et al., J. Biomol. Screen. 2: 67-73, 1999) for the low and high controls of each assay plate (n = 8) was used. ProQinase's criterion for repetition of an assay plate is a Z'-factor below 0.4 (Iversen et al., J. Biomol. Screen. 3: 247-252, 2006).

Their activity has been classified according to two criteria, kinase inhibition potency and kinase selectivity.

The **kinase inhibition potency classification** was done according to the following ranges of IC₅₀ values:
Some compounds have an IC₅₀ activity varying from above 0.050 µM. These compounds correspond to the above-reported class **E**. Some compounds have an IC₅₀ activity varying from 0.025 to 0.050 µM. These compounds correspond to the above-reported class **D**. Some compounds of the invention have an IC₅₀ activity varying from 0.010 to 0.025 µM. These compounds correspond to the above-reported class **C**. Further some particular compounds have an IC₅₀ activity varying from 0.005 to 0.010 µM. These compounds correspond to the above-reported class **B**. Even preferred are compounds of the invention that have an IC₅₀ activity of less than 0.005 µM. These compounds correspond to the above-reported class **A**. This classification was applied to CLK1 and DYRK1A. Said letters A to E were used to quote the activity/efficacy of the compounds of the invention in the following Table 4, 4A and 4B.

The **kinase selectivity classification** was based on comparing the IC₅₀ values of DYRK1A with that of CLK1 or with that of DYRK1B. The classification was done according to the following ranges of values: **I**: ratio of IC₅₀ on CLK1 or DYRK1B over IC₅₀ on DYRK1A ≥ 10 fold (most DYRK1A-selective compounds); **II**: ratio between 2 and 10 fold; **III**: ratio between 0.5 and 2 fold; **IV**: ratio between 0.1 and 0.5 fold; **V**: ratio > 0.1 fold (most CLK1- or DYRK1B-selective compounds). Said numbers I to V were used to quote the relative selectivity of the compounds of the invention in the following Tables.

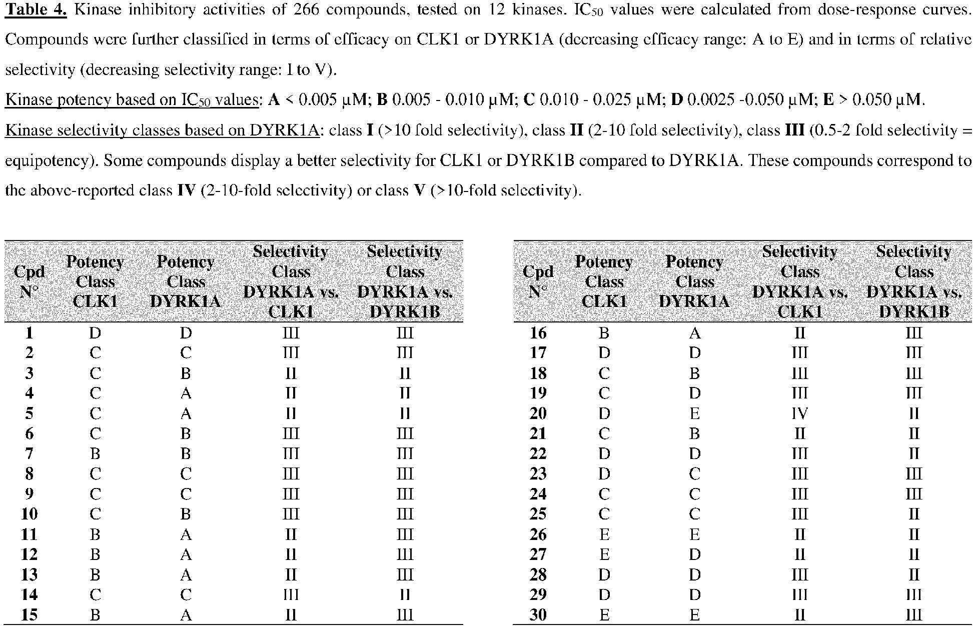

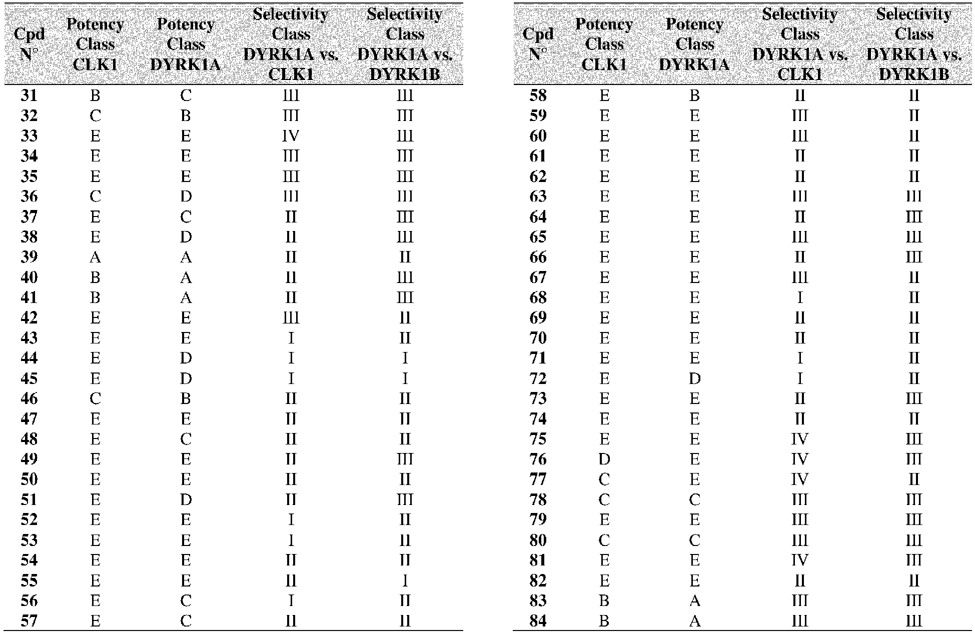

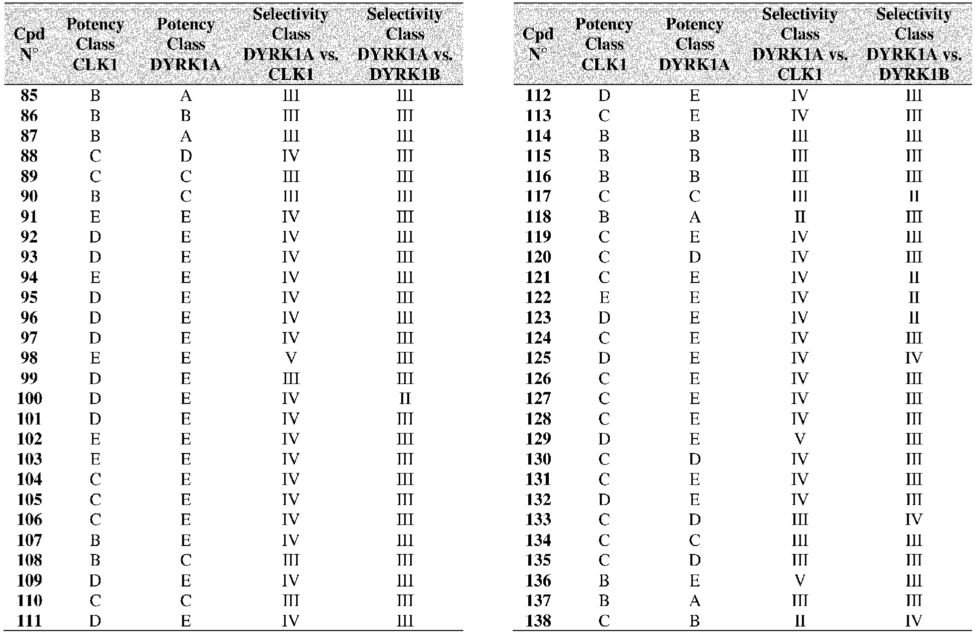

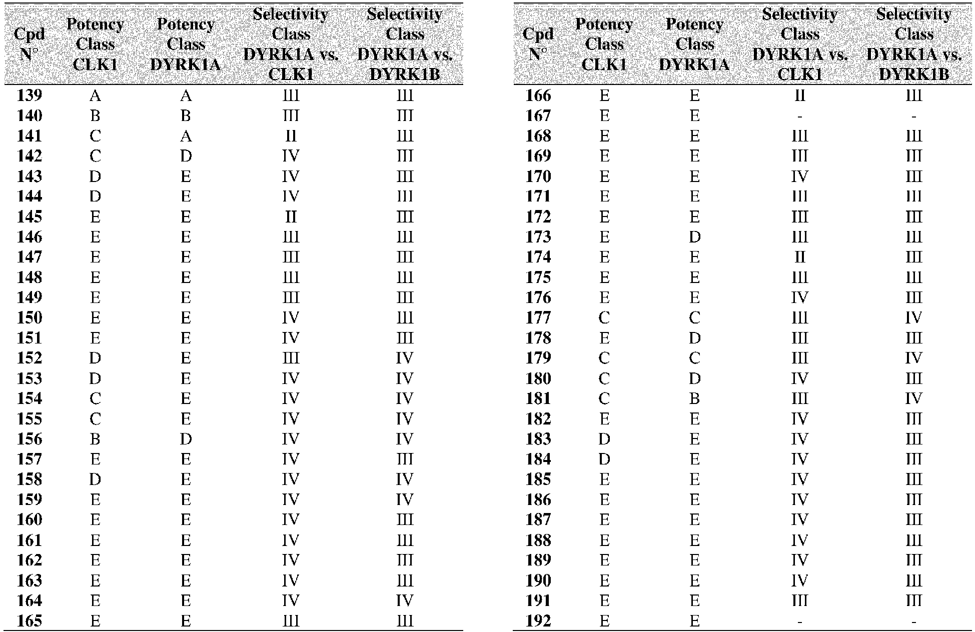

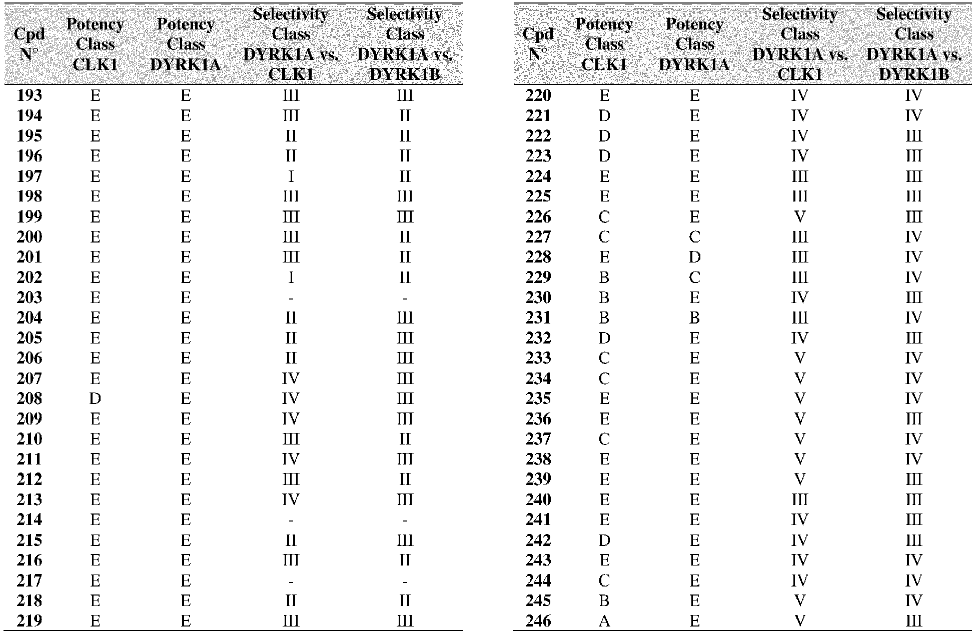

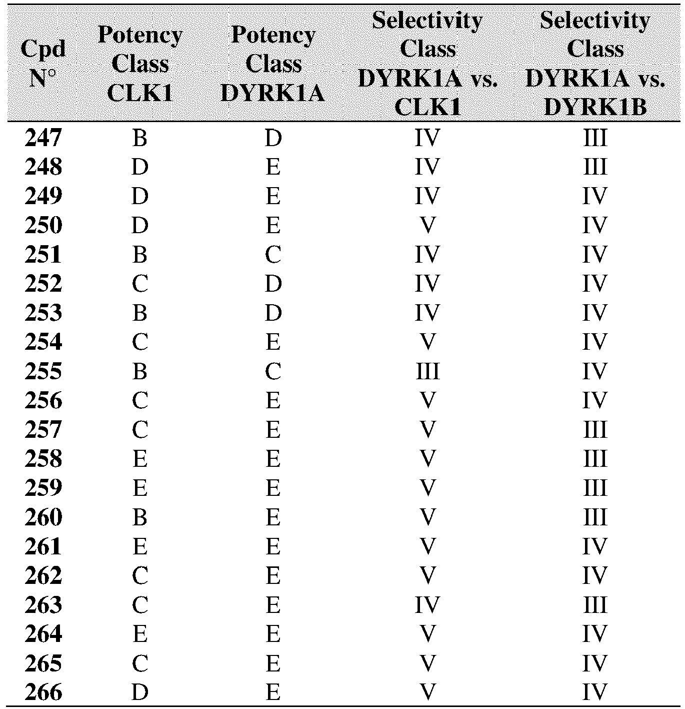

**Table 4A. Most potent CLK1 inhibitors (IC₅₀ ≤ 10 nM). Kinase inhibition potency classes: IC₅₀ values: A ≤ 0.005 µM; B ≤ 0.010 µM; C ≤ 0.025 µM; D ≤ 0.050 µM; E > 0.050 µM. Kinase selectivity classes based on DYRK1A: class I (>10 fold selectivity), class II (2-10 fold selectivity), class III (0.5-2 fold selectivity = equipotency). Some compounds display a better selectivity for CLK1 or DYRK1B compared to DYRK1A. These compounds correspond to the above-reported class IV (2-10-fold selectivity) or class V (>10-fold selectivity).**

| **Cpd N°** | **Potency Class CLK1** | **Potency Class DYRK1A** | **Selectivity Class DYRK1A vs. CLK1** |
|---|---|---|---|
| **39** | A | A | II |
| **139** | A | A | III |
| **246** | A | E | V |
| **245** | B | E | V |
| **84** | B | A | III |
| **87** | B | A | III |
| **83** | B | A | III |
| **12** | B | A | II |
| **31** | B | C | III |
| **251** | B | C | IV |
| **85** | B | A | III |
| **118** | B | A | II |
| **16** | B | A | II |
| **231** | B | B | III |
| **137** | B | A | III |
| **86** | B | B | III |
| **253** | B | D | IV |
| **140** | B | B | III |
| **41** | B | A | II |
| **40** | B | A | II |
| **136** | B | E | V |
| **7** | B | B | III |
| **255** | B | C | III |
| **13** | B | A | II |
| **247** | B | D | IV |
| **114** | B | B | III |
| **107** | B | E | IV |
| **108** | B | C | III |
| **15** | B | A | II |
| **11** | B | A | II |
| **116** | B | B | III |
| **229** | B | C | III |
| **90** | B | C | III |
| **230** | B | E | IV |
| **115** | B | B | III |
| **156** | B | D | IV |

The most potent CLK1 inhibitors, with an IC50 lower or equal to 10 nM are compounds (7), (11), (12), (13), (15), (16), (31), (39), (40), (41), (83), (84), (85), (86), (87), (90), (107), (108), (114), (115), (116), (118), (136), (137), (139), (140), (156), (247), (229), (230), (231), (245), (246), (251), (253) and (255).

The most potent CLK2 inhibitors, with an IC₅₀ lower or equal to 10 nM are compounds (11), (15), (16), (83), (84), (87), (114), (115), (116), (118), (137), (138), (139), (140), (141), (156), (227), (228), (229), (231), (251), (252), (253) and (255).

The most potent CLK3 inhibitors, with an IC50 lower or equal to 250 nM are compounds (24), (27), (29), (72), (100), (114), (134), (136), (152) and (168).

The most potent CLK4 inhibitors, with an IC50 lower or equal to 10 nM are compounds (3), (4), (5), (6), (7), (8), (9), (10), (11), (12), (13), (14), (15), (16), (18), (19), (21), (22), (23), (24), (25), (31), (39), (40), (41), (76), (77), (78), (80), (83), (84), (85), (86), (87), (88), (89), (90), (92), (95), (100), (101), (104), (105), (106), (107), (108), (110), (111), (112), (113), (114), (115), (116), (117), (118), (119), (120), (121), (124), (128), (129), (130), (131), (137), (139), (140), (141), (156), (227), (229), (230), (231), (246), (247), (251), (252), (253), (254) and (255).

**Table 4B. Most potent DYRK1A inhibitors (IC₅₀ ≤ 10 nM). Kinase Inhibition Efficacy Classes and Kinase Selectivity Classes as described in Table 4A.**

| **Cpd N°** | **Potency Class CLK1** | **Potency Class DYRK1A** | **Selectivity Class DYRK1A vs. CLK1** | **Selectivity Class DYRK1A vs. DYRK1B** |
|---|---|---|---|---|
| **12** | B | A | II | III |
| **13** | B | A | II | III |
| **11** | B | A | II | III |
| **41** | B | A | II | III |
| **118** | B | A | II | III |
| **40** | B | A | II | III |
| **39** | A | A | II | II |
| **139** | A | A | III | III |
| **141** | C | A | II | III |
| **15** | B | A | II | III |
| **16** | B | A | II | III |
| **83** | B | A | III | III |
| **84** | B | A | III | III |
| **85** | B | A | III | III |
| **87** | B | A | III | III |
| **4** | C | A | II | II |
| **5** | C | A | II | II |
| **137** | B | A | III | III |
| **140** | B | B | III | III |
| **7** | B | B | III | III |
| **86** | B | B | III | III |
| **21** | C | B | II | II |
| **114** | B | B | III | III |
| **46** | C | B | II | II |
| **3** | C | B | II | II |
| **10** | C | B | III | III |
| **6** | C | B | III | III |
| **18** | C | B | III | III |
| **138** | C | B | II | IV |
| **32** | C | B | III | III |
| **116** | B | B | III | III |
| **115** | B | B | III | III |
| **58** | E | B | II | II |
| **181** | C | B | III | IV |
| **231** | B | B | III | IV |

The most potent DYRK1A inhibitors, with an IC₅₀ lower or equal to 10 nM are compounds (3), (4), (5), (6), (7), (10), (11), (12), (13), (15), (16), (18), (21), (32), (39), (40), (41), (46), (58), (83), (84), (85), (86), (87), (114), (115), (116), (118), (137), (138), (139), (140), (141), (181) and (231).

The most potent DYRK1B inhibitors, with an IC₅₀ lower or equal to 10 nM are compounds (4), (5), (6), (7), (10), (11), (12), (13), (15), (16), (32), (39), (40), (41), (83), (84), (85), (86), (87), (114), (116), (118), (134), (137), (138), (139), (140), (141), (177), (179), (181), (227), (229), (231), (251), (253) and (255).

The most potent DYRK2 inhibitors, with an IC₅₀ lower or equal to 25 nM are compounds (118), (135), (137), (139), (140), (141), (154), (179), (229), (251), (252) and (253).

The most potent DYRK3 inhibitors, with an IC₅₀ lower or equal to 25 nM are compounds (41), (83), (84), (87), (114), (118), (137), (138), (139), (141), (181), (227), (228), (229), (231), (251), (252) and (255).

The most potent DYRK4 inhibitors, with an IC₅₀ lower or equal to 100 nM are compounds (114), (115), (116), (118), (135), (137), (139), (140), (225), (227), (228), (229), (251), (252), (253) and (255).

**Table 4C. Most DYRK1A selective inhibitors compared to CLK1. Kinase Selectivity Classes as described in Table 4A.**

| **Cpd N°** | **Selectivity Class DYRK1A** vs. **CLK1** |
|---|---|
| **71** | I |
| **68** | I |
| **56** | I |
| **202** | I |
| **43** | I |
| **197** | I |
| **52** | I |
| **44** | I |
| **72** | I |
| **53** | I |
| **45** | I |

**Table 4D. Most CLK1 selective inhibitors compared to DYRK1A. Kinase Selectivity Classes as described in Table 4A.**

| **Cpd N°** | **Selectivity Class DYRK1A vs. CLK1** | | **Cpd N°** | **Selectivity Class DYRK1A vs. CLK1** |
|---|---|---|---|---|
| **256** | V | | **233** | V |
| **262** | V | | **235** | V |
| **245** | V | | **237** | V |
| **250** | V | | **258** | V |
| **259** | V | | **239** | V |
| **266** | V | | **261** | V |
| **264** | V | | **98** | V |
| **246** | V | | **254** | V |
| **129** | V | | **226** | V |
| **136** | V | | **236** | V |
| **257** | V | | **238** | V |
| **234** | V | | **265** | V |
| **260** | V | | | |

**Table 4E. Most DYRK1A selective inhibitors compared to DYRK1B. Kinase Selectivity Classes as described in Table 4A.**

| **Cpd N°** | **Selectivity Class DYRK1A vs. DYRK1B** | | **Cpd N°** | **Selectivity Class DYRK1A vs. DYRK1B** |
|---|---|---|---|---|
| **44** | I | | **43** | II |
| **55** | I | | **201** | II |
| **45** | I | | **194** | II |
| **25** | II | | **48** | II |
| **14** | II | | **5** | II |
| **100** | II | | **22** | II |
| **200** | II | | **77** | II |
| **28** | II | | **71** | II |
| **117** | II | | **21** | II |
| **42** | II | | **46** | II |
| **210** | II | | **39** | II |
| **59** | II | | **72** | II |
| **195** | II | | **61** | II |
| **123** | II | | **27** | II |
| **3** | II | | **216** | II |
| **4** | II | | **196** | II |
| **122** | II | | **202** | II |
| **212** | II | | **50** | II |
| **218** | II | | **60** | II |
| **121** | II | | **57** | II |
| **20** | II | | **67** | II |
| **68** | II | | **26** | II |
| **54** | II | | **53** | II |
| **197** | II | | **69** | II |
| **52** | II | | **58** | II |
| **62** | II | | **70** | II |
| **47** | II | | **74** | II |
| **82** | II | | **56** | II |

**Table 4F. Most DYRK1B selective inhibitors compared to DYRK1A. Kinase Selectivity Classes as described in Table 4A.**

| **Cpd N°** | **Selectivity Class DYRK1A vs. DYRK1B** | | **Cpd N°** | **Selectivity Class DYRK1A vs. DYRK1B** |
|---|---|---|---|---|
| **152** | IV | | **179** | IV |
| **251** | IV | | **254** | IV |
| **227** | IV | | **262** | IV |
| **125** | IV | | **155** | IV |
| **244** | IV | | **138** | IV |
| **249** | IV | | **250** | IV |
| **264** | IV | | **220** | IV |
| **228** | IV | | **238** | IV |
| **261** | IV | | **255** | IV |
| **265** | IV | | **252** | IV |
| **154** | IV | | **181** | IV |
| **253** | IV | | **256** | IV |
| **164** | IV | | **231** | IV |
| **266** | IV | | **234** | IV |
| **229** | IV | | **237** | IV |
| **158** | IV | | **235** | IV |
| **153** | IV | | **233** | IV |
| **159** | IV | | **245** | IV |
| **156** | IV | | **243** | IV |
| **177** | IV | | **221** | IV |
| **133** | IV | | | |

### RESULTS

Most compounds of the present invention present an IC₅₀ activity of less than 2 µM on CLKs or DYRKs. All the compounds were inhibitors of DYRK1A and CLK1 (Table 4).

Compounds were preferably inhibitory on CLK1 (Table 4A), CLK4, DYRK1A (Table 4B) and DYRK1B.

Some are most selective for DYRK1A vs. CLK1 (Table 4C), DYRK1A vs. DYRK1B (Table 4E), DYRK1B vs. DYRK1A (Table 4F) or CLK1 vs. DYRK1A (Table 4D). Some compounds display a better selectivity for DYRK1A compared to CLK1 or DYRK1B. These compounds correspond to the above-reported class **I** (>10-fold selectivity) or class **II** (2 to 10-fold selectivity). Some compounds are equipotent. These compounds correspond to the above-reported class **III** (0.5 to 2-fold selectivity). Some compounds display a better selectivity for CLK1 or DYRK1B compared to DYRK1A. These compounds correspond to the above-reported class **IV** (2 to 10-fold selectivity) or class **V** (>10-fold selectivity).

### CONCLUSION

Based on the previous results, it can be concluded that the compounds of formula (I) are suitable chemical compounds in the prevention and/or treatment of cognitive deficits and neuroinflammation associated with Down syndrome (Trisomy 21), Alzheimer's disease and related diseases, dementia and/or tauopathies; other neurodegenerative diseases (Parkinson's disease; Pick disease, including Niemann-Pick Type C Disease); CDKL5 Deficiency Disorder; type 1 and type 2 diabetes; abnormal folate and methionine metabolism; tendinopathy and osteoarthritis, in particular knee osteoarthritis; Duchenne muscular dystrophy; several cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), malaria, Leishmaniasis, Chagas and sleeping sickness (*Trypanosoma* sp.), and cattle diseases due to unicellular pathogens, viral infections, such as caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and in the regulation of body temperature.

It may further be concluded that some compounds of formula (I) are further suitable for treating and/or preventing Phelan-McDermid syndrome; autism; further viral infections, such as caused by Hepatitis C virus, Chikungunya virus, Dengue virus, Influenza virus and Severe acute respiratory syndrome (SARS) coronavirus, Cytomegalovirus and Human papillomavirus; further cancers, such as tissue cancer, including liposarcoma, Hedgehog/GLI-dependent cancer, liver cancer, including Hepatocellular carcinoma, neuroinflammation, anemia, infections caused by unicellular parasites, such as malaria, Leishmaniasis, Chagas and sleeping sickness (*Trypanosoma* sp.), and cattle diseases due to unicellular pathogens.

Leucettinibs display much increased efficacy (down to sub-micromolar and single-digit micromolar IC₅₀ values) compared to reference compounds. They also display a large range of selectivity towards DYRK1A or CLK1/CLK4. And some products being equipotent on CLKs and DYRKs may also find applications as dual-specificity inhibitors.

The present invention further relates to a pharmaceutical composition comprising at least one compound of formula (I) as defined above or any of its pharmaceutically acceptable salts or at least any of compounds (1) to (266) as defined above or any of its pharmaceutically acceptable salts.

The present invention further relates to a pharmaceutical composition comprising at least one compound of formula (I) as defined above or any of its pharmaceutically acceptable salts or at least any of compounds (1) to (266) as defined above or any of its pharmaceutically acceptable salts and also at least one pharmaceutically acceptable excipient.

Pharmaceutical compositions of the invention can contain one or more compound(s) of the invention in any form described herein.

Still a further object of the present invention consists of a compound of formula (I) as defined above, and compounds (1) to (266) as defined above, or one of their pharmaceutically acceptable salts for use in the treatment and/or prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such malaria, Leishmaniasis, Chagas and sleeping sickness (*Trypanosoma* sp.), and cattle diseases due to unicellular pathogens, and for regulating body temperature.

Still a further object of the present invention consists of a compound of formula (I) as defined above, and compounds (1) to (266) as defined above, or one of their pharmaceutically acceptable salts for use in the treatment and/or prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease; Pick disease, including Niemann-Pick Type C Disease; CDKL5 Deficiency Disorder; type 1 and type 2 diabetes; abnomral folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness (*Trypanosoma* sp.), and cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of a compound of formula (I), as defined above, and compounds (1) to (266) as defined above, or one of their pharmaceutically acceptable salts for use in the treatment and/or prevention of a disease selected from type 1 and type 2 diabetes; viral infections, in partiuclar as mentioned above; tendinopathy and osteoarthritis; cancers, in particular as mentioned above; infections caused by unicellular parasites, such malaria, Leishmaniasis, Chagas and sleeping sickness (*Trypanosoma* sp.); cattle diseases due to unicellular pathogens,and to regulate body temperature.

Still a further object of the present invention consists of a compound of formula (I), as defined above, and compounds (1) to (266) as defined above, or one of their pharmaceutically acceptable salts for use in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions and type 1 and type 2 diabetes.

Still a further object of the present invention consists of the use of at least one compound of formula (I) as defined above, and compounds (1) to (266) as defined above, or one of their pharmaceutically acceptable salts according to the present invention for preparing a drug for preventing and/or treating a disease selected from cognitive deficits and neuroinflammation associated with Down syndrome (Trisomy 21); Alzheimer's disease and related diseases; dementia; tauopathies; and other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, such as megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such as malaria, Leishmaniasis, Chagas and sleeping sickness (*Trypanosoma* sp.), cattle diseases due to unicellular pathogens, and for regulating body temperature.

Still a further object of the present invention consists of the use of at least one compound of formula (I) as defined above, and compounds (1) to (266) as defined above, or one of their pharmaceutically acceptable salts according to the present invention for preparing a drug for preventing and/or treating a disease selected from cognitive deficits and neuroinflammation associated with Down syndrome (Trisomy 21), Alzheimer's disease and related diseases, dementia and/or tauopathies; other neurodegenerative diseases such as Parkinson's disease and Pick disease, in particular Niemann-Pick Type C Disease; CDKL5 Deficiency Disorder; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness (*Trypanosoma* sp.), cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of the use of at least one compound of formula (I), as defined above, and compounds (1) to (266) as defined above, or one of their pharmaceutically acceptable salts according to the present invention for preparing a drug for combatting a disease selected from type 1 and type 2 diabetes, viral infections, in particular as mentioned above, osteoarthritis and tendinopathy, cancers and leukemias, in particular as mentioned above, infections caused by unicellular parasites, such malaria, Leishmaniasis, Chagas and sleeping sickness (*Trypanosoma* sp.), cattle diseases due to unicellular pathogens, and to regulate body temperature.

Another object of the invention relates to a therapeutic method for the treatment and/or for the prevention of a disease selected from cognitive deficits and neuroinflammation associated with Down syndrome (Trisomy 21); Alzheimer's disease and related diseases; dementia; tauopathies; and other neurodegenerative diseases such as Parkinson's disease; Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer and breast cancer, such as Triple-negative breast cancer (TNBC), infections caused by unicellular parasites, such malaria, Leishmaniasis, Chagas and sleeping sickness (*Trypanosoma* sp.), and cattle diseases due to unicellular pathogens, and viral infections, such as infections caused by Human immunodeficiency virus type 1 (HIV-1), Human cytomegalovirus (HCMV), Influenza A, Herpes virus, rhesus macaque cytomegalovirus, varicella-zoster virus and herpes simplex virus (HSV) and for regulating body temperature.

Still a further object of the present invention consists of the use of at least one compound of formula (I), as defined above, and compounds (1) to (266) as defined above, or one of their pharmaceutically acceptable salts according to the present invention for preparing a drug for combatting a disease selected from type 1 and type 2 diabetes, viral infections, in partiuclar as mentioned above, tendinopathy and osteoarthritis, cancers, in particular as mentioned above, infections caused by unicellular parasites, such malaria, Leishmaniasis, Chagas and sleeping sickness (*Trypanosoma* sp.), cattle diseases due to unicellular pathogens, and to regulate body temperature.

According to a particular embodiment, the treatment is continuous or noncontinuous.

A "continuous treatment" means a long-term treatment which can be implemented with various administration frequencies, such as once or twice every day, every three days, once a week, or once every two weeks or once every month or by transdermal patch delivery.

According to one embodiment, the compound of formula (I), or any of its pharmaceutically acceptable salts, is administered at a dose varying from 0.1 to 1000 mg. in particular varying from 0.5 to 500 mg, or for example varying from 5 to 100 mg.

Another object of the invention relates to a therapeutic method for the treatment and/or for the prevention of a disease selected from cognitive deficits and neuroinflammation associated with Down syndrome (Trisomy 21), Alzheimer's disease and related diseases, dementia and/or tauopathies as well as other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia, head and neck squamous cell carcinoma, pancreatic cancer, including pancreatic ductal adenocarcinoma, prostate cancer, gastrointestinal cancer, breast cancer, such as Triple-negative breast cancer (TNBC), tissue cancer, including liposarcoma, Hedgehog/GLI-dependent cancer, liver cancer, including Hepatocellular carcinoma and viral infections, such as infections caused by Human immunodeficiency virus type 1 (HIV-1), Human cytomegalovirus (HCMV), Influenza A, Herpes virus, rhesus macaque cytomegalovirus, varicella-zoster virus, herpes simplex virus (HSV), Hepatitis C virus, Chikungunya virus, Dengue virus, Influenza virus and Severe acute respiratory syndrome (SARS) coronavirus, Cytomegalovirus and Human papillomavirus; neuroinflammation; anemia; infections caused by unicellular parasites, such malaria, Leishmaniasis, Chagas and sleeping sickness (*Trypanosoma* sp.), cattle diseases due to unicellular pathogens, and for the regulation of the body temperature, in a patient in need thereof, comprising at least a step of administering a therapeutically effective amount of a compound of formula (I) or of compounds (1) to (266), as defined above. or one of their acceptable salts.

In a specific embodiment, the invention provides a use of a compound of formula (I) according to the invention or a pharmaceutically acceptable salt thereof or a pharmaceutically active derivative thereof or a method according to the invention wherein the compound of formula (I) is to be administered in combination with a co-agent useful in anyone of the hereabove mentioned diseases.

The compounds can be administered through any mode of administration such as, for example, intramuscular, intravenous, intranasal or oral route, transdermal patch, etc.

The inventive composition can further include one or more additives such as diluents, excipients, stabilizers and preservatives. Such additives are well known to those skilled in the art and are described notably in "Ullmann's Encyclopedia of Industrial Chemistry, 6th Ed." (various editors, 1989-1998, Marcel Dekker) and in "Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.
Oral route of administration is in particular preferred in the prophylaxis or treatment aspect of the invention.

## Claims

1. A compound of formula (I) 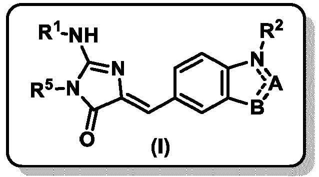 wherein:
A is selected from the group consisting of =CH-, -N= and -NR³-,
B is selected from the group consisting of -O-, =CH-, =N-, -NH- and -NR⁴-,
at least one of A and B is =CH-,
A and B can not both represent a group comprising a C atom,
R² is selected from the group consisting of a hydrogen atom and a (C₁-C₃)alkyl group,
R³ and R⁴ are independently selected from (C₁-C₃)alkyl groups,
R⁵ represents a hydrogen atom, a (C₁-C₄)alkyl group or a (C₃-C₆)cycloalkyl group, and
when A represents =CH- and B represents =N- or -NH-, then R² respectively represents a hydrogen atom or does not exist,
when N is trivalent, then R² does not exist, and
wherein R¹ represents:
(i). a (C₄-C₆)alkyl group substituted by a group selected from a hydroxy group, a halogen and a (C₁-C₃)alkoxy group,
(ii). a (C₃-C₈)cycloalkyl group, optionally substituted by a group selected from a hydroxy group and a (C₁-C₃) alkoxy group,
(iii). a bridged (C₆-C₁₀)cycloalkyl group, optionally substituted by a group selected from a (C₁-C₄)alkyl group, a (C₁-C₄)alkoxy group, a halogen atom and a hydroxy group,
(iv). a fused phenyl group, selected from phenyl groups fused with a (C₅-C₆)cycloalkyl, which (C₅-C₆)cycloalkyl is optionally substituted by a hydroxy group,
(v). a phenyl group, substituted by a (C₄-C₇)heterocycloalkyl group, said (C₄-C₇)heterocycloalkyl group being itself optionally substituted by a (C₁-C₄)alkyl group, or
(vi). a R'-L- group, wherein L is either a single bond or a (C₁-C₃)alkanediyl group, optionnally susbstituted by a group selected from a hydroxy group and a (C₁-C₃)alkoxy group, and
R' represents:
(vi.1). a (C₃-C₈)heterocycloalkyl group, optionally substituted by one or two groups selected from a hydroxy group, and a (C₁-C₄)alkyl group, or
(vi.2). a (C₃-C₈)heteroaryl group, optionally substituted by a (C₁-C₄)alkyl group, or
(vii). a R"-L- group wherein L is a (C₁-C₃)alkanediyl group, optionally substituted by a group selected from a -NR^{b}R^{c} group, a (C₁-C₃)alkoxy group and a hydroxy group, and
R" is a phenyl group, optionally substituted by a fluoro(C₁-C₄)alkyl group,
R^{b} and R^{c} independently represent a (C₁-C₆)alkyl group or a hydrogen atom,
or any of its pharmaceutically acceptable salt.

2. A compound of formula (I) according to claim 1, wherein R¹ represents:
(i). a (C₅-C₆)alkyl group substituted by a group selected from a hydroxy group, a halogen and a (C₁-C₃)alkoxy group,
(ii). a (C₅-C₈)cycloalkyl group, optionally substituted by a group selected from a hydroxy group and a (C₁-C₃) alkoxy group,
(iii). a bridged (C₉-C₁₀)cycloalkyl group, optionally substituted by a group selected from a (C₁-C₄)alkyl group, a (C₁-C₄)alkoxy group, a halogen atom and a hydroxy group,
(iv). a phenyl group fused with a cyclopentyl, which cyclopentyl is optionally substituted by a hydroxy group,
(v). a phenyl group, substituted by a (C₄-C₇)heterocycloalkyl group, said (C₄-C₇)heterocycloalkyl group being itself optionally substituted by a (C₁-C₄) alkyl group, or
(vi). a R'-L- group wherein
• L is either a single bond or a (C₁-C₃)alkanediyl group, optionnally susbstituted by a group selected from a hydroxy group and a (C₁-C₃)alkoxy group, and
• R' represents:
(vi.1). a (C₆-C₇)heterocycloalkyl group, optionally substituted by one or two groups selected from a hydroxy group and a (C₁-C₄)alkyl group, or
(vi.2). a heteroaryl group, optionally substituted by a (C₁-C₄)alkyl group,
(vii). a R"-L- group wherein
• L is a (C₁-C₃)alkanediyl group, optionally substituted by a group selected from a -NR^{b}R^{c} group, a (C₁-C₃)alkoxy group and a hydroxy group, and
• R" is a phenyl group, optionally substituted by a fluoro(C₁-C₄)alkyl group, and
R^{b} and R^{c} independently represent a (C₁-C₆)alkyl group or a hydrogen atom, or any of its pharmaceutically acceptable salt.

3. A compound of formula (I) according to any of claims 1 or 2, wherein R¹ represents:
(i). a (C₅-C₆)alkyl group substituted by a group selected from a hydroxy group, a fluorine atom and a methoxy group,
(ii). a (C₅-C₈)cycloalkyl group in particular a cyclopentyl, a cyclohexyl, a cycloheptyl or a cyclooctyl, optionally substituted by a group selected from a hydroxy group and a methoxy group,
(iii). a bridged (C₉-C₁₀)cycloalkyl group in particular an adamantyl or a noradamantyl, optionally substituted by a group selected from a methoxy group, a fluorine atom and a hydroxy group,
(iv). a phenyl group fused with a cyclopentyl, which cyclopentyl is substituted by a hydroxy group,
(v). a phenyl group, substituted by a N-methylpiperazinyl group, or
(vi). a R'-L- group wherein L is either a single bond or a (C₁-C₃)alkanediyl group, optionnally susbstituted by a group chosen from a hydroxy group and a methoxy group,
and R' is selected from the group consisting of:
(vi.1). a (C₆-C₇)heterocycloalkyl group, in particular a tetrahydropyranyl or an oxepanyl, optionally substituted by one or two groups selected from a hydroxy group and a methyl group,
(vi.2). a heteroaryl group, in particular a pyridinyl or a thiazolyl, optionally substituted by a methyl group, or
(vii). a R"-L- group wherein L is a (C₁-C₃)alkanediyl group, optionally substituted by a group selected from the group consisting of a -NH2 group, a methoxy group, a hydroxy group, a -COOR^{a} group and a halogen atom, in particular a fluorine atom, and
R" is a phenyl group, optionally substituted by a trifluoromethyl group,
or any of its pharmaceutically acceptable salts.

4. A compound of formula (I) according to any of the preceding claims, wherein L is selected from a group consisting of a -CH₂- group, a -CH(CH₂OH)- group, a -CH(CH₂OCH₃)- group, a -CH(OH)-CH₂- group and a -CH(CH₂NH₂)- group, or any of its pharmaceutically acceptable salts.

5. A compound of formula (I) according to any of the preceding claims, wherein:
(vi.1). when R' is a (C₅-C₈)heterocycloalkyl group, L is a -CH₂-group,
(vi.2). when R' is a phenyl, L is selected from the group consisting of a -CH₂- group, a -CH(CH₂OH)- group, a -CH(CH₂OCH₃)- group, a -CH(OH)-CH₂- group and a -CH(CH₂NH₂)- group,
(vi.3). when R' is a heteroaryl group, L is a -CH₂- group, or any of its pharmaceutically acceptable salts.

6. A compound of formula (I) according to any of the preceding claims, chosen from the following compounds 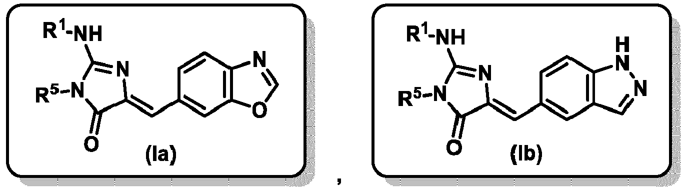 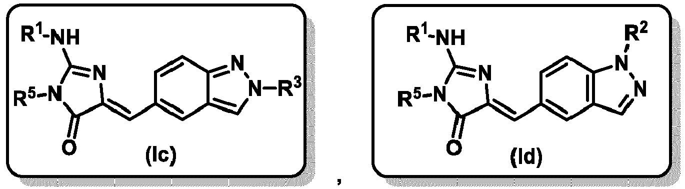 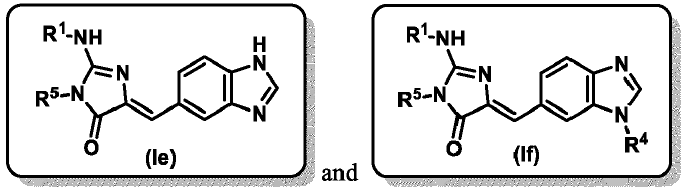 wherein R¹, R², R³ R⁴ and R⁵ are as defined in any of the preceding claims,
or any of its pharmaceutically acceptable salts.

7. A compound of formula (I) according to claim 6, wherein when the compound of formula (I) is chosen from the subformulae (Ib), (Id), (Ie) and (If), R¹ represents:
(i). a (C₄-C₆)alkyl group substituted by a group selected from a hydroxy group, a halogen and a (C₁-C₃)alkoxy group,
(ii). a (C₃-C₈)cycloalkyl group, optionally substituted by a group selected from a hydroxy group and a (C₁-C₃) alkoxy group,
(iii). a bridged (C₆-C₁₀)cycloalkyl group, optionally substituted by a group selected from a (C₁-C₄)alkyl group, a (C₁-C₄)alkoxy group, a halogen atom and a hydroxy group, or
(iv). a R'-L- group, wherein L is either a single bond or a (C₁-C₃)alkanediyl group, optionnally susbstituted by a group selected from a hydroxy group and a (C₁-C₃)alkoxy group, and
R' represents:
(iv.1). a (C₃-C₈)heterocycloalkyl group, optionally substituted by one or two groups selected from a hydroxy group, and a (C₁-C₄)alkyl group, or
(iv.2). a (C₃-C₈)heteroaryl group, optionally substituted by a (C₁-C₄)alkyl group, or
(v). a R"-L- group wherein L is a (C₁-C₃)alkanediyl group, optionally substituted by a group selected from a -NR^{b}R^{c} group, a (C₁-C₃)alkoxy group and a hydroxy group, and
R" is a phenyl group, optionally substituted by a fluoro(C₁-C₄)alkyl group,
R^{b} and R^{c} independently represent a (C₁-C₆)alkyl group or a hydrogen atom, or any of its pharmaceutically acceptable salt.

8. A compound of formula (I) according to any of claims 1 to 7, wherein R¹ represents:
(i). a (C₄-C₆)alkyl group substituted by a group selected from a hydroxy group, a halogen and a (C₁-C₃)alkoxy group,
(ii). a (C₃-C₈)cycloalkyl group, optionally substituted by a group selected from a hydroxy group and a (C₁-C₃) alkoxy group,
(iii). a bridged (C₆-C₁₀)cycloalkyl group, optionally substituted by a group selected from a (C₁-C₄)alkyl group, a (C₁-C₄)alkoxy group, a halogen atom and a hydroxy group, or
(iv). a R'-L- group, wherein L is either a single bond or a (C₁-C₃)alkanediyl group, optionnally susbstituted by a group selected from a hydroxy group and a (C₁-C₃)alkoxy group, and
R' represents:
(iv.1). a (C₃-C₈)heterocycloalkyl group, optionally substituted by one or two groups selected from a hydroxy group, and a (C₁-C₄)alkyl group, or
(iv.2). a (C₃-C₈)heteroaryl group, optionally substituted by a (C₁-C₄)alkyl group, or
(v). a R"-L- group wherein L is a (C₁-C₃)alkanediyl group, optionally substituted by a group selected from a -NR^{b}R^{c} group, a (C₁-C₃)alkoxy group and a hydroxy group, and
R" is a phenyl group, optionally substituted by a fluoro(C₁-C₄)alkyl group,
R^{b} and R^{c} independently represent a (C₁-C₆)alkyl group or a hydrogen atom,
or any pharmaceutically acceptable salt thereof.

9. A compound of formula (I) according to anyone of claims 1 to 6, wherein R⁵ represents a hydrogen atom or a (C₁-C₄)alkyl group.

10. A compound of formula (I) according to anyone of claims 1 to 6 selected from:
(1). (4*Z*)-4-(1,3-Benzoxazol-6-ylmethylene)-2-(cyclohexylamino)-1*H-*imidazol-5-one,
(2). (4*Z*)-4-(1,3-Benzoxazol-6-ylmethylene)-2-(cycloheptylamino)-1*H-*imidazol-5-one,
(3). (4*Z*)-4-(1,3-Benzoxazol-6-ylmethylene)-2-(cycloheptylamino)-1*H-*imidazol-5-one,
(4). (4*Z*)-4-(1,3-Benzoxazol-6-ylmethylene)-2-[[(1*R*)-1-(hydroxymethyl)-3-methyl-butyl]amino]-1*H*-imidazol-5-one,
(5). (4*Z*)-4-(1,3-Benzoxazol-6-ylmethylene)-2-[[(1*R*)-1-(methoxymethyl)-3-methyl-butyl]amino]-1*H*-imidazol-5-one,
(6). (4*Z*)-4-(1,3-Benzoxazol-6-ylmethylene)-2-[[(1*R*,2*R*)-2-methoxycyclopentyl]amino]-1*H*-imidazol-5-one,
(7). (4*Z*)-4-(1,3-Benzoxazol-6-ylmethylene)-2-[[(1*S*,2*S*)-2-methoxycyclopentyl]amino]-1*H*-imidazol-5-one,
(8). (±)-(4*Z*)-4-(1,3-Benzoxazol-6-ylmethylene)-2-[[*trans*-4-hydroxycycloheptyl] amino]-1*H*-imidazol-5-one,
(9). (±)-(4*Z*)-4-(1,3-Benzoxazol-6-ylmethylene)-2-[[*trans*-4-methoxycycloheptyl]amino]-1*H*-imidazol-5-one,
(10). (±)-(4Z)-4-(1,3-Benzoxazol-6-ylmethylene)-2-[[*cis*-3-methoxycycloheptyl]amino]-1*H*-imidazol-5-one,
(11). (4*Z*)-2-(1-Adamantylamino)-4-(1,3-benzoxazol-6-ylmethylene)-1*H-*imidazol-5-one,
(12). (4*Z*)-4-(1,3-Benzoxazol-6-ylmethylene)-2-[(3-hydroxy-1-adamantyl)amino]-1*H*-imidazol-5-one,
(13). (4*Z*)-4-(1,3-Benzoxazol-6-ylmethylene)-2-[(3-methoxy-1-adamantyl)amino]-1*H*-imidazol-5-one.
(14). (4*Z*)-4-(1,3-Benzoxazol-6-ylmethylene)-2-[[2-(trifluoromethyl)phenyl]methylamino]-1*H*-imidazol-5-one,
(15). (4*Z*)-4-(1,3-Benzoxazol-6-ylmethylene)-2-[[(1*S*,2*S*)-2-hydroxyindan-1-yl] amino]-1*H*-imidazol-5-one,
(16). (4*Z*)-4-(1,3-Benzoxazol-6-ylmethylene)-2-[[(1*R*)-2-hydroxy-1-phenyl-ethyl]amino]-1*H*-imidazol-5-one,
(17). (4*Z*)-4-(1,3-Benzoxazol-6-ylmethylene)-2-[[(1*S*)-2-hydroxy-1-phenyl-ethyl]amino]-1*H*-imidazol-5-one,
(18). (4Z)-4-(1,3-Benzoxazol-6-ylmethylene)-2-[[(1*R*)-2-methoxy-1-phenyl-ethyl]amino]-1*H*-imidazol-5-one,
(19). (4*Z*)-4-(1,3-Benzoxazol-6-ylmethylene)-2-[(4-methylthiazol-2-yl)methylamino]-1*H*-imidazol-5-one,
(20). (4Z)-4-(1,3-Benzoxazol-6-ylmethylene)-2-(tetrahydropyran-4-ylmethylamino)-1*H*-imidazol-5-one,
(21). (4*Z*)-4-(1,3-Benzoxazol-6-ylmethylene)-2-[4-(4-methylpiperazin-1-yl)anilino]-1*H*-imidazol-5-one,
(22). (4*Z*)-4-(1,3-Benzoxazol-6-ylmethylene)-2-(2-pyridylamino)-1*H-*imidazol-5-one,
(23). (±)-(4*Z*)-4-(1,3-Benzoxazol-6-ylmethylene)-2-[(6,6-dimethyltetrahydropyran-3-yl)amino]-1*H*-imidazol-5-one.
(24). (4Z)-4-(1,3-Benzoxazol-6-ylmethylene)-2-[[(3*S*)-tetrahydropyran-3-yl]amino]-1*H*-imidazol-5-one,
(25). (±)-(4*Z*)-4-(1,3-Benzoxazol-6-ylmethylene)-2-(oxepan-3-ylamino)-1*H-*imidazol-5-one,
(26). (4*Z*)-2-(Cyclohexylamino)-4-(1*H*-indazol-5-ylmethylene)-1*H*-imidazol-5-one,
(27). 4*Z*)-2-(Cycloheptylamino)-4-(1*H*-indazol-5-ylmethylene)-1*H*-imidazol-5-one,
(28). 4*Z*)-2-(Cyclooctylamino)-4-(1*H*-indazol-5-ylmethylene)-1*H*-imidazol-5-one,
(29). (4*Z*)-2-[[(1*R*)-1-(Hydroxymethyl)-3-methyl-butyl]amino]-4-(1*H-*indazol-5-ylmethylene)-1*H*-imidazol-5-one,
(30). (4Z)-4-(1*H*-Indazol-5-ylmethylene)-2-[[(1*R*)-1-(methoxymethyl)-3-methyl-butyl]amino]-1*H*-imidazol-5-one,
(31). (4*Z*)-2-(1-Adamantylamino)-4-(1*H*-indazol-5-ylmethylene)-1*H-*imidazol-5-one,
(32). (4*Z*)-2-[(3-Hydroxy-1-adamantyl)amino]-4-(1*H*-indazol-5-ylmethylene)-1*H*-imidazol-5-one,
(33). (4*Z*)-4-(1*H*-Indazol-5-ylmethylene)-2-[[(1*R*)-2-methoxy-1-phenyl-ethyl]amino]-1*H*-imidazol-5-one,
(34). (4*Z*)-2-(Cyclohexylamino)-4-[(2-methylindazol-5-yl)methylene]-1*H-*imidazol-5-one,
(35). (4*Z*)-2-(Cycloheptylamino)-4-[(2-methylindazol-5-yl)methylene]-1*H-*imidazol-5-one,
(36). (4*Z*)-2-(Cyclooctylamino)-4-[(2-methylindazol-5-yl)methylene]-1*H-*imidazol-5-one,
(37). (4*Z*)-2-[[(1*R*)-1-(Hydroxymethyl)-3-methyl-butyl]amino]-4-[(2-methylindazol-5-yl)methylene]-1*H*-imidazol-5-one,
(38). (4*Z*)-2-[[(1*R*)-1-(Methoxymethyl)-3-methyl-butyl]amino]-4-[(2-methylindazol-5-yl)methylene]-1*H*-imidazol-5-one,
(39). (4*Z*)-2-(1-Adamantylamino)-4-[(2-methylindazol-5-yl)methylene]-1*H-*imidazol-5-one,
(40). (4*Z*)-2-[(3-Hydroxy-1-adamantyl)amino]-4-[(2-methylindazol-5-yl)methylene]-1*H*-imidazol-5-one,
(41). (4*Z*)-2-[(3-Fluoro-1-adamantyl)amino]-4-[(2-methylindazol-5-yl)methylene]-1*H*-imidazol-5-one,
(42). (4Z)-2-[[(1*R*)-2-Methoxy-1-phenyl-ethyl]amino]-4-[(2-methylindazol-5-yl)methylene]-1*H*-imidazol-5-one,
(43). (4*Z*)-2-(Cyclohexylamino)-4-[(1-methylindazol-5-yl)methylene]-1*H-*imidazol-5-one,
(44). (4*Z*)-2-(Cycloheptylamino)-4-[(1-methylindazol-5-yl)methylene]-1*H-*imidazol-5-one,
(45). (4*Z*)-2-(Cyclooctylamino)-4-[(1-methylindazol-5-yl)methylene]-1*H-*imidazol-5-one,
(46). (4*Z*)-2-[[(1*R*)-1-(Hydroxymethyl)-3-methyl-butyl]amino]-4-[(1-methylindazol-5-yl)methylene]-1*H*-imidazol-5-one,
(47). (4*Z*)-2-[[(1*R*)-1-(Methoxymethyl)-3-methyl-butyl]amino]-4-[(1-methylindazol-5-yl)methylene]-1*H*-imidazol-5-one,
(48). (4*Z*)-2-[[(1*R*)-1-(Fluoromethyl)-3-methyl-butyl]amino]-4-[(1-methylindazol-5-yl)methylene]-1*H*-imidazol-5-one,
(49). (4*Z*)-2-[[(1*S*)-1-(Fluoromethyl)-3-methyl-butyl]amino]-4-[(1-methylindazol-5-yl)methylene]-1*H*-imidazol-5-one,
(50). (4*Z*)-2-[[(1*R*,2*R*)-2-Methoxycyclopentyl]amino]-4-[(1-methylindazol-5-yl)methylene]-1*H*-imidazol-5-one,
(51). (4*Z*)-2-[[(1*S*,2*S*)-2-Methoxycyclopentyl]amino]-4-[(1-methylindazol-5-yl)methylene]-1*H*-imidazol-5-one,
(52). (±)-(4Z)-2-[[*trans*-4-Hydroxycycloheptyl]amino]-4-[(1-methylindazol-5-yl)methylene]-1H-imidazol-5-one,
(53). (±)-(4*Z*)-2-[[*trans*-4-Methoxycycloheptyl]amino]-4-[(1-methylindazol-5-yl)methylene]-1*H*-imidazol-5-one,
(54). (±)-(4*Z*)-2-[[*cis*-3-Methoxycycloheptyl]amino]-4-[(1-methylindazol-5-yl)methylene]-1*H*-imidazol-5-one,
(55). (4*Z*)-2-(1-Adamantylamino)-4-[(1-methylindazol-5-yl)methylene]-1*H-*imidazol-5-one,
(56). (4*Z*)-2-[(3-Hydroxy-1-adamantyl)amino]-4-[(1-methylindazol-5-yl)methylene]-1*H*-imidazol-5-one,
(57). (4Z)-2-[(3-Methoxy-1-adamantyl)amino]-4-[(1-methylindazol-5-yl)methylene]-1*H*-imidazol-5-one,
(58). (4Z)-2-[(3-Fluoro-1-adamantyl)amino]-4-[(1-methylindazol-5-yl)methylene]-1*H*-imidazol-5-one,
(59). (4*Z*)-4-[(1-Methylindazol-5-yl)methylene]-2-[[2-(trifluoromethyl)phenyl]methylamino]-1*H*-imidazol-5-one,
(60). (4*Z*)-2-[[(1*S*,2*S*)-2-Hydroxyindan-1-yl]amino]-4-[(1-methylindazol-5-yl)methylene]-1*H*-imidazol-5-one,
(61). (4*Z*)-2-[[(1*R*)-2-Hydroxy-1-phenyl-ethyl]amino]-4-[(1-methylindazol-5-yl)methylene]-1*H*-imidazol-5-one.
(62). (4*Z*)-2-[[(1*S*)-2-Hydroxy-1-phenyl-ethyl]amino]-4-[(1-methylindazol-5-yl)methylene]-1*H*-imidazol-5-one,
(63). (4*Z*)-2-[[(1*R*)-2-Methoxy-1-phenyl-ethyl]amino]-4-[(1-methylindazol-5-yl)methylene]-1*H*-imidazol-5-one,
(64). (4*Z*)-2-[[(2*R*)-2-Hydroxy-2-phenyl-ethyl]amino]-4-[(1-methylindazol-5-yl)methylene]-1*H*-imidazol-5-one,
(65). (4*Z*)-2-[[(1*R*)-2-Amino-1-phenyl-ethyl]amino]-4-[(1-methylindazol-5-yl)methylene]-1*H*-imidazol-5-one dihydrochloride,
(66). (4*Z*)-2-[[(1*S*)-2-Amino-1-phenyl-ethyl]amino]-4-[(1-methylindazol-5-yl)methylene]-1*H*-imidazol-5-one dihydrochloride,
(67). (4*Z*)-4-[(1-Methylindazol-5-yl)methylene]-2-[(4-methylthiazol-2-yl)methylamino]-1*H*-imidazol-5-one,
(68). (4*Z*)-4-[(1-Methylindazol-5-yl)methylene]-2-(tetrahydropyran-4-ylmethylamino)-1*H*-imidazol-5-one,
(69). (4*Z*)-4-[(1-Methylindazol-5-yl)methylene]-2-[4-(4-methylpiperazin-1-yl)anilino]-1*H*-imidazol-5-one,
(70). (4*Z*)-4-[(1-Methylindazol-5-yl)methylene]-2-(2-pyridylamino)-1*H-*imidazol-5-one,
(71). (4*Z*)-2-[(6,6-Dimethyltetrahydropyran-3-yl)amino]-4-[(1-methylindazol-5-yl)methylene]-1*H*-imidazol-5-one,
(72). (4*Z*)-4-[(1-Methylindazol-5-yl)methylene]-2-[[(3*S*)-tetrahydropyran-3-yl]amino]-1*H*-imidazol-5-one,
(73). (4*Z*)-2-[[(3*R*,4*R*)-4-Hydroxytetrahydropyran-3-yl]amino]-4-[(1-methylindazol-5-yl)methylene]-1*H*-imidazol-5-one,
(74). (±)-(4*Z*)-4-[(1-Methylindazol-5-yl)methylene]-2-(oxepan-3-ylamino)-1*H*-imidazol-5-one,
(75). (4*Z*)-4-(1*H*-Benzimidazol-5-ylmethylene)-2-(cyclohexylamino)-1*H-*imidazol-5-one,
(76). (4*Z*)-4-(1*H*-Benzimidazol-5-ylmethylene)-2-(cycloheptylamino)-1H-imidazol-5-one,
(77). (4*Z*)-4-(1*H*-Benzimidazol-5-ylmethylene)-2-(cyclooctylamino)-1*H-*imidazol-5-one,
(78). (4*Z*)-4-(1*H*-Benzimidazol-5-ylmethylene)-2-[[(1*R*)-1-(hydroxymethyl)-3-methyl-butyl]amino]-1*H*-imidazol-5-one,
(79). (4*Z*)-4-(1*H*-Benzimidazol-5-ylmethylene)-2-[[(1*R*)-1-(methoxymethyl)-3-methyl-butyl]amino]-1*H*-imidazol-5-one,
(80). (4*Z*)-4-(1*H*-Benzimidazol-5-ylmethylene)-2-[[(1*R*)-1-(fluoromethyl)-3-methyl-butyl]amino]-1*H*-imidazol-5-one,
(81). (4*Z*)-4-(1*H*-Benzimidazol-5-ylmethylene)-2-[[(1*S*)-1-(fluoromethyl)-3-methyl-butyl]amino]-1*H*-imidazol-5-one,
(82). (4*Z*)-4-(1*H*-Benzimidazol-5-ylmethylene)-2-[[(1*R*,2*R*)-2-methoxycyclopentyl]amino]-1*H*-imidazol-5-one,
(83). (4*Z*)-2-(3-Noradamantylamino)-4-(1*H*-benzimidazol-5-ylmethylene)-1*H*-imidazol-5-one,
(84). (4*Z*)-2-(1-Adamantylamino)-4-(1*H*-benzimidazol-5-ylmethylene)-1*H-*imidazol-5-one,
(85). (4*Z*)-4-(1*H*-Benzimidazol-5-ylmethylene)-2-[(3-hydroxy-1-adamantyl)amino]-1*H*-imidazol-5-one,
(86). (4*Z*)-4-(1*H*-Benzimidazol-5-ylmethylene)-2-[(3-methoxy-1-adamantyl)amino]-1*H*-imidazol-5-one,
(87). (4*Z*)-4-(1*H*-benzimidazol-5-ylmethylene)-2-[(3-fluoro-1-adamantyl)amino]-1*H*-imidazol-5-one,
(88). (4*Z*)-4-(1*H*-Benzimidazol-5-ylmethylene)-2-[[2-(trifluoromethyl)phenyl]methylamino]-1*H*-imidazol-5-one,
(89). (4*Z*)-4-(1*H*-Benzimidazol-5-ylmethylene)-2-[[(1*S*,2*S*)-2-hydroxyindan-1-yl]amino]-1*H*-imidazol-5-one,
(90). (4*Z*)-4-(1*H*-Benzimidazol-5-ylmethylene)-2-[[(1*R*)-2-hydroxy-1-phenyl-ethyl]amino]-1*H*-imidazol-5-one,
(91). (4*Z*)-4-(1*H*-Benzimidazol-5-ylmethylene)-2-[[(1*S*)-2-hydroxy-1-phenyl-ethyl]amino]-1*H*-imidazol-5-one,
(92). (4*Z*)-4-(1*H*-Benzimidazol-5-ylmethylene)-2-[[(1*R*)-2-methoxy-1-phenyl-ethyl]amino]-1*H*-imidazol-5-one,
(93). (4*Z*)-4-(1*H*-Benzimidazol-5-ylmethylene)-2-[[(2*R*)-2-hydroxy-2-phenyl-ethyl]amino]-1*H*-imidazol-5-one,
(94). (4*Z*)-4-(1*H*-Benzimidazol-5-ylmethylene)-2-[[(2*S*)-2-hydroxy-2-phenyl-ethyl]amino]-1*H*-imidazol-5-one,
(95). (4*Z*)-2-[[(1*R*)-2-Amino-1-phenyl-ethyl]amino]-4-(1*H*-benzimidazol-5-ylmethylene)-1*H*-imidazol-5-one dihydrochloride,
(96). (4*Z*)-2-[[(1*S*)-2-Amino-1-phenyl-ethyl]amino]-4-(1*H*-benzimidazol-5-ylmethylene)-1*H*-imidazol-5-one dihydrochloride,
(97). (4*Z*)-4-(1H-Benzimidazol-5-ylmethylene)-2-[(4-methylthiazol-2-yl)methylamino]-1*H*-imidazol-5-one,
(98). (4*Z*)-4-(1*H*-Benzimidazol-5-ylmethylene)-2-(tetrahydropyran-4-ylmethylamino)-1*H*-imidazol-5-one,
(99). (4*Z*)-4-(1*H*-Benzimidazol-5-ylmethylene)-2-[4-(4-methylpiperazin-1-yl)anilino]-1*H*-imidazol-5-one,
(100). (4*Z*)-4-(1*H*-Benzimidazol-5-ylmethylene)-2-(2-pyridylamino)-1*H-*imidazol-5-one,
(101). (4*Z*)-4-(1*H*-Benzimidazol-5-ylmethylene)-2-[[(3*S*)-tetrahydropyran-3-yl]amino]-1*H*-imidazol-5-one,
(102). (4*Z*)-4-(1*H*-Benzimidazol-5-ylmethylene)-2-[[(3*R*,4*R*)-4-hydroxytetrahydropyran-3-yl]amino]-1*H*-imidazol-5-one,
(103). (4*Z*)-4-(1*H*-Benzimidazol-5-ylmethylene)-2-[[(3*S*,4*S*)-4-hydroxytetrahydropyran-3-yl]amino]-1*H*-imidazol-5-one,
(104). (4*Z*)-4-(1*H*-Benzimidazol-5-ylmethylene)-2-(oxepan-3-ylamino)-1*H-*imidazol-5-one,
(105). (4*Z*)-2-(Cyclohexylamino)-4-[(3-methylbenzimidazol-5-yl)methylene]-1*H*-imidazol-5-one,
(106). (4*Z*)-2-(Cycloheptylamino)-4-[(3-methylbenzimidazol-5-yl)methylene]-1*H*-imidazol-5-one,
(107). (4*Z*)-2-(Cyclooctylamino)-4-[(3-methylbenzimidazol-5-yl)methylene]-1*H*-imidazol-5-one,
(108). (4*Z*)-2-[[(1*R*)-1-(Hydroxymethyl)-3-methyl-butyl]amino]-4-[(3-methylbenzimidazol-5-yl)methylene]-1*H*-imidazol-5-one,
(109). (4*Z*)-2-[[(1*R*)-1-(Methoxymethyl)-3-methyl-butyl]amino]-4-[(3-methylbenzimidazol-5-yl)methylene]-1*H*-imidazol-5-one,
(110). (4*Z*)-2-[[(1*R*)-1-(Fluoromethyl)-3-methyl-butyl]amino]-4-[(3-methylbenzimidazol-5-yl)methylene]-1*H*-imidazol-5-one,
(111). (4*Z*)-2-[[(1*S*)-1-(Fluoromethyl)-3-methyl-butyl]amino]-4-[(3-methylbenzimidazol-5-yl)methylene]-1*H*-imidazol-5-one,
(112). (4*Z*)-2-[[(1*R*,2*R*)-2-Methoxycyclopentyl]amino]-4-[(3-methylbenzimidazol-5-yl)methylene]-1*H*-imidazol-5-one,
(113). (4*Z*)-2-[[(1*S*,2*S*)-2-Methoxycyclopentyl]amino]-4-[(3-methylbenzimidazol-5-yl)methylene]-1*H*-imidazol-5-one,
(114). (4Z)-2-(3-Noradamantylamino)-4-[(3-methylbenzimidazol-5-yl)methylene]-1*H*-imidazol-5-one,
(115). (4*Z*)-2-(1-Adamantylamino)-4-[(3-methylbenzimidazol-5-yl)methylene]-1*H*-imidazol-5-one,
(116). (4*Z*)-2-[(3-Hydroxy-1-adamantyl)amino]-4-[(3-methylbenzimidazol-5-yl)methylene]-1*H*-imidazol-5-one,
(117). (4*Z*)-2-[(3-Methoxy-1-adamantyl)amino]-4-[(3-methylbenzimidazol-5-yl)methylene]-1*H*-imidazol-5-one,
(118). (4*Z*)-2-[(3-Fluoro-1-adamantyl)amino]-4-[(3-methylbenzimidazol-5-yl)methylene]-1*H*-imidazol-5-one,
(119). (4*Z*)-4-[(3-Methylbenzimidazol-5-yl)methylene]-2-[[2-(trifluoromethyl)phenyl]methylamino]-1*H*-imidazol-5-one,
(120). (4*Z*)-2-[[(1*S*,2*S*)-2-Hydroxyindan-1-yl]amino]-4-[(3-methylbenzimidazol-5-yl)methylene]-1*H*-imidazol-5-one,
(121). (4*Z*)-2-[[(1*R*)-2-Hydroxy-1-phenyl-ethyl]amino]-4-[(3-methylbenzimidazol-5-yl)methylene]-1*H*-imidazol-5-one,
(122). (4*Z*)-2-[[(1*S*)-2-Hydroxy-1-phenyl-ethyl]amino]-4-[(3-methylbenzimidazol-5-yl)methylene]-1*H*-imidazol-5-one,
(123). (4*Z*)-2-[[(1*R*)-2-Methoxy-1-phenyl-ethyl]amino]-4-[(3-methylbenzimidazol-5-yl)methylene]-1*H*-imidazol-5-one,
(124). (4*Z*)-2-[[(2*R*)-2-Hydroxy-2-phenyl-ethyl]amino]-4-[(3-methylbenzimidazol-5-yl)methylene]-1*H*-imidazol-5-one,
(125). (4*Z*)-2-[[(2*S*)-2-Hydroxy-2-phenyl-ethyl]amino]-4-[(3-methylbenzimidazol-5-yl)methylene]-1*H*-imidazol-5-one,
(126). (4*Z*)-2-[[(1*R*)-2-amino-1-phenyl-ethyl]amino]-4-[(3-methylbenzimidazol-5-yl)methylene]-1*H*-imidazol-5-one dihydrochloride,
(127). (4*Z*)-2-[[(1*S*)-2-amino-1-phenyl-ethyl]amino]-4-[(3-methylbenzimidazol-5-yl)methylene]-1*H*-imidazol-5-one dihydrochloride,
(128). (4*Z*)-4-[(3-Methylbenzimidazol-5-yl)methylene]-2-[(4-methylthiazol-2-yl)methylamino]-1*H*-imidazol-5-one,
(129). (4*Z*)-4-[(3-Methylbenzimidazol-5-yl)methylene]-2-(tetrahydropyran-4-ylmethylamino)-1*H*-imidazol-5-one,
(130). (4*Z*)-4-[(3-Methylbenzimidazol-5-yl)methylene]-2-[4-(4-methylpiperazin-1-yl)anifino]-1*H*-imidazol-5-one,
(131). (4*Z*)-4-[(3-Methylbenzimidazol-5-yl)methylene]-2-(2-pyridylamino)-1*H*-imidazol-5-one,
(132). (5Z)-5-(1,3-Benzoxazol-6-ylmethylene)-2-(cyclohexylamino)-3-methyl-imidazol-4-one,
(133). (5*Z*)-5-(1,3-Benzoxazol-6-ylmethylene)-2-(cyclooctylamino)-3-methyl-imidazol-4-one,
(134). (5*Z*)-5-(1,3-Benzoxazol-6-ylmethylene)-2-[[(1*R*)-1-(methoxymethyl)-3-methyl-butyl]amino]-3-methyl-imidazol-4-one,
(135). (5*Z*)-5-(1,3-Benzoxazol-6-ylmethylene)-2-[[(1*R*,2*R*)-2-methoxycyclopentyl]amino]-3-methyl-imidazol-4-one,
(136). (5*Z*)-5-(1,3-Benzoxazol-6-ylmethylene)-2-[[(1S*R*,2*S*)-2-methoxycyclopentyl]amino]-3-methyl-imidazol-4-one,
(137). (5*Z*)-2-(3-Noradamantylamino)-5-(1,3-benzoxazol-6-ylmethylene)-3-methyl-imidazol-4-one,
(138). (5*Z*)-2-(1-Adamantylamino)-5-(1,3-benzoxazol-6-ylmethylene)-3-methyl-imidazol-4-one,
(139). (5*Z*)-5-(1,3-Benzoxazol-6-ylmethylene)-2-[(3-hydroxy-1-adamantyl)amino]-3-methyl-imidazol-4-one,
(140). (5*Z*)-5-(1,3-Benzoxazol-6-ylmethylene)-2-[(3-methoxy-1-adamantyl)amino]-3-methyl-imidazol-4-one,
(141). (5*Z*)-5-(1,3-Benzoxazol-6-ylmethylene)-2-[(3-fluoro-1-adamantyl)amino]-3-methyl-imidazol-4-one,
(142). (5*Z*)-5-(1,3-Benzoxazol-6-ylmethylene)-3-methyl-2-[[2-(trifluoromethyl)phenyl]methylamino]imidazol-4-one,
(143). (5*Z*)-5-(1,3-Benzoxazol-6-ylmethylene)-2-[[(1*R*)-2-methoxy-1-phenyl-ethyl]amino]-3-methyl-imidazol-4-one,
(144). (5*Z*)-5-(1,3-Benzoxazol-6-ylmethylene)-3-methyl-2-[(4-methylthiazol-2-yl)methylamino]imidazol-4-one,
(145). (5*Z*)-2-(Cyclohexylamino)-5-(1*H*-indazol-5-ylmethylene)-3-methyl-imidazol-4-one,
(146). (5*Z*)-2-(Cycloheptylamino)-5-(1*H*-indazol-5-ylmethylene)-3-methyl-imidazol-4-one,
(147). (5*Z*)-2-(Cyclooctylamino)-5-(1*H*-indazol-5-ylmethylene)-3-methyl-imidazol-4-one,
(148). (5*Z*)-2-[[(1*R*)-1-(Hydroxymethyl)-3-methyl-butyl]amino]-5-(1*H-*indazol-5-ylmethylene)-3-methyl-imidazol-4-one,
(149). (5*Z*)-5-(1*H*-Indazol-5-ylmethylene)-2-[[(1*R*)-1-(methoxymethyl)-3-methyl-butyl]amino]-3-methyl-imidazol-4-one,
(150). (5*Z*)-5-(1*H*-Indazol-5-ylmethylene)-2-[[(1*R*,2*R*)-2-methoxycyclopentyl]amino]-3-methyl-imidazol-4-one,
(151). (5*Z*)-5-(1*H*-Indazol-5-ylmethylene)-2-[[(1*S*,2*S*)-2-methoxycyclopentyl]amino]-3-methyl-imidazol-4-one,
(152). (5Z)-2-(3-Nordamantylamino)-5-(1*H*-indazol-5-ylmethylene)-3-methyl-imidazol-4-one,
(153). (5*Z*)-2-(1-Adamantylamino)-5-(1*H*-indazol-5-ylmethylene)-3-methyl-imidazol-4-one,
(154). (5*Z*)-2-[(3-Hydroxy-1-adamantyl)amino]-5-(1*H*-indazol-5-ylmethylene)-3-methyl-imidazol-4-one,
(155). (5*Z*)-5-(1*H*-Indazol-5-ylmethylene)-2-[(3-methoxy-1-adamantyl)amino]-3-methyl-imidazol-4-one,
(156). (5*Z*)-2-[(3-Fluoro-1-adamantyl)amino]-5-(1*H*-indazol-5-ylmethylene)-3-methyl-imidazol-4-one,
(157). (5*Z*)-5-(1*H*-Indazol-5-ylmethylene)-3-methyl-2-[[2-(trifluoromethyl)phenyl]methylamino]imidazol-4-one,
(158). (5*Z*)-2-[[(1*S*,2*S*)-2-Hydroxyindan-1-yl]amino]-5-(1*H*-indazol-5-ylmethylene)-3-methyl-imidazol-4-one,
(159). (5*Z*)-2-[[(1*R*)-2-Hydroxy-1-phenyl-ethyl]amino]-5-(1*H*-indazol-5-ylmethylene)-3-methyl-imidazol-4-one,
(160). (5*Z*)-2-[[(1*S*)-2-Hydroxy-1-phenyl-ethyl]amino]-5-(1*H*-indazol-5-ylmethylene)-3-methyl-imidazol-4-one,
(161). (5*Z*)-5-(1*H*-Indazol-5-ylmethylene)-2-[[(1*R*)-2-methoxy-1-phenyl-ethyl]amino]-3-methyl-imidazol-4-one,
(162). (5*Z*)-2-[[(2*R*)-2-Hydroxy-2-phenyl-ethyl]amino]-5-(1*H*-indazol-5-ylmethylene)-3-methyl-imidazol-4-one,
(163). (5*Z*)-2-[[(1*R*)-2-Amino-1-phenyl-ethyl]amino]-5-(1*H*-indazol-5-ylmethylene)-3-methyl-imidazol-4-one dihydrochloride,
(164). (5*Z*)-5-(1*H*-Indazol-5-ylmethylene)-3-methyl-2-[(4-methylthiazol-2-yl)methylamino]imidazol-4-one,
(165). (5*Z*)-5-(1*H*-Indazol-5-ylmethylene)-3-methyl-2-(tetrahydropyran-4-ylmethylamino)imidazol-4-one,
(166). (5*Z*)-5-(1*H*-Indazol-5-ylmethylene)-3-methyl-2-[4-(4-methylpiperazin-1-yl)anilino]imidazol-4-one,
(167). (5*Z*)-5-(1*H*-Indazol-5-ylmethylene)-3-methyl-2-(2-pyridylamino)imidazol-4-one,
(168). (5*Z*)-5-(1*H*-Indazol-5-ylmethylene)-3-methyl-2-[[(3S)-tetrahydropyran-3-yl]amino]imidazol-4-one,
(169). (5*Z*)-2-[[(3*R*,4*R*)-4-Hydroxytetrahydropyran-3-yl]amino]-5-(1*H-*indazol-5-ylmethylene)-3-methyl-imidazol-4-one,
(170). (5*Z*)-2-(Cyclohexylamino)-3-methyl-5-[(2-methylindazol-5-yl)methylene]imidazol-4-one,
(171). (5*Z*)-2-(Cycloheptylamino)-3-methyl-5-[(2-methylindazol-5-yl)methylene]imidazol-4-one,
(172). (5*Z*)-2-(Cyclooctylamino)-3-methyl-5-[(2-methylindazol-5-yl)methylene]imidazol-4-one,
(173). (5*Z*)-2-[[(1*R*)-1-(Hydroxymethyl)-3-methyl-butyl]amino]-3-methyl-5-[(2-methylindazol-5-yl)methylene]imidazol-4-one,
(174). (5*Z*)-2-[[(1*R*)-1-(Methoxymethyl)-3-methyl-butyl]amino]-3-methyl-5-[(2-methylindazol-5-yl)methylene]imidazol-4-one,
(175). (5*Z*)-2-[[(1*R*,2*R*)-2-Methoxycyclopentyl]amino]-3-methyl-5-[(2-methylindazol-5-yl)methylene]imidazol-4-one,
(176). (5*Z*)-2-[[(1*S*,2*S*)-2-Methoxycyclopentyl]amino]-3-methyl-5-[(2-methylindazol-5-yl)methylene]imidazol-4-one,
(177). (5*Z*)-2-(3-Noradamantylamino)-3-methyl-5-[(2-methylindazol-5-yl)methylene]imidazol-4-one,
(178). (5*Z*)-2-(1-Adamantylamino)-3-methyl-5-[(2-methylindazol-5-yl)methylene]imidazol-4-one,
(179). (5*Z*)-2-[(3-Hydroxy-1-adamantyl)amino]-3-methyl-5-[(2-methylindazol-5-yl)methylene]imidazol-4-one,
(180). (5*Z*)-2-[(3-Methoxy-1-adamantyl)amino]-3-methyl-5-[(2-methylindazol-5-yl)methylene]imidazol-4-one,
(181). (5*Z*)-2-[(3-Fluoro-1-adamantyl)amino]-3-methyl-5-[(2-methylindazol-5-yl)methylene]imidazol-4-one,
(182). (5*Z*)-3-Methyl-5-[(2-methylindazol-5-yl)methylene]-2-[[2-(trifluoromethyl)phenyl]methylamino]imidazol-4-one,
(183). (5*Z*)-2-[[(1*S*,2*S*)-2-Hydroxyindan-1-yl]amino]-3-methyl-5-[(2-methylindazol-5-yl)methylene]imidazol-4-one,
(184). (5*Z*)-2-[[(1*R*)-2-Hydroxy-1-phenyl-ethyl]amino]-3-methyl-5-[(2-methylindazol-5-yl)methylene]imidazol-4-one,
(185). (5*Z*)-2-[[(1*S*)-2-Hydroxy-1-phenyl-ethyl]amino]-3-methyl-5-[(2-methylindazol-5-yl)methylene]imidazol-4-one,
(186). (5*Z*)-2-[[(1*R*)-2-Methoxy-1-phenyl-ethyl]amino]-3-methyl-5-[(2-methylindazol-5-yl)methylene]imidazol-4-one,
(187). (5*Z*)-2-[[(2*R*)-2-Hydroxy-2-phenyl-ethyl]amino]-3-methyl-5-[(2-methylindazol-5-yl)methylene]imidazol-4-one,
(188). (5*Z*)-2-[[(1*R*)-2-Amino-1-phenyl-ethyl]amino]-3-methyl-5-[(2-methylindazol-5-yl)methylene]imidazol-4-one dihydrochloride,
(189). (5*Z*)-3-Methyl-5-[(2-methylindazol-5-yl)methylene]-2-[(4-methylthiazol-2-yl)methylamino]imidazol-4-one,
(190). (5*Z*)-3-Methyl-5-[(2-methylindazol-5-yl)methylene]-2-(tetrahydropyran-4-ylmethylamino)imidazol-4-one,
(191). (5*Z*)-3-Methyl-5-[(2-methylindazol-5-yl)methylene]-2-[4-(4-methylpiperazin-1-yl)anilino]imidazol-4-one,
(192). (5*Z*)-3-Methyl-5-[(2-methylindazol-5-yl)methylene]-2-(2-pyridylamino)imidazol-4-one,
(193). (5*Z*)-3-Methyl-5-[(2-methylindazol-5-yl)methylene]-2-[[(3S)-tetrahydropyran-3-yl]amino]imidazol-4-one,
(194). (5*Z*)-2-[[(3*R*,4*R*)-4-Hydroxytetrahydropyran-3-yl]amino]-3-methyl-5-[(2-methylindazol-5-yl)methylene]imidazol-4-one,
(195). (5*Z*)-2-(Cyclohexylamino)-3-methyl-5-[(1-methylindazol-5-yl)methylene]imidazol-4-one,
(196). (5*Z*)-2-(Cycloheptylamino)-3-methyl-5-[(1-methylindazol-5-yl)methylene]imidazol-4-one,
(197). (5*Z*)-2-(Cyclooctylamino)-3-methyl-5-[(1-methylindazol-5-yl)methylene]imidazol-4-one,
(198). (5*Z*)-2-[[(1*R*)-1-(Hydroxymethyl)-3-methyl-butyl]amino]-3-methyl-5-[(1-methylindazol-5-yl)methylene]imidazol-4-one,
(199). (5*Z*)-2-[[(1*R*)-1-(Methoxymethyl)-3-methyl-butyl]amino]-3-methyl-5-[(1-methylindazol-5-yl)methylene]imidazol-4-one,
(200). (5*Z*)-2-[[(1*R*,2*R*)-2-Methoxycyclopentyl]amino]-3-methyl-5-[(1-methylindazol-5-yl)methylene]imidazol-4-one,
(201). (5*Z*)-2-[[(1*S*,2*S*)-2-Methoxycyclopentyl]amino]-3-methyl-5-[(1-methylindazol-5-yl)methylene]imidazol-4-one,
(202). (5*Z*)-2-(3-Noradamantylamino)-3-methyl-5-[(1-methylindazol-5-yl)methylene]imidazol-4-one,
(203). (5*Z*)-2-(1-Adamantylamino)-3-methyl-5-[(1-methylindazol-5-yl)methylene]imidazol-4-one,
(204). (5*Z*)-2-[(3-Hydroxy-1-adamantyl)amino]-3-methyl-5-[(1-methylindazol-5-yl)methylene]imidazol-4-one,
(205). (5*Z*)-2-[(3-Methoxy-1-adamantyl)amino]-3-methyl-5-[(1-methylindazol-5-yl)methylene]imidazol-4-one,
(206). (5*Z*)-2-[(3-Fluoro-1-adamantyl)amino]-3-methyl-5-[(1-methylindazol-5-yl)methylene]imidazol-4-one,
(207). (5*Z*)-3-Methyl-5-[(1-methylindazol-5-yl)methylene]-2-[[2-(trifluoromethyl)phenyl]methylamino]imidazol-4-one,
(208). (5*Z*)-2-[[(1*S*,2*S*)-2-Hydroxyindan-1-yl]amino]-3-methyl-5-[(1-methylindazol-5-yl)methylene]imidazol-4-one,
(209). (5*Z*)-2-[[(1*R*)-2-Hydroxy-1-phenyl-ethyl]amino]-3-methyl-5-[(1-methylindazol-5-yl)methylene]imidazol-4-one,
(210). (5*Z*)-2-[[(1*R*)-2-Hydroxy-1-phenyl-ethyl]amino]-3-methyl-5-[(1-methylindazol-5-yl)methylene]imidazol-4-one,
(211). (5*Z*)-2-[[(1*R*)-2-Methoxy-1-phenyl-ethyl]amino]-3-methyl-5-[(1-methylindazol-5-yl)methylene]imidazol-4-one,
(212). (5*Z*)-2-[[(2*R*)-2-Hydroxy-2-phenyl-ethyl]amino]-3-methyl-5-[(1-methylindazol-5-yl)methylene]imidazol-4-one,
(213). (5*Z*)-2-[[(1*R*)-2-Amino-1-phenyl-ethyl]amino]-3-methyl-5-[(1-methylindazol-5-yl)methylene]imidazol-4-one dihydrochloride,
(214). (5*Z*)-3-Methyl-5-[(1-methylindazol-5-yl)methylene]-2-[(4-methylthiazol-2-yl)methylamino]imidazol-4-one,
(215). (5*Z*)-3-Methyl-5-[(1-methylindazol-5-yl)methylene]-2-(tetrahydropyran-4-ylmethylamino)imidazol-4-one,
(216). (5*Z*)-3-Methyl-5-[(1-methylindazol-5-yl)methylene]-2-[4-(4-methylpiperazin-1-yl)anilino]imidazol-4-one,
(217). (5*Z*)-3-Methyl-5-[(1-methylindazol-5-yl)methylene]-2-(2-pyridylamino)imidazol-4-one,
(218). (5*Z*)-3-Methyl-5-[(1-methylindazol-5-yl)methylene]-2-[[(3*S*)-tetrahydropyran-3-yl]amino]imidazol-4-one,
(219). (5*Z*)-2-[[(3*R*,4*R*)-4-Hydroxytetrahydropyran-3-yl]amino]-3-methyl-5-[(1-methylindazol-5-yl)methylene]imidazol-4-one,
(220). (5*Z*)-5-(1*H*-Benzimidazol-5-ylmethylene)-2-(cyclohexylamino)-3-methyl-imidazol-4-one,
(221). (5*Z*)-5-(1*H*-Benzimidazol-5-ylmethylene)-2-(cycloheptylamino)-3-methyl-imidazol-4-one,
(222). (5*Z*)-5-(1*H*-Benzimidazol-5-ylmethylene)-2-(cyclooctylamino)-3-methyl-imidazol-4-one,
(223). (5*Z*)-5-(1*H*-Benzimidazol-5-ylmethylene)-2-[[(1*R*)-1-(hydroxymethyl)-3-methyl-butyl]amino]-3-methyl-imidazol-4-one,
(224). (5*Z*)-5-(1*H*-Benzimidazol-5-ylmethylene)-2-[[(1*R*)-1-(methoxymethyl)-3-methyl-butyl]amino]-3-methyl-imidazol-4-one,
(225). (5*Z*)-5-(1*H*-Benzimidazol-5-ylmethylene)-2-[[(1*R*,2*R*)-2-methoxycyclopentyl]amino]-3-methyl-imidazol-4-one,
(226). (5*Z*)-5-(1*H*-Benzimidazol-5-ylmethylene)-2-[[(1*S*,2*S*)-2-methoxycyclopentyl]amino]-3-methyl-imidazol-4-one,
(227). (5*Z*)-2-(3-Nordamantylamino)-5-(1*H*-benzimidazol-5-ylmethylene)-3-methyl-imidazol-4-one,
(228). (5*Z*)-2-(1-Adamantylamino)-5-(1*H*-benzimidazol-5-ylmethylene)-3-methyl-imidazol-4-one,
(229). (5*Z*)-5-(1*H*-Benzimidazol-5-ylmethylene)-2-[(3-hydroxy-1-adamantyl)amino]-3-methyl-imidazol-4-one,
(230). (5*Z*)-5-(1*H*-Benzimidazol-5-ylmethylene)-2-[(3-methoxy-1-adamantyl)amino]-3-methyl-imidazol-4-one,
(231). (5*Z*)-5-(1*H*-Benzimidazol-5-ylmethylene)-2-[(3-fluoro-1-adamantyl)amino]-3-methyl-imidazol-4-one,
(232). (5*Z*)-5-(1*H*-Benzimidazol-5-ylmethylene)-3-methyl-2-[[2-(trifluoromethyl)phenyl]methylamino]imidazol-4-one,
(233). (*5Z*)-5-(1*H*-Benzimidazol-5-ylmethylene)-2-[[(1*S*,2*S*)-2-hydroxyindan-1-yl]amino]-3-methyl-imidazol-4-one,
(234). (5*Z*)-5-(1*H*-Benzimidazol-5-ylmethylene)-2-[[(1*R*)-2-hydroxy-1-phenyl-ethyl]amino]-3-methyl-imidazol-4-one,
(235). (5*Z*)-5-(1*H*-Benzimidazol-5-ylmethylene)-2-[[(1*S*)-2-hydroxy-1-phenyl-ethyl]amino]-3-methyl-imidazol-4-one,
(236). (5*Z*)-5-(1*H*-Benzimidazol-5-ylmethylene)-2-[[(1*R*)-2-methoxy-1-phenyl-ethyl]amino]-3-methyl-imidazol-4-one,
(237). (5*Z*)-5-(1*H*-Benzimidazol-5-ylmethylene)-2-[[(2*R*)-2-hydroxy-2-phenyl-ethyl]amino]-3-methyl-imidazol-4-one,
(238). (5*Z*)-5-(1*H*-Benzimidazol-5-ylmethylene)-3-methyl-2-[(4-methylthiazol-2-yl)methylamino]imidazol-4-one,
(239). (5*Z*)-5-(1*H*-Benzimidazol-5-ylmethylene)-3-methyl-2-(tetrahydropyran-4-ylmethylamino)imidazol-4-one,
(240). (5*Z*)-5-(1*H*-Benzimidazol-5-ylmethylene)-3-methyl-2-[4-(4-methylpiperazin-1-yl)anilino]imidazol-4-one,
(241). (5*Z*)-5-(1*H*-Benzimidazol-5-ylmethylene)-3-methyl-2-(2-pyridylamino)imidazol-4-one,
(242). (5*Z*)-5-(1*H*-Benzimidazol-5-ylmethylene)-3-methyl-2-[[(3*S*)-tetrahydropyran-3-yl]amino]imidazol-4-one,
(243). (5*Z*)-5-(1*H*-Benzimidazol-5-ylmethylene)-2-[[(3*R*,4*R*)-4-hydroxytetrahydropyran-3-yl]amino]-3-methyl-imidazol-4-one,
(244). (5*Z*)-2-(Cyclohexylamino)-3-methyl-5-[(3-methylbenzimidazol-5-yl)methylene]imidazol-4-one,
(245). (5*Z*)-2-(Cycloheptylamino)-3-methyl-5-[(3-methylbenzimidazol-5-yl)methylene]imidazol-4-one,
(246). (5*Z*)-2-(Cyclooctylamino)-3-methyl-5-[(3-methylbenzimidazol-5-yl)methylene]imidazol-4-one,
(247). (5*Z*)-2-[[(1*R*)-1-(Hydroxymethyl)-3-methyl-butyl]amino]-3-methyl-5-[(3-methylbenzimidazol-5-yl)methylene]imidazol-4-one,
(248). (5*Z*)-2-[[(1*R*)-1-(Methoxymethyl)-3-methyl-butyl]amino]-3-methyl-5-[(3-methylbenzimidazol-5-yl)methylene]imidazol-4-one,
(249). (5*Z*)-2-[[(1*R*,2*R*)-2-Methoxycyclopentyl]amino]-3-methyl-5-[(3-methylbenzimidazol-5-yl)methylene]imidazol-4-one,
(250). (5*Z*)-2-[[(1*S*,2*S*)-2-Methoxycyclopentyl]amino]-3-methyl-5-[(3-methylbenzimidazol-5-yl)methylene]imidazol-4-one,
(251). (5*Z*)-2-(3-Noradamantylamino)-3-methyl-5-[(3-methylbenzimidazol-5-yl)methylene]imidazol-4-one
(252). (5*Z*)-2-(1-Adamantylamino)-3-methyl-5-[(3-methylbenzimidazol-5-yl)methylene]imidazol-4-one,
(253). (5*Z*)-2-[(3-Hydroxy-1-adamantyl)amino]-3-methyl-5-[(3-methylbenzimidazol-5-yl)methylene]imidazol-4-one,
(254). (5*Z*)-2-[(3-Methoxy-1-adamantyl)amino]-3-methyl-5-[(3-methylbenzimidazol-5-yl)methylene]imidazol-4-one,
(255). (5*Z*)-2-[(3-Fluoro-1-adamantyl)amino]-3-methyl-5-[(3-methylbenzimidazol-5-yl)methylene]imidazol-4-one,
(256). (5*Z*)-3-Methyl-5-[(3-methylbenzimidazol-5-yl)methylene]-2-[[2-(trifluoromethyl)phenyl]methylamino]imidazol-4-one,
(257). (5*Z*)-2-[[(1*R*)-2-Hydroxy-1-phenyl-ethyl]amino]-3-methyl-5-[(3-methylbenzimidazol-5-yl)methylene]imidazol-4-one,
(258). (5*Z*)-2-[[(1*S*)-2-Hydroxy-1-phenyl-ethyl]amino]-3-methyl-5-[(3-methylbenzimidazol-5-yl)methylene]imidazol-4-one,
(259). (5*Z*)-2-[[(1*R*)-2-Methoxy-1-phenyl-ethyl]amino]-3-methyl-5-[(3-methylbenzimidazol-5-yl)methylene]imidazol-4-one,
(260). (5*Z*)-2-[[(2*R*)-2-Hydroxy-2-phenyl-ethyl]amino]-3-methyl-5-[(3-methylbenzimidazol-5-yl)methylene]imidazol-4-one,
(261). (5Z)-3-Methyl-5-[(3-methylbenzimidazol-5-yl)methylene]-2-[(4-methylthiazol-2-yl)methylamino]imidazol-4-one,
(262). (5*Z*)-3-Methyl-5-[(3-methylbenzimidazol-5-yl)methylene]-2-(tetrahydropyran-4-ylmethylamino)imidazol-4-one,
(263). (5*Z*)-3-Methyl-5-[(3-methylbenzimidazol-5-yl)methylene]-2-[4-(4-methylpiperazin-1-yl)anilino]imidazol-4-one,
(264). (5*Z*)-3-Methyl-5-[(3-methylbenzimidazol-5-yl)methylene]-2-(2-pyridylamino)imidazol-4-one,
(265). (5*Z*)-3-Methyl-5-[(3-methylbenzimidazol-5-yl)methylene]-2-[[(3*S*)-tetrahydropyran-3-yl]amino]imidazol-4-one,
(266). (5*Z*)-2-[[(3*R*,4*R*)-4-Hydroxytetrahydropyran-3-yl]amino]-3-methyl-5-[(3-methylbenzimidazol-5-yl)methylene]imidazol-4-one,
or anyone of their pharmaceutically acceptable salts.

11. A pharmaceutical composition comprising at least one compound as defined in anyone of claims 1 to 9 or or any of its pharmaceutically acceptable salt or as defined in claim 10.

12. Synthesis process for manufacturing a compound of formula (I) as defined in anyone of claims 1 to 9 or or any of its pharmaceutically acceptable salt or as defined in claim 10 or any of its pharmaceutically acceptable salts, comprising at least a step of coupling a compound of formula (IX) below 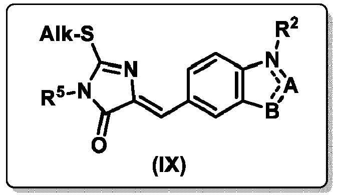
wherein R², R⁵, A and B are as in any one of claims 1 to 9 and Alk is a (C₁-C₅)alkyl,
with an amine of formula R¹NH₂ wherein R¹ is as defined in any one of claims 1 to 9.

13. Synthesis process for manufacturing a compound of formula (I) as defined in anyone of claims 1 to 9 or or any of its pharmaceutically acceptable salt or as defined in claim 10 or any of its pharmaceutically acceptable salts, comprising at least a step of coupling a compound of formula (II) below 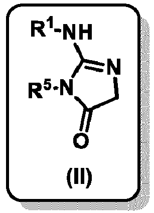
wherein R¹ and R⁵ are as defined in any one of claims 1 to 9
with a compound of formula (III) below 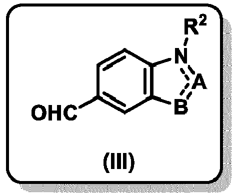
wherein R², A and B are as defined in any one of claims 1 to 9.

14. A synthetic intermediate selected from the compounds below 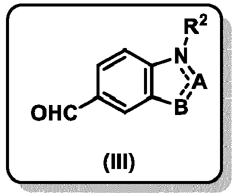 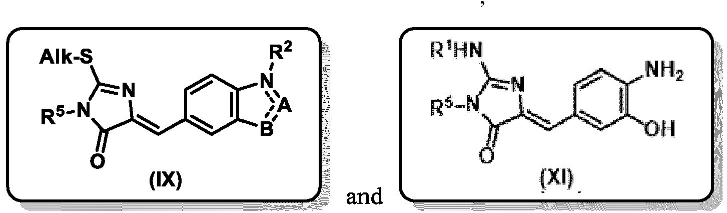 wherein Alk is a (C₁-C₅)alkyl, in particular Alk is selected from the group consisting of an ethyl and a methyl, and wherein R¹, R², R⁵, A and B are as defined in any one of claims 1 to 9.

15. A compound of formula (I) as defined in anyone of claim 1 to 9 or any of its pharmaceutically acceptable salts, or at least any of compounds (1) to (266) as defined in claim 10 or any of its pharmaceutically acceptable salts, for use as a medicament.

16. A compound of formula (I) according to anyone of claims 1 to 9 or any of its pharmaceutically acceptable salts, or at least any of compounds (1) to (266) as defined in claim 10 or any of its pharmaceutically acceptable salts for use in the treatment and/or in the prevention of a disease selected from cognitive deficits and neuroinflammation associated with Down syndrome; Alzheimer's disease and related diseases, dementia and/or tauopathies; as well as other neurodegenerative diseases such as Parkinson's disease and Pick disease, in particular Niemann-Pick Type C Disease; CDKL5 Deficiency Disorder; McDermid syndrome; autism; type 1 and type 2 diabetes; abnormal folate and methionine metabolism; tendinopathy and osteoarthritis, in particular knee osteoarthritis; Duchenne muscular dystrophy; cancers, such as brain cancer, including glioblastoma, leukemia, including megakaryoblastic leukemia and acute lymphoblastic leukemia, head and neck squamous cell carcinoma, pancreatic cancer, including pancreatic ductal adenocarcinoma, prostate cancer, gastrointestinal cancer, breast cancer, such as Triple-negative breast cancer, tissue cancer, including liposarcoma, Hedgehog/GLI-dependent cancer, liver cancer, including Hepatocellular carcinoma and viral infections, such as infections caused by Human immunodeficiency virus type 1, Human cytomegalovirus, Influenza A, Herpes virus, rhesus macaque cytomegalovirus, varicella-zoster virus, herpes simplex virus, Hepatitis C virus, Chikungunya virus, Dengue virus, Influenza virus and Severe acute respiratory syndrome coronavirus, Cytomegalovirus and Human papillomavirus; neuroinflammation; anemia; infections caused by unicellular parasites, such as malaria, Leishmaniasis, Chagas and sleeping sickness, cattle diseases due to unicellular pathogens, and for regulating body temperature.

17. A compound of formula (I) for use according to anyone of claims 1 to 9 or any of its pharmaceutically acceptable salts, or at least any of compounds (1) to (266) as defined in claim 10 or any of its pharmaceutically acceptable salts for use in the treatment and/or prevention of a disease selected from Down syndrome, Alzheimer's disease, dementia, tauopathies, Parkinson's disease, Niemann-Pick Type C Disease, CDKL5 Deficiency Disorder and Phelan-McDermid syndrome and their associated cognitive and motor conditions and type 1 and type 2 diabetes.
